# EUROPEAN PATENT APPLICATION

(11) **EP 3 964 516 A1**
(43) Date of publication of application: **09.03.2022**
(21) Application number: 20798173.9
(22) Date of filing: 28.04.2020
(51) Int. Cl.: C07D 498/14, A61K 31/519, A61P 35/00

(54) **OXAAZAQUINAZOLINE-7(8H)-KETONE COMPOUND, PREPARATION METHOD THERFOR AND PHARMACEUTICAL APPLICATION THEREOF**

(30) Priority: 28.04.2019 CN 201910349813
(71) Applicant: Genfleet Therapeutics (Shanghai) Inc., Shanghai 201203 (CN); Zhejiang Genfleet Therapeutics Co., Ltd., Shaoxing Binhai New City, Zhejiang 312000 (CN)
(72) Inventor: ZHOU, Fusheng, Shanghai 201203 (CN); CAI, Lijian, Shanghai 201203 (CN); JIANG, Tao, Shanghai 201203 (CN); ZHAO, Jichen, Shanghai 201203 (CN); LIU, Yingtao, Shanghai 201203 (CN); ZHAO, Jinzhu, Shanghai 201203 (CN); ZHANG, Leitao, Shanghai 201203 (CN); LIU, Zhubo, Shanghai 201203 (CN); PENG, Ling, Shanghai 201203 (CN); HE, Wan, Shanghai 201203 (CN); YANG, Huabin, Shanghai 201203 (CN); ZHANG, Tao, Shanghai 201203 (CN); DING, Qian, Shanghai 201203 (CN); ZHENG, Biao, Shanghai 201203 (CN); LV, Qiang, Shanghai 201203 (CN); LAN, Jiong, Shanghai 201203 (CN)
(74) Representative: Abel & Imray LLP
(86) International application number: PCT/CN2020/087428
(87) International publication number: WO 2020/221239

(57) **Abstract**

Disclosed are an oxazaazaquinazolin-7(8H)-ketone compound with a selective inhibition effect on KRAS gene mutation and pharmaceutically acceptable salts thereof, stereoisomers, solvent compounds or prodrugs (as shown in formula I or formula II, see the details of the definition to each group in the formulas in the specification), as well as the pharmaceutical composition containing the compound, and the application thereof in preparation of cancer medicine.

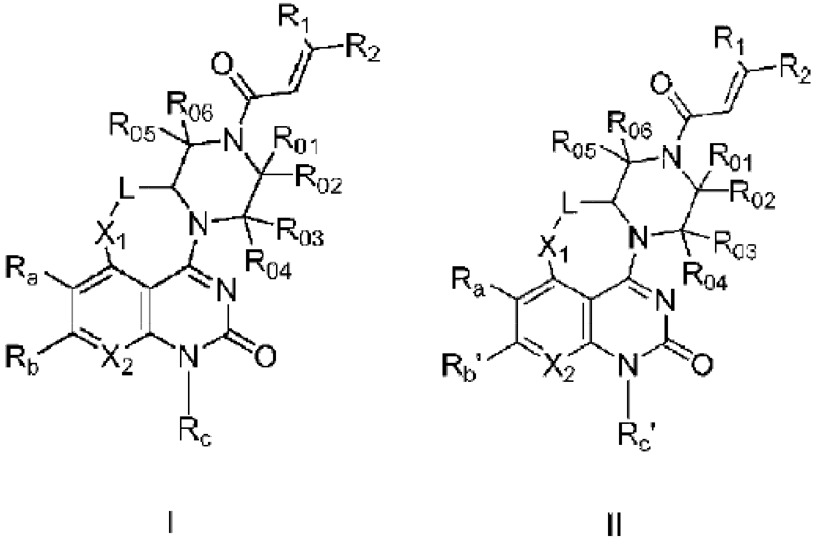

## Description

### Technical Field

The present invention relates to the technical field of medicine, in particular, to a oxaazaquinazolin-7(8H)-one compound, and its use as a selective inhibitor of KRAS gene mutation as well as a pharmaceutical composition prepared therefrom.

### Background of the Invention

Lung cancer is the cancer with the highest incidence in the world. The incidence of lung cancer ranks first among all cancers in China. It is also the cancer with the highest incidence and mortality in China. According to data released by the American Cancer Society in 2016, approximately 1.8 million people suffer from lung cancer, and nearly 80% of lung cancers are non-small cell lung cancer (NSCLC).

RAS is a group of closely related monomeric globular proteins (21 kDa molecular weight), which have 188-189 amino acids and bind to guanosine diphosphate GDP or guanosine triphosphate GTP. Members of the RAS subfamily include HRAS, KRAS and NRAS. RAS acts as a molecular switch, and when RAS contains bound GDP, it is in a dormant or closed position and is "inactive". When cells are exposed to certain growth-promoting stimuli, RAS is induced to convert the bound GDP into GTP. When combined with GTP, RAS is "on" and can interact with other downstream target proteins and activate these proteins. RAS protein itself has a very low inherent ability to hydrolyze GTP and restore it to GDP (thus turning itself into a closed state). The exogenous protein GTPase Activated Protein (GAP) is required to restore it to the closed state. The interaction between GAP and RAS greatly accelerates the conversion of GTP to GDP.

Any mutation in RAS will affect the interaction between RAS and GAP and the ability of the conversion of GTP to GDP. Such mutation will lead to prolonged protein activation time, thereby prolonging cell signaling, which in turn will cause cells to continue to grow and divide. Since such signaling causes cell growth and division, over-activated RAS signaling can eventually lead to cancer.

Among lung cancers, mutations in the RAS genes are confirmed in about 32% of lung cancers. Any one mutation in the three main subtypes of the RAS (HRAS, NRAS, or KRAS) genes can lead to the occurrence of human tumors. It has been reported that the KRAS gene has the highest mutation frequency in the RAS genes, and KRAS mutations are detected in 25-30% of tumors. In comparison, the rates of oncogenic mutations in NRAS and HRAS family members are much lower (8% and 3%, respectively). The most common KRAS mutations are found in residues G12 and G13 as well as residue Q61 in the P loop. The G12C mutation is a frequent mutation of the KRAS gene (mutation of glycine-12 to cysteine). This mutation has been found in about 13% of cancers, about 43% of lung cancers, and almost 100% of MYH-related polyposis (familial colon cancer syndrome).

Therefore, it is a better direction to develop inhibitors that selectively inhibit KRAS mutations. In order to increase the inhibitory activity against KRAS mutations while reducing the inhibitory activity against wild-type KRAS, it has great significance to develop novel selective inhibitors of RAS mutants with higher activity, better selectivity, and lower toxicity.

### Summary of the Invention

The present invention provides a oxaazaquinazolin-7(8H)-one compound, as a selective inhibitor of KRAS mutation, which is advantageous for its high activity, high selectivity and low toxic/side effect and the like.

In one respect, the present invention provides a oxaazaquinazolin-7(8H)-one compound, or a pharmaceutically acceptable salt, stereoisomer, solvate or prodrug thereof, the compound has a structure as represented by formula (I): 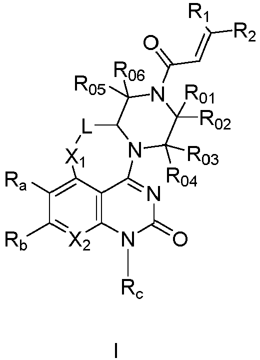 wherein,
R₁, R₂ are each independently hydrogen, cyano, C₁₋₃ alkyl, or -C₁₋₃ alkyl-NR^{a}R^{b};
R₀₁, R₀₂, R₀₃, R₀₄, R₀₅, R₀₆ are each independently hydrogen, C₁₋₆ alkyl, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
or R₀₁, R₀₂ together with the carbon atom attached thereto form C₃₋₆ monocyclic cycloalkyl;
or R₀₃, R₀₄ together with the carbon atom attached thereto form C₃₋₆ monocyclic cycloalkyl;
or R₀₅, R₀₆ together with the carbon atom attached thereto form C₃₋₆ monocyclic cycloalkyl;
L is a bond, (CR_{L1}R_{L2})ₙ, C(O), C(O)C(R_{L1}R_{L2}), or C(R_{L1}R_{L2})C(O);
wherein R_{L1}, R_{L2} are each independently hydrogen, halo, or C₁₋₆ alkyl;
n is 1 or 2;
X₁ is NRₓ₁, O, or CRₓ₂Rₓ₃; wherein Rₓ₁ is hydrogen, or C₁₋₆ alkyl; Rₓ₂, Rₓ₃ are each independently hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{g}R^{h}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
X₂ is N, or CRₓ₄; wherein Rₓ₄ is hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{g}R^{h}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
Rₐ is hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{c}R^{d}, C₂₋₄ alkenyl, C₂₋₄ alkynyl, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
R_{b} is C₆₋₁₀ aryl, or C₅₋₁₀ heteroaryl; the C₆₋₁₀ aryl, C₅₋₁₀ heteroaryl are unsubstituted or substituted by 1, 2, 3, or 4 substituent(s) independently selected from the group S1, the substituents of the group S1 are halo, cyano, nitro, hydroxy, C₁₋₆ alkyl, C₁₋₆ alkoxy, halo C₁₋₆ alkyl, halo C₁₋₆ alkoxy, C₃₋₆ monocyclic cycloalkyl, NRⁱR^{j}, C(O)NR^{e}R^{f}, -SO₂C₁₋₃ alkyl, -SO₂halo C₁₋₃ alkyl, -SC₂NR^{e}R^{f}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-NR^{e}R^{f}, -C₁₋₄ alkyl-C(O)NR^{e}R^{f}, -C₁₋₄ alkyl-SO₂C₁₋₃ alkyl, or C₂₋₄ alkynyl;
R_{c} is C₁₋₆ alkyl, C₆₋₁₀ aryl, C₅₋₁₀ heteroaryl, C₃₋₆ monocyclic cycloalkyl, C₃₋₆ monocyclic heterocyclyl, 7- to 11-membered spirocycloalkyl, -C₁₋₄ alkyl-C₆₋₁₀ aryl, -C₁₋₄ alkyl-C₅₋₁₀ heteroaryl, -NR^{e}-C₆₋₁₀ aryl, -O-C₆₋₁₀ aryl, -C₁₋₄ alkyl-C₃₋₆ monocyclic heterocyclyl, -C₁₋₄ alkyl-C₃₋₆ monocyclic cycloalkyl; wherein
the C₃₋₆ monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione;
the C₃₋₆ monocyclic heterocyclyl is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one;
the -C₁₋₄ alkyl- is unsubstituted or substituted by 1, 2, 3, or 4 substituent(s) independently selected from C₁₋₃ alkyl;
the C₁₋₆ alkyl, C₆₋₁₀ aryl, C₅₋₁₀ heteroaryl, 7- to 11-membered spirocycloalkyl, C₃₋₆ monocyclic cycloalkyl, C₃₋₆ monocyclic heterocyclyl are unsubstituted or substituted by 1, 2, 3, or 4 substituent(s) independently selected from the group S2, the substituents of the group S2 are halo, cyano, hydroxy, C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic heterocyclyl, NRⁱR^{j}, C(O)NR^{e}R^{f}, -SO₂C₁₋₃ alkyl, -SO₂halo C₁₋₃ alkyl, -SC₂NR^{e}R^{f}, -C₁₋₄ alkyl-hydroxy, -C₁₋₄ alkyl-C₂₋₄ alkynyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-C₃₋₆ monocyclic cycloalkyl, -C₁₋₄ alkyl-NR^{e}R^{f}, -C₁₋₄ alkyl-C(O)NR^{e}R^{f}, -C₁₋₄ alkyl-SO₂C₁₋₃ alkyl, or C₂₋₄ alkynyl; wherein the C₃₋₆ monocyclic cycloalkyl in the substituents of the group S2 is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; and the C₁₋₆ alkyl, C₁₋₆ alkoxy, -C₁₋₄ alkyl-, C₃₋₆ monocyclic cycloalkyl, C₃₋₆ monocyclic heterocyclyl in the substituents of the group S2 are optionally substituted by 1, 2, or 3 substituent(s) independently selected from halo, methyl, ethyl, propyl, isopropyl, trifluoromethyl, amino, N(CH₃)₂, hydroxy, carboxyl; wherein the C₃₋₆ monocyclic cycloalkyl is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran;
_{R}^{a}, _{R}^{b}, _{R}^{c}, R^{d}, R^{e}, R^{f}, R^{g}, R^{h} are each independently hydrogen, or C₁₋₃ alkyl;
Rⁱ, R^{j} are each independently hydrogen, C₁₋₃ alkyl, -C(O)C₁₋₃ alkyl, -CO₂C₁₋₃ alkyl.

In one embodiment of the present invention, the compound of formula (I) has a structure as shown in formula (IA) or formula (IB): 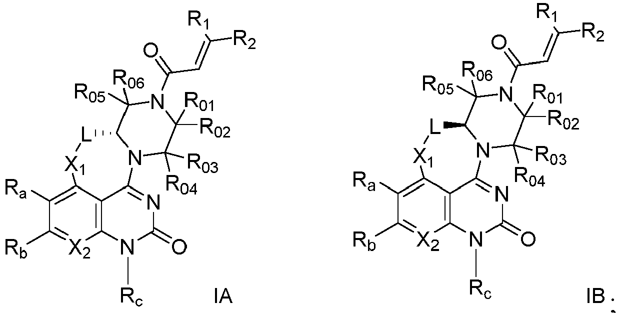 wherein each group is as defined above.

In another aspect, the present invention provides an oxaazaquinazolin-7(8H)-one compound or a pharmaceutically acceptable salt, stereoisomer, solvate or prodrug thereof, the compound has a structure as represented by formula (II): 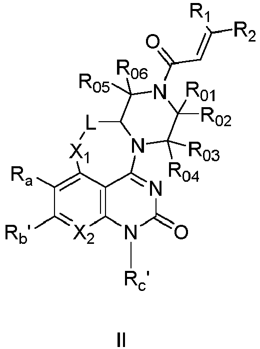 wherein R_{b}' is C₆₋₁₀ aryl, C₅₋₁₀ heteroaryl, C₃₋₆ monocyclic heterocyclyl, pyrimidinonyl, or pyridonyl; the C₆₋₁₀ aryl, C₅₋₁₀ heteroaryl, C₃₋₆ monocyclic heterocyclyl, pyrimidinonyl, and pyridonyl are unsubstituted or substituted by 1, 2, 3, or 4 substituent(s) independently selected from the group S1, or substituted by 1, 2, 3, or 4 substituent(s) independently selected from deuterated C₁₋₆ alkyl and deuterated C₁₋₆ alkoxy; the substituents of the group S1 are halo, cyano, nitro, hydroxy, C₁₋₆ alkyl, C₁₋₆ alkoxy, halo C₁₋₆ alkyl, halo C₁₋₆ alkoxy, C₃₋₆ monocyclic cycloalkyl, NRⁱR^{j}, C(O)NR^{e}R^{f}, -SO₂C₁₋₃ alkyl, -SO₂halo C₁₋₃ alkyl, -SC₂NR^{e}R^{f}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-NR^{e}R^{f}, -C₁₋₄ alkyl-C(O)NR^{e}R^{f}, -C₁₋₄ alkyl-SO₂C₁₋₃ alkyl, or C₂₋₄ alkynyl;
R_{c}' is C₁₋₆ alkyl, C₆₋₁₀ aryl, C₅₋₁₀ heteroaryl, C₃₋₆ monocyclic cycloalkyl, C₃₋₆ monocyclic heterocyclyl, 7- to 11-membered spirocycloalkyl, -C₁₋₄ alkyl-C₆₋₁₀ aryl, -C₁₋₄ alkyl-C₅₋₁₀ heteroaryl, -NR^{e}-C₆₋₁₀ aryl, -O-C₆₋₁₀ aryl, -C₁₋₄ alkyl-C₃₋₆ monocyclic heterocyclyl, -C₁₋₄ alkyl-C₃₋₆ monocyclic cycloalkyl, pyrimidinonyl, or pyridonyl;
wherein,
the C₃₋₆ monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione;
the C₃₋₆ monocyclic heterocyclyl is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one;
the -C₁₋₄ alkyl- is unsubstituted or substituted by 1, 2, 3, or 4 substituent(s) independently selected from C₁₋₃ alkyl;
the C₁₋₆ alkyl, C₆₋₁₀ aryl, C₅₋₁₀ heteroaryl, 7- to 11-membered spirocycloalkyl, C₃₋₆ monocyclic cycloalkyl, C₃₋₆ monocyclic heterocyclyl, pyrimidinonyl, and pyridonyl are unsubstituted or substituted by 1, 2, 3, or 4 substituent(s) independently selected from the group S2, or substituted by 1, 2, 3, or 4 substituent(s) independently selected from deuterated C₁₋₆ alkyl and deuterated C₁₋₆ alkoxy; the substituents of the group S2 are halo, cyano, hydroxy, C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic heterocyclyl, NRⁱR^{j}, C(O)NR^{e}R^{f}, -SO₂C₁₋₃ alkyl, -SO₂halo C₁₋₃ alkyl, -SC₂NR^{e}R^{f}, -C₁₋₄ alkyl-hydroxy, -C₁₋₄ alkyl-C₂₋₄ alkynyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-C₃₋₆ monocyclic cycloalkyl, -C₁₋₄ alkyl-NR^{e}R^{f}, -C₁₋₄ alkyl-C(O)NR^{e}R^{f}, -C₁₋₄ alkyl-SO₂C₁₋₃ alkyl, or C₂₋₄ alkynyl; wherein the C₃₋₆ monocyclic cycloalkyl in the substituents of the group S2 is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; and the C₁₋₆ alkyl, C₁₋₆ alkoxy, -C₁₋₄ alkyl-, C₃₋₆ monocyclic cycloalkyl, C₃₋₆ monocyclic heterocyclyl in the substituents of the group S2 are optionally substituted by 1, 2, or 3 substituent(s) independently selected from halo, methyl, ethyl, propyl, isopropyl, trifluoromethyl, amino, N(CH₃)₂, hydroxy, carboxyl; wherein the C₃₋₆ monocyclic cycloalkyl is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran;
R^{e}, R^{f} are each independently hydrogen, or C₁₋₃ alkyl;
Rⁱ, R^{j} are each independently hydrogen, C₁₋₃ alkyl, -C(O)C₁₋₃ alkyl, -CO₂C₁₋₃ alkyl;
R₁, R₂, R₀₁, R₀₂, R₀₃, R₀₄, R₀₅, R₀₆, L, X₁, X₂, Rₐ are as defined above.

In one embodiment of the present invention, the compound of formula (II) has a structure as shown in formula (IIA) or formula (IIB): 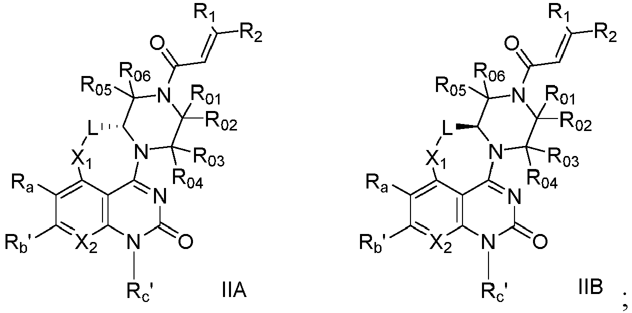 wherein each group is as defined above.

In one embodiment of the present invention, pyridonyl in R_{b}', R_{c}' is pyridinyl-2(1H)-one.

In one embodiment of the present invention, pyrimidinonyl in R_{b}', R_{c}' is pyrimidinyl-4(3H)-one.

In one embodiment of the present invention, the substituents of the group S1 are halo, cyano, nitro, hydroxy, C₁₋₃ alkyl, C₁₋₃ alkoxy, halo C₁₋₃ alkyl, halo C₁₋₃ alkoxy, C₃₋₆ monocyclic cycloalkyl, NRⁱR^{j}, -C(O)NR^{e}R^{f}, -SO₂C₁₋₃ alkyl, -SO₂halo C₁₋₃ alkyl, -SO₂NR^{e}R^{f}, -C₁₋₂ alkyl-hydroxy, -C₁₋₂ alkyl-cyano, -C₁₋₂ alkyl-C₁₋₃ alkoxy, -C₁₋₂ alkyl-halo C₁₋₃ alkyl, -C₁₋₂ alkyl-halo C₁₋₃ alkoxy, -C₁₋₂ alkyl-C₃₋₆ monocyclic heterocyclyl, -C₁₋₂ alkyl-NR^{e}R^{f}, -C₁₋₂ alkyl-C(O)NR^{e}R^{f}, -C₁₋₂ alkyl-SO₂C₁₋₃ alkyl, or C₂₋₄ alkynyl.

In one embodiment of the present invention, the substituents of the group S1 are halo, cyano, nitro, hydroxy, C₁₋₃ alkyl, C₁₋₃ alkoxy, halo C₁₋₃ alkyl, halo C₁₋₃ alkoxy, C₃₋₆ monocyclic cycloalkyl, NRⁱR^{j}, -C(O)NR^{e}R^{f}, -SO₂C₁₋₃ alkyl, -SO₂halo C₁₋₃ alkyl, -SO₂NR^{e}R^{f}, -CH₂-hydroxy, -CH₂-cyano, -CH₂-C₁₋₃ alkoxy, -CH₂-halo C₁₋₃ alkyl, -CH₂-halo C₁₋₃ alkoxy, -CH₂-C₃₋₆ monocyclic heterocyclyl, -CH₂-NR^{e}R^{f}, -CH₂-C(O)NR^{e}R^{f}, -CH₂-SO₂C₁₋₃ alkyl, or C₂₋₄ alkynyl; wherein Rⁱ is hydrogen, C₁₋₃ alkyl, -C(O)CH₃, or -CO₂CH₃; R^{e}, R^{f}, R^{j} are each independently hydrogen, or C₁₋₃ alkyl.

In one embodiment of the present invention, the substituents of the group S1 are halo, cyano, nitro, hydroxy, C₁₋₃ alkyl, C₁₋₃ alkoxy, halo C₁₋₃ alkyl, halo C₁₋₃ alkoxy, C₃₋₆ monocyclic cycloalkyl, NRⁱR^{j}, -C(O)NR^{e}R^{f}, -CH₂-hydroxy, -CH₂-cyano; wherein Rⁱ is hydrogen, -C(O)CH₃, or -CO₂CH₃; R^{e}, R^{f}, R^{j} are each independently hydrogen, or C₁₋₃ alkyl.

In one embodiment of the present invention, the substituents of the group S2 are halo, cyano, hydroxy, C₁₋₆ alkyl, C₁₋₃ alkoxy, halo C₁₋₃ alkyl, halo C₁₋₃ alkoxy, C₃₋₆ monocyclic cycloalkyl, C₃₋₆ monocyclic heterocyclyl, amino, NHCH₃, N(CH₃)₂, C(O)NR^{e}R^{f}, -SO₂C₁₋₃ alkyl, -SO₂halo C₁₋₃ alkyl, -SO₂NR^{e}R^{f}, -C₁₋₂ alkyl-hydroxy, -C₁₋₂ alkyl-ethynyl, -C₁₋₂ alkyl-cyano, -C₁₋₂ alkyl-C₁₋₃ alkoxy, -C₁₋₂ alkyl-halo C₁₋₃ alkyl, -C₁₋₂ alkyl-halo C₁₋₃ alkoxy, -C₁₋₂ alkyl-C₃₋₆ monocyclic heterocyclyl, -C₁₋₂ alkyl-C₃₋₆ monocyclic cycloalkyl, -C₁₋₂ alkyl-NR^{e}R^{f}, -C₁₋₂ alkyl-C(O)NR^{e}R^{f}, -C₁₋₂ alkyl-SO₂C₁₋₃ alkyl, or ethynyl; wherein the C₃₋₆ monocyclic cycloalkyl in the substituents of the group S2 is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; and C₁₋₆ alkyl, C₁₋₃ alkoxy, -C₁₋₂ alkyl-, C₃₋₆ monocyclic cycloalkyl, C₃₋₆ monocyclic heterocyclyl in the substituents of the group S2 are optionally substituted by 1, 2, or 3 substituent(s) independently selected from halo, methyl, ethyl, propyl, isopropyl, trifluoromethyl, amino, N(CH₃)₂, hydroxy, carboxyl; wherein the C₃₋₆ monocyclic cycloalkyl is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1 -dioxide, tetrahydropyran.

In one embodiment of the present invention, the substituents of the group S2 are halo, cyano, hydroxy, C₁₋₄ alkyl, C₁₋₃ alkoxy, halo C₁₋₃ alkyl, halo C₁₋₃ alkoxy, C₃₋₆ monocyclic cycloalkyl, C₃₋₆ monocyclic heterocyclyl, amino, NHCH₃, N(CH₃)₂, C(O)NR^{e}R^{f}, -SO₂C₁₋₃ alkyl, -SO₂halo C₁₋₃ alkyl, -SO₂NR^{e}R^{f}, -CH₂-hydroxy, -CH₂-ethynyl, -CH₂-cyano, -CH₂-C₁₋₃ alkoxy, -CH₂-halo C₁₋₃ alkyl, -CH₂-halo C₁₋₃ alkoxy, -CH₂-C₃₋₆ monocyclic heterocyclyl, -CH₂-C₃₋₆ monocyclic cycloalkyl, -CH₂-NR^{e}R^{f}, -CH₂-C(O)NR^{e}R^{f}, -CH₂-SO₂C₁₋₃ alkyl, or ethynyl; wherein the C₃₋₆ monocyclic cycloalkyl in the substituents of the group S2 is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; and the C₁₋₄ alkyl, C₁₋₃ alkoxy, -CH₂-, C₃₋₆ monocyclic cycloalkyl, C₃₋₆ monocyclic heterocyclyl in the substituents of the group S2 are optionally substituted by 1, 2, or 3 substituent(s) independently selected from halo, methyl, ethyl, propyl, isopropyl, trifluoromethyl, amino, N(CH₃)₂, hydroxy, carboxyl; wherein the C₃₋₆ monocyclic cycloalkyl is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1 -dioxide, tetrahydropyran.

In one embodiment of the present invention, the substituents of the group S2 are halo, cyano, hydroxy, C₁₋₄ alkyl, C₁₋₃ alkoxy, halo C₁₋₃ alkyl, C₃₋₆ monocyclic cycloalkyl, C₃₋₆ monocyclic heterocyclyl, amino, NHCH₃, N(CH₃)₂, -CH₂-hydroxy, -CH2-ethynyl; wherein the C₃₋₆ monocyclic cycloalkyl in the substituents of the group S2 is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; and C₁₋₄ alkyl, C₁₋₃ alkoxy, -CH₂-, C₃₋₆ monocyclic cycloalkyl, C₃₋₆ monocyclic heterocyclyl in the substituents of the group S2 are optionally substituted by 1, 2, or 3 substituent(s) independently selected from halo, methyl, ethyl, propyl, isopropyl, trifluoromethyl, amino, N(CH₃)₂, hydroxy, carboxyl; wherein the C₃₋₆ monocyclic cycloalkyl is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran.

In one embodiment of the present invention, the C₃₋₆ monocyclic cycloalkyl in the substituents of the group S1 is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione.

In one embodiment of the present invention, the C₃₋₆ monocyclic heterocyclyl in the substituents of the group S1 is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one.

In one embodiment of the present invention, the C₃₋₆ monocyclic cycloalkyl in the substituents of the group S2 is selected from the group consisting of: cyclopropyl, cyclobutyl, cyclopentyl, cyclohexyl.

In one embodiment of the present invention, the C₃₋₆ monocyclic heterocyclyl in the substituents of the group S2 is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran.

In one embodiment of the present invention, R₁, R₂ are each independently hydrogen, cyano, C₁₋₃ alkyl, -CH₂NH₂, -CH₂NHCH₃, or -CH₂N(CH₃)₂.

In one embodiment of the present invention, R₁ is hydrogen; R₂ is hydrogen, cyano, C₁₋₃ alkyl, -CH₂NH₂, -CH₂NHCH₃, or -CH₂N(CH₃)₂.

In one embodiment of the present invention, R₁, R₂ are hydrogen.

In one embodiment of the present invention, R₀₁, R₀₂, R₀₃, R₀₄, R₀₅, R₀₆ are each independently hydrogen, C₁₋₃ alkyl, -C₁₋₂ alkyl-hydroxy, -C₁₋₂ alkyl-cyano, -C₁₋₂ alkyl-C₁₋₃ alkoxy, -C₁₋₂ alkyl-halo C₁₋₃ alkyl, -C₁₋₂ alkyl-halo C₁₋₃ alkoxy;
or R₀₁, R₀₂ together with the carbon atom attached thereto form C₃₋₆ monocyclic cycloalkyl;
or R₀₃, R₀₄ together with the carbon atom attached thereto form C₃₋₆ monocyclic cycloalkyl;
or R₀₅, R₀₆ together with the carbon atom attached thereto form C₃₋₆ monocyclic cycloalkyl.

In one embodiment of the present invention, R₀₁, R₀₂, R₀₃, R₀₄, R₀₅, R₀₆ are each independently hydrogen, C₁₋₃ alkyl, -CH₂-hydroxy, -CH₂-cyano, -CH₂-C₁₋₃ alkoxy, -CH₂-halo C₁₋₃ alkyl, -CH₂-halo C₁₋₃ alkoxy;
or R₀₁, R₀₂ together with the carbon atom attached thereto form C₃₋₆ monocyclic cycloalkyl;
or R₀₃, R₀₄ together with the carbon atom attached thereto form C₃₋₆ monocyclic cycloalkyl;
or R₀₅, R₀₆ together with the carbon atom attached thereto form C₃₋₆ monocyclic cycloalkyl.

In one embodiment of the present invention, R₀₅, R₀₆ are hydrogen; R₀₁, R₀₂, R₀₃, R₀₄ are each independently hydrogen, C₁₋₃ alkyl, -CH₂-hydroxy, -CH₂-cyano, -CH₂-C₁₋₃ alkoxy, -CH₂-halo C₁₋₃ alkyl, -CH₂-halo C₁₋₃ alkoxy;
or R₀₁, R₀₂ together with the carbon atom attached thereto form C₃₋₆ monocyclic cycloalkyl;
or R₀₃, R₀₄ together with the carbon atom attached thereto form C₃₋₆ monocyclic cycloalkyl.

In one embodiment of the present invention, R₀₁, R₀₂ together with the carbon atom attached thereto formed C₃₋₆ monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione.

In one embodiment of the present invention, R₀₃, R₀₄ together with the carbon atom attached thereto formed C₃₋₆ monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione.

In one embodiment of the present invention, R₀₅, R₀₆ together with the carbon atom attached thereto formed C₃₋₆ monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione.

In one embodiment of the present invention, R₀₂, R₀₄ are each independently hydrogen, C₁₋₃ alkyl, -CH₂-hydroxy, -CH₂-cyano, -CH₂-C₁₋₃ alkoxy, -CH₂-halo C₁₋₃ alkyl, -CH₂-halo C₁₋₃ alkoxy; R₀₁, R₀₃, R₀₅, R₀₆ are hydrogen.

In one embodiment of the present invention, R₀₂, R₀₄ are each independently hydrogen, CH₃, -CH₂-hydroxy, or -CH₂-cyano; R₀₁, R₀₃, R₀₅, R₀₆ are hydrogen.

In one embodiment of the present invention, R₀₂, R₀₄ are each independently hydrogen, or CH₃; R₀₁, R₀₃,R₀₅, R₀₆ are hydrogen.

In one embodiment of the present invention, R₀₁, R₀₂, R₀₃, R₀₄, R₀₅, R₀₆ are hydrogen.

In one embodiment of the present invention, L is a bond, or (CR_{L1}R_{L2})ₙ; wherein R_{L1}, R_{L2} are each independently hydrogen, halo, or C₁₋₆ alkyl; n is 1 or 2.

In one embodiment of the present invention, L is a bond, or (CR_{L1}R_{L2})ₙ; wherein R_{L1}, R_{L2} are each independently hydrogen, halo, or C₁₋₃ alkyl; n is 1 or 2.

In one embodiment of the present invention, L is a bond, or (CH₂)ₙ; n is 1 or 2.

In one embodiment of the present invention, L is (CR_{L1}R_{L2})ₙ; wherein R_{L1}, R_{L2} are each independently hydrogen, halo, or C₁₋₃ alkyl; n is 1 or 2.

In one embodiment of the present invention, L is CH₂ or CH₂CH₂.

In one embodiment of the present invention, L is CH₂.

In one embodiment of the present invention, X₁ is NRₓ₁ or O; wherein Rₓ₁ is hydrogen, or C₁₋₆ alkyl.

In one embodiment of the present invention, X₁ is NRₓ₁ or O; wherein Rₓ₁ is hydrogen, or C₁₋₃ alkyl.

In one embodiment of the present invention, X₁ is O.

In one embodiment of the present invention, L is CH₂; X₁ is O.

In one embodiment of the present invention, L is CH₂CH₂; X₁ is O.

In one embodiment of the present invention, X₂ is N or CRₓ₄; wherein Rₓ₄ is hydrogen, halo, C₁₋₆ alkyl, C₁₋₆ alkoxy, or halo C₁₋₆ alkyl.

In one embodiment of the present invention, X₂ is N or CRₓ₄; wherein Rₓ₄ is hydrogen, halo, C₁₋₄ alkyl, C₁₋₄ alkoxy, or halo C₁₋₃ alkyl.

In one embodiment of the present invention, X₂ is N.

In one embodiment of the present invention, X₂ is CRₓ₄; wherein Rₓ₄ is hydrogen, halo, C₁₋₄ alkyl, C₁₋₄ alkoxy, or halo C₁₋₃ alkyl.

In one embodiment of the present invention, X₂ is CRₓ₄; wherein Rₓ₄ is hydrogen, fluorine, chlorine, C₁₋₄ alkyl.

In one embodiment of the present invention, Rₐ is hydrogen, halo, cyano, C₁₋₄ alkyl, C₁₋₄ alkoxy, halo C₁₋₃ alkyl, halo C₁₋₃ alkoxy, C₃₋₆ monocyclic cycloalkyl, NR^{c}R^{d}, C₂₋₄ alkenyl, C₂₋₄ alkynyl, -C₁₋₂ alkyl-hydroxy, -C₁₋₂ alkyl-cyano, -C₁₋₂ alkyl-C₁₋₃ alkoxy, -C₁₋₂ alkyl-halo C₁₋₃ alkyl, or -C₁₋₂ alkyl-halo C₁₋₃ alkoxy; wherein R^{c}, R^{d} are each independently hydrogen, or C₁₋₃ alkyl.

In one embodiment of the present invention, Rₐ is hydrogen, halo, cyano, C₁₋₃ alkyl, C₁₋₃ alkoxy, halo C₁₋₃ alkyl, halo C₁₋₃ alkoxy, C₃₋₆ monocyclic cycloalkyl, NR^{c}R^{d}, vinyl, ethynyl, -CH₂-hydroxy, -CH₂-cyano, -CH₂-C₁₋₃ alkoxy, -CH₂-halo C₁₋₃ alkyl, or -CH₂-halo C₁₋₃ alkoxy; wherein R^{c}, R^{d} are each independently hydrogen, or C₁₋₃ alkyl.

In one embodiment of the present invention, the C₃₋₆ monocyclic cycloalkyl in Rₐ is selected from the group consisting of: cyclopropyl, cyclobutyl, cyclopentyl, cyclohexyl.

In one embodiment of the present invention, Rₐ is hydrogen, halo, cyano, C₁₋₃ alkyl, C₁₋₃ alkoxy, halo C₁₋₃ alkyl, NR^{c}R^{d}, ethynyl, -CH₂-hydroxy, -CH₂-C₁₋₃ alkoxy; wherein R^{c}, R^{d} are each independently hydrogen, or C₁₋₃ alkyl.

In one embodiment of the present invention, Rₐ is hydrogen, halo, cyano, C₁₋₃ alkyl, C₁₋₃ alkoxy, halo C₁₋₃ alkyl, amino, NHCH₃, N(CH₃)₂, ethynyl, -CH₂-hydroxy, or -CH₂-C₁₋₃ alkoxy.

In one embodiment of the present invention, Rₐ is hydrogen, halo, cyano, or C₁₋₃ alkyl.

In one embodiment of the present invention, Rₐ is hydrogen, fluorine, chlorine, cyano, methyl, ethyl, propyl, or isopropyl.

In one embodiment of the present invention, the 7- to 11-membered spirocycloalkyl in R_{c}, R_{c}' is a monospirocycloalkyl containing one spiro atom formed by any two monocyclic cycloalkyl rings selected from cyclopropyl ring, cyclobutyl ring, cyclopentyl ring, and cyclohexyl ring.

In one embodiment of the present invention, the C₆₋₁₀ aryl in R_{b}, R_{c}, R_{b}', R_{c}' are each independently phenyl, naphthyl, a 9- or 10-membered aromatic fused bicyclic ring formed by fusing a phenyl to one C₅₋₆ monocyclic heterocyclyl, or a 9- or 10-membered aromatic fused bicyclic ring formed by fusing a phenyl to one C₅₋₆ monocyclic cycloalkyl.

In one embodiment of the present invention, the C₅₋₆ monocyclic heterocyclyl in the 9- or 10-membered aromatic fused bicyclic ring formed by fusing a phenyl to one C₅₋₆ monocyclic heterocyclyl is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one.

In one embodiment of the present invention, the C₅₋₆ monocyclic cycloalkyl in the 9- or 10-membered aromatic fused bicyclic ring formed by fusing a phenyl to one C₅₋₆ monocyclic cycloalkyl is selected from the group consisting of: cyclopentyl, cyclopentenyl, cyclohexyl, cyclohexenyl, cyclohexdienyl, cyclopentanone, cyclopentan-1,3-dione, cyclohexanone, cyclohexan-1,3-dione.

In one embodiment of the present invention, the C₆₋₁₀ aryl in R_{b}, R_{c}, R_{b}', R_{c}' is phenyl.

In one embodiment of the present invention, the C₅₋₁₀ heteroaryl in R_{b}, R_{c}, R_{b}', R_{c}' are each independently a 5- or 6-membered monoheteroaryl, a 9- or 10-membered biheteroaryl formed by fusing a phenyl to a 5- or 6-membered monoheteroaryl, a 8- to 10-membered biheteroaryl formed by fusing a 5- or 6-membered monoheteroaryl to a 5- or 6-membered monoheteroaryl, a 8- to 10-membered biheteroaryl formed by fusing a 5- or 6-membered monoheteroaryl to one C₅₋₆ monocyclic heterocyclyl, or a 8- to 10-membered biheteroaryl formed by fusing a 5- or 6-membered monoheteroaryl to one C₅₋₆ monocyclic cycloalkyl.

In one embodiment of the present invention, when the C₅₋₁₀ heteroaryl in R_{b}, R_{c}, R_{b}', R_{c}' are a 5- or 6-membered monoheteroaryl, the 5- or 6-membered monoheteroaryl are each independently selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine.

In one embodiment of the present invention, when the C₅₋₁₀ heteroaryl in R_{b}, R_{c}, R_{b}', R_{c}' are a 5- or 6-membered monoheteroaryl, the 5- or 6-membered monoheteroaryl are each independently has a structure selected from the group consisting of:

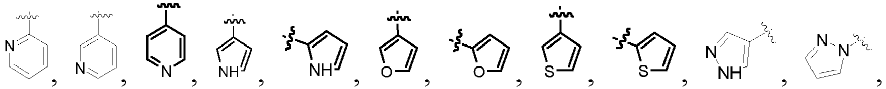

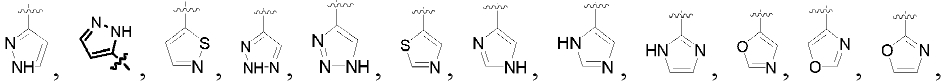

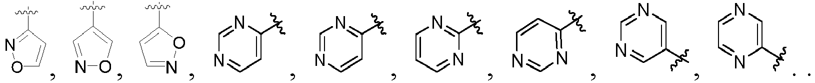

In one embodiment of the present invention, the 5- or 6-membered monoheteroaryl in the 9- or 10-membered biheteroaryl formed by fusing a phenyl to a 5- or 6-membered monoheteroaryl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine.

In one embodiment of the present invention, the 5- or 6-membered monoheteroaryl in the 8- to 10-membered biheteroaryl formed by fusing a 5- or 6-membered monoheteroaryl to a 5- or 6-membered monoheteroaryl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine.

In one embodiment of the present invention, the 5- or 6-membered monoheteroaryl in the 8- to 10-membered biheteroaryl formed by fusing a 5- or 6-membered monoheteroaryl to one C₅₋₆ monocyclic heterocyclyl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine;
the C₅₋₆ monocyclic heterocyclyl is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one.

In one embodiment of the present invention, the 5- or 6-membered monoheteroaryl in the 8- to 10-membered biheteroaryl formed by fusing a 5- or 6-membered monoheteroaryl to one C5-6 monocyclic cycloalkyl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine;
the C₅₋₆ monocyclic cycloalkyl is selected from the group consisting of: cyclopentyl, cyclopentenyl, cyclohexyl, cyclohexenyl, cyclohexdienyl, cyclopentanone, cyclopentan-1,3-dione, cyclohexanone, cyclohexan-1,3-dione.

In one embodiment of the present invention, the 9- or 10-membered biheteroaryl has a structure as shown in formula (a) or formula (b): 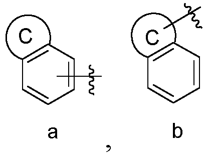 wherein, C ring is a 5- or 6-membered monoheteroaryl; wherein the 5- or 6-membered monoheteroaryl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine.

In one embodiment of the present invention, the C ring has a structure selected from the group consisting of: 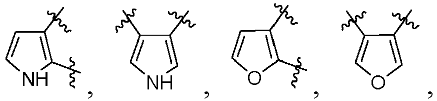 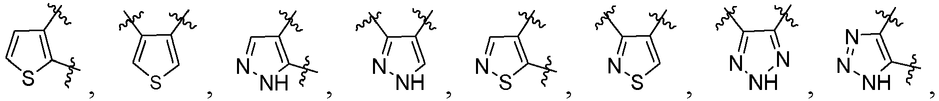 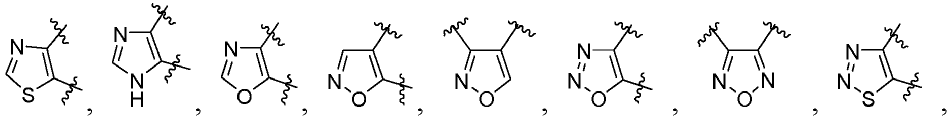 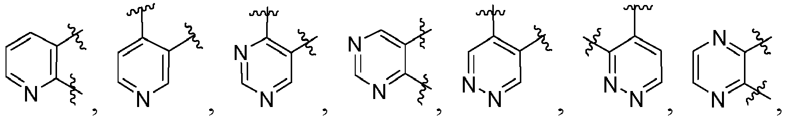 wherein the attached two carbon atoms represented by " 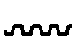 " are a pair of adjacent carbon atoms shared as fused to another ring.

In one embodiment of the present invention, the 9- or 10-membered biheteroaryl formed by fusing a phenyl to a 5- or 6-membered monoheteroaryl is selected from the group consisting of: benzoxazole, benzisoxazole, benzimidazole, benzothiazole, benzisothiazole, benzotriazole, benzofuran, benzothiophene, indole, indazole, isoindole, quinoline, isoquinoline, quinazoline, quinoxaline, cinnoline.

In one embodiment of the present invention, the 9- or 10-membered biheteroaryl formed by fusing a phenyl to a 5- or 6-membered monoheteroaryl is selected from the group consisting of: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline.

In one embodiment of the present invention, the 8- to 10-membered biheteroaryl has a structure as shown in formula (d) or formula (e):

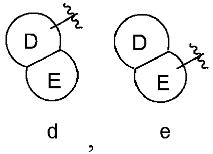

Wherein, D ring, E ring are a 5- or 6-membered monoheteroaryl; wherein the 5- or 6-membered monoheteroaryl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine.

In one embodiment of the present invention, the D ring, E ring have a structure each independently selected from the group consisting of: 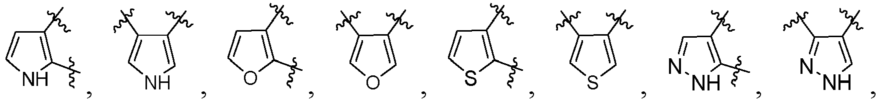 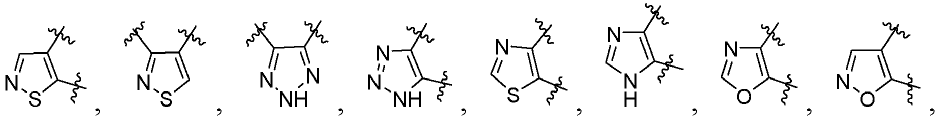 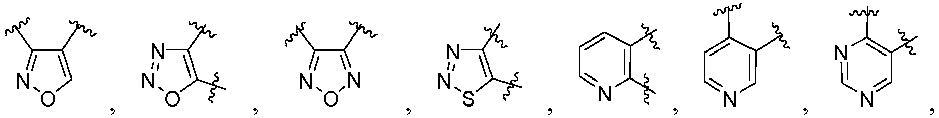 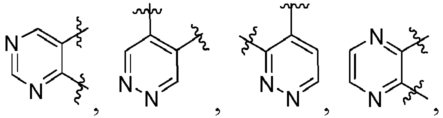 wherein the attached two carbon atoms represented by " 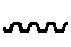 " are a pair of adjacent carbon atoms shared as fused to another ring.

In one embodiment of the present invention, the 8- to 10-membered biheteroaryl formed by fusing a 5- or 6-membered monoheteroaryl to a 5- or 6-membered monoheteroaryl is selected from the group consisting of: pyridopyrimidine and naphthyridine.

In one embodiment of the present invention, the 8- to 10-membered biheteroaryl formed by fusing a 5- or 6-membered monoheteroaryl to a 5- or 6-membered monoheteroaryl is selected from the group consisting of: pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine.

In one embodiment of the present invention, the 9- or 10-membered aromatic fused bicyclic ring formed by fusing a phenyl to one C₅₋₆ monocyclic heterocyclyl has a structure selected from the group consisting of:

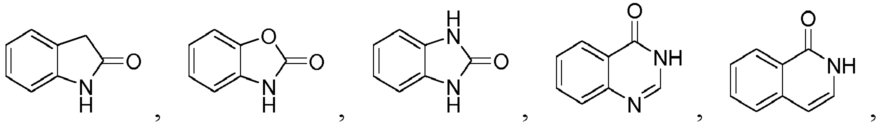

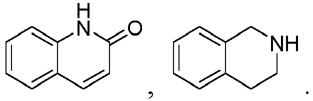

In one embodiment of the present invention, R_{b}, R_{b}' are independently phenyl, naphthyl, a 9- or 10-membered aromatic fused bicyclic ring formed by fusing a phenyl to one C₅₋₆ monocyclic heterocyclyl, a 5- or 6-membered monoheteroaryl, a 9- or 10-membered biheteroaryl formed by fusing a phenyl to a 5- or 6-membered monoheteroaryl, a 8- to 10-membered biheteroaryl formed by fusing a 5- or 6-membered monoheteroaryl to a 5- or 6-membered monoheteroaryl; the phenyl, naphthyl, 9- or 10-membered aromatic fused bicyclic ring, 5- or 6-membered monoheteroaryl, 9- or 10-membered biheteroaryl, 8- to 10-membered biheteroaryl are unsubstituted or substituted by 1, 2, 3, or 4 substituent(s) independently selected from the group S1.

In one embodiment of the present invention, R_{b}, R_{b}' are independently phenyl, or a 9- or 10-membered biheteroaryl formed by fusing a phenyl to a 5- or 6-membered monoheteroaryl; the phenyl, 9- or 10-membered biheteroaryl are unsubstituted or substituted by 1, 2, 3, or 4 substituent(s) independently selected from the group S1.

In one embodiment of the present invention, R_{b}, R_{b}' are independently phenyl, or a 9-membered biheteroaryl formed by fusing a phenyl to a 5-membered monoheteroaryl; the phenyl, 9-membered biheteroaryl are unsubstituted or substituted by 1, 2, 3, or 4 substituent(s) independently selected from the group S1.

In one embodiment of the present invention, when R_{b}, R_{b}' are independently phenyl, R_{b}, R_{b}' have a structure selected from the group consisting of: 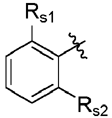 wherein Rₛ₁, Rₛ₂ are each independently selected from the substituent of group S1.

In one embodiment of the present invention, the 9- or 10-membered biheteroaryl formed by fusing a phenyl to a 5- or 6-membered monoheteroaryl has a structure selected from the group consisting of:

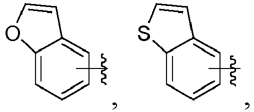

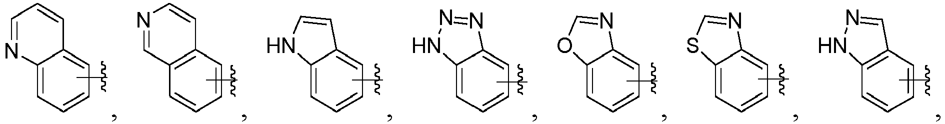

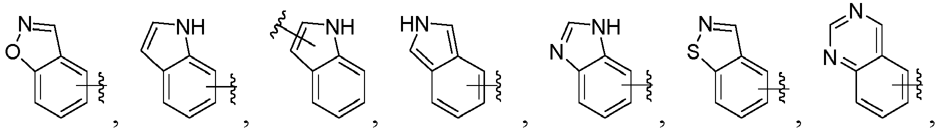

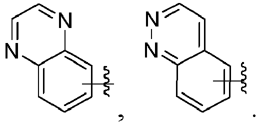

In one embodiment of the present invention, the 8- to 10-membered biheteroaryl formed by fusing a 5- or 6-membered monoheteroaryl to a 5- or 6-membered monoheteroaryl has a structure selected from the group consisting of:

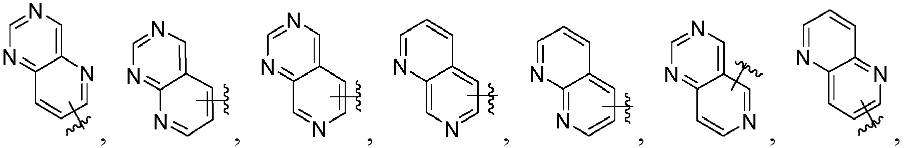

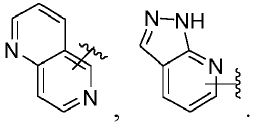

In one embodiment of the present invention, the 9- or 10-membered biheteroaryl formed by fusing a phenyl to a 5- or 6-membered monoheteroaryl has a structure selected from the group consisting of:

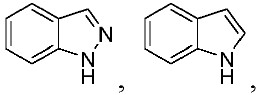

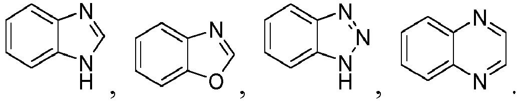

In one embodiment of the present invention, the 8- to 10-membered biheteroaryl formed by fusing a 5- or 6-membered monoheteroaryl to a 5- or 6-membered monoheteroaryl has a structure selected from the group consisting of:

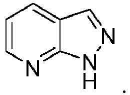

In one embodiment of the present invention, the 9- or 10-membered biheteroaryl formed by fusing a phenyl to a 5- or 6-membered monoheteroaryl or the 8- to 10-membered biheteroaryl formed by fusing a 5- or 6-membered monoheteroaryl to a 5- or 6-membered monoheteroaryl has a structure selected from the group consisting of:

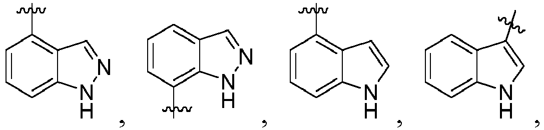

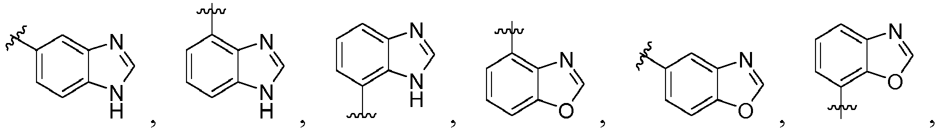

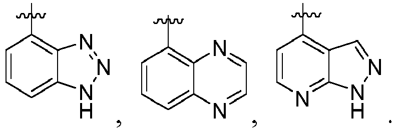

In one embodiment of the present invention, the 9- or 10-membered aromatic fused bicyclic ring formed by fusing a phenyl to one C₅₋₆ monocyclic heterocyclyl has a structure selected from the group consisting of:

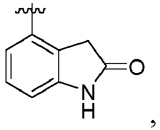

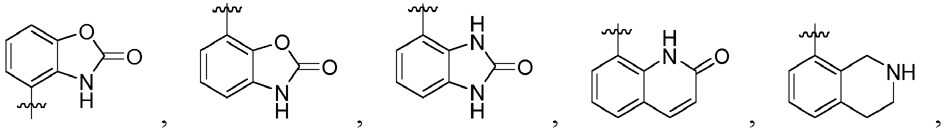

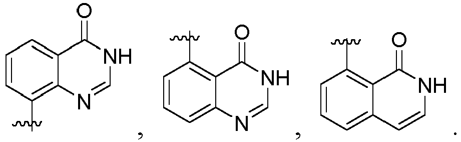

In one embodiment of the present invention, R_{b}, R_{b}' have a structure independently selected from the group consisting of:

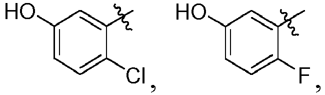

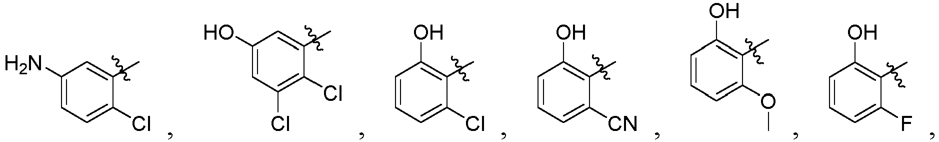

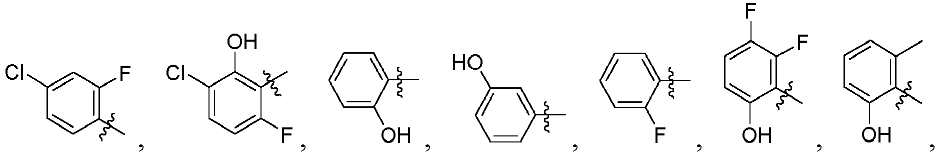

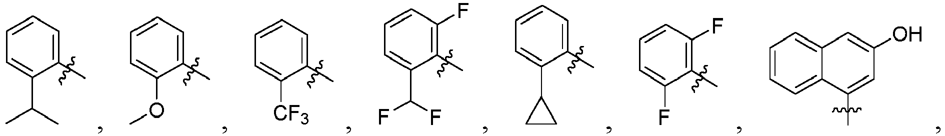

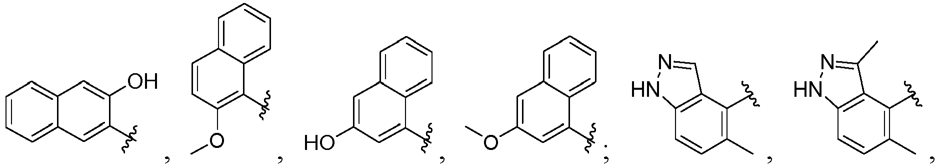

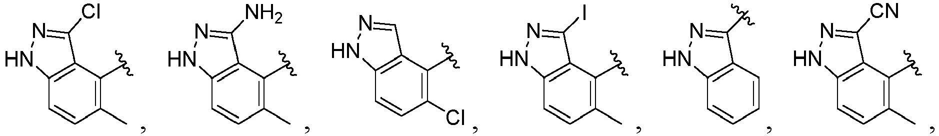

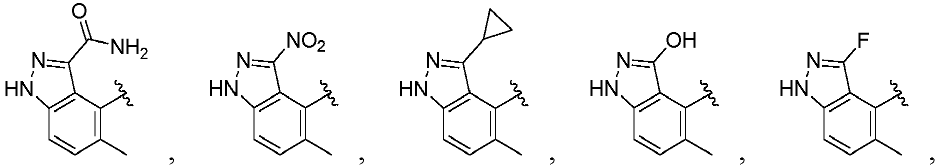

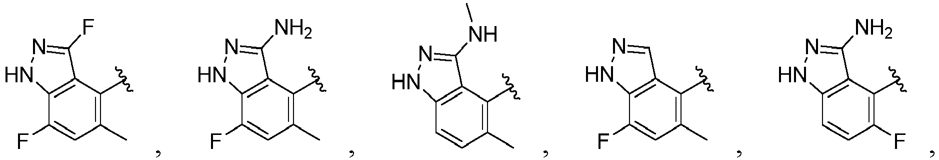

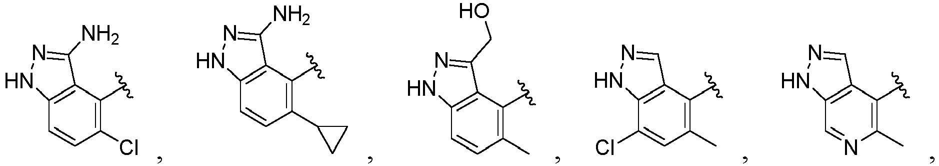

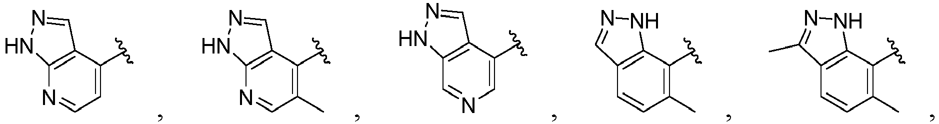

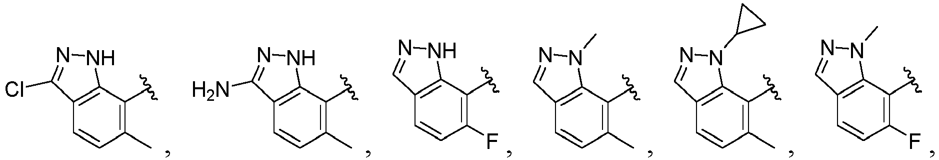

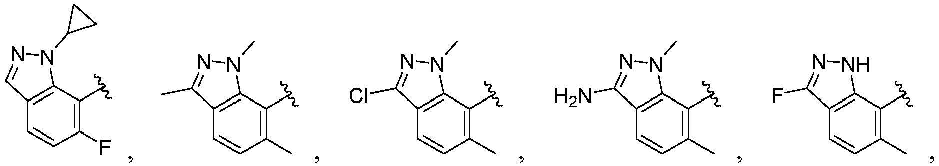

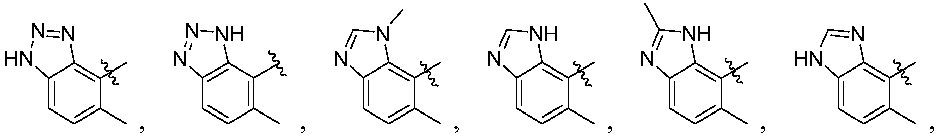

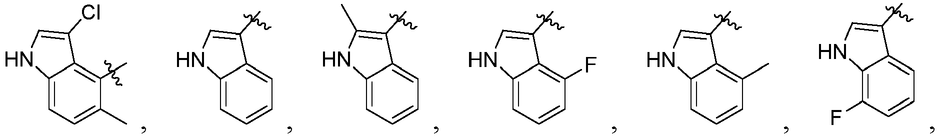

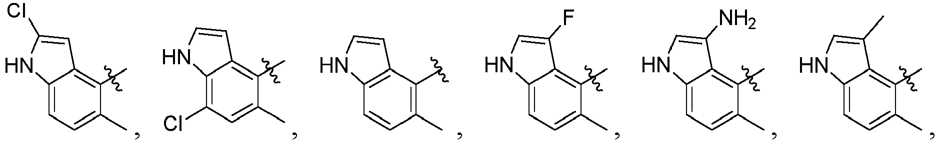

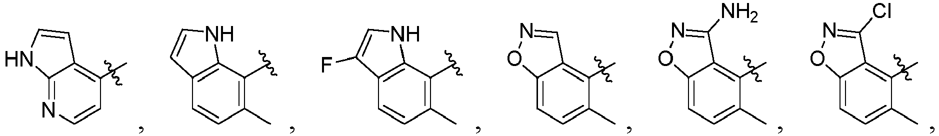

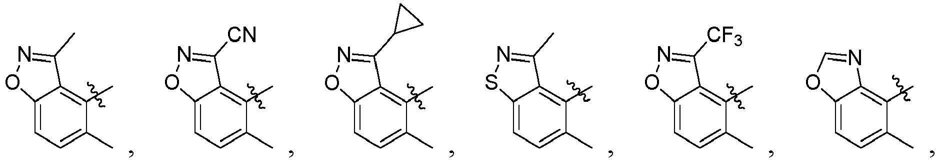

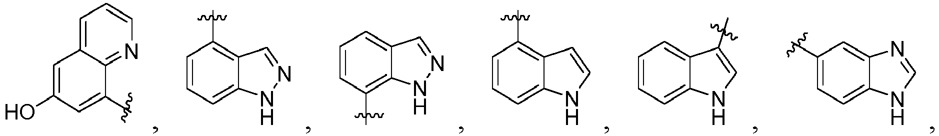

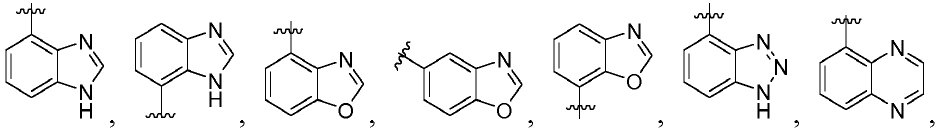

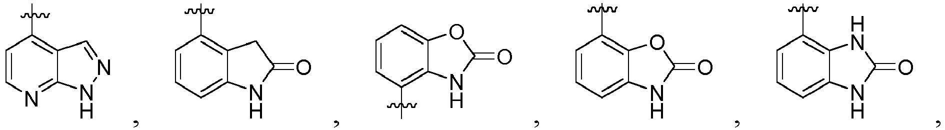

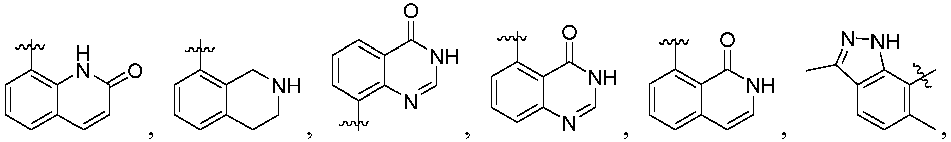

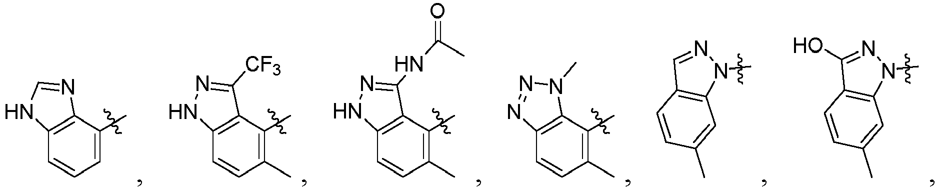

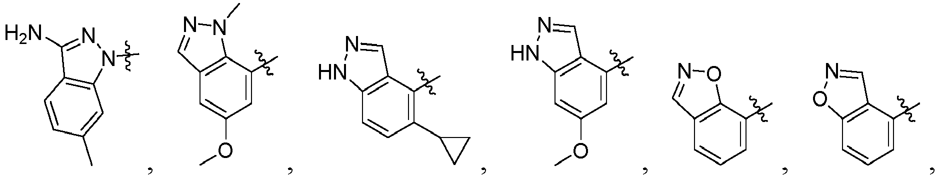

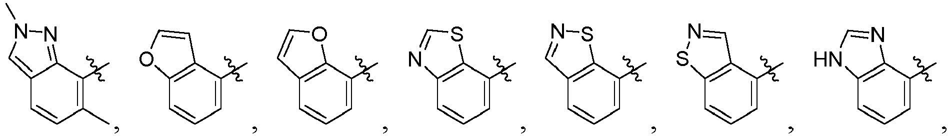

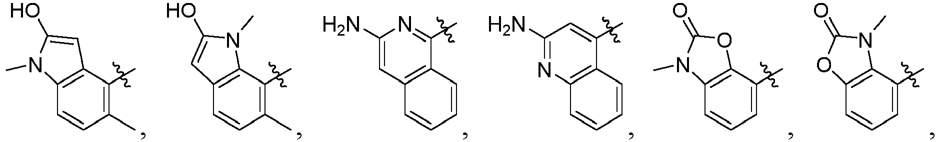

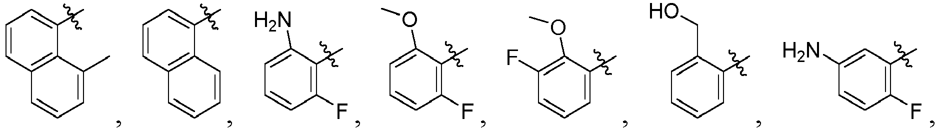

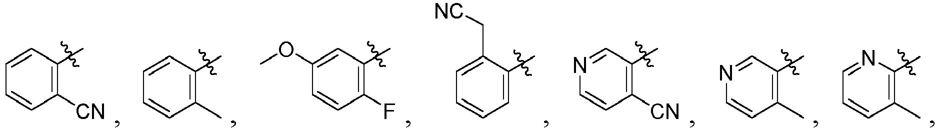

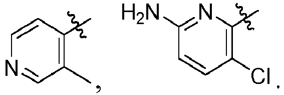

In one embodiment of the present invention, R_{b}, R_{b}' have a structure each independently selected from the group consisting of:

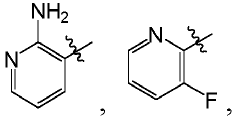

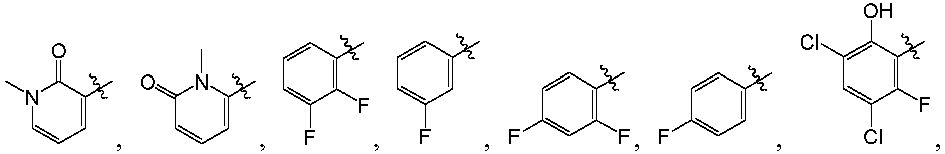

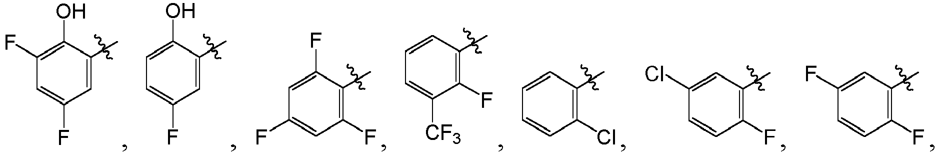

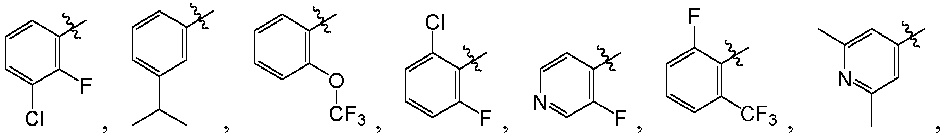

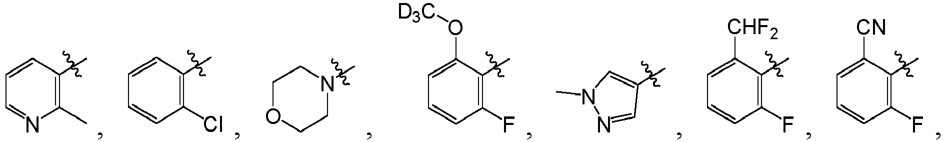

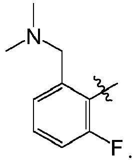

In one embodiment of the present invention, R_{c}, R_{c}' have a structure independently selected from the group consisting of:

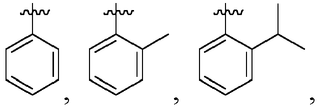

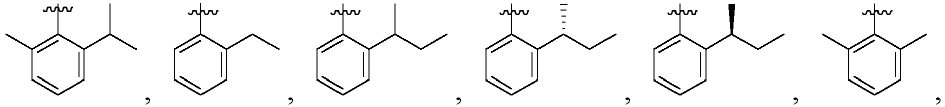

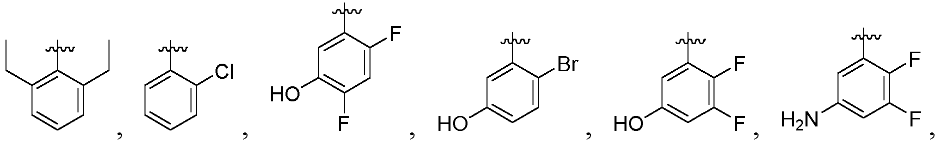

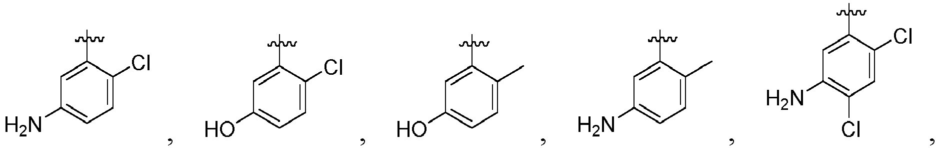

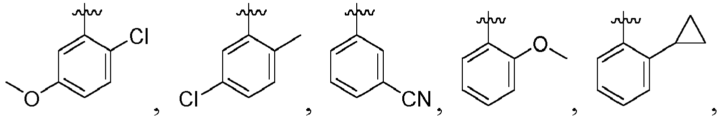

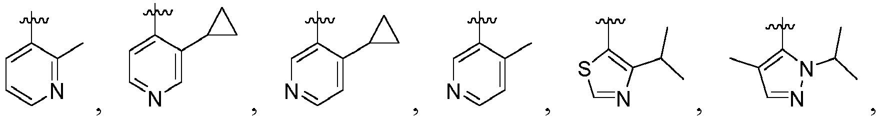

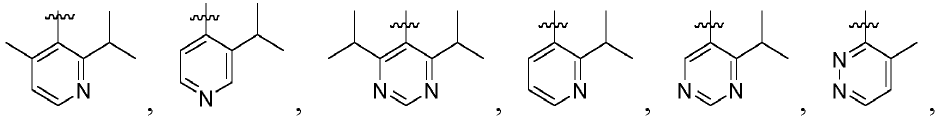

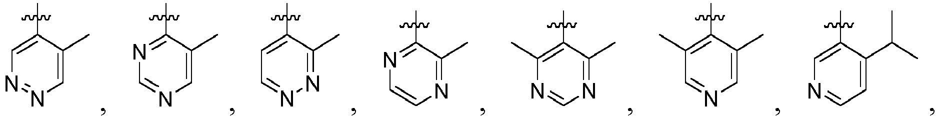

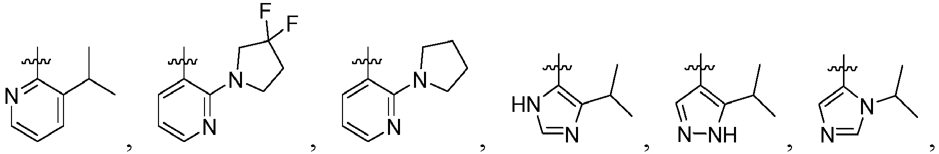

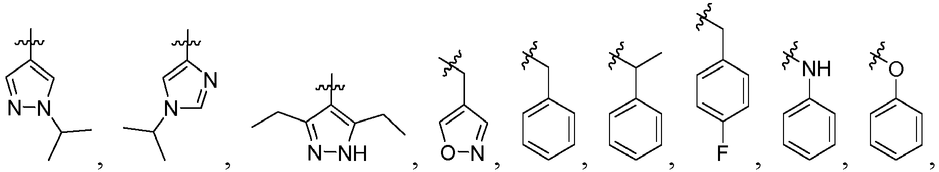

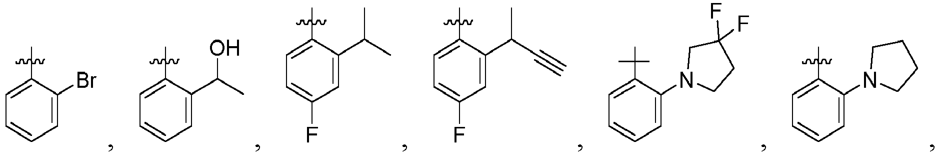

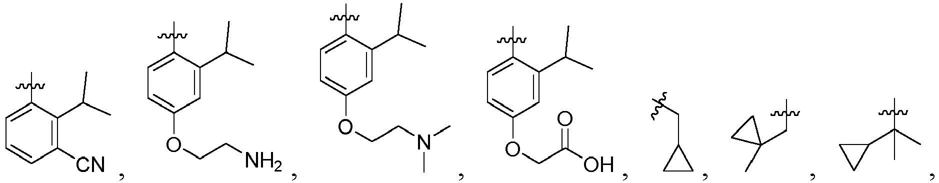

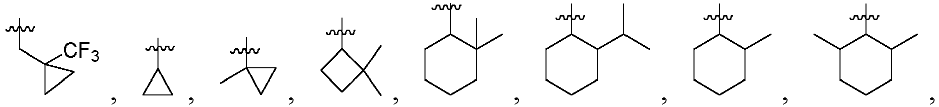

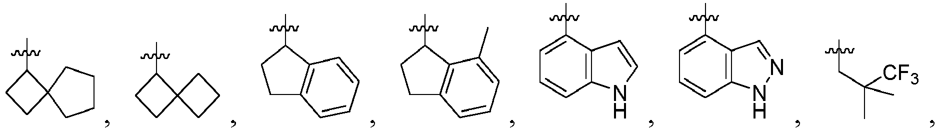

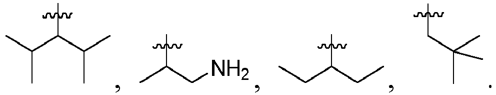

In one embodiment of the present invention, R_{c}, R_{c}' have a structure each independently selected from the group consisting of:

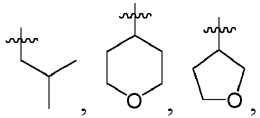

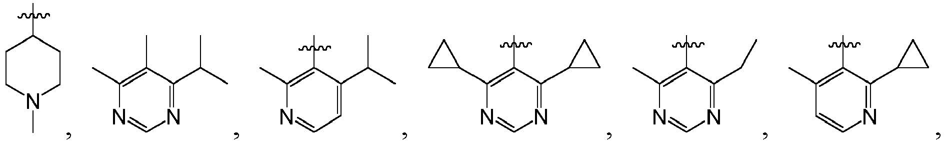

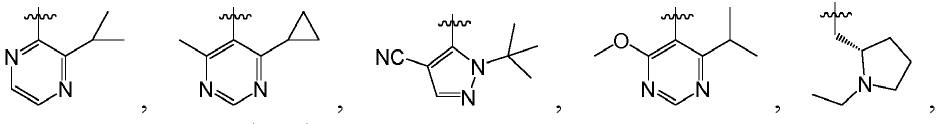

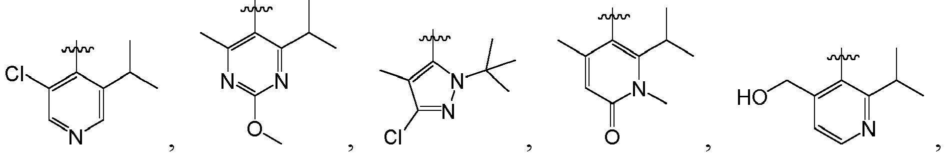

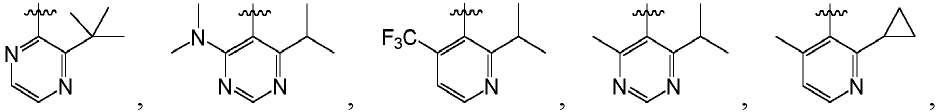

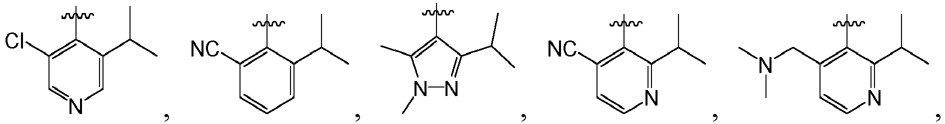

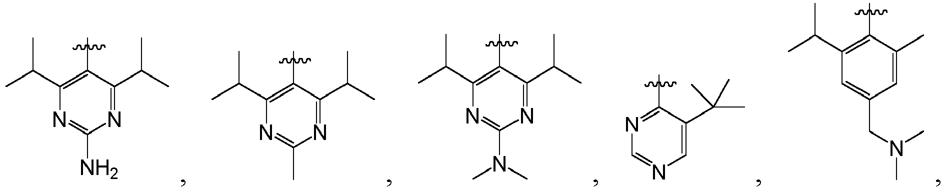

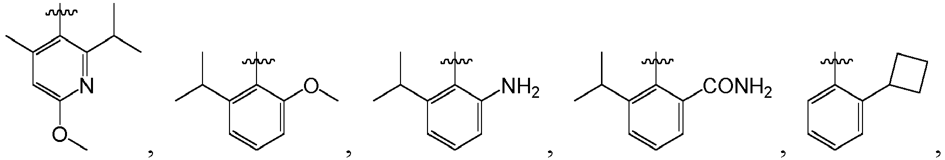

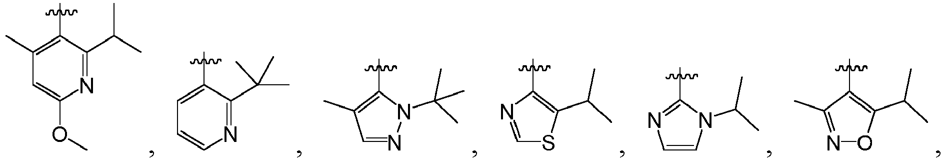

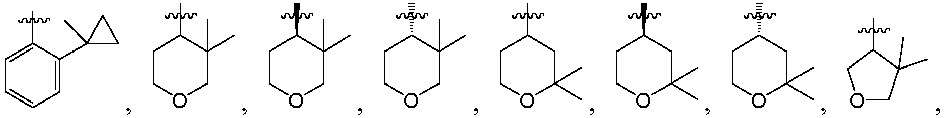

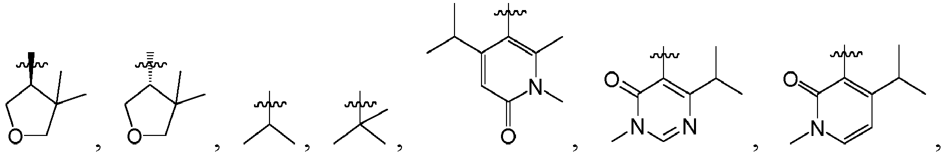

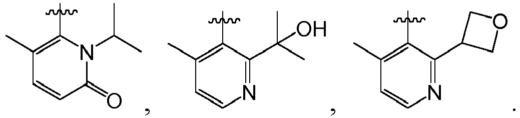

In one embodiment of the present invention, the R₁, R₂, R₀₁, R₀₂, R₀₃, R₀₄, R₀₅, R₀₆, L, X₁, X₂, Rₐ, R_{b}, R_{c}, R_{b}', R_{c}' are each independently the corresponding groups in respective specific compounds in the Examples.

In one embodiment of the present invention, the C₃₋₆ cycloalkyl in any group is selected from the group consisting of: cyclopropyl, cyclobutyl, cyclopentyl, cyclohexyl.

In one embodiment of the present invention, the 3- to 6-membered heterocycloalkyl in any group is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1 -dioxide, tetrahydropyran.

In one embodiment of the present invention, In any group the 5- or 6-membered monocycloheteroaryl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine.

In one embodiment of the present invention, the 8- to 10-membered bicycloheteroaryl in any group is selected from the group consisting of: benzoxazole, benzisoxazole, benzimidazole, benzothiazole, benzisothiazole, benzotriazole, benzofuran, benzothiophene, indole, indazole, isoindole, quinoline, isoquinoline, quinazoline, quinoxaline, cinnoline, pyridopyrimidine, naphthyridine.

In one embodiment of the present invention, the compound of formula (I) is selected from the group consisting of respective specific compounds as noted in the Examples, especially, any compound of Z1 to Z14.

In one embodiment of the present invention, the compound of formula (I) is selected from the group consisting of the compounds as prepared in the Examples of the present application.

In one embodiment of the present invention, the compound of formula (I) is selected from the Table A-1.

In one embodiment of the present invention, the compound of formula (I) is selected from the Table A-2.

In one embodiment of the present invention, the representative compounds of formula (I) and formula (II) include the compounds listed in Table A-1 below, or pharmaceutically acceptable salts, stereoisomers, solvents or prodrugs thereof:

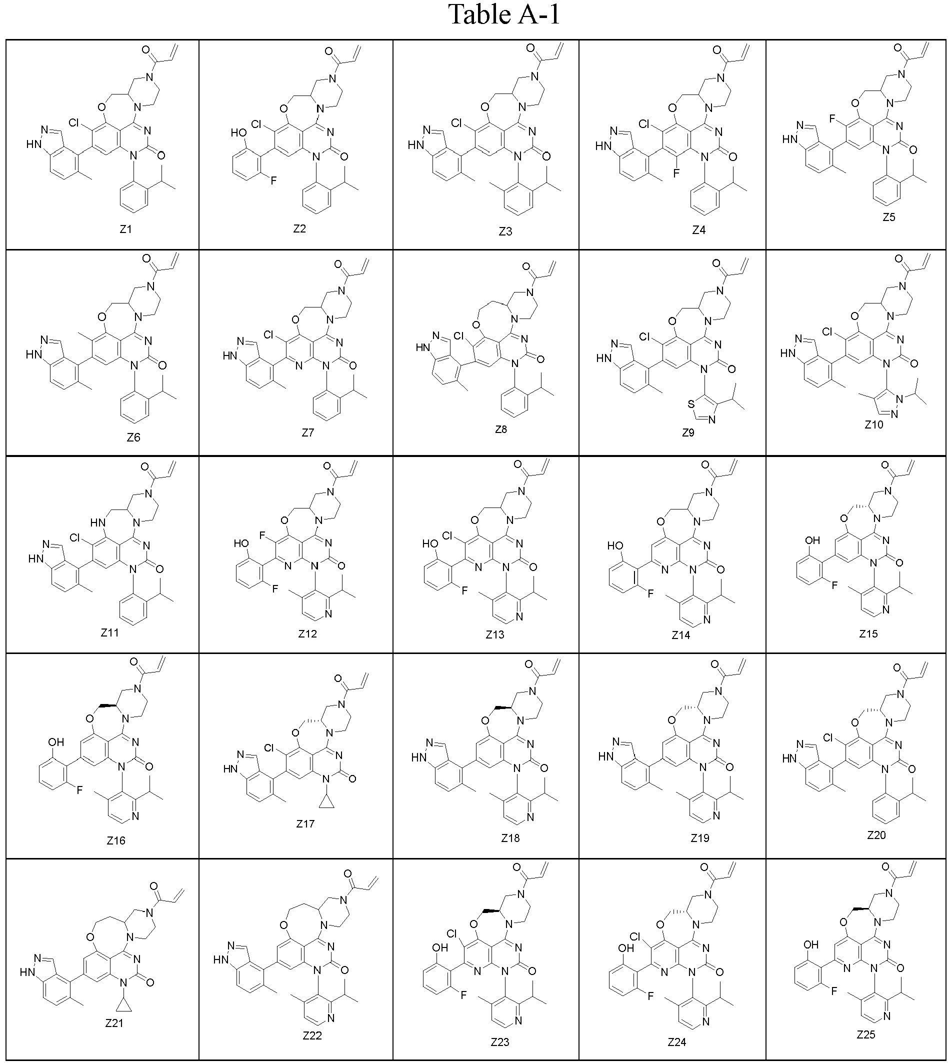

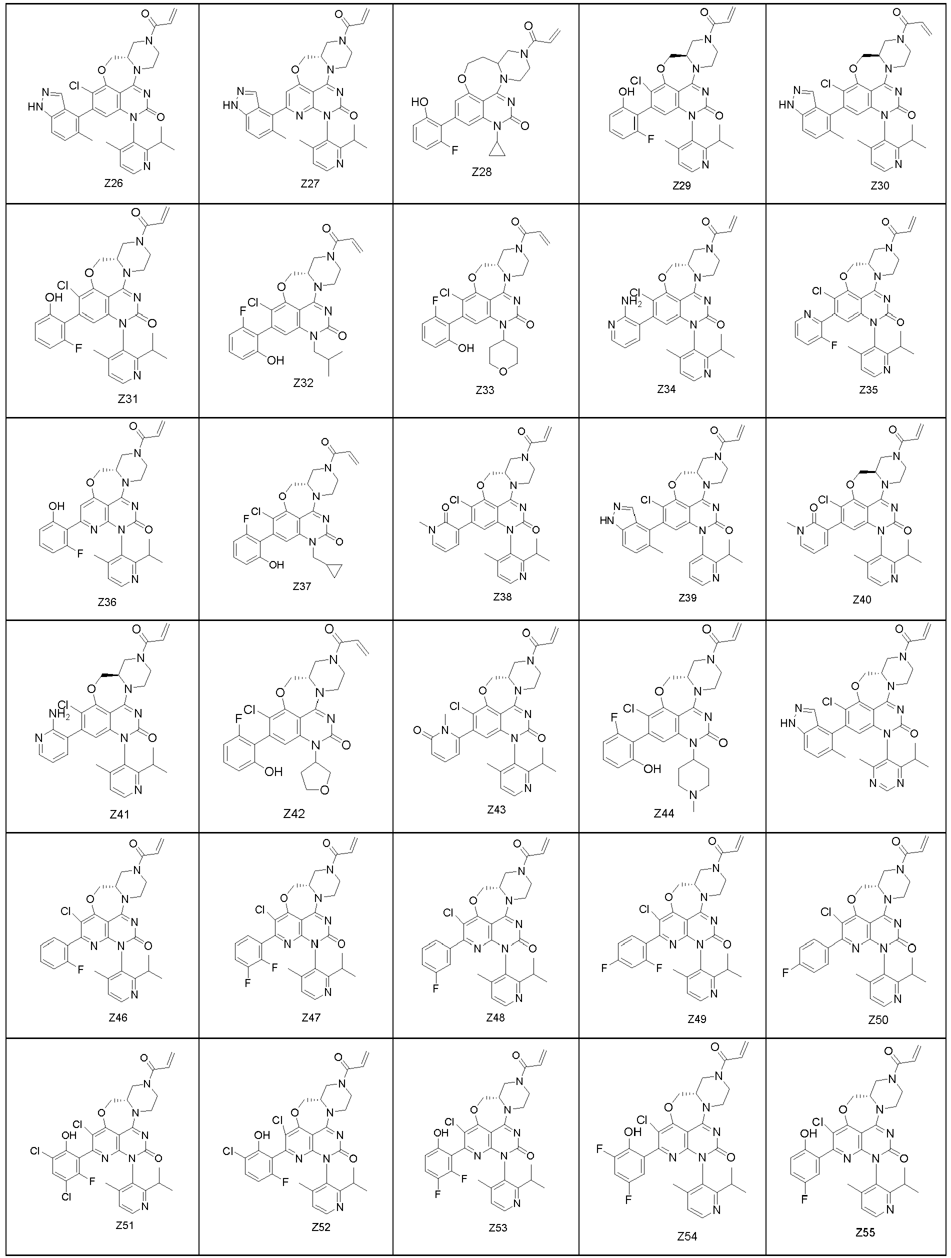

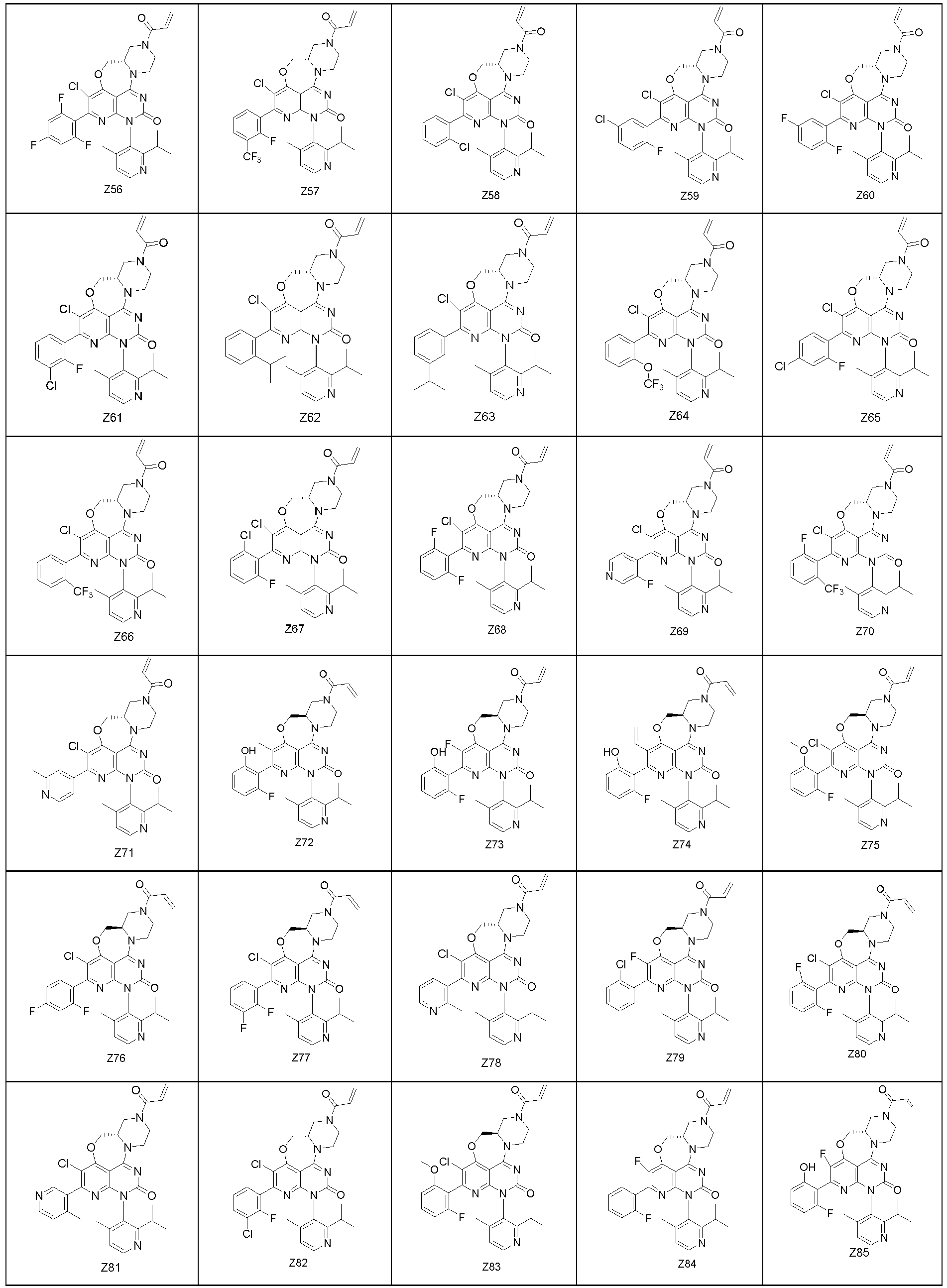

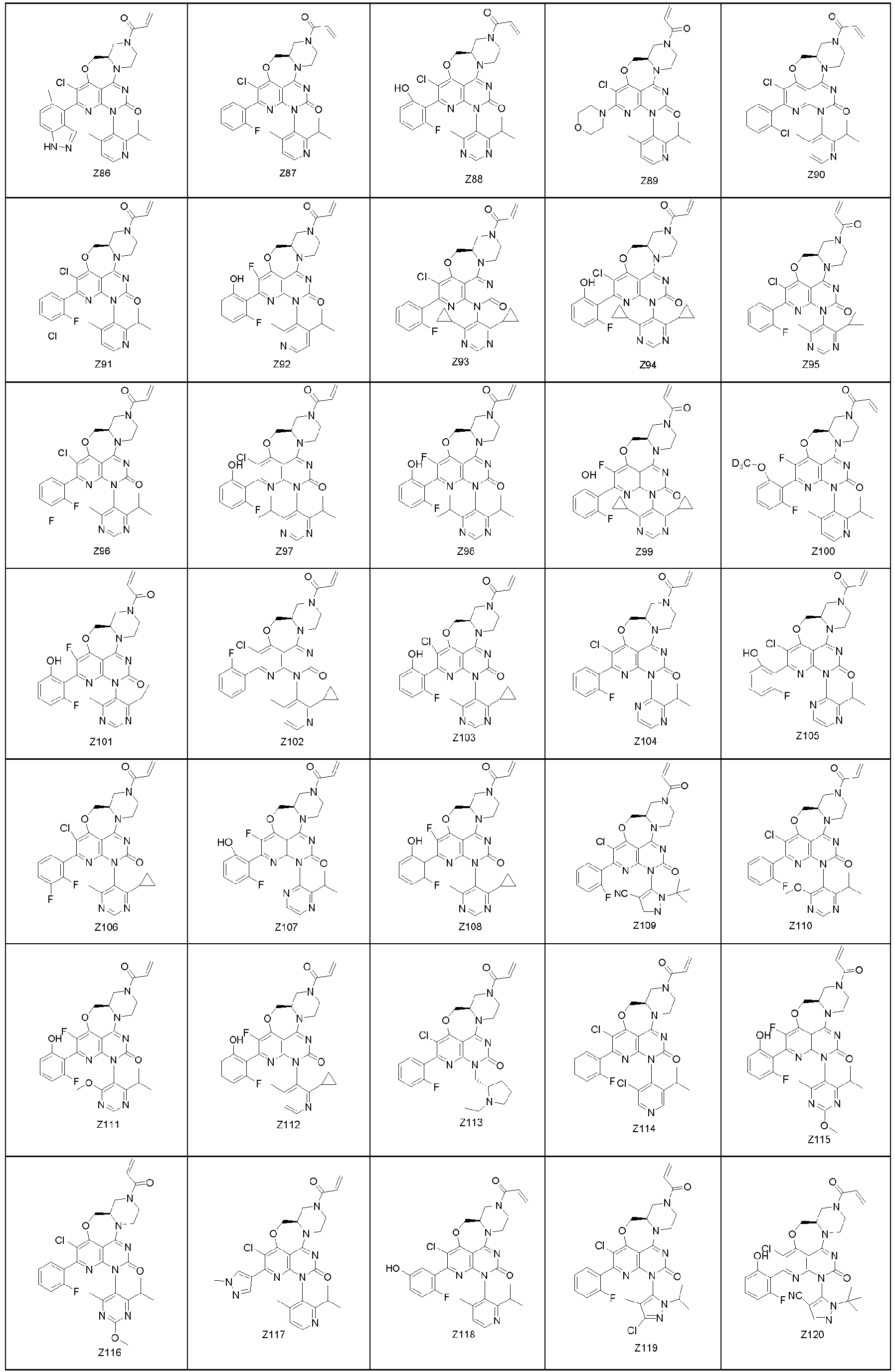

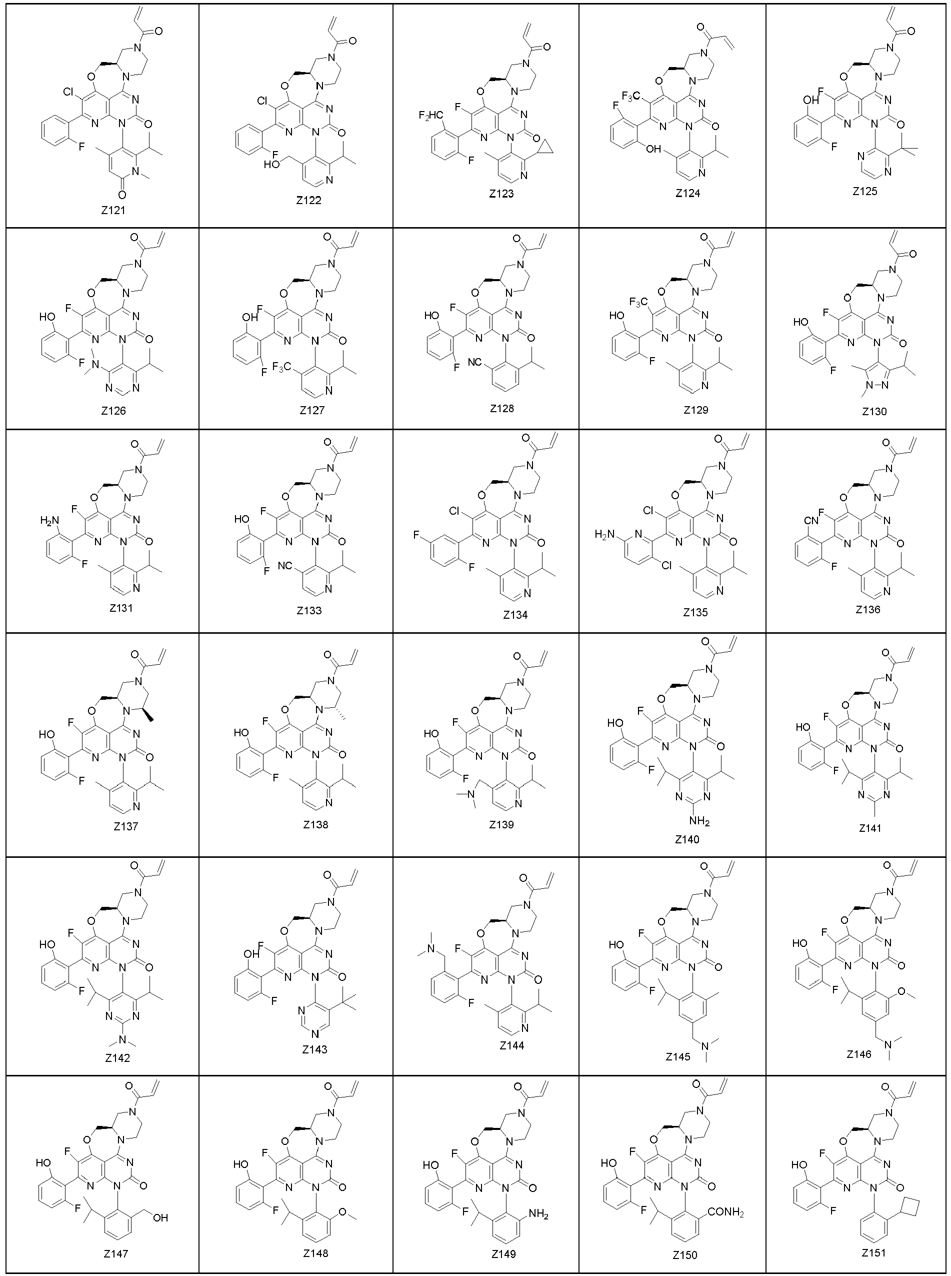

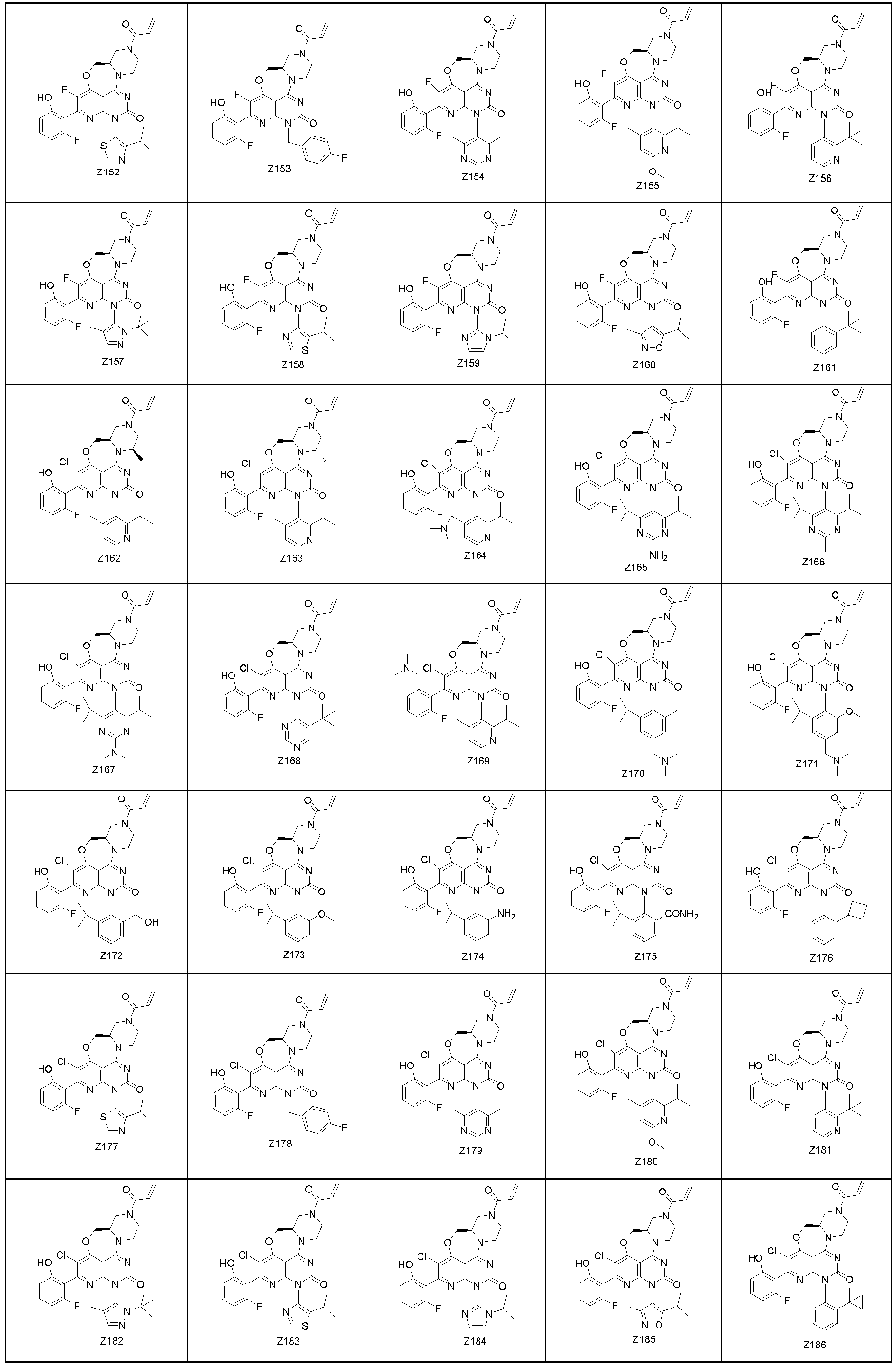

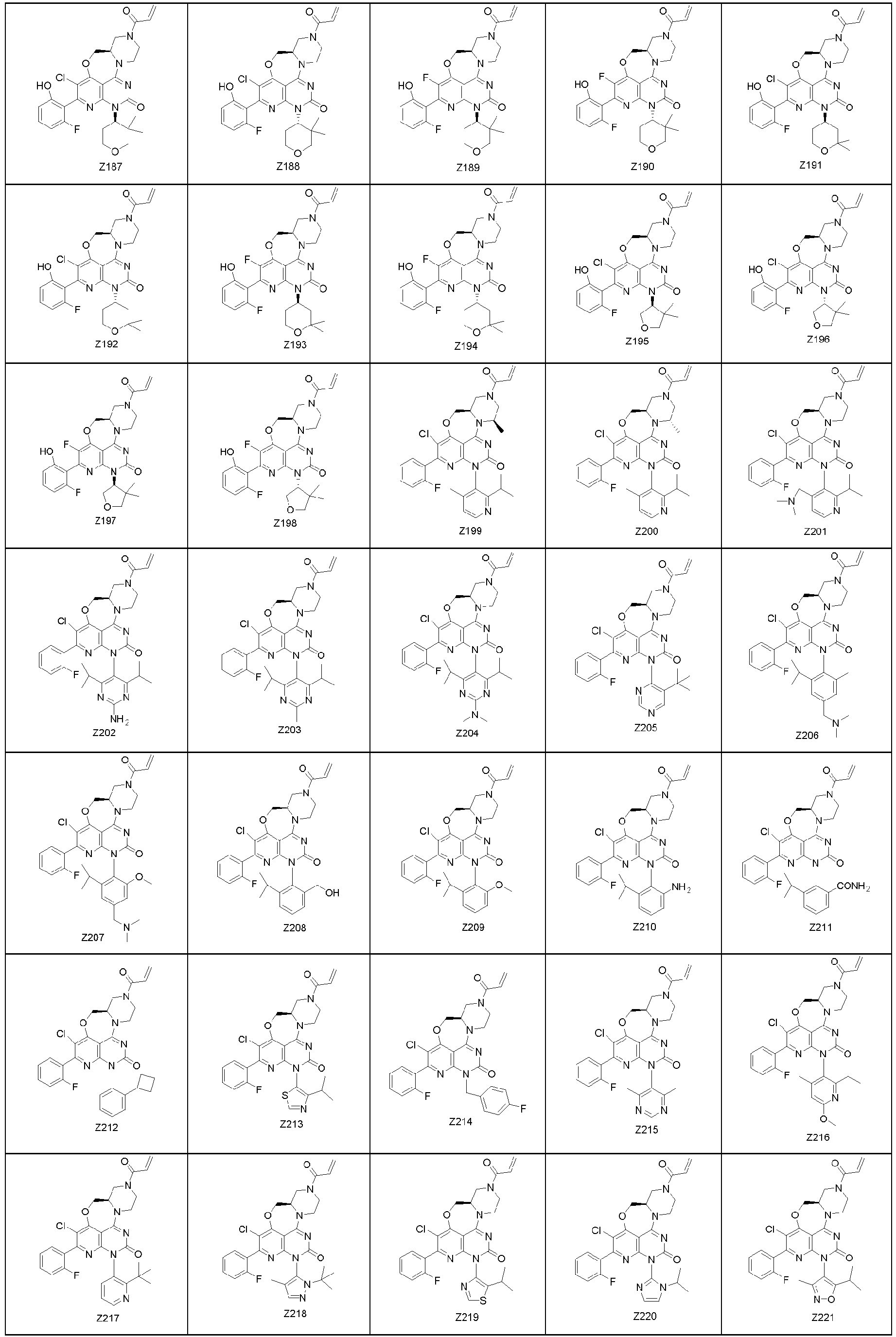

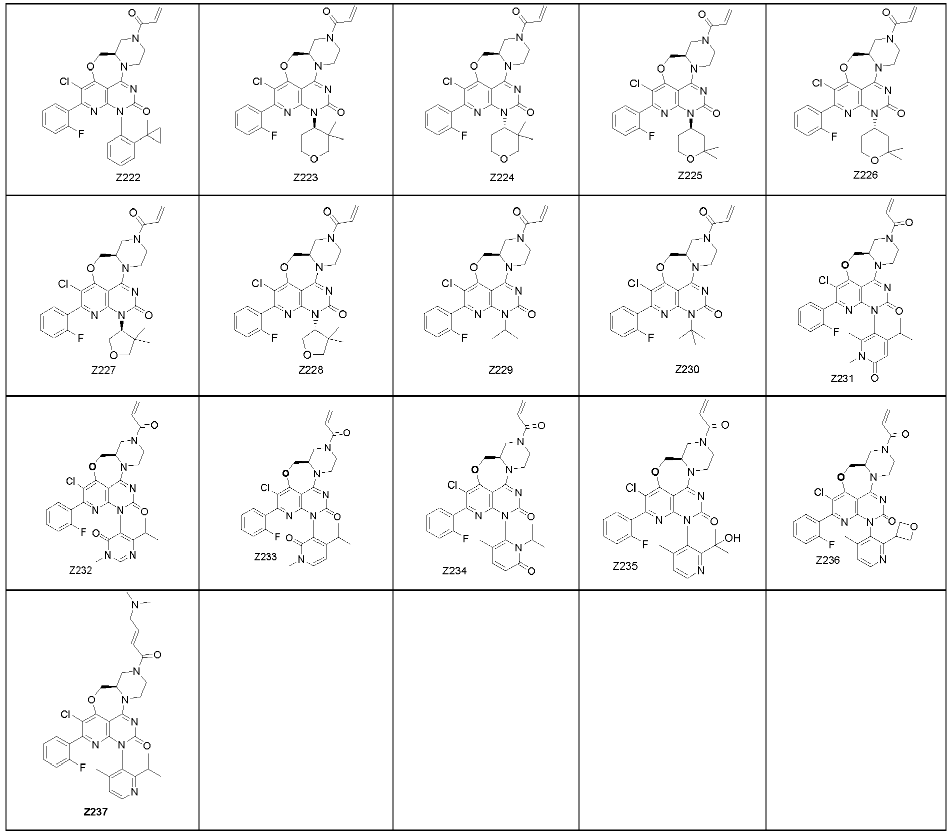

In one embodiment of the present invention, the representative compounds of formula (I) and formula (II) include the compounds listed in Table A-2 below, or pharmaceutically acceptable salts, stereoisomers, solvents or prodrugs thereof:

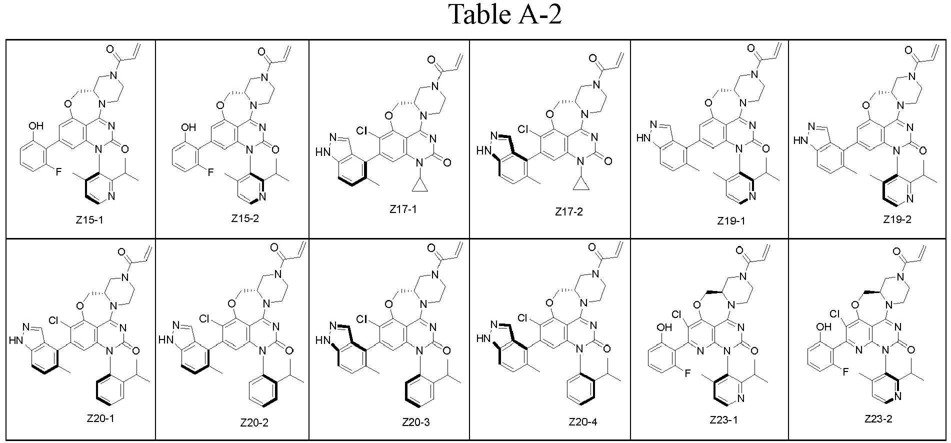

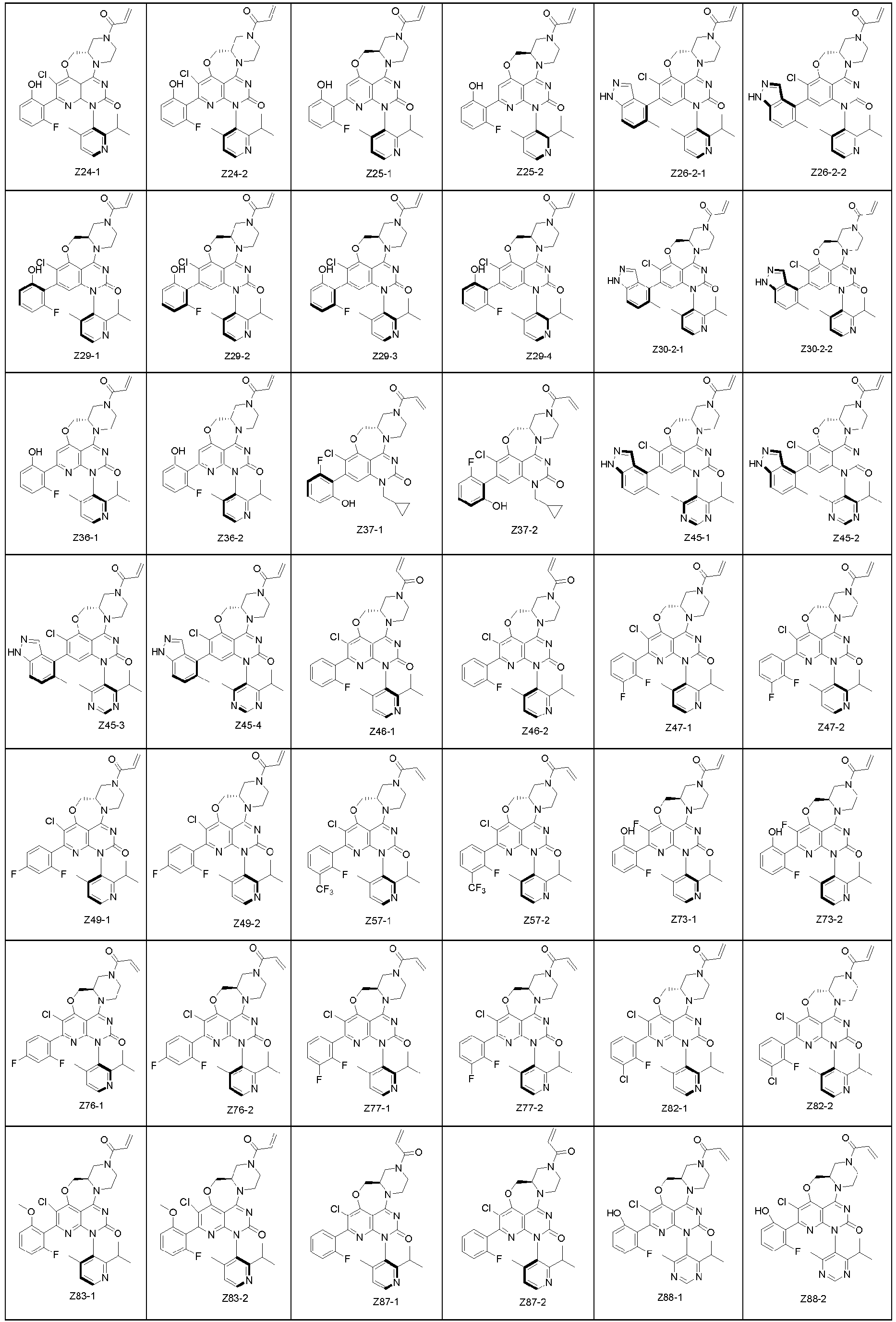

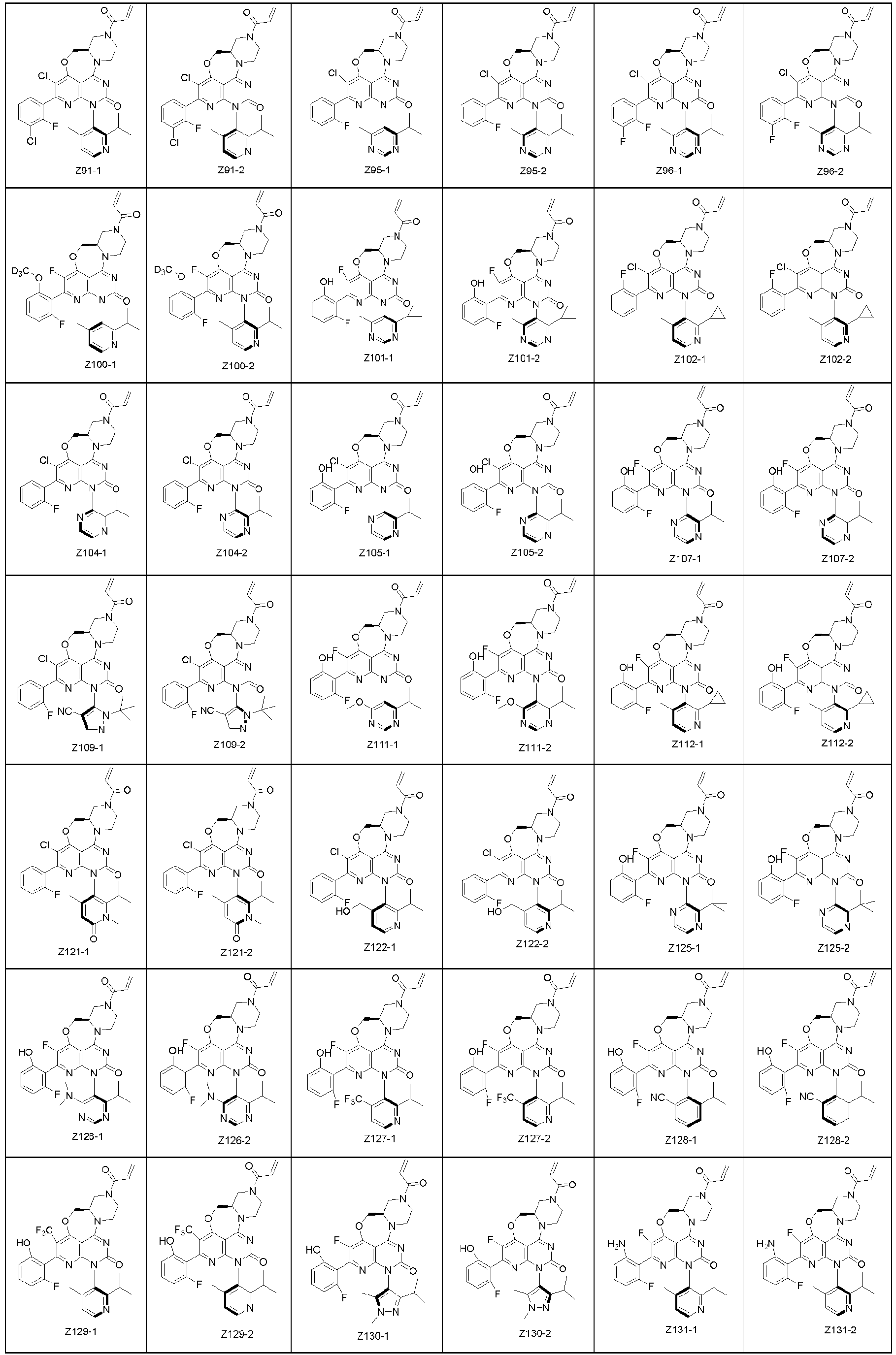

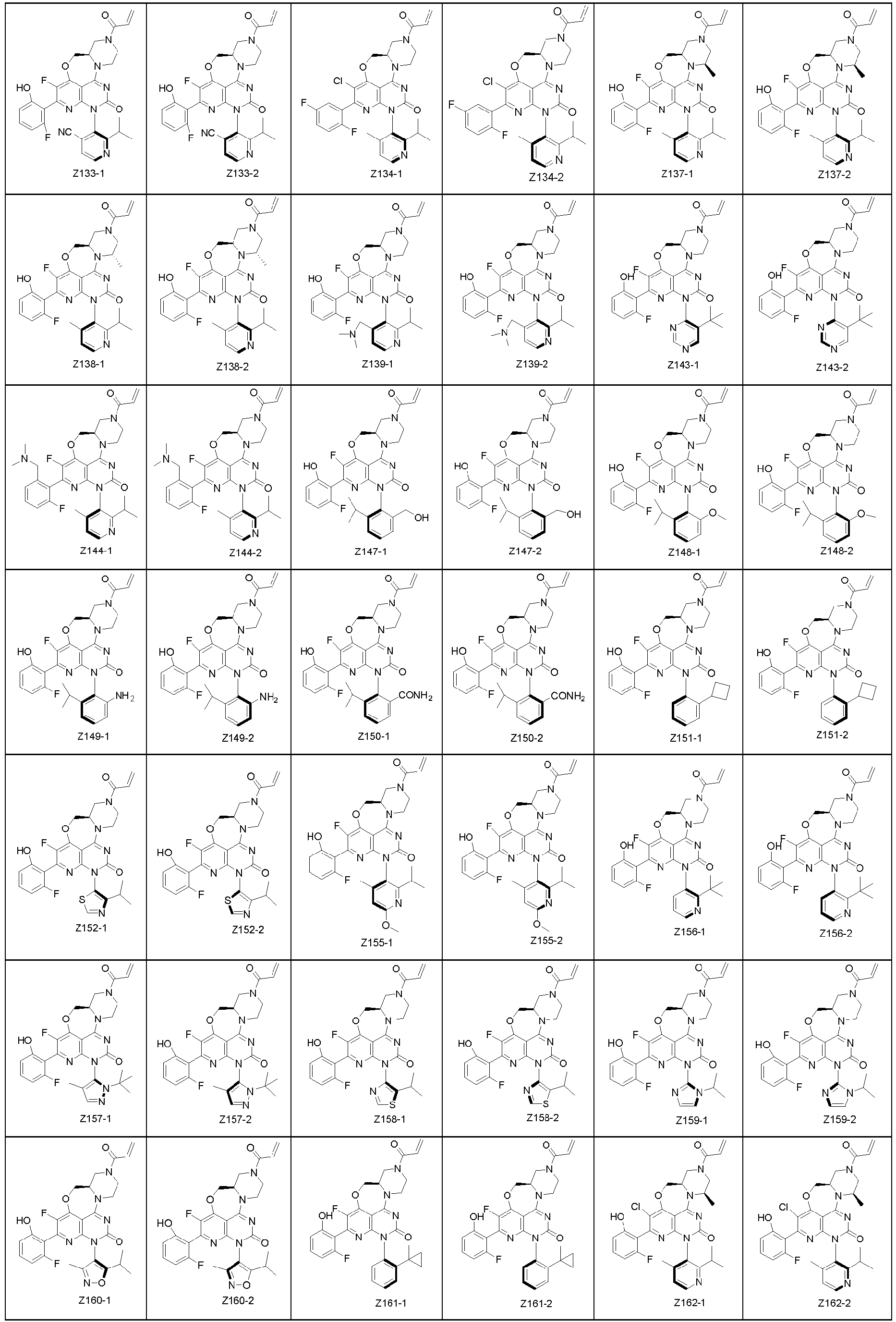

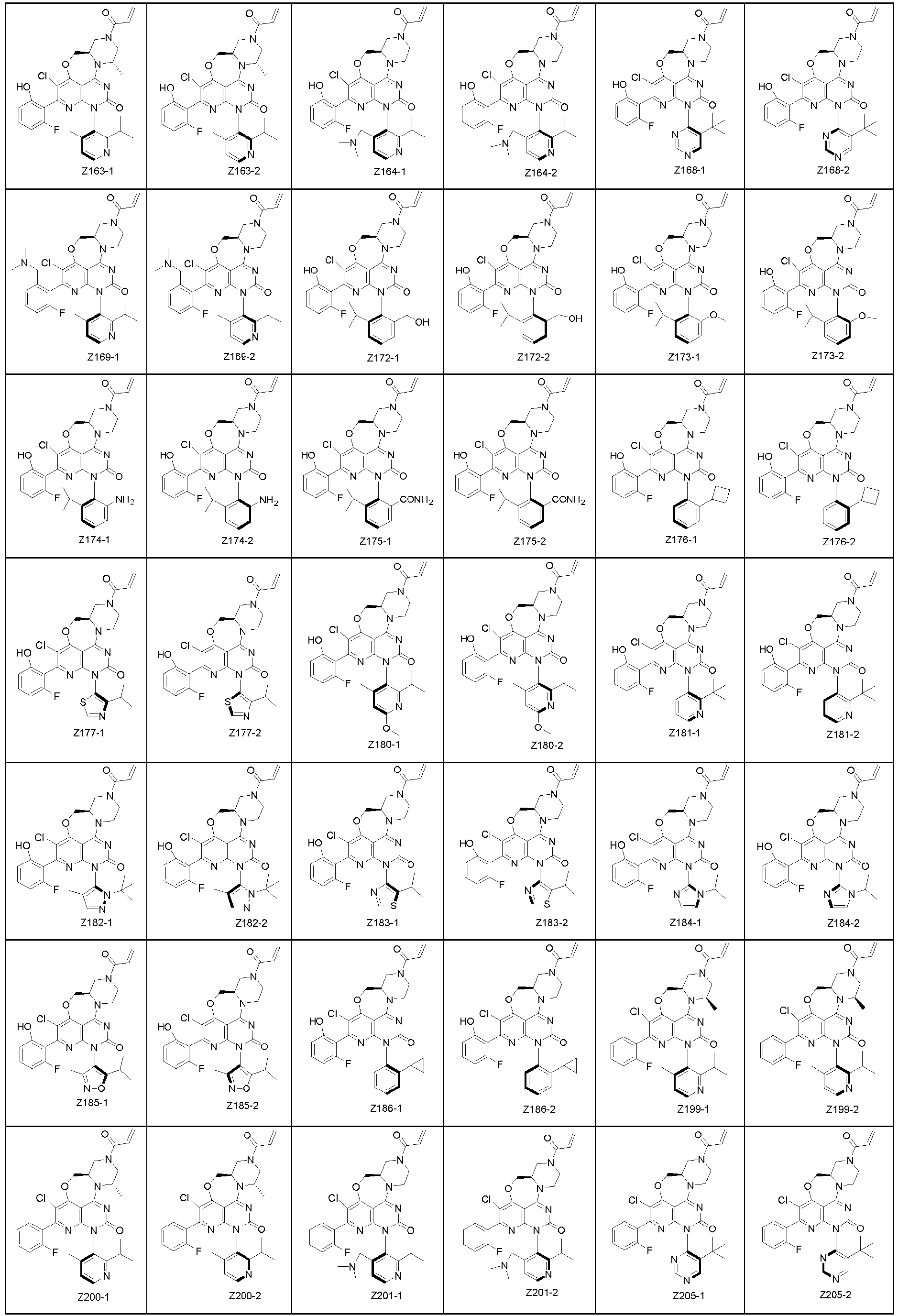

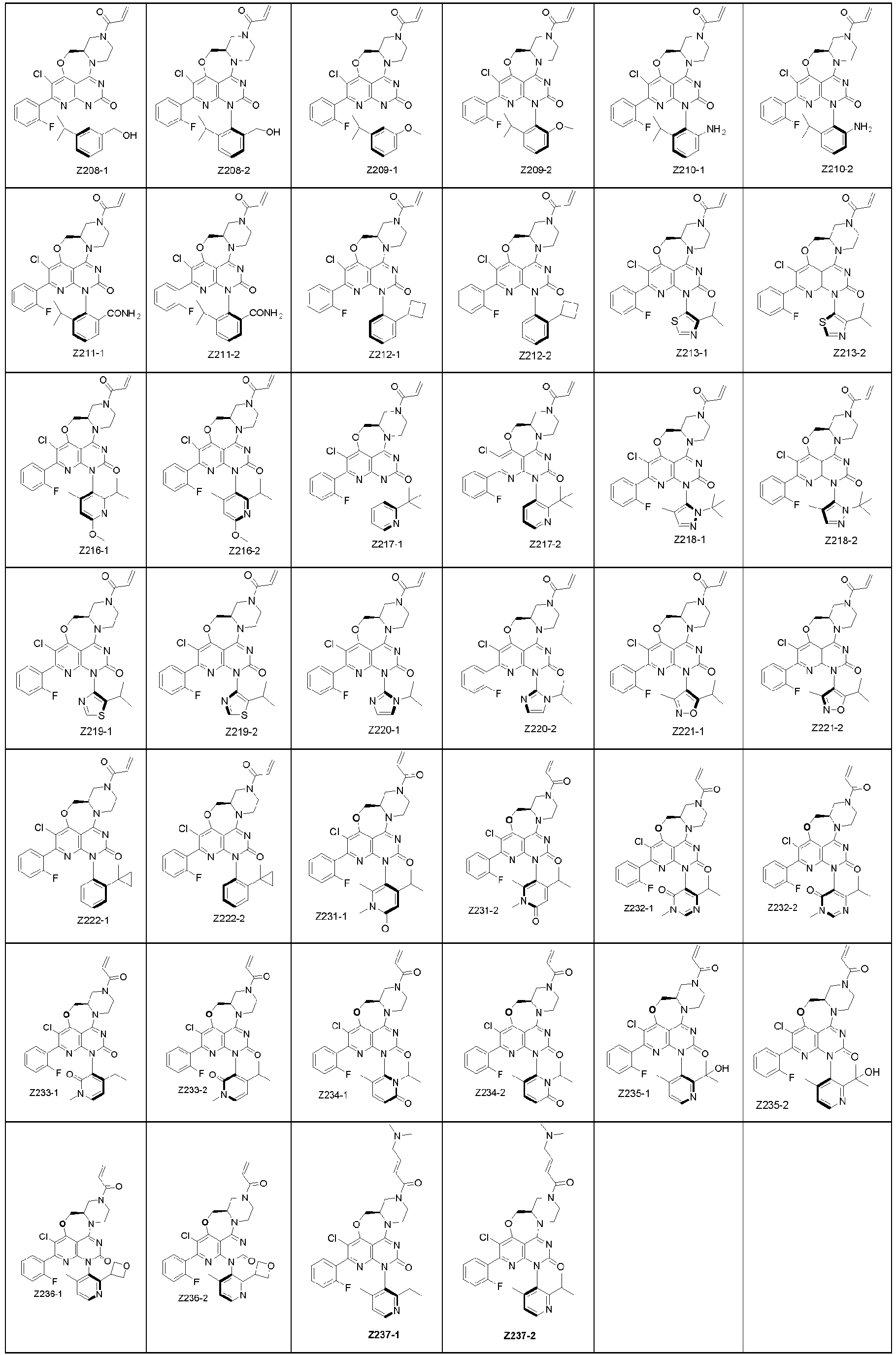

In another respect, the present invention provides a pharmaceutical composition, comprising the compound as decribed above or a pharmaceutically acceptable salt, stereoisomer, solvate or prodrug thereof; and a pharmaceutically acceptable carrier.

As used herein, the term "pharmaceutically acceptable carrier" means any formulation or carrier medium capable of delivering an effective amount of the active substance of the invention without interfering with the biological activity of the active substance and without causing adverse effects to the host or subject. It is a non-toxic, inert, solid, semi-solid substance or liquid filling agent, diluent, packaging material or auxiliary preparation or any type of excipient. Representative carriers include water, oil, vegetables and minerals, cream base, lotion base, ointment base and the like. These bases include suspension agents, viscosifiers, transdermal promoters and the like. Their formulations are known to those skilled in the field of cosmetic or topical medicine.

In an embodiment of the present invention, the pharmaceutical compoisiton may be administered in any form of oral, spray inhalation, rectal administration, nasal administration, buccal administration, topical administration, parenteral administration, such as, subcutaneous, intravenous, intramuscular, intraperitoneal, intrathecal, intraventricular, intrasternal and intracranial injection or infusion, or administered by means of an explanted reservoir. When administered orally, the compound of the present invention may be formulated into any orally acceptable dosage form, including but not limited to tablets, capsules, aqueous solutions or aqueous suspensions. Carriers used in tablets typically include lactose and cornstarch. Lubricants such as magnesium stearate may also be added. Diluents used in capsules typically include lactose and dried cornstarch. Aqueous suspensions are typically formulated by mixing an active ingredient with appropriate emulsifiers and suspension agents. Sweeteners, fragrances or colorants may be added to the oral dosage form as required. When topically administered, especially to the affected surface or organ readily accessible by topical application, such as eye, skin, or lower intestinal neuropathy, the compound of the present invention may be formulated into different topical dosage forms depending on the surface or organs. When topically administered to eyes, the compound of the present invention may be formulated into a dosage form of micronized suspension or solution using an isotonic sterile saline of a certain pH as the carrier, in which preservatives such as benzyl alkoxide chloride may or may not be added. For ocular administration, the compound may be formulated into a form of cream, such as, Vaseline cream. When administered topically to skin, the compound of the present invention may be formulated into a suitable dosage form of ointment, lotion or cream, in which an active ingredient is suspended or dissolved in one or more carriers. The carriers useful in an ointment formulation include but not limited to: mineral oils, liquid vaseline, white vaseline, propylene glycol, polyoxyethylene, polypropylene oxide, emulsified wax and water. The carriers useful in a lotion or cream include but not limited to: mineral oils, sorbitan monostearate, Tween 60, Cetyl ester wax, hexadecenyl aryl alcohol, 2-octyldodecanol, benzyl alcohol and water. The compound of the present invention may be administered in a dosage form of sterile injections, including sterial aqueous injection or oil suspension or sterile injection solution. Useful carriers and solvents include water, Ringer's solution and isotonic sodium chloride solution. Further, sterilized non-volatile oils can also be used as solvents or suspension media, such as monotriglycerides or diglycerides.

In another respect, the present invention provides use of the above oxaazaquinazolin-7(8H)-one compound, or a pharmaceutically acceptable salt, stereoisomer, solvate or prodrug thereof in the preparation of a medicament for preventing and/or treating cancer.

In one embodiment of the present invention, the cancer is pancreatic ductal cancer, colorectal cancer, multiple myeloma, lung cancer, skin melanoma, endometrioid carcinoma, uterine carcinosarcoma, thyroid cancer, acute myeloid leukemia, bladder urothelial cancer, stomach cancer, cervical cancer, head and neck squamous cell carcinoma, diffuse large B-cell lymphoma, esophageal cancer, chronic lymphocytic leukemia, lung squamous cell carcinoma, small cell lung cancer, renal papillary cell carcinoma, adenoid cystic carcinoma, chromophobe renal cell carcinoma, liver cancer, breast invasive carcinoma, cervical squamous cell carcinoma, ovarian serous adenocarcinoma, adrenal cortex carcinoma, prostate cancer, neuroblastoma, brain low-grade glioma, glue Plasmoblastoma, medulloblastoma, esophageal squamous cell carcinoma, renal clear cell carcinoma, osteosarcoma, ovarian small cell carcinoma, rhabdoid tumor, sarcoma, small intestinal neuroendocrine tumor, T-cell prolymphocytic leukemia.

In one embodiment of the present invention, the cancer is lung cancer, preferably non-small cell lung cancer.

In another respect, the present invention provides use of the above oxaazaquinazolin-7(8H)-one compound, or a pharmaceutically acceptable salt, stereoisomer, solvate or prodrug thereof in the preparation of an inhibitor of KRAS mutation (preferably, the KRAS mutation is KRAS G12C mutation).

In another respect, the present invention provides a method for treating cancer, comprising the step of administering to a patient in need thereof a therapeutically effective amount of compound, or a pharmaceutically acceptable salt, stereoisomer, solvate or prodrug thereof, or any combination thereof, or the above pharmaceutical compostition.

As used herein, the term "subject" refers to an aminal, especially a mammal, preferally a human being.

As used herein, the term "effective amount" or "therapeutically effective amount" refers to the sufficient amount of a drug or agent that is non-toxic but has the desired effect. In an embodiment of the present invention, when treating a patient in accordance with the present invention, the amount of a given drug depends on a number of factors, such as the particular dosage regimen, the type of disease or disorder and its severity, and the uniqueness of the subject or the host in need of treatment (e.g., body weight), however, depending on the particular circumstances, including, for example, the particular drug that has been employed, the route of administration, the condition being treated, and the subject or host being treated, the dosage administered can be decided by methods routinely known in the art. Generally, for use in the treatment for an adult, the dosage administered will typically range from 0.02 to 5000 mg/day, for example from about 1 to 1500 mg/day. The desired dose may conveniently be presented as a single dose, or concurrently (or in a short period of time) or in divided doses at appropriate intervals, such as two, three, four or more divided doses per day. It will be understood by those skilled in the art that although the above dosage ranges are given, the specific effective amount can be appropriately adjusted depending on the condition of the patient and in connection with the diagnosis of the physician.

As used therein, the term "pharmaceutically acceptable salts" refers to salts of the the compound of the present invention which are pharmaceutically acceptable, and can retain the biological effectiveness of the free base without other side effects. The type of pharmaceutical acceptable salts includes: acid addition salts formed with inorganic acids (such as hydrochloric acid, hydrobromic acid, sulfuric acid, nitric acid, phosphoric acid, and the like) or organic acids (such as acetic acid, propionic acid, hexanoic acid, cyclopentanepropionic acid, glycolic acid, pyruvic acid, trifluoroacetic acid, formic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, benzenesulfonic acid, naphthalene sulfonic acid, camphor sulfonic acid, gluconic acid, glutamic acid, hydroxynaphthalamic acid, salicylic acid, stearic acid, muconic acid and the like); or salts formed when an acidic proton present in the parent compound either is replaced by a metal ion such as an alkali metal ion or alkaline earth ion, such as sodium salt, potassium salt, calcium salt and magnesium salt, and the like; or coordinates with an organic base such as ethanolamine, diethanolamine, triethanolamine, N-methylglucamine, and the like. The pharmaceutically acceptable salts of the present invention can be synthesized from the parent compound containing acid radicals or base radicals by conventional chemical methods. In general, such salts are prepared by the reaction of these compounds in a form of free acid or base with a stoichiometric amount of the appropriate base or acid in water or an organic solvent or a mixture of the both. In general, non-aqueous media such as ether, ethyl acetate, ethanol, isopropanol or acetonitrile are preferred. In addition to salt forms, the compounds provided herein also exist in prodrug forms. The prodrugs of the compounds described herein are readily chemically altered under physiological conditions to be converted into the compounds of the invention. In addition, prodrugs can be converted to the compounds of the present invention by chemical or biochemical methods in an *in vivo* environment.

As used herein, the term "solvate" refers to a substance formed by combining the compound of the invention with a pharmaceutically acceptable solvent. Pharmaceutical acceptable solvates include water, ethanol, acetic acid and the like. The solvates include stoichiometric solvates and non-stoichiometric solvates, preferably hydrates. Certain compounds of the present invention may be present in unsolvated or solvated forms, including hydrated forms. In general, solvated forms are equivalent to unsolvated forms and both are included within the scope of the present invention.

As used herein, the compound as represented by formula (I) or formula (II) of the present invention may contain one or more chiral centers and exist in different optically active forms. When the compound contains one chiral center, the compound contain enantiomers. The present invention includes these two isomers and mixtures of isomers, such as racemic mixtures. Enantiomers can be resolved by methods known in the art, such as crystallization and chiral chromatography and other methods. When the compound of formula (I) or formula (II) contains more than one chiral center, diastereomers may exist. The present invention includes resolved optically pure specific isomers and mixtures of diastereomers. Diastereomers can be resolved by methods known in the art, such as crystallization and preparative chromatography. As used herein, the term "stereoisomers" include both conformational and configurational isomers, wherein configurational isomers mainly include cis-trans isomers and optical isomers. The compound of the present invention may be present in a stereoisomeric form, and thereby cover all possible stereoisomeric forms, including but not limited to cis-trans isomers, tautomers, enantiomers, diastereomers, atropisomers, and the like. The compound of the present invention can also be present in forms such as any combination or any mixture of the aforementioned stereoisomers, such as a mixture of mesoisomer, racemate, atropisomer in equal amounts, and the like. For example, a single enantiomer, a single non-enantiomer, or a mixture thereof, or a single atropisomer, or a mixture thereof. Where the compound of the invention contains an olefinic double bond, it includes a cis-isomer and trans-isomer, and any combination thereof, unless otherwise specified. The atropisomers of the present invention are stereoisomers with axial or planar chirality based on the restriction of intramolecular rotation. The compound of the present invention have two atropisomers derived from axial asymmetry, which is produced by restricting the rotation of bond when the substituents R_{b} or R_{c}, R_{b}' or R_{c}' are cyclic groups such as C₆₋₁₀ aryl, a 5- or 6-membered monocycloheteroaryl, a 8- to 10-membered bicyclo heteroaryl or pyridonyl (especially when the adjacent positions of two ends of the bond have substituents at the ortho positions) connected to the rings such as substituted naphthalidone, and forming a steric hindrance. Regarding the atropisomer of the present invention, wherein the compound has a structure of formula (I) or formula (II), or the compound of formula (I) or formula (II) has an isomer produced by asymmetric carbon, and the like, it represents any one of a pair of atropisomers present in each isomeric compound. And as a medicine, an atropisomer with excellent activity is preferred. The compound of formula (I) or formula (II) has optical isomers derived from asymmetric carbon, axial asymmetry, and the like, if necessary, a single isomer can be obtained by methods known in the art, such as crystallization or chromatography (e.g., chiral chromatography) and other methods. The atropisomers of the compound of the present invention can be expressed in P or M configuration, and can also be labeled in other commonly used ways known in the art.

As mentioned above, the present invention provides compounds shown in the above-mentioned various structures, or tautomers, cis-trans isomers, mesoisomers, racemates, enantiomers, diastereomers, atropisomers thereof, or the form of a mixture thereof, wherein "the form of a mixture thereof" includes any of the aforementioned stereoisomers (e.g., tautomers, cis-trans isomers, enantiomers, diastereomers, atropisomers) and/or mixtures (mesoisomers, racemates) in any form, such as mixtures of cis-trans isomers, mixtures of enantiomers and diastereomers, mixtures of diastereomers, mixtures of atropisomers, or mixtures of cis-trans isomers and racemates, mixtures of enantiomers and diastereomers, mixtures of atropisomers and diastereomers mixtures, and the like.

As used herein, the term "alkyl" refers to a liner or branched aliphatic hydrocarbon group having 1 to 20 carbon atoms. The term "C₁₋₁₀ alkyl" refers to a liner or branched alkyl group having 1 to 10 carbon atoms, more preferably 1, 2, 3, 4, 5 or 6 carbon atoms, i.e., C₁₋₆ alkyl, more preferably, C₁₋₄ alkyl, the most preferably, C₁₋₃ alkyl. Specific examples include, but are not limited to, methyl, ethyl, *n*-propyl, *iso*-propyl, *n*-butyl, *iso*-butyl, tert-butyl, sec-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and various branched isomers thereof.

As used herein, the term "alkoxy" refers to a group having a structure of -O-alkyl, wherein the alkyl is as defined above. The term "C₁₋₁₀ alkoxy" refers to an alkoxy group having 1 to 10 carbon atoms, preferably, C₁₋₆ alkoxy, more preferably, C₁₋₄ alkoxy, the most preferably, C₁₋₃ alkoxy. Specific examples include, but are not limited to, methoxy, ethoxy, *n*-propoxy, iso-propoxy, *n*-butoxy, *tert*-butoxy, *sec*-butoxy, *n*-pentoxy and the like.

As used herein, the term "alkenyl" refers to an alkyl as defined above having one or more carbon-carbon double bond at any position of the chain. The term "C₂₋₈ alkenyl" refers to an alkenyl group having 2 to 8 carbon atoms and at least one carbon-carbon double bond, prefereably, an alkenyl group having 2 to 6 carbon atoms and 1 to 2 carbon-carbon double bond, i.e., C₂₋₆ alkenyl, more preferably, an alkenyl group having 2 to 4 carbon atoms and 1 to 2 carbon-carbon double bond, i.e., C₂₋₄ alkenyl. Specific examples include, but are not limited to, vinyl, 1-propenyl, 2-propenyl, 1-, 2- or 3-butenyl, pentenyl, hexenyl, butadienyl, and the like.

As used herein, the term "alkynyl" refers to an alkyl as defined above having one or more carbon-carbon triple bond at any position of the chain. The term "C₂₋₈ alkynyl" refers to an alkynyl group having 2 to 8 carbon atoms and at least one carbon-carbon triple bond, prefereably, an alkynyl group having 2 to 6 carbon atoms and 1 to 2 carbon-carbon triple bond, i.e., C₂₋₆ alkynyl, more preferably, an alkynyl group having 2 to 4 carbon atoms and 1 to 2 carbon-carbon triple bond, i.e., C₂₋₄ alkynyl. Specific examples include, but are not limited to, ethynyl, 1-propynyl, 2-propynyl, 1-, 2- or 3-butynyl, and the like.

As used herein, the term "halogen" refers to fluoro, chloro, bromo and iodine.

As used herein, the term "haloalkyl" refers to an alkyl as defined above which is substituted by one or more (1, 2, 3, 4 or 5) halogens. The term "halo C₁₋₁₀ alkyl" refers to a haloalkyl having 1 to 10 carbon atoms, preferably, halo C₁₋₆ alkyl, more preferably, halo C₁₋₄ alkyl, most preferably, halo C₁₋₃ alkyl. Specific examples include, but are not limited to, chloromethyl, dichloromethyl, trichloromethyl, chloroethyl, 1,2-dichloroethyl, trichloroethyl, bromoethyl, fluoromethyl, difluoromethyl, trifluoromethyl, fluoroethyl, difluoroethyl, trifluoroethyl, and the like.

As used herein, the term "haloalkoxy" refers to an alkoxy as defined above which is substituted by one or more (1, 2, 3, 4 or 5) halogens. The term "halo C₁₋₁₀ alkoxy" refers to a haloalkoxy having 1 to 10 carbon atoms, preferably, halo C₁₋₆ alkoxy, more preferably, halo C₁₋₄ alkoxy, most preferably, halo C₁₋₃ alkoxy. Specific examples include, but are not limited to, trifluoromethoxy, trifluoroethoxy, fluoromethoxy, fluoroethoxy, difluoromethoxy, difluoroethoxy, and the like.

As used herein, the term "deuterated alkyl" refers to an alkyl group substituted with one or more (e.g., 1, 2, 3, 4, or 5) deuterium atoms, wherein the definition of the alkyl group is as described above. The term "deuterated C₁₋₁₀ alkyl" refers to a deuterated alkyl having 1 to 10 carbon atoms. It is preferably a deuterated C₁₋₆ alkyl, more preferably a deuterated C₁₋₄ alkyl, and more preferably a deuterated C₁₋₃ alkyl. Specific examples include, but are not limited to, mono-deuterated methyl, di-deuterated methyl, tri-deuterated methyl, mono-deuterated ethyl, 1,2-di-deuterated ethyl, tri-deuterated ethyl, and the like.

As used herein, the term "deuterated alkoxy" refers to an alkoxy group substituted with one or more (e.g., 1, 2, 3, 4, or 5) deuterium atoms, wherein the alkoxy group is as defined above. The term "deuterated C₁₋₁₀ alkoxy" refers to a deuterated alkoxy group having 1 to 10 carbon atoms. It is preferably a deuterated C₁₋₆ alkoxy, more preferably a deuterated C₁₋₄ alkoxy, and more preferably a deuterated C₁₋₃ alkoxy. Specific examples include, but are not limited to, tri-deuterated methoxy, tri-deuterated ethoxy, mono-deuterated methoxy, mono-deuterated ethoxy, di-deuterated methoxy, di-deuterated ethoxy, and the like.

As used herein, the terms "cycloalkyl" and "cycloalkyl ring" refer to saturated or partially unsaturated monocyclic or polycyclic cyclic hydrocarbyl substituents. The cycloalkyl ring contains 3 to 20 carbon atoms (C₃₋₂₀), preferably contains 3 to 12 carbon atoms (C₃₋₁₂), more preferably contains 3 to 10 carbon atoms (C₃₋₁₀), most preferably contains 3 to 6 carbon atoms (C₃₋₆). A ring carbon atom in a cycloalkyl may be optionally substituted by 1, 2 or 3 oxo group(s) to form a structure of cyclic ketone. When it is a monocyclic cycloalkyl, the monocyclic cycloalkyl is saturated or partially unsaturated, preferably a monocyclic cycloalkane containing 3 to 8 ring carbon atoms (i.e., 3- to 8- membered or C₃₋₈), more preferably containing 3 to 6 ring carbon atoms. Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, cyclobutanone, cyclobutan-1,2-dione, cyclopentanone, cyclopentan-1,3-dione, cyclohexanone, cyclohexan-1,3-dione, and the like; the polycyclic cycloalkyl includes spirocycloalkyl, fused cycloalkyl, and bridged cycloalkyl.

The term "spirocycloalkyl" refers to a polycyclic group containing 5 to 20 ring carbon atoms (i.e., 5- to 20-membered or C₅₋₂₀), wherein 3- to 8-membered (i.e., containing 3 to 8 ring carbon atoms or C₃₋₈) single rings share one carbon atom (referred to as spiro atom). Each single ring can contain one or more double bonds, but none of the rings has a fully conjugated π-electron system. It is preferably a 6- to 14-membered (i.e., containing 6 to 14 ring carbon atoms or C₆₋₁₄) spirocycloalkyl, and more preferably a 7- to 11-membered spirocycloalkyl. Depending on the number of the spiro atoms shared between the rings, the spirocycloalkyls are divided into monospirocycloalkyl, bispirocycloalkyl, or polyspirocycloalkyl, preferably a monospirocycloalkyl and a bispirocycloalkyl, more preferably a 7-membered (4-membered monocyclic/4-membered monocyclic), 8-membered (4-membered monocyclic/5-membered monocyclic), 9-membered (4-membered monocyclic/6-membered monocyclic, 5-membered monocyclic/5-membered monocyclic), 10-membered (5-membered monocyclic/6-membered monocyclic) or 11-membered (6-membered monocyclic/6-membered monocyclic) monospirocycloalkyl. Non-limiting examples of spirocycloalkyl include:

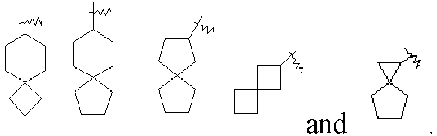

The cycloalkyl ring may be fused to an aryl ring, a heteroaryl ring or a heterocyclyl ring, wherein the ring attached to the parent structure is the cycloalkyl ring. Non-limiting examples include indanyl, tetralyl, benzocycloheptyl, and the like. In the present invention, each of the above types of cycloalkyl may be optionally substituted, where the substituent(s) are preferably one or more substituents as described in the present disclosure.

As used herein, the term "halocycloalkyl" refers to a cycloalkyl as defined above which is substituted by one or more (1, 2, 3, 4 or 5) halogens. The term "halo C₃₋₈ cycloalkyl" refers to a halocycloalkyl having 3 to 8 carbon atoms, preferably, halo C₃₋₆ cycloalkyl, more preferably, halo C₃, halo C₄, halo C₅, or halo C₆ cycloalkyl. Specific examples include, but are not limited to, trifluorocyclopropyl, fluorocyclopropyl, fluorocyclohexyl, difluorocyclopropyl, difluorocyclohexyl, and the like.

As used herein, the term "heterocyclyl" and "heterocyclyl ring" are used exchangeably to refer to a saturated or partially unsaturated monocyclic or polycyclic cyclic hydrocarbyl, containing 3 to 20 ring atoms (i.e., 3- to 20-membered or C₃₋₂₀), wherein one or more (preferably 1 to 4) ring atoms are heteroatoms selected from the group consisting of nitrogen, oxygen or S(O)ₘ, (wherein m is an integer from 0 to 2), but not contain a cyclic moity of -O-O-, -O-S- or -S-S-, and the remaining ring atoms are each carbon. The nitrogen atom may be substituted or unsubstituted (i.e., N or NR, R is hydrogen, or other substituents as defined herein). The ring carbon atoms of the heterocyclyl may be optionally substituted by 1, 2 or 3 oxo group(s) to form a structure of cyclic ketone, cyclic lactone or cyclic lactam. It preferably contains 3 to 12 ring atoms, more preferably 3 to 10 ring atoms, wherein 1 to 4 are heteroatoms.

In certain embodiments of the present invention, "heterocyclyl" refers to a monocyclic heterocyclyl, which is saturated or partially unsaturated, and preferably a monocyclic heterocyclyl containing 3 to 8 ring atoms (i.e., 3- to 8-membered or C₃₋₈), wherein 1 to 3 are heteroatoms, more preferably a monocyclic heterocyclyl group containing 3 to 6 ring atoms, wherein 1 to 2 are heteroatoms, the most preferably a monocyclic heterocyclyl group containing 5 to 6 ring atoms, wherein 1 to 2 are heteroatoms. If the heteroatom is a nitrogen atom, the nitrogen atom may be substituted or unsubstituted (i.e., N or NR, R is hydrogen or other substituents as defined herein). If the heteroatom is a sulfur atom, the sufur atom may be optionally oxidized (i.e., S(O)ₘ, m is an integer from 0 to 2). The ring carbon atom in the monocyclic heterocyclyl may be optionally substituted by 1, 2 or 3 oxo group(s) to form a structure of cyclic ketone, cyclic lactone or cyclic lactam. Specific examples of the monocyclic heterocyclyl includes, but are not limited to, aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, and the like.

The adjacent two ring atoms in the above monocyclic heterocyclyl, including C-C, N-C, may be optionally fused to the cylcoalkyl, heterocyclyl, aryl or heteroaryl as defined herein, such as, monocyclic cycloalkyl ring, monocyclic heterocyclyl ring, monoaryl ring, 5- or 6-membered monoheteroaryl ring and the like, to form a fused polycyclyl. The adjacent two ring atoms in the above monocyclic heterocyclyl fused to another ring is preferably C-C.

In the present invention, each of the above types of heterocyclyl may be optionally substituted. If substituted, the substituent(s) are preferably one or more substituents as described in the present disclosure.

As used herein, the term "aryl" refers to an all-carbon monocyclyl, all-carbon polycyclyl (a ring is linked to another by a covalent bond, non-fused) or all-carbon fused polycyclyl (i.e., a pair of adjacent carbon atoms are shared between the ring) groups containing 6 to 14 ring atoms (i.e., 6- to 14-membered or C₆₋₁₄), and at least one ring in the ring system is aromatic, that is, has a π electron conjugated system. It is preferably an aryl containing 6 to 10 ring atoms (i.e., 6- to 10-membered or C₆₋₁₀). Each ring in the ring system contains 5 or 6 ring atoms. The terms "aryl" and "aryl ring" are used interchangeably.

In certain embodiments of the present invention, "aryl" refers to a monoaryl or polyaryl ring, and non-limiting examples thereof include: phenyl, biphenyl, and the like.

In certain embodiments of the present invention, "aryl" refers to an aromatic fused polycyclyl, the aromatic fused polycyclyl maybe a polycyclyl group formed by fusing a monoaryl ring to one or more monoaryl rings. Non-limiting examples include naphthyl, anthryl, and the like.

In certain embodiments of the present invention, "aryl" refers to an aromatic fused polycyclyl (preferably a 9- or 10-membered aromatic fused polycyclyl), the aromatic fused polycyclyl may be a polycyclyl group formed by fusing a monoaryl ring (preferably phenyl) to one or more non-aromatic rings, wherein the ring attached to the parent structure is an aromatic or non-aromatic ring. The non-aromatic ring includes, but is not limited to, a 3-to 6-membered monocyclic heterocyclyl ring, preferably a 5- or 6-membered monocyclic heterocyclyl ring (the ring carbon atom in the monocyclic heterocyclyl may be substituted by 1 or 2 oxo group(s) to form a structure of cyclic lactam or cyclic lactone), a 3- to 6-membered monocyclic cycloalkyl ring, preferably a 5- or 6-membered monocyclic cycloalkyl ring (the ring carbon atom in the monocyclic cycloalkyl may be substituted by 1 or 2 oxo goup(s) to form a structure of cyclic ketone), and the like.

The above polycyclyl group formed by fusing a monoaryl ring to one or more non-aromatic rings may be linked to other moiety or the parent structure through a nitrogen atom or carbon atom. The ring attached together to the parent structure is an aromatic or non-aromatic ring, and non-limiting examples include:

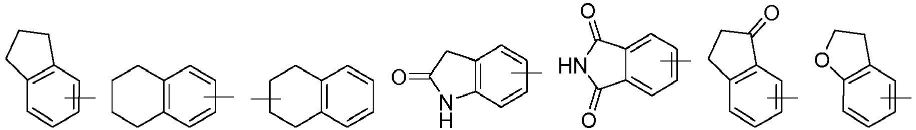

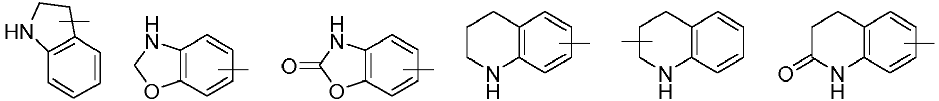

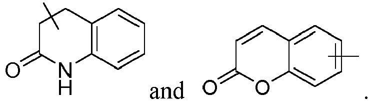

In the present invention, each of the above types of aryl may be substituted or unsubstituted. If substituted, the substituent(s) are preferably one or more groups as described in the present disclosure.

As used herein, the term "heteroaryl" refers to a monocyclic or fused polycyclic (that is, rings sharing adjacent carbon atoms or pairs of heteroatoms) groups containing 1 to 4 heteroatoms, having 5 to 14 ring atoms (i.e., 5- to 14-membered or C₅₋₁₄), preferably 5 to 10 ring atoms (i.e., 5- to 10-membered or C₅₋₁₀), more preferably 5, 6, 8, 9 or 10 ring atoms, wherein the heteroatoms are selected from the group consisting of oxygen, sulfur and nitrogen, wherein the nitrogen and sulfur atoms may be optionally oxidized, and the nitrogen atom may be optionally quaternized. The heteroaryl in the ring system has 6, 10 or 14 π electrons as shared. At least one ring in the ring system is aromatic. The terms "heteroaryl" and "heteroaryl ring" are used interchangeably.

In certain embodiments of the present invention, "heteroaryl" refers to a monoheteroaryl ring (preferably a 5- or 6-membered monoheteroaryl ring), and non-limiting examples of the monoheteroaryl include: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, and the like.

In certain embodiments of the present invention, "heteroaryl" refers to a fused polyheteroaryl ring (preferably a 8- to 10-membered biheteroaryl ring). The fused polyheteroaryl ring either inclues a polycyclic group (preferably a 9- or 10-membered biheteroaryl ring) formed by fusing a monoaryl ring (preferably, phenyl) and a monoheteroaryl ring (preferably a 5- or 6-membered monoheteroaryl ring), or include a polycyclic group (preferably a 8- to 10-membered biheteroaryl ring) formed by fusing a monoheteroaryl (preferably a 5- or 6-membered monoheteroaryl) and a monoheteroaryl (preferably a 5- or 6-membered monoheteroaryl).

Any 2 adjacent ring atoms in the above monoheteroaryl ring, including C-C, N-C, N-N, may be fused to the cycloalkyl, heterocyclyl, aryl or heteroaryl such as the monocyclic cycloalkyl ring, monocyclic heterocyclyl ring, monoaryl ring, 5- or 6-membered monoheteroaryl ring and the like as define in the present disclosure, to form a fused polycyclyl. The 2 adjacent ring atoms in the monoheteroaryl ring, which are fused to another ring to form a fused ring, are preferably C-C, and include in a non-limiting way the forms of:

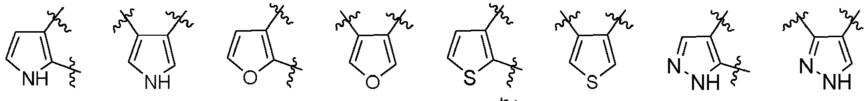

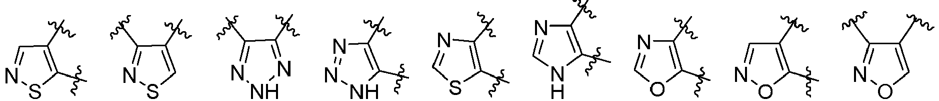

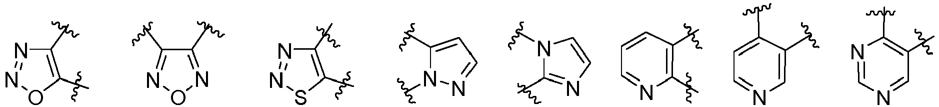

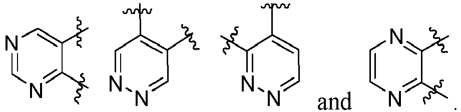

Non-limiting examples of the fused polyheteroaryl ring include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, and the like.

The above monoheteroaryl, or polycyclyl group formed by fusing a monoaryl ring and a monoheteroaryl ring, or polycyclyl group formed by fusing a monoheteroaryl and a monoheteroaryl may be linked to other moiety or the parent structure through a nitrogen atom or carbon atom. If it is a polycyclyl group, the ring attached together to the parent structure is a heteroaryl ring, an aryl ring, a monocyclic cycloalkyl ring or a monocyclic heterocyclyl ring, and non-limiting examples thereof include:

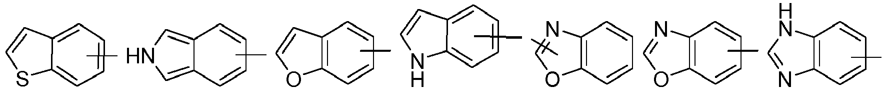

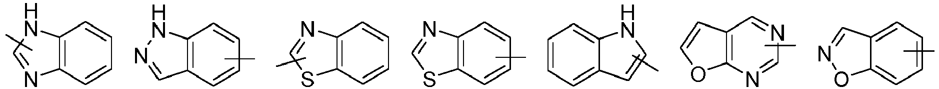

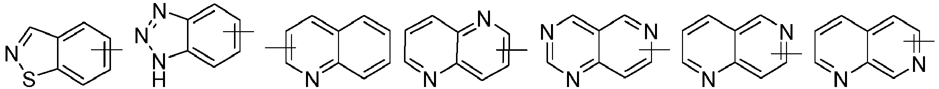

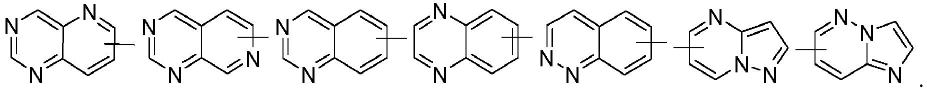

In certain embodiments of the present invention, "heteroaryl" refers to a fused polyheteroaryl ring (preferably a 8- to 10-membered biheteroaryl ring). The fused polyheteroaryl ring is a polycyclyl group formed by fusing a monoheteroaryl ring (preferably a 5- or 6-membered monoheteroaryl ring) and one or more non-aromatic ring(s), wherein the ring attached together to the parent structure is a heteroaryl ring or a non-aromatic ring, the non-aromatic ring includes, but not limited to: a 3- to 6-membered (preferably a 5- or 6-membered) monocyclic heterocyclyl ring (the ring carbon atoms in the monocyclic heterocyclyl ring may be substituted by 1 or 2 oxo group(s) to form a structure of cyclic lactam or cyclic lactone), a 3- to 6-membered (preferably a 5- or 6-membered) monocyclic cycloalkyl ring (the ring carbon atoms in the monocyclic cycloalkyl ring may be substituted by 1 or 2 oxo group(s) to form a structure of cyclic ketone), and the like.

The above polycyclyl group formed by fusing a monoheteroaryl ring and one or more non-aromatic ring(s) may be linked to other moiety or the parent structure through a nitrogen atom or carbon atom, the ring attached together to the parent structure is a heteroaryl ring or a non-aromatic ring, and non-limiting examples thereof include:

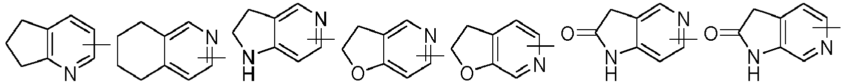

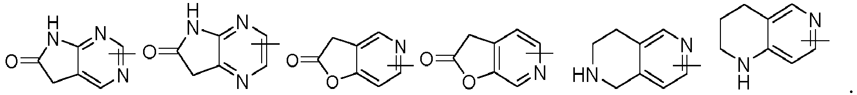

In the present invention, each of the above types of heteroaryl may be substituted or unsubstituted. When substituted, the substituent(s) are preferably one or more substituents as described in the present disclosure.

As used herein, the term "hydroxyl" refers to -OH.

As used herein, the term "hydroxylmethyl" refers to -CH₂OH, and "hydroxyethyl" refers to -CH₂CH₂OH or -CH(OH)CH₃.

As used herein, the term "cyanomethyl" refers to -CH₂CN, and "cyanoethyl" refers to -CH₂CH₂CN or -CHCNCH₃.

As used herein, the term "amino" refers to -NH₂.

As used herein, the term "cyano" refers to -CN.

As used herein, the term "nitro" refers to -NO₂.

As used herein, the term "benzyl" refers to -CH₂-phenyl.

As used herein, the term "oxo group" refers to =O.

As used herein, the term "carboxyl" refers to -C(O)OH.

As used herein, the term "carboxylic ester group" refers to -C(O)O(alkyl) or -C(O)O(cycloalkyl).

As used herein, the term "acetyl" refers to -COCH₃.

As used herein, the term "-C₁₋₄ alkyl-C₆₋₁₀ aryl" refers to C₁₋₄ alkyl is substituted by C₆₋₁₀ aryl, preferably substituted by one C₆₋₁₀ aryl, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-C₅₋₁₀ heteroaryl" refers to C₁₋₄ alkyl is substituted by C₅₋₁₀ heteroaryl, preferably substituted by one C₅₋₁₀ heteroaryl, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-C₃₋₆ monocyclic heterocyclyl" refers to C₁₋₄ alkyl is substituted by C₃₋₆ monocyclic heterocyclyl, preferably substituted by one C₃₋₆ monocyclic heterocyclyl, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-C₃₋₆ monocyclic cycloalkyl" refers to C₁₋₄ alkyl is substituted by C₃₋₆ monocyclic cycloalkyl, preferably substituted by one C₃₋₆ monocyclic cycloalkyl, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-hydroxy" refers to C₁₋₄ alkyl is substituted by hydroxy, preferably substituted by one hydroxy, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-, and the examples of -C₁₋₄ alkyl-hydroxy include hydroxymethyl, hydroxyethyl.

As used herein, the term "-C₁₋₄ alkyl-cyano" refers to C₁₋₄ alkyl is substituted by cyano, preferably substituted by one cyano, wherein -C₁₋₄ alkyl-represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-, and the examples of -C₁₋₄ alkyl-cyano include cyanomethyl, cyanoethyl.

As used herein, the term "-C₁₋₄ alkyl-C₁₋₆ alkoxy" refers to C₁₋₄ alkyl is substituted by C₁₋₆ alkoxy, preferably substituted by one C₁₋₆ alkoxy, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-halo C₁₋₆ alkyl" refers to C₁₋₄ alkyl is substituted by halo C₁₋₆ alkyl, preferably substituted by one halo C₁₋₆ alkyl, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-halo C₁₋₆ alkoxy" refers to C₁₋₄ alkyl is substituted by halo C₁₋₆ alkoxy, preferably substituted by one halo C₁₋₆ alkoxy, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-NR^{e}R^{f}" refers to C₁₋₄ alkyl is substituted by NR^{e}R^{f}, preferably substituted by one NR^{e}R^{f}, wherein -C₁₋₄ alkyl-represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-C(O)NR^{e}R^{f}" refers to C₁₋₄ alkyl is substituted by C(O)NR^{e}R^{f}, preferably substituted by one C(O)NR^{e}R^{f}, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-SO₂C₁₋₃ alkyl" refers to C₁₋₄ alkyl is substituted by SO₂C₁₋₃ alkyl, preferably substituted by one SO₂C₁₋₃ alkyl, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "-C₁₋₄ alkyl-C₂₋₄ alkynyl" refers to C₁₋₄ alkyl is substituted by C₂₋₄ alkynyl, preferably substituted by one C₂₋₄ alkynyl, wherein -C₁₋₄ alkyl- represents an alkylene or alkylidene radical formed by substitution, and is preferably -C₁₋₄ alkyl-, more preferably -C₁₋₃ alkyl-, more preferably -C₁₋₂ alkyl-, such as -CH₂-CH₂-, the most preferably -CH₂-.

As used herein, the term "substituted" means that any one or more hydrogen atoms on a particular atom are replaced with substituents, including deuterium and hydrogen variants, as long as the valence of a particular atom is normal and the substituted compound is stable. When the substituent is an oxo group (i.e., =O), it means that two hydrogen atoms are replaced. Replacement of an oxo group does not occur on aromatic groups. The term "optionally substituted" means that it may or may not be substituted. Unless otherwise specified, the type and number of substituents may be arbitrary on the basis of being chemically achievable.

When any variant (e.g., R) occurs more than once in the constitution or structure of a compound, its definition in each case is independent. Thus, for example, if a group is substituted by 0-2 R, the group may optionally be substituted with up to two R, and R in each case has an independent option. In addition, combinations of substituents and/or variants thereof are permissible only if such combinations result in stable compounds.

The compound represented by the formula (I) or formula (II) of the present invention may be prepared by using a synthetic method known in the art or using a method known in the art in combination with the method described in the present invention. The solvent, temperature and other reaction conditions provided by the present invention are exemplary and may be changed according to methods well known in the art. The compounds described in the Examples of the present invention may be synthesized by using appropriate starting materials according to the methods described in the Examples based on their specific structures. It may be synthesized using a method similar to that described in the Examples. The starting materials used to synthesize the compounds in the Examples of the present invention may be prepared by known synthetic methods or similar methods described in the literature or obtained from commercial sources. If necessary, the compounds in the Examples may be further resolved to obtain its stereoisomers by methods well known in the art, such as crystallization, chromatography, and the like, and the resolution conditions are easily obtained by those skilled in the art through conventional means or limited experiments.

As a further illustration, the compound of formula ((I-a) of the present invention may be synthesized by the following method, wherein the solvent, temperature and other reaction conditions in each step may be the same as or similar to those described in the following Examples, or the reaction conditions known in the art are used; 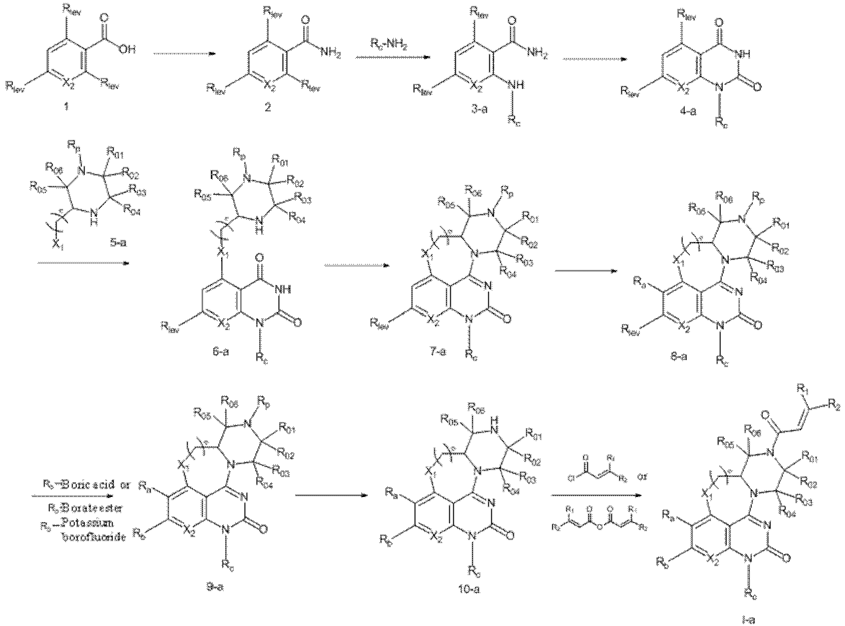 wherein, Rₗₑᵥ is a well known leaving group in the art, such as trifluoromethanesulfonate; chlorine, bromine, iodine; sulfonate group, such as mesylate, tosylate, p-toluenesulfonate, and the like; acyloxy group, such as acetoxy, trifluoroacetoxy, and the like. Rₚ is an amino protecting group well known in the art, such as formyl; acyl, such as alkanoyl (such as acetyl, trichloroacetyl or trifluoroacetyl); alkoxycarbonyl, such as tert-butoxycarbonyl (Boc); arylmethoxycarbonyl, such as benzyloxycarbonyl (Cbz) and 9-fluorenylmethyloxycarbonyl (Fmoc); arylmethyl, such as benzyl (Bn), trityl (Tr), 1,1-di-(4'-methoxyphenyl)methyl; silyl, such as trimethylsilyl (TMS) and tert-butyldimethylsilyl (TBS), and the like. R₁, R₂, R₀₁, R₀₂, R₀₃, R₀₄, R₀₅, R₀₆, n, X₁, X₂, Rₐ, R_{b}, R_{c} are as defined above.

As a further illustration, the compound of formula ((II-a) of the present invention may be synthesized by the following method, wherein the solvent, temperature and other reaction conditions in each step may be the same as or similar to those described in the following Examples, or the reaction conditions known in the art are used; 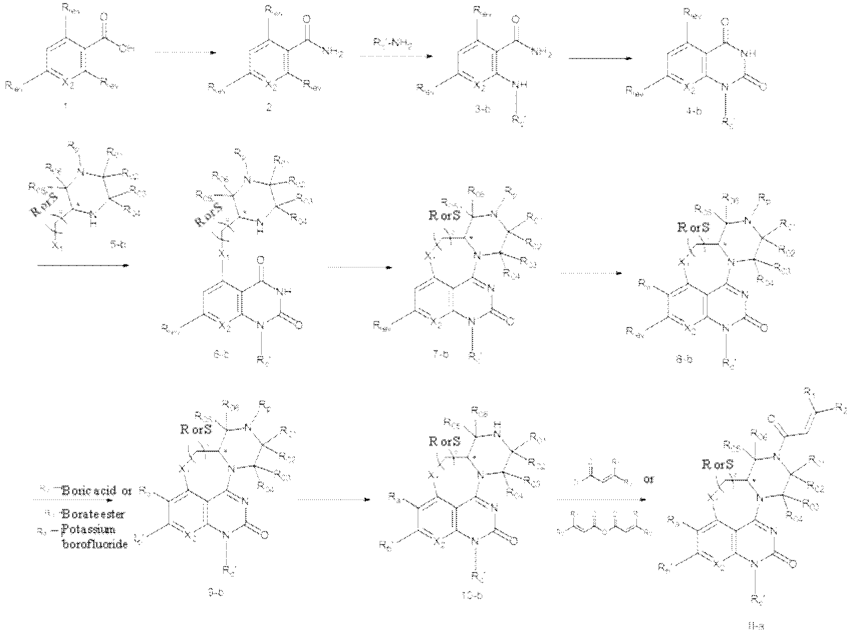 wherein, Rₗₑᵥ is a well known leaving group in the art, such as trifluoromethanesulfonate; chlorine, bromine, iodine; sulfonate group, such as mesylate, tosylate, p-toluenesulfonate, and the like; acyloxy group, such as acetoxy, trifluoroacetoxy, and the like. Rₚ is an amino protecting group well known in the art, such as formyl; acyl, such as alkanoyl (such as acetyl, trichloroacetyl or trifluoroacetyl); alkoxycarbonyl, such as tert-butoxycarbonyl (Boc); arylmethoxycarbonyl, such as benzyloxycarbonyl (Cbz) and 9-fluorenylmethyloxycarbonyl (Fmoc); arylmethyl, such as benzyl (Bn), trityl (Tr), 1,1-di-(4'-methoxyphenyl)methyl; silyl, such as trimethylsilyl (TMS) and tert-butyldimethylsilyl (TBS), and the like. R₁, R₂, R₀₁, R₀₂, R₀₃, R₀₄, R₀₅, R₀₆, n, X₁, X₂, Rₐ, R_{b}', R_{c}' are as defined above.

### Detailed Description of the Invention

The compound of the present invention can be prepared by a variety of synthetic methods well known to those skilled in the art, including the specific embodiments listed below, the embodiments obtained by combining those listed below with other chemical synthesis methods, and the equivalent alternatives well known to those skilled in the art. Preferred embodiments include, but are not limited to, the Examples of the present invention.

It should be understood that the absolute configuration of the stereoisomer separated in the following Examples is arbitrarily specified, and the separation method may be chromatographic separation, for example, including but not limited to column chromatography, thin layer chromatography, liquid chromatography, and the like.

The present invention is illustrated in details by means of the Examples, but is not meant to be unfavorably limited thereto. In the present disclosure, the present invention has been described in details, wherein specific embodiments thereof are also disclosed. It will be apparent to those skilled in the art that various changes and modifications can be made to the specific embodiments of the present invention without departing from the spirit and scope of the present invention. If specific conditions are not indicated in the Examples, it shall be carried out in accordance with conventional conditions or those recommended by the manufacturer. The reagents or instruments without their manufacturers indicated are all conventional products that can be purchased commercially.

The abbreviations of reagents used in the following Examples are as follows: CDI is N,N'-carbonyl diimidazole, DBU is 1,8-diazabicyclo[5.4.0]undec-7-ene, PyBop is 1H-benzotriazol-1-yloxytripyrrolidinyl hexafluorophosphate, SPhos is 2-dicyclohexylphosphino-2',6'-dimethoxybiphenyl, SPhos-Pd-G2 is chloro(2-dicyclohexylphosphino-2' ,6' -dimethoxy-1,1' -biphenyl) [2-(2 ' -amino-1,1-biphenyl)]palladium(II), LiHMDS is lithium bis(trimethylsilyl)amide, Selectfluor is 1-chloromethyl-4-fluoro-1,4-diazoniabicyclo[2.2.2]octane bis(tetrafluoroborate), XantPhos-Pd-G2 is chloro[(4,5-bis(diphenylphosphino)-9,9-dimethylxanthene)-2-(2'-amino-1,1'-bi phenyl)]palladium(II), xantphos is 4,5-bis(diphenylphosphino)-9,9-dimethylxanthene, Pd(dppf)Cl₂ is [1,1'-bis(diphenylphosphino)ferrocene]palladium(II) dichloride, NCS is N-chlorosuccinimide, NaHMDS is sodium bis(trimethylsilyl)amide, T₃P is 1-propylphosphonic anhydride, DIEA is N,N- diisopropylethylamine.

### Preparation of Intermediate a

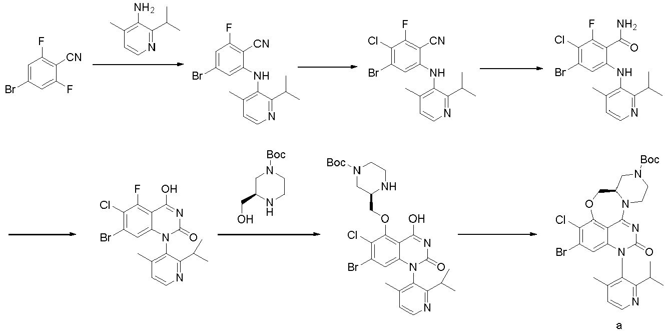

Step 1: 2-isopropyl-4-methylpyridine-3-amine (50 g, 333.3 mmol) was dissolved in tetrahydrofuran (500 mL) and the reaction solution was cooled to -65°C, then NaHMDS (333.3 mL, 2M, 666.6 mmol) was slowly dropped, and the reaction solution was kept lower than -60°C during the dropping process. After the completion of the dropping, the reaction proceeded at -60°C for 30 minutes, then 4-bromo-2,6-difluorobenzonitrile in tetrahydrofuran (500 mL) was slowly dropped to the reaction solution, and the reaction solution was kept lower than -60°C during the dropping process. After the completion of the dropping, the reaction solution returned to room temperature and the reaction proceeded overnight. After the completion of the reaction, the saturated ammonium chloride solution was added to quench the reaction. The reaction solution was extracted with ethyl acetate, the organic phase was washed with brine, dried with anhydrous sodium sulfate, and concentrated to dryness under reduced pressure, to obtain a crude product of 4-bromo-2-fluoro-6-((2-isopropyl-4-methylpyridin-3-yl)amino)benzonitrile (133 g, yield: 100%). ES-API:[M+H]⁺= 348.1.

Step 2: 4-bromo-2-fluoro-6-((2-isopropyl-4-methylpyridin-3-yl)amino)benzonitrile(13 3 g, the crude product, 333.3 mmol) was dissolved in acetonitrile (1400 mL), and NCS (89.1 g, 666.6 mmol) was added. The reaction solution was heated to 80°C to react for 2 hours, cooled to room temperature, and water was added to quench the reaction. The reaction solution was extracted with ethyl acetate, the organic phase was washed with the saturated brine, dried with anhydrous sodium sulfate, concentrated to dryness under reduced pressure, and purified by column to obtain 4-bromo-3-chloro-2-fluoro-6-((2-isopropyl-4-methylpyridin-3-yl)amino)benzo nitrile (13.91 g, yield of two steps:11%). ES-API:[M+H]⁺= 382.1.

Step 3: 4-bromo-3-chloro-2-fluoro-6-((2-isopropyl-4-methylpyridin-3-yl)amino)benzo nitrile (13.91 g,36.5 mmol) was dissolved in DMSO (130 mL), then potassium carbonate (10.0 g, 73.2 mmol) was added, hydrogen peroxide (14.91 g, 131.41 mmol) was dropped under the ice bath, and the reaction proceeded at room temperature for 2 hours. After the completion of the reaction, 10% sodium sulfite solution (100 mL) was slowly added under the ice bath to quench the reaction. The reaction solution was extracted with ethyl acetate, the organic phase was washed with the saturated brine, dried with anhydrous sodium sulfate, and concentrated to dryness under reduced pressure to obtain the product 4-bromo-3-chloro-2-fluoro-6-((2-isopropyl-4-methylpyridin-3-yl)amino)benza mide (12.5 g, yield:85.8%). ES-API:[M+H]⁺= 400.1.

Step 4: 4-bromo-3-chloro-2-fluoro-6-((2-isopropyl-4-methylpyridin-3-yl)amino)benza mide (12.5 g, 31.33 mmol) in tetrahydrofuran (150ml) was dropped in a solution of sodium hydride (12.5 g, 156.66 mmol) in tetrahydrofuran (40 mL) under a dry ice ethanol bath to control the temperature below 0°C, the reaction proceeded for 15 minutes under a dry ice ethanol bath, and then carbonyl diimidazole (7.6 g, 47.1 mmol) in tetrahydrofuran (100 mL) was dropped. After the completion of the dropping, dry ice reacted with ethanol for 15 minutes. After the completion of the reaction, the saturated ammonium chloride solution was added to quench the reaction. The reaction solution was extracted with ethyl acetate, the organic phase was washed with the saturated brine, dried with anhydrous sodium sulfate, concentrated to dryness under reduced pressure, and the crude product was slurried with (petroleum ether/ethyl acetate=10:1) to obtain the target product: 7-bromo-6-chloro-5-fluoro-1-(2-isopropyl-4-methylpyridin-3-yl)quinazolin- 2,4 (1H,3H)-dione (10.8 g, yield:81.1%), ES-API:[M+H]⁺= 426.1.

Step 5: tert-butyl (R) 3-(hydroxymethyl)piperazin-1-carboxylate (3.0 g, 14.07 mmol) was dissolved in THF (60 mL) at 0oC, sodium hydride (1.88 g, 46.9 mmol) was added in batches, the reaction was continued at 0oC for 30 minutes, then 7-bromo-6-chloro-5-fluoro-4-hydroxy-1-(2- isopropyl-4-methylpyridin-3-yl)qui nazolin-2-one (4.0g, 9.38 mmol) was added in batches, and the reaction solution reacted at 0oC~room temperature for 1.5 hours. After the completion of the reaction, the reaction solution was diluted with ethyl acetate (100 mL) at 0oC, then the ice water was added, the product was extracted with ethyl acetate (3 * 100 mL), washed with the saturated brine (3 * 80 mL), dried with anhydrous sodium sulfate, and concentrated to obtain the crude product. The crude product was purified by a silica gel column to obtain tert-butyl (R)-3-(((7-bromo-6-chloro-4-hydroxy-1-(2-isopropyl-4-methylpyridin-3-yl)-2-oxo-1,2-dihydroquinazolin-5-yl)oxy)methyl)piperazin-1-carboxylate (a brown oil, 4.5 g, yield:77.59%). ES-API:[M+H]⁺=622, 624.

Step 6: tert-butyl (R)-3-(((7-bromo-6-chloro-4-hydroxy-1-(2-isopropyl-4-methylpyridin-3-yl)-2-oxo-1,2-dihydroquinazolin-5-yl)oxy)methyl)piperazin-1-carboxylate (4.0 g, 6.42 mmol) was dissolved in DMF (100 mL) at 0oC, PyBOP (8.35 g, 16.05 mmol) was added in batches, and then DBU (4.88 g, 32.10 mmol) was dropped. After addition was completed, the reaction solution reacted at room temperature for 2 hours. After the completion of the reaction, the reaction solution was diluted with ethyl acetate (100 mL) at 0oC, then the ice water was added, the product was extracted with ethyl acetate (3 * 100 mL), washed with the saturated brine (3 *100 mL), dried with anhydrous sodium sulfate, and concentrated to obtain the crude product. The crude product was purified by a silica gel column to obtain Intermediate a (a brown oil, 3.6 g, yield:92.78%). ES-API:[M÷H]⁺=603, 605.

### Preparation of Intermediate b

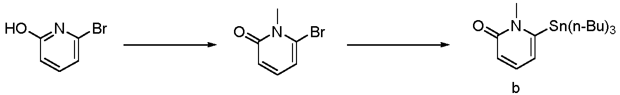

Step 1: 6-bromopyridin-2-ol (866 mg, 4.98 mmol) was dissolved in 5 mL acetonitrile, potassium carbonate (2.06 g, 14.93 mmol) and methyl iodide (1.06 g, 7.47 mmol) were added, and stirred at 60°C for 2 hours. The reaction solution was extracted with ethyl acetate, and concentrated under reduced pressure. The crude product was purified by column chromatography (petroleum ether/ethyl acetate=1/1) to obtain 6-bromo-1-methylpyridin-2(1H)-one (a white solid, 630 mg, yield:73%). ES-API:[M+H]⁺=188.0.

Step 2: 6-bromo-1-methylpyridin-2(1H)-one (2 g, 10.6 mmol) was dissolved in tetrahydrofuran (20 mL), and cooled to -78°C under nitrogen protection, n-butyl lithium (5.1 mL, 12.7 mmol, 2.5 M in tetrahydrofuran) was dropped. Under stirring, the reaction proceeded for 15 minutes at -78°C, and then tributyltin chloride (5.19 g, 16.0 mmol) was dropped, and the reaction slowly rised to room temperature. The reaction solution was quenched with the saturated ammonium chloride solution, extracted with ethyl acetate, concentrated under reduced pressure. The crude product was purified by column chromatography (petroleum ether/ethyl acetate=10/1-2/1) to obtain 1-methyl-6-(tributyltinalkyl)pyridin-2(1H)-one (light yellow liquid, 1.6 g, yield: 38%). ES-API:[M+H]⁺=400.2.

### Preparation of Intermediate c

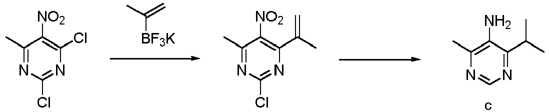

Step 1: 2,4-dichloro-6-methyl-5-nitropyrimidine (20 g, 97.08 mmol) was dissolved in dioxane (400 ml), isopropylalkenyl potassium trifluoroborate (14.98 g,101.9 mmol), bis(triphenylphosphine)palladium dichloride (3.4 g, 4.85 mmol) were added in sequence, sodium carbonate (20 g) dissolved in 100 mL of water was dropped thereto. After addition was completed, the nitrogen was used for replacement for three times, and the reaction proceeded for 2 hours at 80°C. After the completion of the reaction, the reaction system was cooled to room temperature, added into 500 ml ice water, extracted with ethyl acetate for three times, the organic phase was washed with brine once, dried with anhydrous sodium sulfate, and purified by manual column chromatography (pure petroleum ether) to obtain 2-chloro-4-methyl-5-nitro-6-(prop-1-en-2-yl)pyrimidine (8 g, yield: 38%). ES-API:[M+l]⁺=214.02.

Step 2: 2-chloro-4-methyl-5-nitro-6-(prop-1-en-2-yl)pyrimidine (8 g, 37.55 mmol) was dissolved in methanol (50 ml), sodium acetate (6.15 g, 75.11 mmol) and 800 mg palladium on carbon were added in sequence. The reaction was connected to hydrogen balloon and the hydrogen was used for replacement for three times. The reaction was stirred at room temperature for 24 hours. The point board was found that the raw materials disappeared, there were products and by-products that had not removed Cl. The reaction solution was filtered, the sample was mixed and passed the column machine (120 g column) to purify (petroleum ether/ethyl acetate=3/1) and obtain 4-isopropyl-6-methylpyrimidin-5-amine (4 g, yield:71%). ES-API:[M+1]⁺= 152.1.

### Example 1: Preparation of Compound Z 1

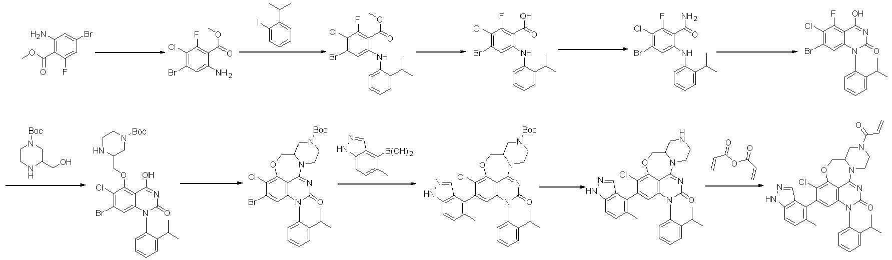

Z1 Step 1: methyl 2-amino-4-bromo-6-fluorobenzoate (2.6 g, 10.48 mmol) and 50 mL isopropanol were added to a 250 mL round bottom flask. After the temperature of the system raising to 60°C, N-chlorosuccinimide (1.7g, 12.7mmol) was added to the reaction solution. The temperature of the system rised to 80°C and reacted at this temperature for 20 minutes. After the completion of the reaction, the reaction solution was concentrated. The crude product was purified by a fast silica gel column (ethyl acetate/petroleum ether: 0-8%) to obtain the target product: methyl 6-amino-4-bromo-3-chloro-2-fluorobenzoate 1.2 g, yield of 39%. ES-API:[M+H]⁺=282.0. HNMR (400MHz, DMSO-*d₆*): 7.05(s, 1H), 6.87(s, 2H), 3.82(s, 3H).

Step 2: methyl 6-amino-4-bromo-3-chloro-2-fluorobenzoate (1.1 g, 3.89 mmol), 1-iodo-2-isopropylbenzene (1.44 g, 5.84 mmol), Pd₂(dba)₃ (0.36 g, 0.39 mmol), xantphos (0.56 g, 0.97 mmol), cesium carbonate (2.55 g, 7.78 mmol) and 20 mL of dioxane were added to a 100 mL round bottom flask. The system was replaced with the nitrogen for three times, and then protected with a nitrogen ball. The reaction was stirred at 70°C for 16 hours. The completion of the reaction was detected by LC-MS. 30 mL of water was added to the reaction. The reaction solution was extracted with 30 mL of ethyl acetate for 3 times, and the organic phase was dried and concentrated. The crude product was purified by a fast silica gel column (ethyl acetate/petroleum ether: 0-5%) to obtain the target product: methyl 4-bromo-3-chloro-2-fluoro-6-((2-isopropylphenyl)amino)benzoate 380mg, yield of24%. ES-API: [M+H]⁺=400.1.

Step 3: methyl 4-bromo-3-chloro-2-fluoro-6-((2-isopropylphenyl)amino)benzoate (380mg, 0.95mmol), sodium hydroxide (114mg, 2.84mmol), methanol (5mL), tetrahydrofuran (10mL) and water (5mL) were added to a 100 mL round bottom flask. The reaction was stirred at room temperature for 3 hours. The completion of the reaction was detected by LC-MS. 30 mL of water was added to the reaction, and the pH of the reaction was adjusted to 7 with 1M aqueous hydrochloric acid. The reaction solution was extracted with 30 mL of ethyl acetate for 3 times, and the organic phase was dried and concentrated to obtain the crude product: 4-bromo-3-chloro-2-fluoro-6-((2-isopropylphenyl)amino)benzoic acid 367mg. ES-API: [M+H]⁺=386.1.

Step 4: 4-bromo-3-chloro-2-fluoro-6-((2-isopropylphenyl)amino)benzoic acid (280mg, 0.72mmol), thionyl chloride (862mg,7.2mmol) and toluene (5mL) were added to a 100 mL round bottom flask. The reaction was stirred at 100°C for 1 hour. The reaction solution was concentrated and dried to obtain a yellow oil. The yellow oil was dissolved in tetrahydrofuran, and then it was dropped to 28% ammonia water (5mL). The reaction solution was stirred at room temperature for 1 hour. The completion of the reaction was detected by LC-MS. 30 mL of water was added to the reaction. The reaction solution was extracted with 30 mL of ethyl acetate for 3 times, and the organic phase was dried and concentrated. The crude product was purified by a fast silica gel column (ethyl acetate/petroleum ether: 0-40%) to obtain the target product: 4-bromo-3-chloro-2-fluoro-6-((2-isopropylphenyl)amino)benzamide (300mg), purity of 89%, yield of 96%. ES-API: [M+H]⁺=385.3.

Step 5: 60% NaH (146mg, 3.65mmol), tetrahydrofuran (6mL) and DMF (2mL) were added to a 100 mL round bottom flask. The reaction was cooled to 0°C, and 4-bromo-3-chloro-2-fluoro-6-((2-isopropylphenyl)amino)benzamide (280mg,0.73mmol) in tetrahydrofuran (2mL) was dropped thereto. The reaction was stirred at 0°C for 5 minutes, and then N,N'-carbonyl diimidazole (141mg, 0.87mmol) in tetrahydrofuran (1mL) was dropped to the reaction solution. After the completion of the dropping, the reaction was stirred at 0°C for 15 minutes. The completion of the reaction was detected by LC-MS. 30mL of the saturated NH₄Cl aqueous solution was added into the reaction solution. The reaction solution was extracted with 20mL of ethyl acetate for 3 times, and the organic phase was dried and concentrated. The crude product was purified by a fast silica gel column (ethyl acetate/petroleum ether:0-25%) to obtain the target product: 7-bromo-6-chloro-5-fluoro-4-hydroxy-1-(2-isopropylphenyl)quinazolin-2(1H)-one (150mg), a yellow solid, yield of 50%. ES-API: [M+H]⁺=411.2.

Step 6: 60% NaH (24mg, 0.6mmol), tert-butyl 3-(hydroxymethyl)piperazin-1-carboxylate (67mg, 0.31mmol), tetrahydrofuran (6mL) were added to a 100 mL round bottom flask. The reaction was cooled to 0°C, and 7-bromo-6-chloro-5-fluoro-4-hydroxy-1-(2-isopropylphenyl)quinazolin-2(1H)-one (100mg, 0.24mmol) in tetrahydrofuran (2mL) was dropped thereto. The reaction was stirred at 0°C for 5 minutes, and then the reaction was stirred under a 65°C oil bath for 1 hour. The completion of the reaction was detected by LC-MS. 30mL of the saturated NH₄Cl aqueous solution was added into the reaction solution. The reaction solution was extracted with 40m ethyl acetate for 3 times, and the organic phase was dried and concentrated. The crude product was purified by a fast silica gel column (methanol/dichloromethane: 0-5%) to obtain the target product: tert-butyl 3-(((7-bromo-6-chloro-4-hydroxy-1-(2-isopropylphenyl)-2-oxo-1,2-dihydroqui nazolin-5-yl)oxy)methyl)piperazin-1-carboxylate (130mg), a yellow solid, purity of 84%, yield of 75%. ES-API: [M+H]⁺=607.1.

Step 7: tert-butyl 3-(((7-bromo-6-chloro-4-hydroxy-1-(2-isopropylphenyl)-2-oxo-1,2-dihydroqui nazolin-5-yl)oxy)methyl)piperazin-1-carboxylate (100mg, 0.16mmol), N,N-dimethylformamide (1mL) and 50% T₃P in ethyl acetate (523mg, 0.82mmol) were added to a 50 mL round bottom flask. DBU (146mg, 0.96mmol) was added all at once while stirring. The reaction was stirred at room temperature for 16 hours. The completion of the reaction was detected by LC-MS. The reaction was directly purified by a C18 reversed-phase column (acetonitrile/water (1% trifluoroacetic acid):0-70%) to obtain the target product: tert-butyl 10-bromo-11-chloro-8-(2-isopropylphenyl)-7-oxo-3,4,7,8,13,13a-hexahydropyr azine[2',1':3,4][1, 4]oxazepine[5,6,7-de]quinazolin-2(1H)-carboxylate (50mg), a yellow solid, purity of 90%, yield of 53%. ES-API: [M+H]⁺=589.1.

Step 8: tert-butyl 10-bromo-11-chloro-8-(2-isopropylphenyl)-7-oxo-3,4,7,8,13,13a-hexahydropyr azine[2',1':3,4][1,4]oxazepine[5,6,7-de]quinazolin-2(1H)-carboxylate (40mg, 0.068mmol), (5-methyl-1H-indazol-4-yl)boronic acid (18mg, 0.1mmol), SPhos (7mg, 0.017mmol), Pd₂(dba)₃ (12mg, 0.014mmol), potassium phosphate (43mg, 0.2mmol), 2mL of dioxane and 0.2mL of water were added to a 10mL microwave reaction tube. The microwave reaction tube was placed in a microwave reactor at 115°C and the reaction proceeded for 1 hour, and the reaction stopped. 20mL of water was added to the reaction solution. The reaction solution was extracted with 20mL of ethyl acetate for 3 times, and the organic phase was dried and concentrated. The crude product was purified by a fast silica gel column (ethyl acetate/petroleum ether:0-100%) to obtain the target product: tert-butyl 11-chloro-8-(2-isopropylphenyl)-10-(5-methyl-1H-indazol-4-yl)-7-oxo-3,4,7,8, 13,13a-hexahydropyrazine[2',1':3,4][1,4]oxazepine[5,6,7-de]quinazolin-2(1H)-carboxylate (40mg), a yellow solid, purity of 70%, the crude product yield of 64%. ES-API: [M+H]⁺=641.2.

Step 9: tert-butyl 11-chloro-8-(2-isopropylphenyl)-10-(5-methyl-1H-indazol-4-yl)-7-oxo-3,4,7,8, 13,13a-hexahydropyrazine[2',1':3,4][1,4]oxazepine[5,6,7-de]quinazolin-2(1H)-carboxylate (40mg, 0.062mmol), 2mL dichloromethane and 2mL of trifluoroacetic acid were added to a 25 mL round bottom flask. The reaction was stirred at room temperature for 0.5 hour. The completion of the reaction was detected by LC-MS. The reaction solution was concentrated at 40°C to obtain the crude product: 11-chloro-8-(2-isopropylphenyl)-10-(5-methyl-1H-indazol-4-yl)-1,2,3,4,13,13a -hexahydropyrazino[2',1':3,4][1,4]oxazepine [5,6,7-de]quinazolin-7(8H)-one (39mg), a yellow solid, purity of 72%. ES-API:[M+H]⁺=541.2.

Step 10: 11-chloro-8-(2-isopropylphenyl)-10-(5-methyl-1H-indazol-4-yl)-1,2,3,4,13,13a -hexahydropyrazino[2',1':3,4][1,4]oxazepine[5,6,7-de]quinazolin-7(8H)-one (39mg, 0.06mmol), 2mL dichloromethane and triethylamine (30mg, 0.3mmol) were added to a 25 mL round bottom flask. The reaction was cooled to 0°C, and acrylic anhydride in dichloromethane (5mg, 0.04mmol, 0.5mL) was dropped to the reaction solution. The reaction was stirred at 0°C for 20 minutes. 10mL of the saturated NaHCO₃ aqueous solution was added to the reaction solution, extracted with 10mL dichloromethane for 3 times, the organic phase was dried and concentrated, and the crude product was purified by Preparative HPLC to obtain the target product: Z1(4.44mg), a white solid, the reaction yield of 12%. ES-API:[M+H]⁺=595.2. (400MHz,CDCl₃): 7.52-7.34(m,4H), 7.28-7.26(m,2H), 7.11-7.09(m,1H), 6.68-6.61(m,1H), 6.44-6.40(m,1H), 6.21-6.20(m,1H), 5.83(d,*J*=10Hz,1H), 5.05-5.01(m,1H), 4.72-4.65(m,3H), 4.08-3.98(m,2H), 3.62-3.58(m,1H), 3.21-3.12(m,2H), 2.76-2.71(m,1H), 2.12-2.10(m,3H), 1.22-1.20(m,3H), 1.05-1.01(m,3H).

### Example 2-14

Compounds Z2 to Z14 were prepared by referring to the similar method of Compound Z1, wherein the starting materials of each Compound can be prepared by commercially available or by referring to existing methods well known to one skilled in the art, and similar synthetic methods of Intermediates are easily available for one skilled in the art by referring existing methods.

| Example No. | Compound Structure | MS [M+H]⁺ | Example No. | Compound Structure | MS [M+H]⁺ |
|---|---|---|---|---|---|
| 2 | 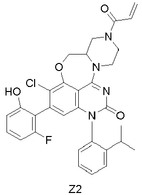 | 575.2 | 3 | 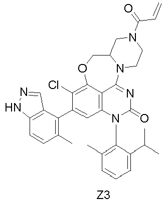 | 609.2 |
| 4 | 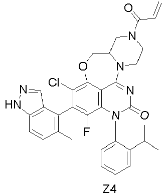 | 613.2 | 5 | 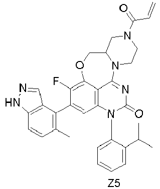 | 579.2 |
| 6 | 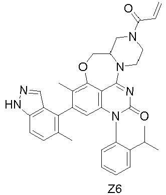 | 575.2 | 7 | 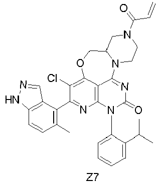 | 596.2 |
| 8 | 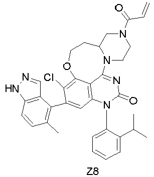 | 609.2 | 9 | 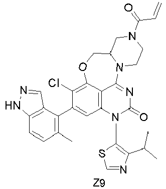 | 602.1 |
| 10 | 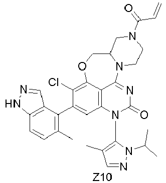 | 599.2 | 11 | 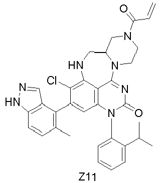 | 594.2 |
| 12 | 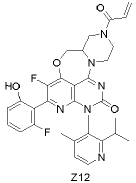 | 575.2 | 13 | 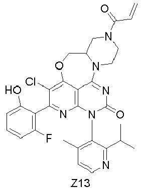 | 591.2 |
| 14 | 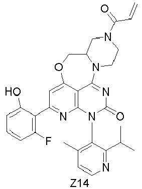 | 557.2 | | | |

### Example 15: Preparation of Compound Z15, Z15-1 and Z15-2

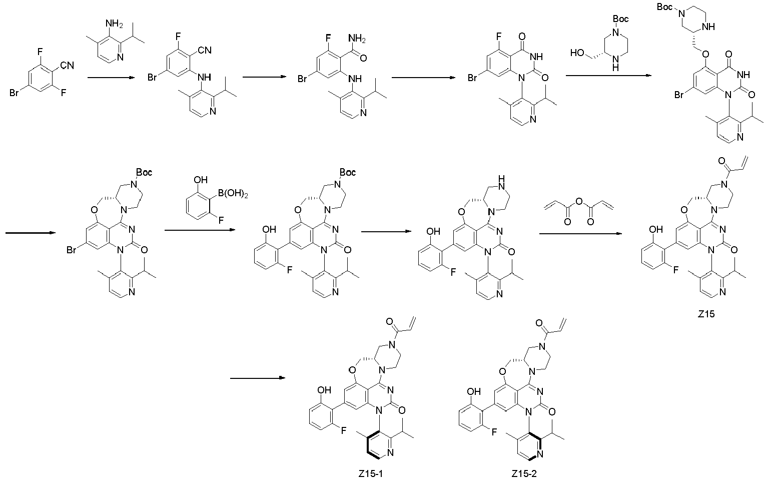

Step 1: 2-isopropyl-4-methylpyridin-3-amine (1.5 g, 9.99 mmol) and 50 mL of tetrahydrofuran were added to a 250 mL round bottom flask. After the system cooling to 0°C, sodium bis(trimethylsilyl)amide (12.5mL, 2.5 M in tetrahydrofuran, 25 mmol) was dropped to the reaction solution. After the completion of the dropping, the reaction was stirred at -65°C for 30 minutes, and then 4-bromo-2,6-difluorobenzonitrile (2.39g, 10.98 mmol) in tetrahydrofuran (50 mL) was dropped to the reaction solution. After the completion of the dropping, the reaction was stirred at 0°C overnight. After the completion of the reaction, the reaction solution was poured into ice water, and extracted with ethyl acetate. The organic phase was washed with 1M hydrochloric acid, the saturated aqueous sodium bicarbonate solution, and the saturated brine. The organic phase was dried with anhydrous sodium sulfate, filtered, and the filtrate was concentrated and dried to obtain the product: 4-bromo-2-fluoro-6-(((2-isopropyl-4-methylpyridin-3-yl)amino)benzonitrile (1.7g, 49%), a yellow solid. ES-API: [M+H]⁺=349.3.

Step 2: 4-bromo-2-fluoro-6-(((2-isopropyl-4-methylpyridin-3-yl)amino)benzonitrile (1.7 g, 4.88 mmol) and 50 mL of dimethyl sulfoxide were added to a 250 mL three-necked round bottom flask, cooled to 0-5°C under the ice bath, potassium carbonate (1.34g, 9.76mmol) and hydrogen peroxide (6.57g, 30% aqueous solution , 58mmol) were added. The reaction proceeded overnight at this temperature. The completion of the reaction was detected by LCMS. The reaction solution was poured into about 150 mL of ice water, and a solid precipitated out. It was filtered to obtain filter cake. The filter cake was dried to obtain 4-bromo-2-fluoro-6-(((2-isopropyl-4-methylpyridin-3-yl)amino)benzamide(1.8 g, the crude product), a white solid, the crude product was directly used in the next step. ES-API:[M+H]⁺=367.2.

Step 3: 4-bromo-2-fluoro-6-(((2-isopropyl-4-methylpyridin-3-yl)amino)benzamide(1.8 g, 4.9 mmol) and 80 mL of dry tetrahydrofuran were added to a 250 mL three-necked round bottom flask, cooled to 0-5°C under the ice bath, sodium hydride was added in batches (980mg, 24.5 mmol), and the reaction proceeded at this temperature for 10 minutes. A suspension of CDI (1.59g, 9.8 mmol) in tetrahydrofuran (40mL) was dropped to the above solution, and then the reaction proceeded at this temperature for 20 minutes. The completion of the reaction was detected by LCMS. The reaction solution was poured into about 150 mL of ice water, and the pH was adjusted to about 4 with 3 M hydrochloric acid. The reaction solution was extracted with ethyl acetate. The organic phase was washed with the saturated brine, dried with anhydrous sodium sulfate, filtered. The filtrate was concentrated and dried to obtain 7-bromo-5-fluoro-1-(2-isopropyl-4-methylpyridin-3-yl)quinazolin-2,4(1H, 3H)-dione (1.46g, 76%), a yellow solid, the crude product was directly used in the next step. ES-API:[M+H]⁺=393.3.

Step 4: tert-butyl (S)-3-(hydroxymethyl)piperazin-1-carboxylate (1.04g, 4.81 mmol) was added to a suspension of 60% sodium hydride (1.11g, 18.5 mmol) in tetrahydrofuran (50 mL) at 0°C, reacted at 0°C for 30 minutes, and 7-bromo-5-fluoro-1-(2-isopropyl-4-methylpyridin-3-yl)quinazolin-2,4(1H, 3H)-dione (1.46 g, 3.7 mmol) in tetrahydrofuran (20 mL) was dropped thereto. The reaction was stirred at 0°C for 30 minutes. The completion of the reaction was detected by LC-MS. The reaction solution was poured into 100mL of ice water. The reaction solution was extracted with ethyl acetate for 3 times. The organic phase was dried and concentrated, and the crude product was purified by a fast silica gel column (ethyl acetate/petroleum ether:20-100%) to obtain tert-butyl (S)-3-((((7-bromo-1-(2-isopropyl-4-methylpyridin-3-yl)-2,4-dioxo-1,2,3,4-tetra hydroquinazolin-5-yl)oxy)methyl)piperazin-1-formate (1.34g, 62%), a yellow solid. ES-API: [M+H]⁺=589.1.

Step 5: tert-butyl (S)-3-((((7-bromo-1-(2-isopropyl-4-methylpyridin-3-yl)-2,4-dioxo-1 ,2,3,4-tetra hydroquinazolin-5-yl)oxy)methyl)piperazin-1-formate (1.34g, 2.29 mmol), DBU (2.01g, 13.74 mmol), PyBop (3.57g, 6.87 mmol) dichloromethane (100 mL) were added to a round bottom flask. The reaction was stirred at room temperature for 30 minutes. The completion of the reaction was detected by LC-MS. 100 mL of dichloromethane was added to the reaction, and the organic phase was washed with 30 mL of hydrochloric acid (1M) and 100 mL of the saturated sodium bicarbonate aqueous solution in sequence. The organic phase was dried and concentrated, and the crude product was purified by a fast silica gel column (methanol /dichloromethane:0-3%) to obtain tert-butyl (S)-10-bromo-8-(2-isopropyl-4-methylpyridin-3-yl)-7-oxo-3,4,7,8,13,13a-hexa hydropyrazino[2',1':3,4][1,4]oxazepine[5,6,7-de]quinazolin-2(1H)-carboxylate (1.04g, 80%). ES-API: [M+H]⁺=571.4.

Step 6: tert-butyl (S)-10-bromo-8-(2-isopropyl-4-methylpyridin-3-yl)-7-oxo-3,4,7,8,13,13a-hexa hydropyrazino[2',1':3,4][1,4]oxazepine[5,6,7-de]quinazolin-2(1H)-carboxylate (1.04g, 1.83mmol), (2-fluoro-6-hydroxyphenyl)boronic acid (429mg, 2.75mmol), SPhos (73mg, 0.18 mmol), SPhos-Pd-G2 (129mg, 0.18 mmol), potassium phosphate (1.16g, 5.49 mmol), 60 mL of dioxane and 12 mL of water were added to a 100mL reaction flask. The reaction was stirred at 115°C for 2 hours under nitrogen protection, and the reaction stopped. 50 mL of water was added to the reaction solution. The reaction solution was extracted with 50mL of ethyl acetate for 3 times, the organic phase was dried and concentrated, and the crude product was purified by a fast silica gel column (ethyl acetate/petroleum ether:0-100%) to obtain the product: tert-butyl (S)-10-(2-fluoro-6-hydroxyphenyl)-8-(2-isopropyl-4-methylpyridin-3-yl)-7 -oxo -3,4,7,8,13,13a-hexahydropyrazino[2',1':3,4][1,4]oxazepine[5,6,7-de]quinazoli n-2(1H)-carboxylate (560mg, 51%), a yellow solid. ES-API: [M+H]⁺= 602.1.

Step 7: tert-butyl (S)-10-(2-fluoro-6-hydroxyphenyl)-8-(2-isopropyl-4-methylpyridin-3-yl)-7-oxo -3,4,7,8,13,13a-hexahydropyrazino[2',1':3,4][1,4]oxazepine[5,6,7-de]quinazoli n-2(1H)-carboxylate (560mg, 0.93 mmol), 3 mL of trifluoroacetic acid and 6 mL of dichloromethane were added to a round bottom flask. The reaction was stirred at room temperature for 1 hour. The completion of the reaction was detected by LC-MS. The reaction solution was concentrated to obtain (S)-10-(2-fluoro-6-hydroxyphenyl)-8-(2-isopropyl-4-methylpyridin-3-yl)-1,2,3, 4,13,13 a-hexahydropyrazino[2',1':3,4][1,4]oxazepine[5,6,7-de]quinazolin-7(8 H)-one (582 mg), a yellow solid. The crude product was directly used in the next step. ES-API: [M+H]⁺=502.1.

Step 8: (S)-10-(2-fluoro-6-hydroxyphenyl)-8-(2-isopropyl-4-methylpyridin-3-yl)-1,2,3, 4,13,13a-hexahydropyrazino[2',1':3,4][1,4]oxazepine[5,6,7-de]quinazolin-7(8H )-one (582 mg, 1.16mmol), 6 mL of dichloromethane and triethylamine (1.17g, 11.6mmol) were added to a round bottom flask. The reaction was cooled to 0°C, and acrylic anhydride in dichloromethane (117 mg, 0.93 mmol, 1 mL) was dropped to the reaction solution. The reaction was stirred at 0°C for 10 minutes. 40 mL of the saturated sodium bicarbonate aqueous solution was added to the reaction solution, and the reaction solution was extracted with 20 mL of dichloromethane for 3 times . The organic phase was dried and concentrated, and the crude product was purified by Preparative HPLC to obtain the target product: (S)-2-acryloyl-10-(2-fluoro-6-hydroxyphenyl)-8-(2-isopropyl-4-methylpyridin-3-yl)-1,2,3,4,13,13a-hexahydropyrazino[2',1':3,4][1,4]oxazepine[5,6,7-de]quin azolin-7(8H)-one Z15 (257mg, 40%). ES-API: [M+H]⁺=556.1. ¹H NMR (400 MHz, DMSO-*d₆*) δ 10.08 (d, *J* = 1.8 Hz, 1H), 8.50 (d,*J*= 5.0 Hz, 1H), 7.28 (d, *J* = 5.0 Hz, 1H), 7.16 (dd, *J* = 15.0, 8.2 Hz, 1H), 6.84 (s, 2H), 6.72 (d, *J* = 8.2 Hz, 1H), 6.69-6.60 (m, 1H), 6.20 (d, *J* = 16.4 Hz, 1H), 5.98 (s, 1H), 5.76 (s, 1H), 4.75 (s, 1H), 4.61 (s, 2H), 4.52-4.26 (m, 2H), 4.17-3.97 (m, 2H), 2.82-2.68(m, 1H), 1.95 (d, *J =* 3.4 Hz, 4H), 1.24 (s, 3H), 1.11-1.06 (m, 4H), 1.05-1.00 (m, 4H).

Step 9: Compound Z15 (257mg, 0.46mmol) was resolved chirally (mobile phase: methanol (0.2% ammonia methanol)); column type: Cellulose-SC (4.6^{∗}100^{∗}5um); flow rate: 1.8 ml/min; column temperature: 40.2 °C) to obtain:
Compound Z15-1 (112mg, retention time: 1.6min, purity: 100 %, de value: 99 %). ES-API: [M+H]⁺= 556.2. and Compound Z15-2 (121mg, retention time: 2.81min, purity:100%, de value: 99%). ES-API:[M+H]⁺= 556.2.

### Example 16: Preparation of Compound Z16

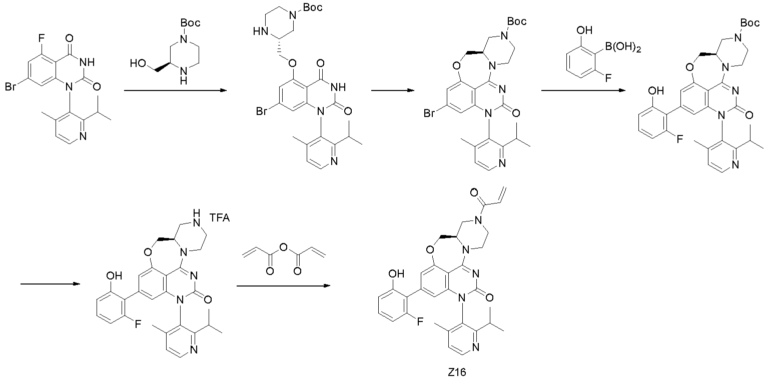

Step 1: sodium hydride (0.81g, 20.25mmol, 60%) was suspended in 15ml of tetrahydrofuran solution and cooled to 0 °C, and 7-bromo-5-fluoro-1-(2-isopropyl-4-methylpyridin-3-yl)quinazolin-2,4(1H, 3H)-dione (1.44g, 6.658mmol), 10ml of tetrahydrofuran solution was slowly dropped to the solution. After stirring for 5 minutes, tert-butyl (R)-3-(hydroxymethyl)piperazin-1-carboxylate (1.8g, 4.589mmol), 15ml of tetrahydrofuran solution was dropped. After heating to 65°C, the reaction was stirred for 1 hour. 30mL of ammonium chloride solution was added to the reaction solution. The reaction solution was extracted twice with 50ml of ethyl acetate, dried and concentrated. The crude product was purified by a fast silica gel column (ethyl acetate/petroleum ether:0-35%) to obtain the target product: tert-butyl (R)-3-((((7-bromo-1-(2-isopropyl-4-methylpyridin-3-yl)-2,4-dioxo-1,2,3,4-tetra hydroquinazolin-5-yl)oxy)methyl)piperazin-1-carboxylate (1.5g, yield:75%), a white-like solid. ES-API: [M+H]⁺=588.

Step 2: tert-butyl (R)-3-((((7-bromo-1-(2-isopropyl-4-methylpyridin-3-yl)-2,4-dioxo-1,2,3,4-tetra hydroquinazolin-5-yl)oxy)methyl)piperazin-1-carboxylate (1.5g,2.55 mmol) was dissolved in N,N-dimethylformamide (50 mL), benzotriazole-1-yl-oxytripyrrolidinophosphonium hexafluorophosphate (3.97g, 7.64mmol), 1,8-diazabicycloundec-7-ene (2.33g, 15.29mmol) were added in sequence. The reaction was stirred at room temperature for 1 hour. 150 mL of ethyl acetate was added to the reaction solution, the reaction solution was washed with the saturated brine for 3 times, dried and concentrated. The crude product was purified by a fast silica gel column (ethyl acetate/petroleum ether: 0-20%) to obtain the target product: tert-butyl (R)-10-bromo-8-(2-isopropyl-4-methylpyridin-3-yl)-7-oxo-3,4,7,8,13,13a-hexa hydropyrazino[2',1':3,4][1,4]oxazepine[5,6,7-de]quinazolin-2(1H)-carboxylate (1.0g, yield: 69%), a light yellow solid. ES-API: [M+H]⁺=571.

Step 3: tert-butyl (R)-10-bromo-8-(2-isopropyl-4-methylpyridin-3-yl)-7-oxo-3,4,7,8,13,13a-hexa hydropyrazino[2',1':3,4][1,4]oxazepine[5,6,7-de]quinazolin-2(1H)-carboxylate (200mg, 0.35mmol), (2-fluoro-6-hydroxyphenyl)boronic acid (82mg, 0.526mmol), chloro(2-dicyclohexylphosphino-2',6'-dimethoxy-1,1'-biphenyl)(2'-amino-1,1'-b iphenyl-2-yl)palladium(II) (25.24mg, 0.035mmol), 2-dicyclohexylphosphino-2' ,6' -dimethoxy-biphenyl (14.37mg, 0.035mmol), potassium phosphate (223mg, 0.105mmol), 5ml of 1, 4-dioxane and 1mL of water were added to a 100ml three-necked round bottom flask. The system was replaced with the nitrogen for three times, and then protected with a nitrogen ball. The reaction was stirred at 110°C for 1 hour. 150 mL of ethyl acetate was added to the reaction solution, the reaction solution was washed with the saturated brine for 3 times, dried and concentrated. The crude product was purified by a fast silica gel column (ethyl acetate/petroleum ether:0-50%) to obtain the target product: tert-butyl (R)-10-(2-fluoro-6-hydroxyphenyl)-8-(2-isopropyl-4-methylpyridin-3-yl)-7 -ox o-3,4,7,8,13,13a-hexahydropyrazino[2',1':3,4][1,4]oxazepine[5,6,7-de]quinazo lin-2(1H)-carboxylate (160mg, yield: 76%), a light yellow solid. ES-API: [M+H]⁺=602.

Step 4: tert-butyl (R)-10-(2-fluoro-6-hydroxyphenyl)-8-(2-isopropyl-4-methylpyridin-3-yl)-7-ox o-3,4,7,8,13,13a-hexahydropyrazino[2',1':3,4][1,4]oxazepine[5,6,7-de]quinazo lin-2(1H)-carboxylate (160 mg, 0.266 mmol) was dissolved in dichloromethane (5 mL), and trifluoroacetic acid (2 mL) was added. The reaction was stirred at room temperature for 2 hours, and the reaction solution was concentrated to obtain the target product: (R)-10-(2-fluoro-6-hydroxyphenyl)-8-(2-isopropyl-4-methylpyridin-3-yl)-1,2,3, 4,13,13a-hexahydropyrazino[2',1':3,4][1,4]oxazepine[5,6,7-de]quinazolin-7(8 H)-one (175mg). The crude product was directly used in the next step. ES-API: [M+H]⁺=502.

Step 5: (R)-10-(2-fluoro-6-hydroxyphenyl)-8-(2-isopropyl-4-methylpyridin-3-yl)-1,2,3, 4,13,13 a-hexahydropyrazino[2',1':3,4][1,4]oxazepine[5,6,7-de]quinazolin-7(8 H)-one (175mg, 0.266 mmol) was dissolved in dichloromethane (15 mL), and triethylamine (1.0mL, 7.21mmol) was added. The reaction was cooled to 0°C, and acrylic anhydride (26.83 mg, 0.213mmol) was dropped to the reaction solution. The reaction was stirred at 0°C for 15 minutes. 80 mL of dichloromethane was added to the reaction solution, the reaction solution was washed with 100mL of the saturated NaHCO₃ aqueous solution, 80mL of the saturated brine, dried and concentrated. The crude product was purified by a fast silica gel column (ethyl acetate/petroleum ether:0-60%) to obtain the target product:
(R)-2-acryloyl-10-(2-fluoro-6-hydroxyphenyl)-8-(2-isopropyl-4-methylpyridin-3-yl)-1,2,3,4,13,13a-hexahydropyrazino[2',1':3,4][1,4]oxazepine[5,6,7-de]quin azolin-7(8H)-one Z16 (60mg, yield:67%), a yellow solid. ES-API: [M+H]⁺=556. ¹H NMR (500 MHz, DMSO-*d₆*) δ 10.05 (d, J = 92.9 Hz, 1H), 8.50 (d, J = 4.9 Hz, 1H), 7.35-7.24 (m, 1H), 7.16 (td, J = 8.3, 6.7 Hz, 1H), 6.90 (t, J = 13.6 Hz, 1H), 6.84 (d, J = 2.9 Hz, 1H), 6.71 (d, J = 8.3 Hz, 1H), 6.65 (dd, J = 9.9, 8.4 Hz, 1H), 6.20 (d, J = 16.7 Hz, 1H), 5.98 (dt, J = 4.7, 1.4 Hz, 1H), 5.77 (dd, J = 10.4, 2.4 Hz, 1H), 4.76 (d, J = 18.0 Hz, 1H), 4.67-4.54 (m, 2H), 4.34 (d, J = 17.2 Hz, 1H), 4.05 (s, 1H), 3.51 (dd, J = 20.8, 11.2 Hz, 1H), 3.29-2.98 (m, 2H), 2.76 (dp, J = 26.7, 6.7 Hz, 1H), 1.95 (d, J = 4.5 Hz, 3H), 1.08 (t, J = 6.7 Hz, 3H), 1.03 (dd, J = 6.7, 4.2 Hz, 3H).

### Example 17: Preparation of Compound Z17, Z17-1 and Z17-2

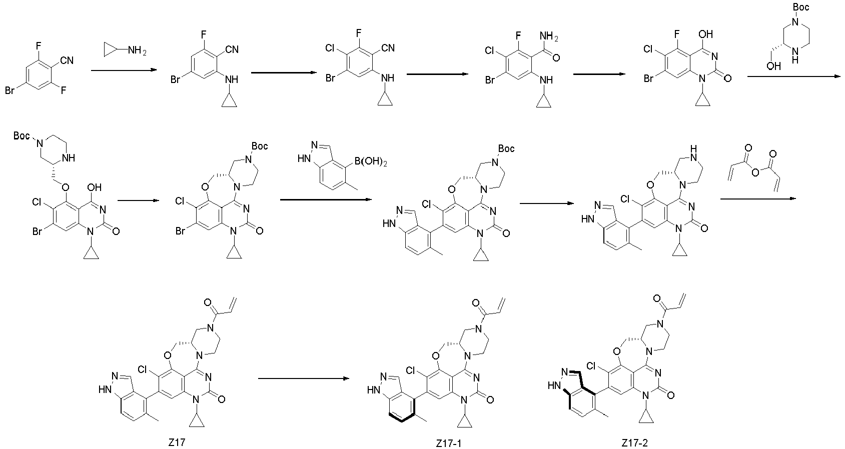

Step 1: 4-bromo-2,6-difluorobenzonitrile (10 g, 45.87 mmol), 40 mL of isopropanol and cyclopropylamine (13 g, 229 mmol) were added to the sealed tube. The reaction was heated under a 70°C oil bath for 2 hours. The completion of the reaction was detected by LC-MS. The reaction solution was concentrated. The crude product was washed with petroleum ether to obtain the product: 4-bromo-2-(cyclopropylamino)-6-fluorobenzonitrile (11 g, 94%), a white solid. The crude product was directly used in the next step. ES-API: [M+H]⁺=255.1.

Step 2: 4-bromo-2-(cyclopropylamino)-6-fluorobenzonitrile (10 g, 39.21 mmol), N-chlorosuccinimide (5.7 g, 43.13 mmol) and acetonitrile (150 mL) were added to a round bottom flask. The reaction was stirred at 65°C for 0.5 hour. sodium thiosulfate aqueous solution was added to the reaction solution . The reaction solution was extracted with ethyl acetate. The organic phase was dried and concentrated. The crude product was purified by a fast silica gel column (ethyl acetate/petroleum ether: 0-5%) to obtain 4-bromo-3-chloro-6-(cyclopropylamino)-2-fluorobenzonitrile (3.97 g, 35%), a white solid. HNMR (400MHz, CDCl₃) 7.17 (s, 1H), 5.13 (s, 1H), 2.50-2.48 (m, 1H), 0.92-0.90 (m, 2H), 0.62-0.60 (m, 2H).ES-API: [M+H]⁺=289.1.

Step 3: 4-bromo-3-chloro-6-(cyclopropylamino)-2-fluorobenzonitrile (5 g, 17.30 mmol), potassium carbonate (4.77 g, 34.60 mmol) and dimethyl sulfoxide (30 mL) were added to a round bottom flask. 30% hydrogen peroxide (3.47 mL, 34.60 mmol) was added under stirring. The reaction was stirred at room temperature for 3 hours. Ice water was added to the reaction solution, and a solid precipitated out. It was filtered and the filter cake was vacuum-dried to obtain 4-bromo-3-chloro-6-(cyclopropylamino)-2-fluorobenzamide (4.7 g, 89%), a white solid. HNMR (400MHz, DMSO-*d₆*) 7.91 (s, 1H), 7.85 (s, 1H), 7.15 (s, 1H), 6.96 (s, 1H), 2.502-2.45 (m, 1H), 0.80-0.75 (m, 2H), 0.47-0.43 (m, 2H).ES-API: [M+H]⁺=306.9.

Step 4: 4-bromo-3-chloro-6-(cyclopropylamino)-2-fluorobenzamide (4.7 g, 16.7 mmol) and 80 mL of dry tetrahydrofuran were added to a round bottom flask, cooled to 0-5°C under the ice bath, sodium hydride (3.3 g, 83.5 mmol) was added in batches, the reaction proceeded at this temperature for 5 minutes. A suspension of CDI (3.2 g, 20.04 mmol) in tetrahydrofuran (40 mL) was dropped to the above solution, and then the reaction proceeded at this temperature for 15 minutes. The completion of the reaction was detected by LCMS. The reaction solution was poured into about 150 mL of ice water, and the pH was adjusted to about 3 with 3 M hydrochloric acid. The reaction solution was extracted with ethyl acetate. The organic phase was washed with the saturated brine, dried with anhydrous sodium sulfate, and filtered. The filtrate was concentrated and dried to obtain 7-bromo-6-chloro-1cyclopropyl-5-fluoro-4-hydroxyquinazolin-2(1H)-one (3g, 55%), a yellow solid, the crude product was directly used in the next step. ES-API: [M+H]⁺=333.1.

Step 5: 60% NaH (180 mg, 4.5 mmol), tert-butyl (S)-3-(hydroxymethyl)piperazin-1-carboxylate (291 mg, 1.35 mmol), tetrahydrofuran (12 mL) were added to a round bottom flask. The reaction was cooled to 0°C, and a solution of 7-bromo-6-chloro-1-cyclopropyl-5-fluoro-4-hydroxyquinazolin-2(1H)-one (300 mg, 0.90 mmol) in tetrahydrofuran (3 mL) was dropped thereto. The reaction was stirred at 0°C for 5 minutes, and then the reaction was stirred under a 60°C oil bath for 1 hour. The completion of the reaction was detected by LC-MS. 30 mL of the saturated NH₄Cl aqueous solution was added into the reaction solution. The reaction solution was extracted with 40 mL of ethyl acetate for 3 times. The organic phase was dried and concentrated. The crude product was purified by a fast silica gel column (methanol /dichloromethane:0-5%) to obtain the target product: tert-butyl (S)-3-((((7-bromo-6-chloro-1-cyclopropylmethyl-4-hydroxy-2-oxo-1,2-dihydro quinazolin-5-yl)oxy)methyl)piperazin-1-carboxylate (470 mg), a yellow solid, yield of 98%. ES-API:[M+H]⁺=529.1.

Step 6: tert-butyl (S)-3-((((7-bromo-6-chloro-1-cyclopropylmethyl-4-hydroxy-2-oxo-1,2-dihydro quinazolin-5-yl)oxy)methyl)piperazin-1-carboxylate (450 mg, 0.85 mmol), 1H-benzotriazol-1-yloxytripyrrolidinyl hexafluorophosphate (1.3 g, 2.55 mmol) N,N-dimethylformamide (8 mL) were added to a round bottom flask. DBU (646 mg, 4.25 mmol) was added under stirring. The reaction was stirred at room temperature for 2 hours. The completion of the reaction was detected by LC-MS. 20 mL of water was added to the reaction solution. The reaction solution was extracted with 20 mL of ethyl acetate for 3 times. The organic phase was washed with the saturated brine for three times, dried and concentrated. The crude product was purified by a fast silica gel column (ethyl acetate/petroleum ether:0-100%) to obtain the target product: tert-butyl (S)-10-bromo-11-chloro-8-cyclopropyl-7-oxo-3,4,7,8,13,13a-hexahydropyrazin o[2',1:3,4][1,4]oxazepine[5,6,7-de]quinazolin-2(1H)-carboxylate (420 mg), a yellow solid, yield of 96%. ES-API: [M+H]⁺=511.0.

Step 7: tert-butyl (S)-10-bromo-11-chloro-8-cyclopropyl-7-oxo-3,4,7,8,13,13a-hexahydropyrazin o[2',1':3,4][1,4]oxazepine[5,6,7-de]quinazolin-2(1H)-carboxylate (370 mg, 0.72 mmol), (5-methyl-1H-indazol-4-yl)boronic acid (190 mg, 1.08 mmol), SPhos (29 mg, 0.072 mmol), SPhos-Pd-G₂ (52 mg, 0.072 mmol), potassium phosphate (457 mg, 2.16 mmol), 8 mL of dioxane and 2 mL of water were added to a round bottom flask. The round bottom flask was placed in a microwave reactor at 105°C and the reaction proceeded for 2 hours, and the reaction stopped. 20 mL of water was added to the reaction solution. The reaction solution was extracted with 20 mL of ethyl acetate for 3 times, and the organic phase was dried and concentrated. The crude product was purified by a fast silica gel column (methanol /dichloromethane:0-2%) to obtain the target product: tert-butyl (13aS)-11-chloro-8-cyclopropyl-10-(5-methyl-1H-indazol-4-yl)-7-oxo-3,4,7,8, 13,13a-hexahydropyrazino[2',1': 3,4][1,4]oxazepine[5,6,7-de]quinazolin-2(1H) -carboxylate (400 mg), a yellow solid, purity of 60%, yield of 59%. ES-API: [M+H]⁺=563.2.

Step 8: tert-butyl (13aS)-11-chloro-8-cyclopropyl-10-(5-methyl-1H-indazol-4-yl)-7-oxo-3,4,7,8, 13,13a-hexahydropyrazino[2',1':3,4][1,4]oxazepine[5,6,7-de]quinazolin-2(1H) -carboxylate (400 mg, 0.44 mmol), 3 mL of dichloromethane and 3 mL of trifluoroacetic acid were added to a round bottom flask. The reaction was stirred at room temperature for 1 hour. The completion of the reaction was detected by LC-MS. The reaction solution was concentrated to obtain the crude product: (13aS)-11-chloro-8-cyclopropyl-10-(5-methyl-1H-indazol-4-yl)-1,2,3,4,13,13a-hexahydropyrazino[2',1':3,4][1,4]oxazepine[5,6,7-de]quinazolin-7(8H)-one (203 mg), directly used in the next step. ES-API:[M+H]⁺=463.2.

Step 9: (13aS)-11-chloro-8-cyclopropyl-10-(5-methyl-1H-indazol-4-yl)-1,2,3,4,13,13a-hexahydropyrazino[2',1':3,4][1,4]oxazepine[5,6,7-de]quinazolin-7(8H)-one (203 mg, 0.44 mmol), 5 mL of dichloromethane and triethylamine (222 mg, 2.2 mmol) were added to a round bottom flask. The reaction was cooled to 0°C, acrylic anhydride in dichloromethane (39mg, 0.3 mmol,0.5 mL) was dropped to the reaction solution . The reaction was stirred at 0°C for 10 minutes. 10 mL of the saturated NaHCO₃ aqueous solution was added to the reaction solution, and the reaction solution was extracted with 10 mL of dichloromethane for 3 times. The organic phase was dried and concentrated, and the crude product was purified by Preparative HPLC to obtain the target product: Z17 (55.94 mg, 24%), a white solid. ¹HNMR (400MHz, CDCl₃) 7.62 (s, 1H), 7.53-7.35 (m, 3H), 6.60-6.56 (m, 1H), 6.42-6.38 (m, 1H), 5.83-5.80 (m, 1H), 4.90-4.87 (m, 1H), 4.60-4.55 (m, 3H), 4.10-3.90 (m, 2H), 3.56-3.26 (m, 3H), 2.82-2.81 (m, 1H), 2.26-2.25 (m, 3H), 1.22-1.15 (m, 2H), 0.88-0.80 (m, 2H). ES-API: [M+H]⁺= 517.0.

Step 10: Compound Z17(0.3g) was resolved chirally (column type: Chiralpak IH 250mm^{∗}4.6mm^{∗}5um; mobile phase: acetonitrile: isopropanol: ammonia methanol =90:10:0.2; flow rate: 1ml/min; column temperature=30 °C) to obtain:
Compound Z17-1 (80 mg, retention time 5.666 min; peak 1), a white solid. ¹HNMR (400MHz, CDCl₃) 7.68 (s, 1H), 7.58 (d, *J*=4 Hz, 1H), 7.43-7.41 (m, 2H), 6.62-6.56 (m, 1H), 6.42-6.39 (m, 1H), 5.82-5.80 (m, 1H), 4.90-4.87 (m, 1H), 4.60-4.51 (m, 3H), 4.10-3.91 (m, 2H), 3.60-3.29 (m, 3H), 2.83-2.82 (m, 1H), 2.26 (s, 3H), 1.22-1.20 (m, 2H), 0.90-0.82 (m, 2H). ES-API:[M+H]⁺ =517.1.
and Compound Z17-2 (85 mg, retention time 6.661 min; peak 2), a white solid. ¹HNMR (400MHz, CDCl₃) 7.74-7.41 (m, 4H), 6.60-6.58 (m, 1H), 6.42-6.39 (m, 1H), 5.82-5.80 (m, 1H), 4.89-4.88 (m, 1H), 4.60-4.57 (m, 3H), 4.06-3.96 (m, 2H), 3.60-3.30 (m, 3H), 2.83-2.82 (m, 1H), 2.26 (s, 3H), 1.20-1.16 (m, 2H), 0.92-0.82 (m, 2H). ES-API:[M+H]⁺=517.1.

### Example 18: Preparation of Compound Z18

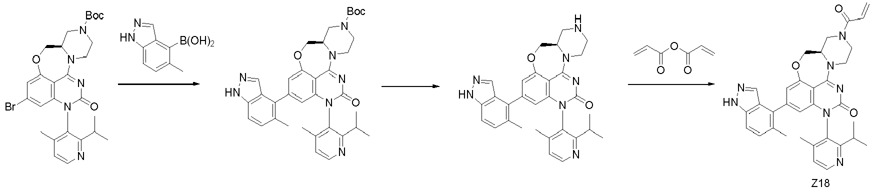

Step 1: tert-butyl (S)-10-bromo-8-(2-isopropyl-4-methylpyridin-3-yl)-7-oxo-3,4,7,8,13,13a-hexa hydropyrazino[2',1':3,4][1,4]oxazepine [5,6,7-de]quinazolin-2(1H)-carboxylate (500mg, 0.9mmol), (5-methyl-1H-indazol-4-yl)boronic acid (238mg, 1.35mmol), SPhos (37mg, 0.09 mmol), SPhos-Pd-G2 (65mg, 0.09 mmol), potassium phosphate (600mg, 2.25 mmol), 60 mL of dioxane and 12 mL of water were added to a 100mL reaction flask. The reaction was stirred at 115°C for 2 hours under nitrogen protection, and the reaction stopped. 50 mL of water was added to the reaction solution. The reaction solution was extracted with 50mL of ethyl acetate for 3 times, and the organic phase was dried and concentrated. The crude product was purified by a fast silica gel column (ethyl acetate/petroleum ether:0-100%) to obtain the product: tert-butyl (R)-8-(2-isopropyl-4-methylpyridin-3-yl)-10-(5-methyl-1H-indazol-4-yl)-7-oxo -3,4,7,8,13,13a-hexahydropyrazino[2',1':3,4][1,4]oxazepine[5,6,7-de]quinazoli n-2(1H)-carboxylate (346mg, 62%), a yellow solid. ES-API:[M+H]⁺=622.1.

Step 2: tert-butyl (R)-8-(2-isopropyl-4-methylpyridin-3-yl)-10-(5-methyl-1H-indazol-4-yl)-7-oxo -3,4,7,8,13,13a-hexahydropyrazino[2',1':3,4][1,4]oxazepine[5,6,7-de]quinazoli n-2(1H)-carboxylate (346mg, 0.56 mmol), 3 mL of trifluoroacetic acid and 6 mL of dichloromethane were added to a round bottom flask. The reaction was stirred at room temperature for 1 hour. The completion of the reaction was detected by LC-MS. The reaction solution was concentrated to obtain (R)-8-(2-isopropyl-4-methylpyridin-3-yl)-10-(5-methyl-1H-indazol-4-yl)-1,2,3, 4,13,13a-hexahydropyrazino[2',1':3,4][1,4]oxazepine[5,6,7-de]quinazolin-7(8 H)-one (361 mg), a yellow solid. The crude product was directly used in the next step. ES-API: [M+H]⁺=522.1.

Step 3: (R)-8-(2-isopropyl-4-methylpyridin-3-yl)-10-(5-methyl-1H-indazol-4-yl)-1,2,3, 4,13,13 a-hexahydropyrazino[2',1':3,4][1,4]oxazepine[5,6,7-de]quinazolin-7(8 H)-one (361 mg, 0.69mmol), 10 mL of dichloromethane and triethylamine (700mg, 6.9mmol) were added to a round bottom flask. The reaction was cooled to 0°C, and acrylic anhydride in dichloromethane (69 mg, 0.55 mmol, 1 mL) was dropped to the reaction solution. The reaction was stirred at 0°C for 10 minutes. 40 mL of the saturated sodium bicarbonate aqueous solution was added to the reaction solution, and the reaction solution was extracted with 20 mL of dichloromethane for 3 times. The organic phase was dried and concentrated, and the crude product was purified by Preparative HPLC to obtain the target product: (R)-2-acryloyl-8-(2-isopropyl-4-methylpyridin-3-yl)-10-(5-methyl-1H-indazol-4-yl)-1,2,3,4,13,13a-hexahydropyrazino[2',1':3,4][1,4]oxazepine[5,6,7-de]quin azolin-7(8H)-one Z18 (134mg, 35%). ES-API: [M+H]⁺=576.1. ¹H NMR (400 MHz, DMSO-*d₆*) δ 13.12 (s, 1H), 8.44 (d, *J =* 4.8 Hz, 1H), 7.55-7.39 (m, 2H), 7.25 (dd, *J* = 19.8, 6.0 Hz, 2H), 6.87 (s, 2H), 6.22 (d, *J* = 16.6 Hz, 1H), 5.85 (s, 1H), 5.78 (d, *J =* 10.4 Hz, 1H), 4.70 (d, *J =* 12.0 Hz, 3H), 4.53-4.28 (m, 2H), 4.13 (s, 2H), 3.54 (s, 2H), 2.93-2.72 (m, 1H), 2.13 (s, 3H), 2.01 (d,*J*= 5.6 Hz, 3H), 1.08 (d,*J*= 6.1 Hz, 3H), 0.97 (dd,*J*= 12.3, 6.7 Hz, 4H).

### Example 19: Preparation of Compound Z19, Z19-1 and Z19-2

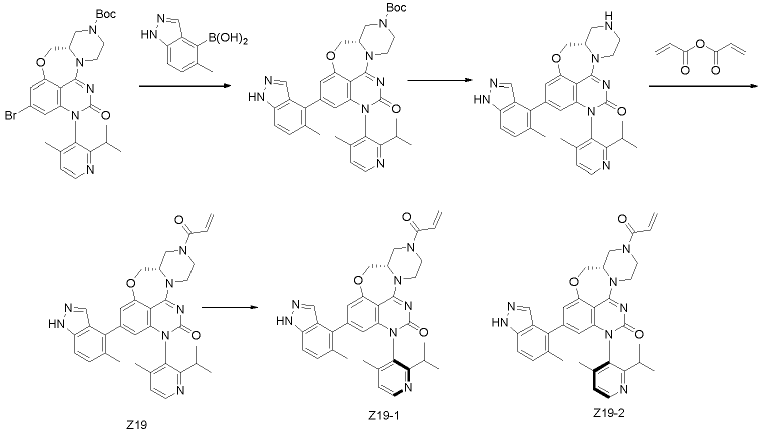

Step 1: tert-butyl (S)-10-bromo-8-(2-isopropyl-4-methylpyridin-3-yl)-7-oxo-3,4,7,8,13,13a-hexa hydropyrazino[2',1':3,4][1,4]oxazepine [5,6,7-de]quinazolin-2(1H)-carboxylate (500mg, 0.9mmol), (5-methyl-1H-indazol-4-yl)boronic acid (238mg, 1.35mmol), SPhos (37mg, 0.09 mmol), SPhos-Pd-G2 (65mg, 0.09 mmol), potassium phosphate (600mg, 2.25 mmol), 60 mL of dioxane and 12 mL of water were added to a 100mL reaction flask. The reaction was stirred at 115°C for 2 hours under nitrogen protection, and the reaction stopped. 50 mL of water was added to the reaction solution. The reaction solution was extracted with 50mL of ethyl acetate for 3 times, and the organic phase was dried and concentrated. The crude product was purified by a fast silica gel column (ethyl acetate/petroleum ether:0-100%) to obtain the product: tert-butyl (S)-8-(2-isopropyl-4-methylpyridin-3-yl)-10-(5-methyl-1H-indazol-4-yl)-7-oxo -3,4,7,8,13,13a-hexahydropyrazino[2',1':3,4][1,4]oxazepine[5,6,7-de]quinazoli n-2(1H)-carboxylate (381mg, 68%), a yellow solid. ES-API:[M+H]⁺=622.1.

Step 2: tert-butyl (S)-8-(2-isopropyl-4-methylpyridin-3-yl)-10-(5-methyl-1H-indazol-4-yl)-7-oxo -3,4,7,8,13,13a-hexahydropyrazino[2',1':3,4][1,4]oxazepine[5,6,7-de]quinazoli n-2(1H)-carboxylate (381mg, 0.61 mmol), 3 mL of trifluoroacetic acid and 6 mL of dichloromethane were added to a round bottom flask. The reaction was stirred at room temperature for 1 hour. The completion of the reaction was detected by LC-MS. The reaction solution was concentrated to obtain (S)-8-(2-isopropyl-4-methylpyridin-3-yl)-10-(5-methyl-1H-indazol-4-yl)-1,2,3, 4,13,13a-hexahydropyrazino[2',1':3,4][1,4]oxazepine[5,6,7-de]quinazolin-7(8 H)-one (401 mg), a yellow solid. The crude product was directly used in the next step. ES-API: [M+H]⁺=522.1.

Step 3: (S)-8-(2-isopropyl-4-methylpyridin-3-yl)-10-(5-methyl-1H-indazol-4-yl)-1,2,3, 4,13,13a-hexahydropyrazino[2',1':3,4][1,4]oxazepine[5,6,7-de]quinazolin-7(8 H)-one (401 mg, 0.96mmol), 10 mL of dichloromethane and triethylamine (971mg, 9.6mmol) were added to a round bottom flask. The reaction was cooled to 0°C, and a solution of acrylic anhydride in dichloromethane (97 mg, 0.77 mmol, 1 mL) was dropped to the reaction solution. The reaction was stirred at 0°C for 10 minutes. 40 mL of the saturated sodium bicarbonate aqueous solution was added to the reaction solution, and the reaction solution was extracted with 20 mL of dichloromethane for 3 times. The organic phase was dried and concentrated, and the crude product was purified by Preparative HPLC to obtain the target product: (S)-2-acryloyl-8-(2-isopropyl-4-methylpyridin-3-yl)-10-(5-methyl-1H-indazol-4-yl)-l,2,3,4,13,13a-hexahydropyrazino[2',1':3,4][1,4]oxazepine[5,6,7-de]quin azolin-7(8H)-one Z19 (248mg, 45%). ES-API: [M+H]⁺=576.1.

Step 4: the above-obtained Compound Z19 (248mg, 0.43 mmol) was resolved chirally (mobile phase: n-hexane (0.1% ammonia methanol):ethanol (0.1% ammonia methanol)=50:50); column type: AY-H (250^{∗}4.6mm 5um) ; flow rate: 1.0 ml/min; column temperature: 30°C) to obtain:
Compound Z19-1 (105mg, retention time: 8.821min, purity: 99 %, de value: 99 %). ES-API:[M+H]⁺= 576.3.
and Compound Z19-2 (115mg, retention time: 11.79min, purity:99 %, de value: 99 %). ES-API:[M+H]⁺=576.2. ¹H NMR (400 MHz, DMSO-*d₆*) δ 13.12 (s, 1H), 8.44 (d,*J =* 4.8 Hz, 1H), 7.45 (dd, *J* = 16.7, 6.5 Hz, 2H), 7.27 (d, *J* = 3.2 Hz, 1H), 7.22 (d, *J* = 8.6 Hz, 1H), 6.88 (t, *J* = 10.8 Hz, 2H), 6.21 (d, *J =* 16.3 Hz, 1H), 5.84 (d, *J* = 1.7 Hz, 1H), 5.78 (dd, *J* = 10.5, 2.4 Hz, 1H), 4.85-4.60 (m, 3H), 4.53-4.29 (m, 2H), 4.24-3.95 (m, 2H), 2.79 (ddd, *J =* 20.1, 13.5, 6.8 Hz, 1H), 2.12 (s, 3H), 2.00 (d, *J* = 5.8 Hz, 4H), 1.09-1.05 (m, 4H), 0.99-0.91 (dd, *J* = 12.0, 6.8 Hz,3H).

### Example 20: Preparation of Compound Z20, Z20-1, Z20-2, Z20-3 and Z20-4

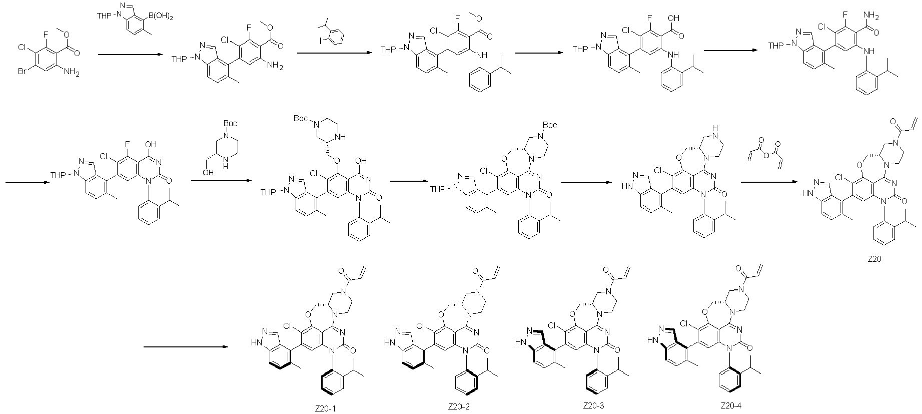

Step 1: methyl 6-amino-4-bromo-3-chloro-2-fluorobenzoate (6.3 g, 22.35 mmol), (5-methyl-1-(tetrahydro-2H-pyran-2-yl)-1H-indazol-4-yl)boronic acid (9.3 g, 35.77 mmol), 2-bicyclohexylphosphin-2',6'-dimethoxybiphenyl (734 mg, 1.78 mmol), SPhos-Pd-G2 (1.27 g, 1.78 mmol), potassium phosphate (14.2 g, 67.05 mmol), 100 mL of dioxane and 20 mL of water were added to a reaction flask. The reaction proceeded under a 90°C oil bath for 2 hours under nitrogen protection, and the reaction stopped. 100 mL of water was added to the reaction solution. The reaction solution was extracted with 100 mL of ethyl acetate for 3 times, and the organic phase was dried and concentrated. The crude product was purified by a fast silica gel column (ethyl acetate/petroleum ether:0-25%) to obtain the target product: methyl 6-amino-3-chloro-2-fluoro-4-(5-methyl-1-(tetrahydro-2H-pyran-2-yl)-1H-indaz ol-4-yl)benzoate (6.7 g), a yellow solid, purity of 58%. ES-API:[M+H]⁺=418.0.

Step 2: methyl 6-amino-3-chloro-2-fluoro-4-(5-methyl-1-(tetrahydro-2H-pyran-2-yl)-1H-indaz ol-4-yl)benzoate (6.2 g, 14.8 mmol), 1-iodo-2-isopropylbenzene (5.4 g, 22.2 mmol), XantPhos-Pd-G2 (0.92 g,1.03 mmol), 4,5-bisdiphenylphosphino-9,9-dimethylxanthene (0.60 g, 1.03 mmol), cesium carbonate (9.7 g,29.6 mmol), and 120 mL of toluene were added to a round bottom flask. The system was replaced with the nitrogen for three times, and then protected with a nitrogen ball. The reaction was stirred at 90°C for 21 hours. The completion of the reaction was detected by LC-MS. Water was added to the reaction solution. The reaction solution was extracted with ethyl acetate for 3 times, and the organic phase was dried and concentrated. The crude product was purified by a fast silica gel column (ethyl acetate/petroleum ether:0-10%) to obtain the target product: methyl 3-chloro-2-fluoro-6-(((2-isopropylphenyl)amino)-4-(5-methyl-1-(tetrahydro-2H -pyran-2-yl)-1H-indazol-4-yl)benzoate (5.55 g), yield of 70%. ES-API: [M+H]+=536.1.

Step 3: methyl 3-chloro-2-fluoro-6-(((2-isopropylphenyl)amino)-4-(5-methyl-1-(tetrahydro-2H -pyran-2-yl)-1H-indazol-4-yl)benzoate (6.5 g, 12.1 mmol), sodium hydroxide (1.5 g,36.4 mmol), methanol (50 mL), tetrahydrofuran (50 mL) and water (20 mL) were added to a round bottom flask. The reaction was stirred at 45°C for 1 hour. The completion of the reaction was detected by LC-MS. The pH of the reaction was adjusted to 8 with 6M aqueous hydrochloric acid, and then the organic solvent was concentrated and removed. Water was added, and the reaction solution was extracted with ethyl acetate for 3 times. The organic phase was dried and concentrated to obtain 3-chloro-2-fluoro-6-((2-isopropylphenyl)amino)-4-(5-methyl-1-(tetrahydro-2H-pyran-2-yl)-1H-indazol-4-yl)benzoic acid (5.9 g, 94%). ES-API: [M+H]⁺=522.1.

Step 4: 3-chloro-2-fluoro-6-((2-isopropylphenyl)amino)-4-(5-methyl-1-(tetrahydro-2H-pyran-2-yl)-1H-indazol-4-yl)benzoic acid (6.3 g,12.1 mmol), ammonium chloride (1.9 g, 36.2 mmol), 2-(7-azobenzotriazole)-N,N,N',N'-tetramethylurea hexafluorophosphate (6.9 g, 18.2 mmol), dichloromethane (40 mL), N,N-dimethylformamide (20 mL) and triethylamine (6.1 g, 60.5 mmol) were added to the round bottom flask in sequence at 0°C. The reaction was stirred at room temperature for 4 hours. Water was added to the reaction solution. The reaction solution was extracted with ethyl acetate for 3 times, and the organic phase was dried and concentrated. The crude product was purified by reversed-phase column (acetonitrile/water: 20%-100%) to obtain the target product: 3-chloro-2-fluoro-6-(((2-isopropylphenyl)amino)-4-(5-methyl-1-(tetrahydro-2H -pyran-2-yl)-1H-indazol-4-yl)benzamide (6.1 g,95%), a yellow solid. ES-API: [M+H]+=521.2.

Step 5: 60% NaH (2.3 g, 58.5 mmol) and tetrahydrofuran (60 mL) were added to a round bottom flask. The reaction was cooled to 0°C, and a solution of 3-chloro-2-fluoro-6-(((2-isopropylphenyl)amino)-4-(5-methyl-1-(tetrahydro-2H -pyran-2-yl)-1H-indazol-4-yl)benzamide (6.1 g, 11.7 mmol) in tetrahydrofuran (20 mL) was dropped thereto. The reaction was stirred at 0°C for 5 minutes, and then a solution of N,N'-carbonyl diimidazole (2.8 g,17.5 mmol) in tetrahydrofuran (40 mL) was dropped to the reaction solution. After the completion of the dropping, the reaction was stirred at 0°C for 30 minutes. The completion of the reaction was detected by LC-MS. The reaction solution was poured into icy saturated sodium bicarbonate aqueous solution. The reaction solution was extracted with ethyl acetate for 3 times. The organic phase was dried and concentrated to obtain the target product: 6-chloro-5-fluoro-4-hydroxy-1-(2-isopropylphenyl)-7 -(5-methyl-1-(tetrahydro-2H-pyran-2-yl)-1H-indazol-4-yl)quinazolin-2(1H)-one (6.2 g, 98%), a yellow solid. ES-API: [M+H]⁺=547.2.

Step 6: 60% NaH (2.3 g, 57.5mmol), tert-butyl (S)-3-(hydroxymethyl)piperazin-1-carboxylate (4 g, 18.4 mmol), tetrahydrofuran (100 mL) were added to a round bottom flask. The reaction was cooled to 0°C, and a solution of 6-chloro-5-fluoro-4-hydroxy-1-(2-isopropylphenyl)-7-(5-methyl-1-(tetrahydro-2H-pyran-2-yl)-1H-indazol-4-yl)quinazolin-2(1H)-one (6.3 g,11.5 mmol) in tetrahydrofuran (20 mL) was dropped thereto. The reaction was stirred at 0°C for 5 minutes, and then the reaction was stirred under a 65°C oil bath for 0.5 hour. The completion of the reaction was detected by LC-MS. The reaction solution was poured into icy saturated sodium bicarbonate aqueous solution. The reaction solution was extracted with ethyl acetate for 3 times. The organic phase was dried and concentrated. The crude product was purified by a fast silica gel column (methanol /dichloromethane:0-7%) to obtain the target product: tert-butyl (3S)-3-((((6-chloro-4-hydroxy-1-(2-isopropylphenyl)-7-(5-methyl-1-(tetrahydro -2H-pyran-2-yl)-1H-indazol-4-yl)-2-oxo-1,2-dihydroquinazolin-5-yl)oxy)meth ylpiperazin-1-formate (6.7g, 79%), a yellow solid. ES-API: [M+H]⁺=743.2.

Step 7: tert-butyl (3S)-3-((((6-chloro-4-hydroxy-1-(2-isopropylphenyl)-7-(5-methyl-1-(tetrahydro -2H-pyran-2-yl)-lH-indazol-4-yl)-2-oxo-1,2-dihydroquinazolin-5-yl)oxy)meth ylpiperazin-1-formate (6 g, 8.1 mmol), N,N-dimethylformamide(40 mL) and 1H-benzotriazol-1-yloxytripyrrolidinyl hexafluorophosphate (10.5 g, 20.2 mmol) were added to a round bottom flask. DBU was added all at once while stirring (6.1 g,40.4 mmol). The reaction was stirred at room temperature for 1 hour. The completion of the reaction was detected by LC-MS. The reaction was directly purified by a C18 reversed-phase column (acetonitrile /water (0.5% trifluoroacetic acid):0-80%) to obtain the target product: tert-butyl (13aS)-11-chloro-8-(2-isopropylphenyl)-10-(5-methyl-1-(tetrahydro-2H-pyran-2-yl)-1H-indazol-4-yl)-7 oxo 3,4,7,8,13,13a hexahydropyrazino[2',1':3,4][1,4]oxazepine[5,6,7-de]quinazolin-2(1H)-carboxy late (4.1 g, 70%), a yellow solid. ES-API: [M+H]⁺=589.1. ES-API:[M+H]⁺=725.2.

Step 8: tert-butyl (13aS)-11-chloro-8-(2-isopropylphenyl)-10-(5-methyl-1-(tetrahydro-2H-pyran-2-yl)-1H-indazol-4-yl)-7 oxo 3,4,7,8,13,13a hexahydropyrazino[2',1':3,4][1,4]oxazepine [5,6,7 -de]quinazolin-2(1H)-carboxy late (4.1 g, 5.6 mmol), 5 mL of dichloromethane and 20 mL of trifluoroacetic acid were added to a round bottom flask. The reaction was stirred at room temperature for 2 hours. The completion of the reaction was detected by LC-MS. The reaction solution was concentrated at 40°C to obtain the crude product: (13aS)-11-chloro-8-(2-isopropylphenyl)-10-(5-methyl-1H-indazol-4-yl )-1,2,3,4,13,13a-hexahydropyrazino[2',1':3,4][1,4]oxazepine[5,6,7-de]quinazol in-7(8H)-one (3 g), a yellow solid, directly used in the next step. ES-API: [M+H]⁺=541.2.

Step 9: (13aS)-11-chloro-8-(2-isopropylphenyl)-10-(5-methyl-1H-indazol-4-yl)-1,2,3,4 ,13,13a-hexahydropyrazino[2',1':3,4][1,4]oxazepine[5,6,7-de]quinazolin-7(8H) -one (3 g, 5.6 mmol), 20 mL of dichloromethane and triethylamine (2.8 g, 28 mmol) were added to a round bottom flask. The reaction was cooled to 0°C, and acrylic anhydride (635 mg, 5.04 mmol) was added to the reaction solution. The reaction was stirred at 0°C for 20 minutes. 100 mL of the saturated NaHCO₃ aqueous solution was added to the reaction solution, and the reaction solution was extracted with dichloromethane for 3 times. The organic phase was dried and concentrated, and the crude product was purified by Preparative HPLC to obtain the target product: Z20 (1.1 g, 33%), a white solid. ¹HNMR(400MHz, CDCl₃):7.50-7.30 (m, 4H), 7.28-7.26 (m, 2H), 7.11-7.09 (m, 1H), 6.68-6.61 (m, 1H), 6.44-6.40 (m, 1H), 6.21-6.20 (m, 1H), 5.84-5.81 (m, 1H), 5.05-5.01 (m, 1H), 4.72-4.65 (m, 3H), 4.08-3.98 (m, 2H), 3.62-3.58 (m, 1H), 3.21-3.12 (m, 2H), 2.76-2.70 (m, 1H), 2.12-2.10 (m, 3H), 1.22-1.20 (m, 3H), 0.88-0.86 (m, 3H). ES-API:[M+H]⁺=595.2.

Step 10: Compound Z20(1.1 g) was resolved chirally (column type: Chiralpak IA 250mm^{∗}4.6mm^{∗}5um; mobile phase: n-hexane: ethanol: ammonia methanol =50:50:0.2; flow rate: 1ml/min; column temperature=30 °C) to obtain:
Compound Z20-1 (151.17 mg, retention time 6.439 min; peak 1), a white solid. ¹HNMR (400MHz, CDCl₃) 7.42-7.30 (m, 4H), 7.22-7.16 (m, 2H), 7.10-7.00 (m, 1H), 6.56-6.53 (m, 1H), 6.33-6.32 (m, 1H), 6.06 (s, 1H), 5.74-5.71 (m, 1H), 4.94-4.91 (m, 1H), 4.67-4.57 (m, 3H), 4.00-3.92 (m, 2H), 3.54-3.22 (m, 3H), 2.66-2.63 (m, 1H), 2.03 (s, 3H), 0.94 (d, *J*=4Hz, 3H), 0.88 (d, *J*=4Hz, 3H). ES-API: [M+H]⁺=595.2.

Compound Z20-2 (110.33 mg; peak 4, retention time: 10.952 min), a white solid. ¹HNMR (400MHz, CDCl₃) 7.38-7.21 (m, 4H), 7.20-7.19 (m, 2H), 7.10-7.00 (m, 1H), 6.56-6.53 (m, 1H), 6.33-6.32 (m, 1H), 6.13 (s, 1H), 5.74-5.71 (m, 1H), 4.95-4.92 (m, 1H), 4.67-4.57 (m, 3H), 4.00-3.92 (m, 2H), 3.54-3.22 (m, 3H), 2.66-2.63 (m, 1H), 2.03 (s, 3H), 1.13 (d, *J*=4Hz, 3H), 0.95 (d, *J*=4Hz, 3H). ES-API: [M+H]⁺=595.2.

Compound Z20-3 (232.96 mg; peak 2, retention time:7.026 min), a white solid. ¹HNMR (400MHz, CDCl₃) 7.47-7.32 (m, 4H), 7.20-7.19 (m, 2H), 7.10-7.05 (m, 1H), 6.66-6.60 (m, 1H), 6.40-6.38 (m, 1H), 6.20 (s, 1H), 5.84-5.81 (m, 1H), 5.00-4.90 (m, 1H), 4.69-4.57 (m, 3H), 4.00-3.92 (m, 2H), 3.73-3.32 (m, 3H), 2.71-2.68 (m, 1H), 2.10 (s, 3H), 1.22 (d, *J*=4Hz, 3H), 1.01 (d, *J*=4Hz, 3H). ES-API: [M+H]⁺=595.2.
and Compound Z20-4 (196.33 mg; peak 3, retention time:8.800 min), a white solid. ¹HNMR (400MHz, CDCl₃) 7.64-7.24 (m, 6H), 7.14-7.05 (m, 1H), 6.66-6.62 (m, 1H), 6.40-6.38 (m, 1H), 6.21 (s, 1H), 5.86-5.81 (m, 1H), 5.03-4.92 (m, 1H), 4.75-4.57 (m, 3H), 4.10-4.00 (m, 2H), 3.70-3.25 (m, 3H), 2.71-2.68 (m, 1H), 2.10 (s, 3H), 1.22 (d, *J*=4Hz, 3H), 1.05 (d, *J*=4Hz, 3H). ES-API: [M+H]⁺=595.2.

### Example 21: Preparation of Compound Z21

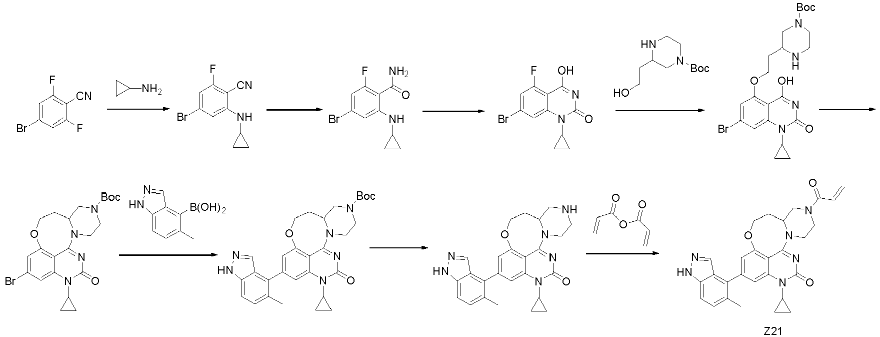

Step 1: 4-bromo-2,6-difluorobenzonitrile (50.0g, 0.229mol), cyclopropylamine (17.1 g, 1.146mol) and 500mL of isopropanol were added to a 1000 mL three-necked round bottom flask, and the reaction was stirred at 70°C for 2 hours. After the completion of the reaction, the reaction solution was cooled to room temperature, a solid precipitated out, and filtered to obtain the target product: 4-bromo-2-(cyclopropylamino)-6-fluorobenzonitrile (53g, yield: 91%) ES-API: [M+H]⁺=255.0.

Step 2: 4-bromo-2-(cyclopropylamino)-6-fluorobenzonitrile (52.0g, 0.204mol), potassium carbonate (55.5g, 0.408mol) and 500mL of dimethyl sulfoxide were added to a 1000 mL three-necked round bottom flask. 30% hydrogen peroxide (93.0g, 0.734mol) was slowly dropped at room temperature, The reaction solution was stirred for 3 hours at room temperature. 500mL of water was added to the reaction solution, a large amount of solid precipitated out, filtered to obtain the target product: 4-bromo-2-(cyclopropylamino)-6-fluorobenzamide (55g, yield: 97%) ES-API: [M+H]+=273.0.

Step 3: 4-bromo-2-(cyclopropylamino)-6-fluorobenzonitrile (13.2g,0.048mol) and 150 mL of anhydrous tetrahydrofuran were added to a 500 mL three-necked round bottom flask. The system was replaced with the nitrogen for three times, and then protected with a nitrogen ball. sodium hydride (9.62g, 0.240mol) was added at 0°C, the reaction proceeded for 0.5 hour keeping this temperature, and a solution of N,N'-carbonyl diimidazole in anhydrous tetrahydrofuran (1.44mol/L, 50mL) was quickly added. After 5min for the completion of the reaction, the reaction solution was added to ice water, extracted with ethyl acetate for three times, the organic phase was dried with anhydrous sodium sulfate and filtered, and the filtrate was concentrated to obtain the crude product. The crude product was slurried with 100mL of methyl tert-butyl ether overnight and filtered to obtain the target product: 7-bromo-1-cyclopropyl-5-fluoro-4-hydroxyquinolin-2(1H)-one (9g, yield: 63%) ES-API: [M+H]⁺=299.0s.

Step 4: tert-butyl 3-(2-hydroxyethyl)piperazin-1-formate (6.1g, 0.026mol) and 80mL of anhydrous tetrahydrofuran were added to a 250 mL three-necked round bottom flask, the system was replaced with the nitrogen for three times, and then protected with a nitrogen ball. Sodium hydride (4.5g,0.115mol) was added at 0°C, the reaction proceeded for 0.5 hour keeping this temperature, a solution of 7-bromo-1-cyclopropyl-5-fluoro-4-hydroxyquinolin-2(1H)-one in anhydrous tetrahydrofuran (1.12mol/L,20mL) was added, and the reaction proceeded at room temperature for 1.5 hours. The completion of the reaction was detected by mass spectrometry. The reaction solution was added to ice water, extracted with ethyl acetate for three times, the organic phase was dried with anhydrous sodium sulfate and filtered, and the filtrate was concentrated to obtain the target product: 3-(2-(((7-bromo-1-cyclopropyl-4-hydroxy-2-oxo -1,2-dihydroquinazolin-5-yl)oxy)ethyl)piperazine-1-formate tert-butyl (9g, yield:78%)ES-API: [M+H]⁺=509.1.

Step 5: tert-butyl 3-(2-(((7-bromo-1-cyclopropyl-4-hydroxy-2-oxo -1,2-dihydroquinazolin-5-yl)oxy)ethyl)piperazine-1-formate (3.0g, 0.006mol), 1H-benzotriazol-1-yloxytripyrrolidinyl hexafluorophosphate (9.3g, 0.018mol), 1,8-diazabicyclo[5.4.0]undec-7-ene (4.56g, 0.03mol) and 50mL of N,N-dimethylformamide were added to a 100 mL three-necked round bottom flask, and stirred at room temperature for 2 hours. 80 mL of ethyl acetate was added to the reaction solution, the reaction solution was washed with 80 mL of the saturated brine for three times. The phase in ethyl acetate was dried and concentrated, and the crude product was purified by a fast silica gel column to obtain the target product: tert-butyl 10-bromo-8-cyclopropyl-7-oxo-1,3,4,7,8,13,14,14a-octahydro-2H-pyrazino[1', 2':5,6][1,5]oxazoline[4,3,2-de]quinazolin-2-carboxylate (0.65g, yield: 23%). ES-API: [M+H]⁺=490.3.

Step 6: tert-butyl 10-bromo-8-cyclopropyl-7-oxo-1,3,4,7,8,13,14,14a-octahydro-2H-pyrazino[1', 2':5,6][1,5]oxazoline[4,3,2-de]quinazolin-2-carboxylate (0.49g, 0.001mol), (5-methyl-1H-indazol-4-yl)boronic acid (0.260g, 0.0015mol), chloro(2-dicyclohexylphosphino-2',6'-dimethoxy-1,1'-biphenyl)(2'-amino-1,1'-b iphenyl-2-yl)palladium(II) (0.072g, 0.0001mol), SPhos (0.041g, 0.0001mol), potassium phosphate (0.64g, 0.003mol), 10mL of dioxane and 2mL of water were added to a 100 mL three-necked round bottom flask. The system was replaced with the nitrogen for three times, and then protected with a nitrogen ball. The reaction proceeded at 110°C for 2 hours. 30 mL of ethyl acetate was added to the reaction solution, the reaction solution was washed with 30 mL of the saturated brine for 3 times, dried and concentrated. The crude product was purified by a fast silica gel column to obtain the target product: tert butyl 8-cyclopropyl-10-(5-methyl-1H-indazol-4-yl)-7-oxo-1,3,4,7,8,13,14,14a-octah ydro-2H-pyrazino[1',2':5,6][1,5]oxazolidine[4,3,2-de]quinazolin-2-carboxylate (550mg, yield: 83%). ES-API: [M+H]+=543.3.

Step 7: tert-butyl 8-cyclopropyl-10-(5-methyl-1H-indazol-4-yl)-7-oxo-1,3,4,7,8,13,14,14a-octah ydro-2H-pyrazino[1',2':5,6][1,5]oxazolidine[4,3,2-de]quinazolin-2-carboxylate (550 mg, 0.001mol) was dissolved in dichloromethane (8 mL), and trifluoroacetic acid (2 mL) was added. The reaction was stirred at room temperature for 2 hours, and the reaction solution was concentrated to obtain the target product: 8-cyclopropyl-10-(5-methyl-1H-indazol-4-yl)-1,3,4,13,14,14a-hexahydro-2H-p yrazino[1',2':5,6][1,5]oxazolidine[4,3,2-de]quinazolin-7(8H)-one (500 mg), the crude product was directly used in the next step. ES-API: [M+H]⁺=443.3.

Step 8: 8-cyclopropyl-10-(5-methyl-1H-indazol-4-yl)-1,3,4,13,14,14a-hexahydro-2H-p yrazino[1',2':5,6][1,5]oxazolidine[4,3,2-de]quinazolin-7(8H)-one (500mg, 0.0011 mol) was dissolved in dichloromethane (15 mL), and triethylamine (0.034g, 0.0003mol) was added. The reaction was cooled to 0°C, and acrylic chloride (138 mg, 0.0011mol) was dropped to the reaction solution. The reaction was stirred at 0°C for 15 minutes. 80 mL of dichloromethane was added to the reaction solution, the reaction solution was washed with 100mL of the saturated NaHCO₃ aqueous solution, 80mL of the saturated brine, dried and concentrated. The crude product was purified by a fast silica gel column to obtain the target product: 2-acryloyl-8-cyclopropyl-10-(5-methyl-1H-indazol-4-yl)-1,3,4,13,14,14a-hexa hydro-2H-pyrazino[1',2':5,6][1,5]oxazolidine[4,3,2-de]quinazolin-7(8H)-one Z21(80mg,yield:14%). ES-API: [M+H]⁺=497.2. ¹H NMR (500 MHz, DMSO-*d₆*) δ 13.13 (s, 1H), 7.81 (s, 1H), 7.50 (d, *J=* 8.3 Hz, 1H), 7.28 (s, 2H), 6.78 (s, 2H), 6.19 (t,*J* = 14.8 Hz, 1H), 5.75 (d, *J*= 8.9 Hz, 1H), 4.85-4.58 (m, 1H), 4.48-3.97 (m, 5H), 3.60 (d, *J* = 13.2 Hz, 1H), 3.05 (s, 2H), 2.70 (s, 1H), 2.37 (s, 3H), 2.01 (dd, *J* = 81.0, 33.5 Hz, 2H), 1.06 (d, *J* = 56.8 Hz, 2H), 0.67 (d, *J=* 72.2 Hz, 2H). ES-API: [M+H]⁺=497.2.

### Example 22: Preparation of Compound Z22

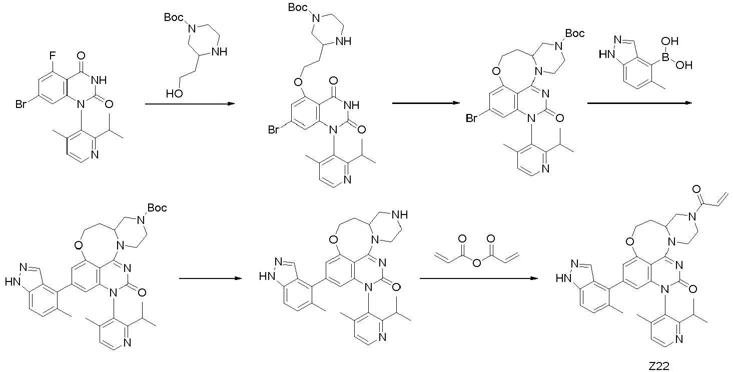

Step 1: Tert-butyl 3-(2-hydroxyethyl)piperazin-1-formate (1.76 g, 7.65 mmol) was added to a suspention of sodium hydride (404 mg, 10.20 mmol) in tetrahydrofuran (40 mL) under the ice bath, and stirred for 5 minutes. Then 7-bromo-5-fluoro-1-(2-isopropyl-4-methylpyridin-3-yl)quinazolin-2,4(1H, 3H)-dione (2 g, 5.10 mmol) was added to the reaction solution, heated to 60°C, and stirred for 2 minutes. After cooling to room temperature, the reaction solution was quenched with the saturated ammonium chloride solution (40 mL), extracted with ethyl acetate(50 mL^{∗}3), the organic phases were combined and dried, concentrated and purified by a fast silica gel column.(0-10%methanol /dichloromethane) to obtain a white solid of tert-butyl 3-(2-((7-bromo-1-(2-isopropyl-4-methylpyridin-3-yl)-2,4-dioxo-1 ,2,3,4-tetrahy droquinazolin-5-yl)oxy)ethyl)piperazin-1-carboxylate (1.5 g, yield: 44%). ES-API:[M+H]⁺=602.0.

Step 2: tert-butyl 3-(2-((7-bromo-1-(2-isopropyl-4-methylpyridin-3-yl)-2,4-dioxo-1,2,3,4-tetrahy droquinazolin-5-yl)oxy)ethyl)piperazin-1-carboxylate (1.5 g, 2.49 mmol) was dissolved in N,N-dimethylformamide (50 mL), 1, 8-diazabicycloundec-7-ene (1.89 g, 12.45 mmol) and 1H-benzotriazol-1-yloxytripyrrolidinyl hexafluorophosphate (3.89 g, 7.47 mmol) were added in sequence, and stirred for 2 hours at room temperature. The reaction solution was poured into the saturated brine (50 mL), extracted with ethyl acetate (50 mL^{∗}3), the organic phases were combined and dried, concentrated and purified by normal phase colum (0-100%acetonitrile /water (1‰ trifluoroacetic acid)) to obtain a yellow oily compound of tert-butyl 10-bromo-8-(2-isopropyl-4-methylpyridin-3-yl)-7-oxo-1,3,4,7,8,13,14,14a-octa hydro-2H-pyrazino[1',2':5,6][1,5]oxazolidine[4,3,2-de]quinazolin-2-carboxylat e (800 mg, yield: 55%). ES-API:[M+H]⁺=584.0.

Step 3: tert-butyl 10-bromo-8-(2-isopropyl-4-methylpyridin-3-yl)-7-oxo-1,3,4,7,8,13,14,14a-octa hydro-2H-pyrazino[1',2':5,6][1,5]oxazolidine[4,3,2-de]quinazolin-2-carboxylat e (200 mg, 0.34 mmol), (5-methyl-1H-indazol-4-yl)boronic acid (90 mg, 0.51 mmol), chloro(2-dicyclohexylphosphino-2',6'-dimethoxy-1,1'-biphenyl)(2'-amino-1,1'-b iphenyl-2-yl)palladium(II) (24 mg, 0.03 mmol), 2-bicyclohexylphosphin-2',6'-dimethoxybiphenyl (14 mg, 0.03 mmol) and potassium phosphate (218 mg, 1.03 mmol) in a mixture of 1,4-dioxane (4 mL) and water (1 mL) was stirred for 1 hour at 120°C under nitrogen protection. the reaction solution was dissolved in ethyl acetate (20 mL), washed with the saturated brine (10 mL^{∗}3), the obtained organic phase was dried and concentrated, and purified by a fast silica column (0-10% methanol /dichloromethane) to obtain a white solid of tert-butyl 8-(2-isopropyl-4-methylpyridin-3-yl)-10-(5 -methyl-1H-indazol-4-yl)-7-oxo-1,3 ,4,7,8,13,14,14a-octahydro-2H-pyrazino[1',2':5,6][1,5]oxazolidino[4,3,2-de]qui nazolin-2-carboxylate (120 mg, purity:44%). ES-API:[M+H]⁺=636.3.

Step 4: trifluoroacetic acid(0.5 mL) was added to a solution of tert-butyl 8-(2-isopropyl-4-methylpyridin-3-yl)-10-(5-methyl-1H-indazol-4-yl)-7-oxo-1,3 ,4,7,8,13 , 14,14a-octahydro-2H-pyrazino[1',2':5,6][1,5]oxazolidino[4,3,2-de]quinazolin-2-carboxylate (120 mg, 0.19 mmol) in dichloromethane (2 mL) under the ice bath, stirred for 2 hours at room temperature, and concentrated to obtain a yellow oily compound of 8-(2-isopropyl-4-methylpyridin-3-yl)-10-(5-methyl-1H-indazol-4-yl)-1,3,4,13,1 4,14a-hexahydro-2H-pyrazino[1',2':5,6][1,5]oxazolidine[4,3,2-de]quinazolin-7( 8H)-one, directly used in the next step. ES-API:[M+H]⁺=536.2.

Step 5: under the ice bath, triethylamine (191 mg, 1.89 mmol) was added to the above solution of the compound 8-(2-isopropyl-4-methylpyridin-3-yl)-10-(5-methyl-1H-indazol-4-yl)-1,3,4,13,1 4,14a-hexahydro-2H-pyrazino[1',2':5,6][1,5]oxazolidine[4,3,2-de]quinazolin-7( 8H)-one in dichloromethane (2 mL). After the reaction solution was clarified, acrylic anhydride (22 mg, 0.18 mmol) was dropped, and stirred for 5 minutes. The reaction solution was washed with the saturated sodium bicarbonate solution (5 mL), the organic phase was dried and concentrated, and then purified by Preparative HPLC (ammonium bicarbonate system) to obtain a white solid of Z22 (43.39 mg, purity: 100%, yield:39%). ¹H NMR (500 MHz, DMSO-*d₆*) δ 13.10 (s, 1H), 8.42 (dd, *J* = 11.3, 4.8 Hz, 1H), 7.50 (s, 1H), 7.42 (d, J = 8.6 Hz, 1H), 7.26 (t, J = 5.5 Hz, 1H), 7.21 (d, J = 8.4 Hz, 1H), 6.91-6.76 (m, 2H), 6.26-6.17 (m, 1H), 5.82-5.71 (m, 2H), 4.94-4.76 (m, 1H), 4.48 (s, 1H), 4.42-4.29 (m, 1H), 4.27-4.05 (m, 3H), 3.84-3.67 (m, 1H), 3.67-3.42 (m, 3H), 2.96-2.82 (m, 1H), 2.67-2.52 (m, 1H), 2.14 (d, J = 2.5 Hz, 3H), 2.08 (s, 1H), 1.90 (s, 2H), 1.07 (d, J = 6.6 Hz, 3H), 1.02 (d, J = 6.6 Hz, 2H), 0.85 (d, J = 6.7 Hz, 1H). ES-API: [M+H]⁺= 590.3.

### Example 23: Preparation of Compound Z23, Z23-1 and Z23-2

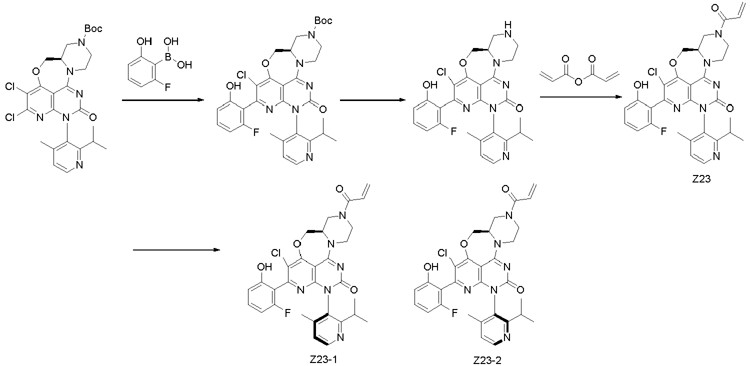

Step 1: tert-butyl (R)-2,3-dichloro-12-(2-isopropyl-4-methylpyridin-3-yl)-11-oxo-5a,6,8,9, 11,12-hexahydro-4-oxo-1,7,9a,10,12-pentazabenzo[4,5]cyclohepta[1,2,3-de]naphthal en-7(5H)-carboxylate (0.900mg, 1.607 mmol), (2-fluoro-6-hydroxyphenyl)boronic acid (752mg, 4.821 mmol), SPhos-Pd-G2 (115.7mg, 0.1607mmol), SPhos (66.0mg, 0.1607mmol), potassium phosphate (1.02g, 4.821mmol), dioxane (30mL) and water (5.0mL) were added to a single-neck flask, nitrogen was used for replacement for 3 times, and the reaction proceeded at 75°C for 0.5∼1 hour. After the completion of the reaction, 100mL of the saturated brine was added to the reaction solution, extracted with ethyl acetate twice (100mL^{∗}2). The phase in ethyl acetate was dried with anhydrous sodium sulfate, filtered, spin-dried and purified by column [dichloromethane:methanol =100:0~80:20,(V/V)] to obtain tert-butyl (5aR)-3 -chloro-2-(2- fluoro-6-hydroxyphenyl)-12-(2-isopropyl-4-methylpyridin -3-yl)-11-oxo-5a,6,8,9,11,12-hexahydro-4-oxo-1,7,9a,10,12-pentazabenzo[4,5] cyclohepta[1,2,3-de]naphthalen-7(5H)-carboxylate (0.660g, yield:58.1%). API: [M+H]⁺=637.2.

Step 2: tert-butyl (5aR)-3 -chloro-2-(2- fluoro-6-hydroxyphenyl)-12-(2-isopropyl-4-methylpyridin -3-yl)-11-oxo-5a,6,8,9,11,12-hexahydro-4-oxo-1,7,9a,10,12-pentazabenzo[4,5] cyclohepta[1,2,3-de]naphthalen-7(5H)-carboxylate (0.660g, 1.037mmol) and methanol (5mL) were added to a 100mL single-neck round bottom flask, then dioxane hydrochloride solution (3.0mL, 4M, 12.0mmol) was added, and the reaction proceeded at room temperature for 2 hours. After the completion of the reaction, the solvent was spin-dried under reduced pressure to obtain the crude product: (5aR)-3 -chloro-2-(2- fluoro-6-hydroxyphenyl)-12-(2- isopropyl-4-methylpyridin -3-yl)-5,5a,6,7,8,9-hexahydro-4-oxo-1,7,9a,10,12-pentazabenzo[4,5]cyclohepta [1,2,3-de]naphthalen-11(12H)-one (0.720g, the crude product), API: [M+H]⁺=537.2. The crude product was directly used in the next step.

Step 3: (5aR)-3-chloro-2-(2-fluoro-6-hydroxyphenyl)-12-(2-isopropyl-4-methylpyridin -3-yl)-5,5a,6,7,8,9-hexahydro-4-oxo-1,7,9a,10,12-pentazabenzo[4,5]cyclohepta [1,2,3-de]naphthalen-11(12H)-one (0.720g, 1.037mmol) was dissolved in dichloromethane (20 mL), and triethylamine (1.04g, 10.37mmol) was added. The reaction was cooled to 0°C, acrylic anhydride (117 mg, 0.933mmol) was dropped to the reaction solution. The reaction was stirred at 0°C for 15 minutes. 80 mL of dichloromethane was added to the reaction solution, the reaction solution was washed with 100mL of the saturated NaHCO₃ aqueous solution, 80mL of the saturated brine, dried and concentrated, and prepared to obtain (5aR)-7-acryloyl-3-chloro-2-(2-fluoro-6-hydroxyphenyl)-12-(2-isopropyl-4-me thylpyridin-3-yl)-5,5a,6,7,8,9-hexahydro-4-oxo-1,7,9a,10,12-pentazabenzo[4,5] cyclohepta[1,2,3-de]naphthalen-11(12H)-one Z23 (395mg,yield:65%). ES-API: [M+H]+=591.1. ¹H NMR (500 MHz, DMSO-*d*₆)δ 10.05 (t, *J =* 13.1 Hz, 1H), 9.60 (s, 1H), 8.35 (d, *J =* 4.8 Hz, 1H), 7.28-7.14 (m, 2H), 6.94-6.75 (m, 1H), 6.72-6.58 (m, 2H), 6.21 (dd, *J* = 16.7, 2.3 Hz, 1H), 5.84-5.74 (m, 1H), 4.87 (dt, *J =* 11.8, 10.7 Hz, 2H), 4.56 (s, 1H), 4.42 (d, *J =* 51.3 Hz, 1H), 4.28 (s, 1H), 3.65 (d, *J* = 13.9 Hz, 2H), 2.75 (dd, *J* = 25.2, 18.5 Hz, 1H), 1.92 (dd,*J* = 12.4, 7.7 Hz, 3H), 1.19 (dd, *J* = 13.7, 7.1 Hz, 8H).

Step 4: Compound Z23 (395 mg, 0.6695mmol) was resolved (column type: IF, 250mm^{∗}4.6mm^{∗}5um, mobile phase: n-hexane:ethanol=50:50, flow rate: 1ml/min, column temperature=30°C) to obtain:
Compound Z23-1 (106mg, peak 1, retention time:6.792 min, yield:27%), a light yellow solid, ES-API: [M+H]⁺=591.1. ¹H NMR (500 MHz, DMSO-*d*₆)δ 10.03 (s, 1H), 8.35 (d, *J =* 4.8 Hz, 1H), 7.27-7.12 (m, 2H), 6.95-6.77 (m, 1H), 6.72-6.59 (m, 2H), 6.21 (dd, *J =* 16.7, 2.3 Hz, 1H), 5.78 (dd, *J =* 10.4, 2.2 Hz, 1H), 4.87 *(*t*, J* = 20.3 Hz, 2H), 4.42 (dd, *J* = 99.0, 41.8 Hz, 3H), 4.08 (d*, J =* 41.4 Hz, 1H), 3.70 (d, *J* = 52.0 Hz, 2H), 3.36 (d, *J* = 14.7 Hz, 1H), 2.77 (dt, *J*= 13.3, 6.7 Hz, 1H), 1.91 (d, *J* = 9.3 Hz, 3H), 1.07 (d, *J* =6.9 Hz, 3H), 0.93 (dd, *J* = 6.5, 3.5 Hz, 3H).
and Z23-2 (102.7mg, peak 2, retention time: 11.512 min, yield: 26%), a light yellow solid, ES-API: [M+H]⁺=591.1. ¹H NMR (500 MHz, DMSO-*d₆*) δ 10.02 (d, *J* =30.9 Hz, 1H), 8.35 (d, *J* = 4.7 Hz, 1H), 7.28-7.11 (m, 2H), 6.95-6.79 (m, 1H), 6.66 (ddd, *J* = 24.8, 12.3, 5.9 Hz, 2H), 6.21 (dd, *J* = 16.7, 2.2 Hz, 1H), 5.78 (dd, *J* = 10.4, 2.2 Hz, 1H), 4.94-4.83 (m, 2H), 4.42 (dd, *J* = 102.4, 44.1 Hz, 3H), 4.04 (s, 1H), 3.70 (d, *J* = 35.8 Hz, 2H), 3.36 (d, *J =* 9.8 Hz, 1H), 2.70 (dt, *J*= 13.6, 6.8 Hz, 1H), 1.93 (d, *J* = 5.8 Hz, 3H), 1.07 (d, *J* = 6.9 Hz, 3H), 0.93 (dd, *J* = 6.5, 3.8 Hz, 3H).

### Example 24: Preparation of Compound Z24, Z24-1 and Z24-2

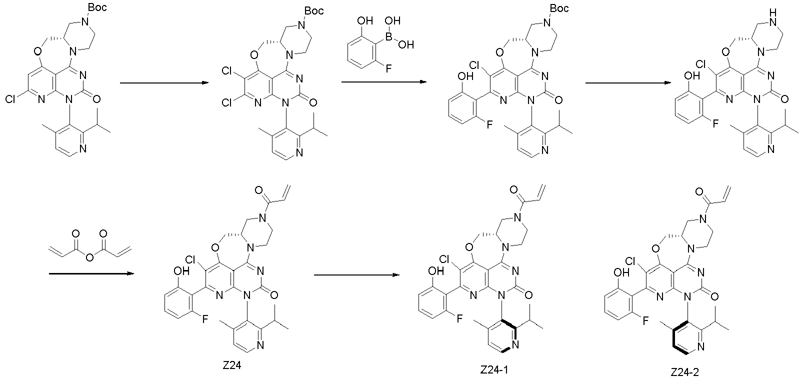

Step 1: tert-butyl (S)-2-chloro-12-(2-isopropyl-4-methylpyridin-3-yl)-11-oxo-5a,6,8,9,11,12-hex ahydro-4-oxo-1,7,9a,10,12-pentazabenzo[4,5]cyclohepta[1,2,3-de]naphthalen-7 (5H)-carboxylate (500mg, 0.95 mmol), N-chlorosuccinimide (255mg, 1.9 mmol) and acetonitrile (50 mL) were added to a round bottom flask. The reaction was stirred at 80°C for 2 hours. Sodium thiosulfate aqueous solution was added to the reaction solution. The reaction solution was extracted with ethyl acetate. The organic phase was dried and concentrated, and the crude product was purified by a fast silica gel column (methanol /dichloromethane:0-3%) to obtain the product: tert-butyl (S)-2,3-dichloro-12-(2-isopropyl-4-methylpyridin-3-yl)-11-oxo-5a,6,8,9, 11,12-hexahydro-4-oxa-1,7,9a,10,12-pentazabenzo[4,5]cyclohepta[1,2,3-de]naphthale n-7(5H)-carboxylate (533mg, the crude product), a yellow solid. ES-API: [M+H]+=562.2.

Step 2: tert-butyl (S)-2,3-dichloro-12-(2-isopropyl-4-methylpyridin-3-yl)-11-oxo-5a,6,8,9, 11,12-hexahydro-4-oxa-1,7,9a,10,12-pentazabenzo[4,5]cyclohepta[1,2,3-de]naphthale n-7(5H)-carboxylate (533 mg, 0.95mmol), (2-fluoro-6-hydroxyphenyl)boronic acid (222mg, 1.43 mmol), tetratriphenylphosphine palladium (109mg, 0.095mmol), sodium carbonate (302 mg, 2.85 mmol), 30 mL of dioxane and 6 mL of water were added to a 100mL reaction flask. The reaction was stirred at 90°C for 2 hours under nitrogen protection, and the reaction stopped. 30 mL of water was added to the reaction solution. The reaction solution was extracted with 30 mL of ethyl acetate for 3 times, the organic phase was dried and concentrated, and the crude product was purified by a fast silica gel column (ethyl acetate/petroleum ether:0-100%) to obtain the product: tert-butyl (5aS)-3-chloro-2-(2-fluoro-6-hydroxyphenyl)-12-(2-isopropyl-4-methylpyridin -3-yl)-11-oxo-5a,6,8,9,11,12-hexahydro-4-oxa-1,7,9a,10,12-pentazabenzo[4,5]c yclohepta[1,2,3-de]naphthalen-7(5H)-carboxylate (309mg, 51%), a yellow solid. ES-API:[M+H]⁺= 638.2.

Step 3: tert-butyl (5aS)-3-chloro-2-(2- fluoro-6-hydroxyphenyl)-12-(2-isopropyl-4-methylpyridin -3-yl)-11-oxo-5a,6,8,9,11,12-hexahydro-4-oxa-1,7,9a,10,12-pentazabenzo[4,5]c yclohepta[1,2,3-de]naphthalen-7(5H)-carboxylate (309mg, 0.48mmol), 1 mL of methanol and 3 mL of hydrogen chloride/dioxane solution (4 M) were added to a round bottom flask. The reaction was stirred at room temperature for 1 hour. The completion of the reaction was detected by LC-MS. The reaction solution was concentrated to obtain (5aS)-3-chloro-2-(2-fluoro-6-hydroxyphenyl)-12-(2-isopropyl-4-methylpyridin -3-yl)-5,5a,6,7,8,9-hexahydro-4-oxo-1,7,9a,10,12-pentazabenzo[4,5]cyclohepta [1,2,3-de]naphthalen-11(12H)-one (317 mg), a yellow solid. The crude product was directly used in the next step. ES-API: [M+H]⁺=538.2.

Step 4: (5aS)-3-chloro-2-(2-fluoro-6-hydroxyphenyl)-12-(2-isopropyl-4-methylpyridin -3-yl)-5,5a,6,7,8,9-hexahydro-4-oxo-1,7,9a,10,12-pentazabenzo[4,5]cyclohepta [1,2,3-de]naphthalen-11(12H)-one (317 mg, 0.59mmol), 10mL of dichloromethane and triethylamine (597 mg, 5.9mmol) were added to a round bottom flask. The reaction was cooled to 0°C, and acrylic anhydride in dichloromethane (59 mg, 0.47 mmol, 0.5 mL) was dropped to the reaction solution. The reaction was stirred at 0°C for 10 minutes. 40 mL of the saturated sodium bicarbonate aqueous solution was added to the reaction solution, and the reaction solution was extracted with 20 mL of dichloromethane for 3 times. The organic phase was dried and concentrated, and the crude product was purified by Preparative HPLC to obtain the target product: (5aS)-7-acryloyl-3-chloro-2-(2-fluoro-6-hydroxyphenyl)-12-(2-isopropyl-4-met hylpyridin-3-yl)-5,5a,6,7,8,9-hexahydro-4-oxo-1,7,9a,10,12-pentazabenzo[4,5] cyclohepta[1,2,3-de]naphthalen-11(12H)-one Z24 (108mg, 40%). ES-API:

[M+H]+=592.2.

Step 5: the above-obtained Compound Z24 (108mg, 0.18 mmol) was resolved chirally (mobile phase: n-hexane-ethanol=40-60); column type: IC (250^{∗}4.6mm 5um); flow rate: 1.0 ml/min; column temperature: 30 °C) to obtain:
Compound Z24-1 (45mg, retention time: 6.91min, purity:99 %, de value: 99 %). ES-API: [M+H]⁺= 592.3;
and Compound Z24-2 (55mg, retention time: 11.77min, purity:99 %, de value: 99 %). ES-API:[M+H]⁺= 592.2.

### Example 25: Preparation of Compound Z25, Z25-1 and Z25-2

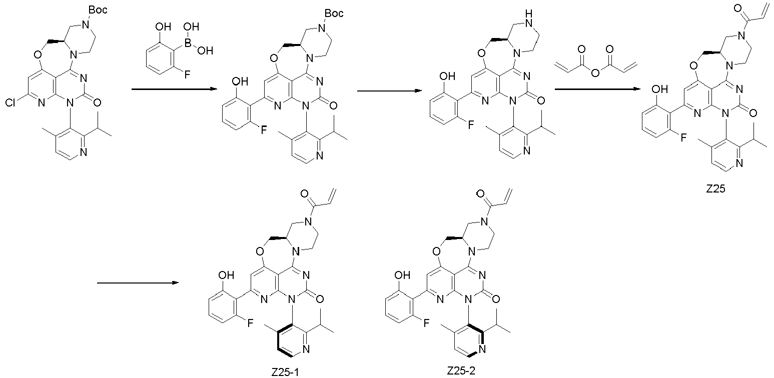

Step 1: tert-butyl (R)-2-chloro-12-(2-isopropyl-4-methylpyridin-3-yl)-11-oxo-5a,6,8,9,11,12-hex ahydro-4-oxo-1,7,9a,10,12-pentazabenzo[4,5]cyclohepta[1,2,3-de]naphthalen-7 (5H)-carboxylate (200mg, 0.3800 mmol), (2-fluoro-6-hydroxyphenyl)boronic acid (90.0mg, 0.5769 mmol), SPhos-Pd-G2 (30mg, 0.04167mmol), SPhos(20mg, 0.0487mmol), potassium phosphate (0.240g, 01.132mmol), dioxane(10mL) and water (2.0mL) were added to a single-neck flask, nitrogen was used for replacement for 3 times, and the reaction proceeded at 120°C for 0.5~1 hour. After the completion of the reaction, 100mL of the saturated brine was added to the reaction solution. The reaction solution was extracted with ethyl acetate twice (100mL ^{∗}2). The phase in ethyl acetate was dried with anhydrous sodium sulfate, filtered, spin-dried and purified by column [dichloromethane:methanol =100:0~80:20,(V/V)] to obtain tert-butyl (R)-2-(2-fluoro-6-hydroxyphenyl)-12-(2-isopropyl-4-methylpyridin-3-yl)-11-o xo-5a,6,8,9,11,12-hexahydro-4-oxo-1,7,9a,10,12-pentazabenzo[4,5]cyclohepta[ 1,2,3-de]naphthalen-7(5H)-carboxylate (0.210g, yield: 92%). API: [M+H]⁺=603.2.

Step 2: Tert-butyl (R)-2-(2-fluoro-6-hydroxyphenyl)-12-(2-isopropyl-4-methylpyridin-3-yl)-11-o xo-5a,6,8,9,11,12-hexahydro-4-oxo-1,7,9a,10,12-pentazabenzo[4,5]cyclohepta [1,2,3-de]naphthalen-7(5H)-carboxylate (0.210g, 0.3487mmol) and methanol (5mL) were added to a 100mL single-neck round bottom flask, then dioxane hydrochloride solution (3.0mL, 4M, 12.0mmol) was added, and the reaction proceeded at room temperature for 2 hours. After the completion of the reaction, the solvent was spin-dried under reduced pressure to obtain the crude product: (R)-2-(2-fluoro-6-hydroxyphenyl)-12-(2-isopropyl-4-methylpyridin-3-yl)-5,5a ,6,7,8,9-hexahydro-4-oxo-1,7,9a,10,12-pentazabenzo[4,5]cyclohepta[1,2,3-de] naphthalen-11(12H)-one (0.234g,the crude product), API:[M+H]⁺=503.2. The crude product was directly used in the next step.

Step 3: (R)-2-(2-fluoro-6-hydroxyphenyl)-12-(2-isopropyl-4-methylpyridin-3-yl)-5,5a, 6,7,8,9-hexahydro-4-oxo-1,7,9a,10,12-pentazabenzo[4,5]cyclohepta[1,2,3-de]n aphthalen-11(12H)-one (0.234g, 0.3487mmol) was dissolved in dichloromethane (15 mL), triethylamine (3.0mL, 21.62mmol) was added. The reaction was cooled to 0°C, and acrylic anhydride (39.5 mg, 0.3138mmol) was dropped to the reaction solution. The reaction was stirred at 0°C for 15 minutes. 80 mL of dichloromethane was added to the reaction solution, the reaction solution was washed with 100mL of the saturated NaHCO₃ aqueous solution, 80mL of the saturated brine, dried and concentrated, and prepared to obtain (R)-7-acryloyl-2-(2-fluoro-6-hydroxyphenyl)-12-(2-isopropyl-4-methylpyridin-3-yl)-5,5a,6,7,8,9-hexahydro-4-oxo-1,7,9a,10,12-pentazabenzo[4,5]cyclohepta[ 1,2,3-de]naphthalen-11(12H)-one Z25 (152mg, yield:78%). ES-API: [M+H]⁺=557.2.

Step 4: Compound Z25 (152mg,0.2733mmol) was resolved chirally (column type: Chiralpak IC 250mm^{∗}4.6mm 5um, mobile phase: acetonitrile :isopropanol: ammonia methanol=60:40:0.2, flow rate: 1ml/min, column temperature=30°C) to obtain:
Compound Z25-1 (21mg, yield: 13.8%), ¹H NMR (500 MHz, DMSO-*d*₆) δ 11.72 (s, 1H), 8.51 (d, *J* = 4.8 Hz, 1H), 7.37-7.20 (m, 3H), 6.95-6.81 (m, 1H), 6.72 (dd, *J* = 11.9, 8.4 Hz, 1H), 6.60 (d, *J* = 8.3 Hz, 1H), 6.21 (dd, *J* = 16.7, 2.2 Hz, 1H), 5.78 (dd, *J* = 10.4, 2.2 Hz, 1H), 4.91-4.69 (m, 2H), 4.60 (s, 1H), 4.48-4.33 (m, 1H), 4.27 (s, 1H), 4.04 (s, 1H), 3.91-3.62 (m, 2H), 3.40 (d, *J* = 10.6 Hz, 1H), 2.80 (dt, *J* = 13.4, 6.7 Hz, 1H), 1.95 (s, 3H), 1.09 (d, *J* = 6.7 Hz, 3H), 0.96 (d, *J* = 6.7 Hz, 3H).
and Compound Z25-2(19mg,12.5%), ¹H NMR(500 MHz, DMSO-*d*₆) δ 11.76 (s, 1H), 8.52 (d, *J* = 4.9 Hz, 1H), 7.35-7.21 (m, 3H), 6.85 (ddd, *J* = 27.3, 16.4, 10.3 Hz, 1H), 6.72 (dd, *J* = 12.2, 8.2 Hz, 1H), 6.60 (d, *J* = 8.4 Hz, 1H), 6.21 (dd, *J* = 16.7, 2.1 Hz, 1H), 5.81-5.73 (m, 1H), 4.86-4.71 (m, 2H), 4.59 (s, 1H), 4.39 (dd, *J* = 53.3, 12.2 Hz, 1H), 4.25 (s, 1H), 4.14-3.98 (m, 1H), 3.91-3.58 (m, 2H), 3.45-3.37 (m, 2H), 2.71 (dt, *J* = 13.3, 6.6 Hz, 1H), 1.99 (s, 3H), 1.09 (d, *J* = 6.6 Hz, 3H), 0.93 (d, *J* = 6.7 Hz, 3H).

### Example 26: Preparation of Compound Z26, Z26-1, Z26-2, Z26-2-1 and Z26-2-2

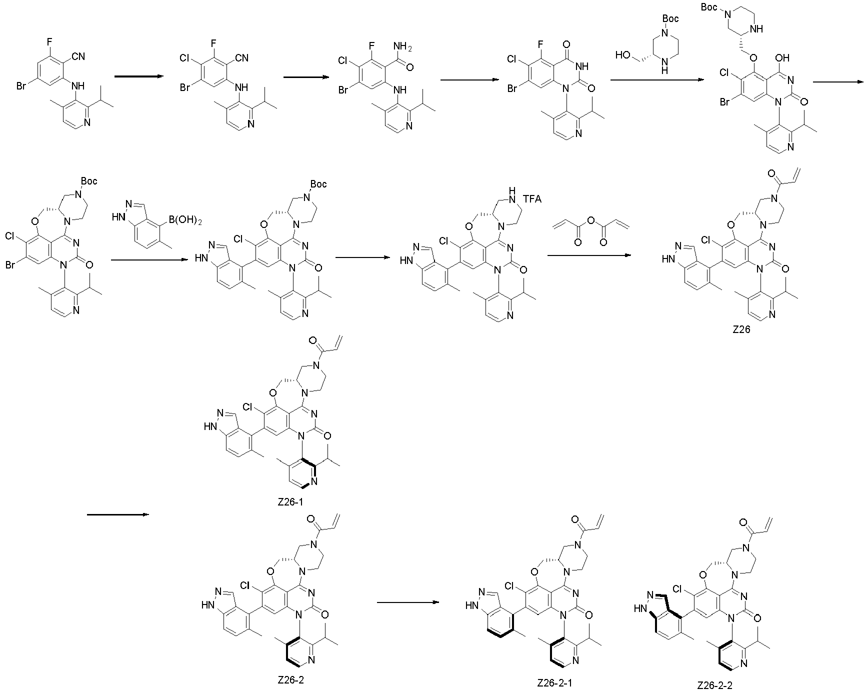

Step 1: Compound 4-bromo-2-fluoro-6-(((2-isopropyl-4-methylpyridin-3-yl)amino)benzonitrile(2. 0g, 5.6mmol) was dissolved in acetonitrile (20ml), then NCS (1.5g, 2.0eq) was added, the reaction proceeded at 80°C for four hours and completed, returned to room temperature, washed with water, extracted with ethyl acetate, and separated by column chromatography (PE:EA=10:1) to obtain the product: 4-bromo-3-chloro-2-fluoro-6-((2-isopropyl-4-methylpyridin-3-yl)amino)benzo nitrile (880mg, yield:40%). ES-API:[M+H]⁺=383.2.

Step 2: 4-bromo-3-chloro-2-fluoro-6-((2-isopropyl-4-methylpyridin-3-yl)amino)benzo nitrile (880mg,2.3 mmol) and 50 mL of dimethyl sulfoxide were added to a 250 mL three-necked round bottom flask, cooled to 0-5°C under the ice bath, potassium carbonate (634mg, 4.6mmol) and hydrogen peroxide (938mg, 30% aqueous solution , 8.28mmol) waere added, and the reaction proceeded overnight at this temperature. The completion of the reaction was detected by LCMS. The reaction solution was poured into about 15 mL of ice water, and a solid precipitated out. It was filtered to obtain filter cake. The filter cake was dried to obtain 4-bromo-3-chloro-2-fluoro-6-((2-isopropyl-4-methylpyridin-3-yl)amino)benza mide (1.01g, the crude product), a white solid, the crude product was directly used in the next step. ES-API:[M+H]⁺=401.2.

Step 3: 4-bromo-3-chloro-2-fluoro-6-((2-isopropyl-4-methylpyridin-3-yl)amino)benza mide(1.01g, 2.49 mmol) and 20 mL of dry tetrahydrofuran were added to a 250 mL three-necked round bottom flask, cooled to 0-5°C under the ice bath, sodium hydride (500mg, 12.45 mmol) was added in batches, and the reaction proceeded at this temperature for 10 minutes. A suspension of CDI (806mg, 4.98 mmol) in tetrahydrofuran (20mL) was dropped to the above solution, and then the reaction proceeded at this temperature for 20 minutes. The completion of the reaction was detected by LCMS. The reaction solution was poured into about 50 mL of ice water, and the pH was adjusted to about 4 with 3 M hydrochloric acid. The reaction solution was extracted with ethyl acetate. The organic phase was washed with the saturated brine, dried with anhydrous sodium sulfate, filtered. The filtrate was concentrated and dried to obtain 7-bromo-6-chloro-5-fluoro-1-(2-isopropyl-4-methylpyridin-3-yl)quinazolin-2,4 (1H,3H)-dione (1.1g, the crude product), a yellow solid, the crude product was directly used in the next step. ES-API: [M+H]⁺=427.3.

Step 4: tert-butyl (S)-3-(hydroxymethyl)piperazin-1-carboxylate (557mg, 2.58 mmol) was added to a suspension of 60% sodium hydride (412mg, 10.32mmol) in tetrahydrofuran (50 mL) at 0°C, the reaction proceeded at 0°C for 30 minutes, a solution of 7-bromo-6-chloro-5-fluoro-1-(2-isopropyl-4-methylpyridin-3-yl)quinazolin-2,4 (1.1 g, 2.58 mmol) in tetrahydrofuran (20 mL) was dropped thereto. The reaction was stirred at 0°C for 30 minutes. The completion of the reaction was detected by LC-MS. 50mL of ice water was added into the reaction solution. The reaction solution was extracted with ethyl acetate for 3 times. The organic phase was dried and concentrated, and the crude product was purified by a fast silica gel column (ethyl acetate/petroleum ether:20-100%) to obtain tert-butyl (S)-3-((((7-bromo-6-chloro-4-hydroxy-1-(2-isopropyl-4-methylpyridin-3-yl)-2-oxo-1,2-dihydroquinazolin-5-yl)oxy)methyl)piperazin-1-carboxylate (1.04g, 64%), a yellow solid. ES-API: [M+H]⁺=623.3.

Step 5: tert-butyl (S)-3-((((7-bromo-6-chloro-4-hydroxy-1-(2-isopropyl-4-methylpyridin-3-yl)-2-oxo-1,2-dihydroquinazolin-5-yl)oxy)methyl)piperazin-1-carboxylate (1.04g, 1.67 mmol), DBU (1.46g, 10.02 mmol), PyBop (2.59g, 6.87 mmol) dichloromethane (50 mL) were added to a round bottom flask. The reaction was stirred at room temperature for 30 minutes. The completion of the reaction was detected by LC-MS. 100 mL of dichloromethane was added to the reaction, and the organic phase was washed with 30 mL of hydrochloric acid (1M) and 100 mL of the saturated sodium bicarbonate aqueous solution in sequence. The organic phase was dried and concentrated, and the crude product was purified by a fast silica gel column (methanol /dichloromethane:0-3%) to obtain tert-butyl (S)-10-bromo-11-chloro-8-(2-isopropyl-4-methylpyridin-3-yl)-7-oxo-3,4,7,8,13 ,13a-hexahydropyrazino[2',1':3,4][1,4]oxazepine[5,6,7-de]quinazolin-2(1H)-ca rboxylate (806mg, 80%). ES-API: [M+H]⁺=605.4.

Step 6: tert-butyl (S)-10-bromo-11-chloro-8-(2-isopropyl-4-methylpyridin-3-yl)-7-oxo-3,4,7,8,13 ,13a-hexahydropyrazino[2',1':3,4][1,4]oxazepine[5,6,7-de]quinazolin-2(1H)-ca rboxylate (806mg, 1.34mmol), (5-methyl-1H-indazol-4-yl)boronic acid (353mg, 2.01mmol), SPhos (55mg, 0.134 mmol), SPhos-Pd-G2 (96mg, 0.134 mmol), potassium phosphate (852mg, 4.02 mmol), 60 mL of dioxane and 12 mL of water were added to a 100mL reaction flask. The reaction was stirred at 115°C for 2 hours under nitrogen protection, and the reaction stopped. 50 mL of water was added to the reaction solution. The reaction solution was extracted with 50mL of ethyl acetate for 3 times, the organic phase was dried and concentrated, and the crude product was purified by a fast silica gel column (ethyl acetate/petroleum ether:0-100%) to obtain the product: tert-butyl (13aS)-11-chloro-8-(2-isopropyl-4-methylpyridin-3-yl)-10-(5-methyl-1H-indaz ol-4-yl)-7-oxo-3,4,7,8,13,13a-hexahydropyrazino[2',1':3,4][1,4]oxazepine[5,6, 7-de]quinazolin-2(1H)-carboxylate (395mg, 45%), a yellow solid. ES-API:[M+H]⁺= 657.1.

Step 7: tert-butyl (13aS)-11-chloro-8-(2-isopropyl-4-methylpyridin-3-yl)-10-(5-methyl-1H-indaz ol-4-yl)-7-oxo-3,4,7,8,13,13a-hexahydropyrazino[2',1':3,4][1,4]oxazepine[5,6, 7-de]quinazolin-2(1H)-carboxylate (395mg, 0.6 mmol), 3 mL of trifluoroacetic acid and 6 mL of dichloromethane were added to a round bottom flask. The reaction was stirred at room temperature for 1 hour. The completion of the reaction was detected by LC-MS. The reaction solution was concentrated to obtain (13aS)-11-chloro-8-(2-isopropyl-4-methylpyridin-3-yl)-10-(5-methyl-1H-indaz ol-4-yl)-1,2,3,4,13,13a-hexahydropyrazino[2',1':3,4][1,4]oxazepine[5,6,7-de]q uinazolin-7(8H)-one (401 mg), a yellow solid, the crude product was directly used in the next step. ES-API: [M+H]⁺=557.1.

Step 8: (13aS)-11-chloro-8-(2-isopropyl-4-methylpyridin-3-yl)-10-(5-methyl-1H-indaz ol-4-yl)-1,2,3,4,13,13a-hexahydropyrazino[2',1':3,4][1,4]oxazepine[5,6,7-de]q uinazolin-7(8H)-one (401mg, 0.71mmol), 6 mL of dichloromethane and triethylamine (718mg, 7.1mmol) were added to a round bottom flask. The reaction was cooled to 0°C, and acrylic anhydride in dichloromethane (72 mg, 0.57 mmol, 1 mL) was dropped to the reaction solution. The reaction was stirred at 0°C for 10 minutes. 40 mL of the saturated sodium bicarbonate aqueous solution was added to the reaction solution, and the reaction solution was extracted with 20 mL of dichloromethane for 3 times. The organic phase was dried and concentrated to obtain Compound Z26.

Step 9: The crude product of Compound Z26 was purified by Preparative HPLC to obtain: Compound Z26-1 (108mg, 24%), HPLC: retention time: 6.13/6.19 min, ES-API: [M+H]⁺=611.2. and Compound Z26-2(104mg, 24%), HPLC: retention time: 6.26/6.31 min, ES-API: [M+H]⁺=611.2.

Step 10: Compound Z26-2 (106mg, 0.18mmol) was resolved chirally (mobile phase: n-hexane-ethanol-40-60); column type: IB (250mm^{∗}4.6mm 5um) ; flow rate: 1.0 ml/min; column temperature: 30 °C) to obtain: Compound Z26-2-1 (35mg, retention time: 6.824min, purity:100 %, de value: 100 %). ES-API:[M+H]⁺= 611.2. and Compound Z26-2-2 ((31mg, retention time: 9.384min, purity: 100 %, de value: 99%). ES-API:[M+H]⁺= 611.2.

### Example 27: Preparation of Compound Z27

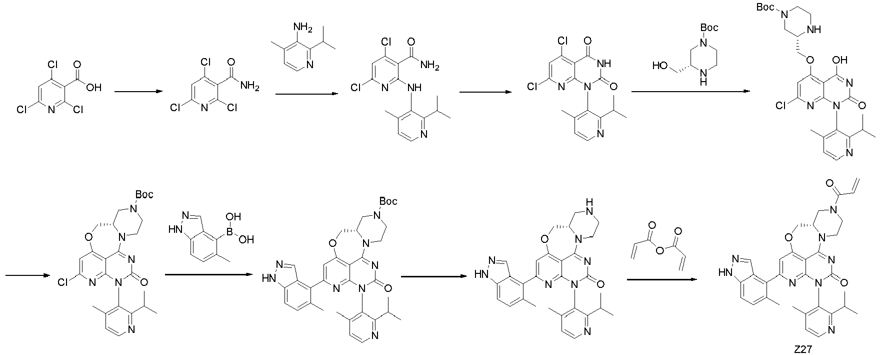

Step 1: 2,4,6-trichloronicotinic acid (8g, 35.5mmoL) was dissolved in 150mL of dichloromethane and cooled to 0°C, and oxalyl chloride (9.3ml, 106.6mmol) was added. After 30 minutes for the completion of the dropping, DMF (0.5ml) was dropped, the reaction rised to room temperature and proceeded for 1 hour. After the completion of the reaction, the reaction solution was concentrated and diluted with 150mL of dichloromethane, cooled to 0°C, 8mL of ammonia water was slowly dropped, and the reaction was stirred at room temperature for 2h. After the completion of the reaction, the reaction solution was concentrated and slurried with ethyl acetate, filtered to obtain the crude product: 2,4,6-Trichloronicotinamide 6g (6.5g, yield82%). ES-API: [M+H]⁺= 224.9.

Step 2: 2-isopropyl-4-methylpyridin-3-amine (4.4g, 29.1mmol) was dissolved in 80mL of tetrahydrofuran, LiHMDS (106.4ml, 1M) was dropped under the ice water bath under nitrogen protection, stirred for 30 minutes, 2,4,6-Trichloronicotinamide (6.5g, 29.1mmol) dissolved in 20mL of tetrahydrofuran, was added to the above reaction solution, slowly rised to room temperature, and reacted for 2 hours, cooled to room temperature, and dilute hydrochloric acid was dropped to pH of 7~8. The reaction solution was extracted with ethyl acetate, and concentrated to obtain a gray solid, slurried with ethyl acetate, and filtered to obtain the crude product 4,6-dichloro-2-((2-isopropyl-4-methylpyridin-3-yl)amino)nicotinamide (1.77g, yield18%). ES-API:[M+H]⁺= 339.0.

Step 3: 4,6-dichloro-2-((2-isopropyl-4-methylpyridin-3-yl)amino)nicotinamide (1.77g, 5.24mmoL) was dissolved in 30ml of tetrahydrofuran, 60% NaH (3g, 15.72moml) was added under the ice water bath, stirred for 15 minutes, CDI (0.63g, 7.86mmol) was added, the reaction proceeded under the ice water bath for 1 hour, raw material disappeared, the reaction solution was poured into ice water, pH was adjusted to 5-6 with dilute hydrochloric acid. The reaction solution was extracted with ethyl acetate, washed with water and the saturated brine in sequence, concentrated under reduced pressure to obtain 5,7-dichloro-1-(2-isopropyl-4-methylpyridin-3-yl)pyridine[2,3-d]pyrimidin-2,4 (1H,3H)-dione (1.72g, yield 90%). ES-API:[M+H]⁺=365.0.

Step 4: tert-butyl (S)-3-(hydroxymethyl)piperazin-1-carboxylate (1.02g, 4.72mmoL) was dissolved in tetrahydrofuran, 60% NaH (0.567g, 14.2mml) was added under the ice water bath, stirred for 10 minutes, 5,7-dichloro-1-(2-isopropyl-4-methylpyridin-3-yl)pyridine[2,3-d]pyrimidin-2,4 (1H,3H)-dione (1.72g, 4.72mmoL) was added, and stirred for 20 minutes. After the completion of the reaction, the reaction solution was poured into ice water, extracted with ethyl acetate for 3 times, the organic phases was combined, dried and concentrated under reduced pressure, purified by column chromatography (methanol /dichloromethane: 0~10%) to obtain tert-butyl (S)-3-((((7-chloro-4-hydroxy-1-(2-isopropyl-4-methylpyridin-3-yl)-2-oxo-1,2-d ihydropyrido[2,3-d]pyrimidin-5-yl)oxy)methyl)piperazin-1-formate (1.93g, yield 75%). ES-API:[M+H]⁺=545.2.

Step 5: tert-butyl (S)-3-((((7-chloro-4-hydroxy-1-(2-isopropyl-4-methylpyridin-3-yl)-2-oxo-1,2-d ihydropyrido[2,3-d]pyrimidin-5-yl)oxy)methyl)piperazin-1-formate (1.93g, 3.54mmoL) was dissolved in DMF, PyBOP (9.2 g, 17.7mmoL) and DBU (2.69 g, 17.7mmoL) was dropped under the ice water bath, and the reaction proceeded at room temperature for 2 hours. The completion of the reaction was detected. The reaction solution was poured into ice water, extracted with ethyl acetate for 3 times, the organic phases was combined, dried and concentrated under reduced pressure, purified by column chromatography (methanol /dichloromethane:0~10%) to obtain tert-butyl (S)-2-chloro-12-(2-isopropyl-4-methylpyridin-3-yl)-11-oxo-5a,6,8,9,11,12-hex ahydro-4-oxa-3,7,9a,10,12-pentazabenzo[4,5]cyclohepta[1,2,3-de]naphthalen-7 (5H)-carboxylate (0.745g, yield 40%). ES-API:[M+H]⁺= 527.2.

Step 6: tert-butyl (S)-2-chloro-12-(2-ethyl-4-methylpyridin-3-yl)-11-oxo-5a,6,8,9,11,12-hexahyd ro-4-oxo-1,7,9a,10,12-pentazabenzo[4,5]cyclohepta[1,2,3-de]naphthalen-7(5H) -carboxylate (0.745g, 1.42mmoL), (5-methyl-1H-indazol-4-yl)boronic acid (500 mg, 2.84mmol), Pd(PPh₃)₄ (164mg, 0.142mmol) and potassium carbonate (588mg, 4.26mmol) was dissolved in 8mL of dioxane and 2mL of water, nitrogen was used for replacement, the reaction proceeded at 120°C for 1.5 hours, cooled to room temperature, filtered, washed with water and the saturated brine, concentrated, column chromatography to obtain tert-butyl (S)-12-(2-isopropyl-4-methylpyridin-3-yl)-2-(5-methyl-1H-indazol-4-yl)-11-ox o-5a,6,8,9,11,12-hexahydro-4-oxo-1,7,9a,10,12-pentazabenzo[4,5]cyclohepta[1 ,2,3-de]naphthalen-7(5H)-carboxylate (371mg, yield 42%). ES-API:[M+H]⁺ =623.3.

Step 7: tert-butyl (S)-12-(2-isopropyl-4-methylpyridin-3-yl)-2-(5-methyl-1H-indazol-4-yl)-11-ox o-5a,6,8,9,11,12-hexahydro-4-oxo-1,7,9a,10,12-pentazabenzo[4,5]cyclohepta[1 ,2,3-de]naphthalen-7(5H)-carboxylate (371mg, 0.6mmoL) was dissolved in 10mL of dichloromethane, 4mL of trifluoroacetic acid was slowly dropped at room temperature, reacted for 5 hours, 10mL of dichloromethane was added, concentrated under reduced pressure to obtain (S)-12-(2-isopropyl-4-methylpyridin-3-yl)-2-(5-methyl-1H-indazol-4-yl)-5,5a,6 ,7,8,9-hexahydro-4-oxo-1,7,9a,10,12-pentazabenzo[4,5]cyclohepta[1,2,3-de]na phthalen-11(12H)-one (313mg, the crude product), directly used in the next step. ES-API:[M+H]⁺ =523.3

Step 8: (S)-12-(2-isopropyl-4-methylpyridin-3-yl)-2-(5-methyl-1H-indazol-4-yl)-5,5a,6 ,7,8,9-hexahydro-4-oxo-1,7,9a,10,12-pentazabenzo[4,5]cyclohepta[1,2,3-de]na phthalen-11(12H)-one (313mg, 0.6mmol) was dissolved in dichloromethane, triethylamine (303mg, 3 mmol) and acrylic anhydride (60mg, 0.48mmol) was dropped under the ice water bath, and stirred for 10 minutes under the ice bath. after the completion of the reaction, the reaction solution was concentrated at room temperature under reduced pressure, and purified by preparative HPLC to obtain Compound Z27(6.05mg, yield1.3%), ES-API:[M+H]⁺ =577.2.

### Example 28: Preparation of Compound Z28

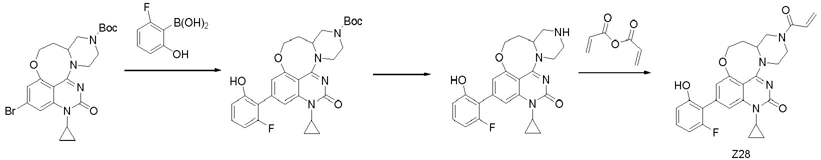

Step 1: tert-butyl 10-bromo-8-cyclopropyl-7-oxo-1,3,4,7,8,13,14,14a-octahydro-2H-pyrazino[1', 2':5,6][1,5]oxazolidine[4,3,2-de]quinazolin-2-carboxylate (100mg, 0.2mmol), (2-fluoro-6-hydroxyphenyl)boronic acid (48mg, 0.3mmol), chloro(2-dicyclohexylphosphino-2',6'-dimethoxy-1,1'-biphenyl)(2'-amino-1,1'-b iphenyl-2-yl)palladium(II) (15mg, 0.02mmol), SPhos (8.4mg, 0.02mmol), potassium phosphate (130mg, 0.6mol), 10mL of dioxane and 2mL of water were added to a 100 mL three-necked round bottom flask. The system was replaced with the nitrogen for three times, and then protected with a nitrogen ball. The reaction proceeded at 110°C for 2 hours. 30 mL of ethyl acetate was added to the reaction solution, the reaction solution was washed with 30 mL of the saturated brine for 3 times, dried and concentrated. The crude product was purified by a fast silica gel column to obtain the target product: tert-butyl 8-cyclopropyl-10-(2-fluoro-6-hydroxyphenyl)-7-oxo-1,3,4,7,8,13,14,14a-octah ydro-2H-pyrazino[1',2':5,6][1,5]oxazolidine[4,3,2-de]quinazolin-2-carboxylate (100mg, yield: 93%). ES-API: [M+H]+=523.2.

Step 2: tert-butyl 8-cyclopropyl-10-(2-fluoro-6-hydroxyphenyl)-7-oxo-1,3,4,7,8,13,14,14a-octah ydro-2H-pyrazino[1',2':5,6][1,5]oxazolidine[4,3,2-de]quinazolin-2-carboxylate (100 mg,0.19mol) was dissolved in dichloromethane (8 mL), and trifluoroacetic acid (2 mL) was added. The reaction was stirred at room temperature for 2 hours. The reaction solution was concentrated to obtain the target product: 8-cyclopropyl-10-(2-fluoro-6-hydroxyphenyl)-1,3,4,13,14,14a-hexahydro-2H-p yrazino[1',2':5,6][1,5]oxazolidine[4,3,2-de]quinazolin-7(8H)-one (70 mg, the crude product), was directly used in the next step. ES-API: [M+H]⁺=443.3.

Step 3: 8-cyclopropyl-10-(2-fluoro-6-hydroxyphenyl)-1,3,4,13,14,14a-hexahydro-2H-p yrazino[1',2':5,6][1,5]oxazolidine[4,3,2-de]quinazolin-7(8H)-one (70mg, 0.165mmol) was dissolved in dichloromethane (15 mL), triethylamine (50mg, 0.5mmol) was added. The reaction was cooled to 0°C, and acrylic chloride (21 mg, 0.016mol) was dropped to the reaction solution. The reaction was stirred at 0°C for 15 minutes. 40 mL of dichloromethane was added to the reaction solution, the reaction solution was washed with 50mL of the saturated NaHCO₃ aqueous solution, 40mL of the saturated brine, dried and concentrated. The crude product was purified by a fast silica gel column to obtain the target product: Z28 (30mg, yield:38%). ES-API: [M+H]⁺=477.1. ¹H NMR (500 MHz, DMSO-*d*₆) δ 10.07 (s, 1H), 7.36-7.12 (m, 2H), 6.98-6.63 (m, 4H), 6.17 (m*, J* = 14.3 Hz, 1H), 5.74 (d, *J* = 9.9 Hz, 1H), 4.71 (d, *J* = 33.4 Hz, 1H), 4.41-3.89 (m, 5H), 3.57 (d, *J* = 13.6 Hz, 0.5H), 3.31-3.19 (m, 2H), 3.03 (s, 0.5H), 2.64 (s, 1H), 2.13-1.80 (m, 2H), 1.18-0.99 (m, 2H), 0.78-0.44 (m, 2H). ES-API: [M+H]⁺=477.1.

### Example 29: Preparation of Compound Z29, Z29-1, Z29-2, Z29-3 and Z29-4

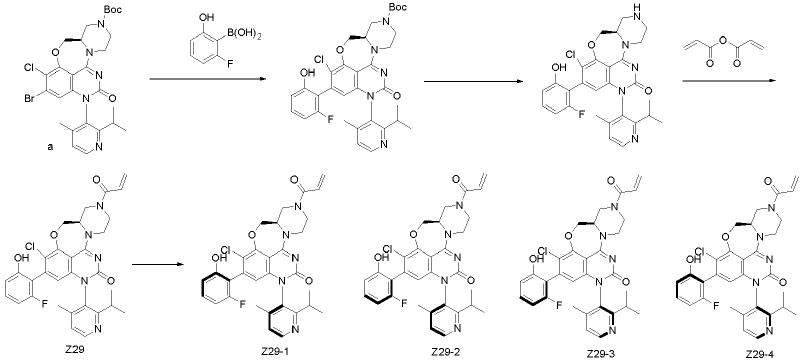

Step 1: tert-butyl (R)-10-bromo-11-chloro-8-(2-isopropyl-4-methylpyridin-3-yl)-7-oxo-3,4,7,8,1 3,13a-hexahydropyrazino[2',1':3,4][1,4]oxazepine[5,6,7-de]quinazolin-2(1H)-c arboxylate (149mg, 0.25mmol), (2-fluoro-6-hydroxyphenyl)boronic acid (59mg, 0.38mmol), SPhos (10mg, 0.025 mmol), SPhos-Pd-G2 (18mg, 0.025 mmol), potassium phosphate (159mg, 0.75mmol), 60 mL of dioxane and 12 mL of water were added to a 100mL reaction flask. The reaction was stirred at 115°C for 2 hours under nitrogen protection, and the reaction stopped. 50 mL of water was added to the reaction solution. The reaction solution was extracted with 50mL of ethyl acetate for 3 times, and the organic phase was dried and concentrated. The crude product was purified by a fast silica gel column (ethyl acetate/petroleum ether: 0-100%) to obtain the product: tert-butyl (13aR)-11-chloro-10-(2-fluoro-6-hydroxyphenyl)-8-(2-isopropyl-4-methylpyrid in-3-yl)-7-oxo-3,4,7,8,13,13a-hexahydropyrazino[2',1:3,4][1,4]oxazepine[5,6, 7-de]quinazolin-2(1H)-carboxylate (111mg, 70%), a yellow solid. ES-API:[M+H]⁺= 637.1.

Step 2: Tert-butyl (13aR)-11-chloro-10-(2-fluoro-6-hydroxyphenyl)-8-(2-isopropyl-4-methylpyrid in-3-yl)-7-oxo-3,4,7,8,13,13a-hexahydropyrazino[2',1':3,4][1,4]oxazepine[5,6, 7-de]quinazolin-2(1H)-carboxylate (111mg, 0.18mmol), 3 mL of trifluoroacetic acid and 6 mL of dichloromethane were added to a round bottom flask. The reaction was stirred at room temperature for 1 hour. The completion of the reaction was detected by LC-MS. The reaction solution was concentrated to obtain (13aR)-11-chloro-10-(2-fluoro-6-hydroxyphenyl)-8-(2-isopropyl-4-methylpyrid in-3-yl)-1,2,3,4,13,13a-hexahydropyrazino[2',1':3,4][1,4]oxazepine[5,6,7-de]q uinazolin-7(8H)-one (121mg), a yellow solid. The crude product was directly used in the next step. ES-API: [M+H]⁺=537.1.

Step 3: (13aR)-11-chloro-10-(2-fluoro-6-hydroxyphenyl)-8-(2-isopropyl-4-methylpyrid in-3-yl)-1,2,3,4,13,13a-hexahydropyrazino[2',1':3,4][1,4]oxazepine[5,6,7-de]q uinazolin-7(8H)-one (121 mg, 0.21mmol), 6 mL of dichloromethane and triethylamine (212mg, 2.1mmol) were added to a round bottom flask. The reaction was cooled to 0°C, and acrylic anhydride in dichloromethane (21 mg, 0.168 mmol, 1 mL) was dropped to the reaction solution. The reaction was stirred at 0°C for 10 minutes. 40 mL of the saturated sodium bicarbonate aqueous solution was added to the reaction solution, and the reaction solution was extracted with 20 mL of dichloromethane for 3 times. The organic phase was dried and concentrated. The crude product was purified by Preparative HPLC to obtain the target product: Z29(28mg, 23%). ES-API: [M+H]+=591.1.

Step 4: the above obtained Compound Z29 (28mg, 0.31mmol) was resolved chirally (mobile phase: n-hexane:ethanol: ammonia methanol=60:40:0.2; column type: IE (250mm^{∗}4.6mm^{∗}5um); flow rate: 1 ml/min; column temperature: 30°C) to obtain:
Compound Z29-1 (6mg, retention time: 9.598min, purity: 100 %, de value: 98 %). ES-API: [M+H]⁺= 591.1.
Compound Z29-2 (5.5mg, retention time: 10.377min, purity: 100.0%, de value: 97.1%). ES-API:[M+H]⁺= 591.1.
Compound Z29-3 (4.3mg, retention time: 12.512min, purity: 99.1%, de value: 97.5%). ES-API:[M+H]⁺= 591.1.
Compound Z29-4 (4.2mg, retention time: 13.741min, purity: 98.6%, de value: 98.2%). ES-API:[M+H]⁺= 591.1.

### Example 30: Preparation of Compound Z30, Z30-1, Z30-2, Z30-2-1 and Z30-2-2

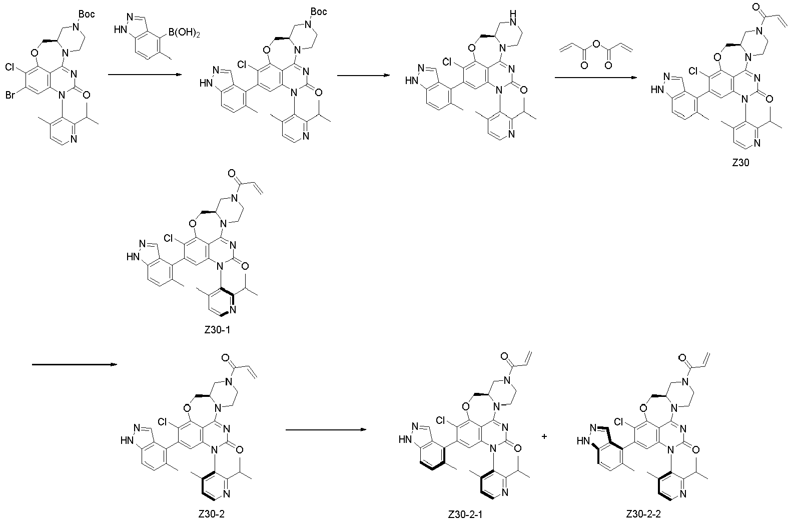

Step 1: tert-butyl (R)-10-bromo-11-chloro-8-(2-isopropyl-4-methylpyridin-3-yl)-7-oxo-3,4,7,8,1 3,13a-hexahydropyrazino[2',1':3,4][1,4]oxazepine[5,6,7-de]quinazolin-2(1H)-c arboxylate (500mg, 0.83mmol), (5-methyl-1H-indazol-4-yl)boronic acid (224mg, 1.27mmol), SPhos (34mg, 0.084 mmol), SPhos-Pd-G2 (60mg, 0.084 mmol), potassium phosphate (535mg, 2.52mmol), 60 mL of dioxane and 12 mL of water were added to a 100mL reaction flask. The reaction was stirred at 115°C for 2 hours under nitrogen protection, and the reaction stopped. 50 mL of water was added to the reaction solution. The reaction solution was extracted with 50mL of ethyl acetate for 3 times, and the organic phase was dried and concentrated. The crude product was purified by a fast silica gel column (ethyl acetate/petroleum ether: 0-100%) to obtain the product: tert-butyl (13aR)-11-chloro-8-(2-isopropyl-4-methylpyridin-3-yl)-10-(5-methyl-1H-indaz ol-4-yl)-7-oxo-3,4,7,8,13,13a-hexahydropyrazino[2',1':3,4][1,4]oxazepine[5,6, 7-de]quinazolin-2(1H)-carboxylate (418mg, 76%), a yellow solid. ES-API:[M+H]⁺= 656.1.

Step 2: tert-butyl (13aR)-11-chloro-8-(2-isopropyl-4-methylpyridin-3-yl)-10-(5-methyl-1H-indaz ol-4-yl)-7-oxo-3,4,7,8,13,13a-hexahydropyrazino[2',1':3,4][1,4]oxazepine[5,6, 7-de]quinazolin-2(1H)-carboxylate (418mg, 0.64mmol), 3 mL of trifluoroacetic acid and 6 mL of dichloromethane were added to a round bottom flask. The reaction was stirred at room temperature for 1 hour. The completion of the reaction was detected by LC-MS. The reaction solution was concentrated to obtain (13aR)-11-chloro-8-(2-isopropyl-4-methylpyridin-3-yl)-10-(5-methyl-1H-indaz ol-4-yl)-1,2,3,4,13,13a-hexahydropyrazino[2',1':3,4][1,4]oxazepine[5,6,7-de]q uinazolin-7(8H)-one (501mg), a yellow solid. The crude product was directly used in the next step. ES-API: [M+H]⁺=556.1.

Step 3: (13aR)-11-chloro-8-(2-isopropyl-4-methylpyridin-3-yl)-10-(5-methyl-1H-indaz ol-4-yl)-1,2,3,4,13,13a-hexahydropyrazino[2',1':3,4][1,4]oxazepine[5,6,7-de]q uinazolin-7(8H)-one (501mg, 0.9mmol), 6 mL of dichloromethane and triethylamine (450mg, 4.5mmol) were added to a round bottom flask. The reaction was cooled to 0°C, and acrylic anhydride in dichloromethane (91 mg, 0.72mmol, 1 mL) was dropped to the reaction solution. The reaction was stirred at 0°C for 5 minutes. 40 mL of the saturated sodium bicarbonate aqueous solution was added to the reaction solution, and the reaction solution was extracted with 20 mL of dichloromethane for 3 times. The organic phase was dried and concentrated, and the crude product was purified by Preparative HPLC to obtain the target product: Z30 (188mg, 34%). ES-API: [M+H]+=611.2.

Step 4: Compound Z30 was purified by Preparative HPLC to obtain: Compound Z30-1 (188mg, 34%), HPLC: retention time: 7.36/7.41 min, ES-API: [M+H]⁺=611.2. and Compound Z30-2 (88mg, 16%), HPLC: retention time: 7.45/7.51 min, ES-API: [M+H]⁺=611.2.

Step 5: Compound Z30-2 (88mg, 0.14mmol) was resolved chirally (mobile phase: n-heptane:ethanol (0.1% ammonia methanol)=70-30); column type: AS-H (inner diameter: 0.46cm, length: 15cm); flow rate: 0.5 ml/min; column temperature: 25°C) to obtain: Compound Z30-2-1 (36mg, retention time:4.612 min, purity: 100 %, de value: 98 %). ES-API:[M+H]⁺= 611.2; and Compound Z30-2-2((31mg, retention time: 4.861min, purity: 100 %, de value: 98%). ES-API:[M+H]⁺= 611.2.

### Example 31: Preparation of Compound Z31

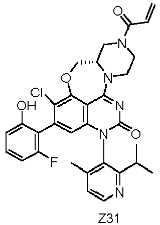

Compound Z31 was prepared with tert-butyl (S)-3-(hydroxymethyl)piperazin-1-carboxylate as the raw material according to the method of Example 29. ES-API: [M+H]⁺=591.1. ¹H NMR (500 MHz, DMSO-*d*₆), δ 10.02 (d, *J* = 2.2 Hz, 1H), 8.48 (dd, *J* = 4.8, 2.5 Hz, 1H), 7.30-7.26 (m, 1H), 7.23-7.16 (m, 1H), 6.97-6.81 (m, 1H), 6.75-6.55 (m, 2H), 6.21 (d, *J* = 16.1 Hz, 1H), 5.83 (dd, *J* = 7.4, 4.1 Hz, 1H), 5.77 (d, *J* = 12.5 Hz, 1H), 4.75 (s, 3H), 4.55-4.00 (m, 3H), 3.54 (d, *J* = 12.4 Hz, 1H), 3.14 (s, 1H), 2.88-2.65 (m, 1H), 1.96 (dd, *J* = 14.7, 8.8 Hz, 3H), 1.14-0.90 (m, 6H).

### Example 32: Preparation of Compound Z32

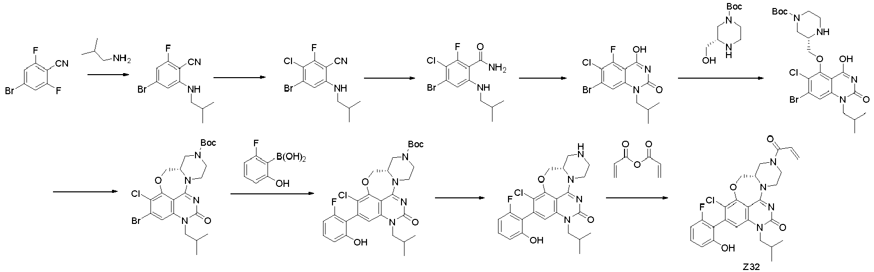

Step 1: 4-bromo-2,6-difluorobenzonitrile (10 g, 45.87 mmol) was added to isopropanol (200 mL), then 2-methylprop-1-amine (5 g, 68.8 mmol) was added, and the reaction solution was heated to 50°C to react for two hours. The completion of the reaction was detected by LCMS, iPrOH was spin-dried, 100mL of water was added. The reaction solution was extracted with ethyl acetate (100 mL^{∗}3), and the extracted organic phase was spin-dried to obtain the crude product, and the crude product was passed through a fast column (120g silica gel column, ethyl acetate/petroleum ether=20/1~10/1) to obtain the product: 4-bromo-2-fluoro-6-(isobutylamino)benzonitrile (12.7 g, yield:96.7%). ES-API:[M+1]⁺= 271.0, 273.0.

Step 2: 4-bromo-2-fluoro-6-(isobutylamino)benzonitrile (10 g, 37 mmol), was dissolved in isopropanol(100 mL), NCS (5.4 g, 40.7 mmol) was added, the reaction solution was heated to 50°C to react for 3 hours. The completion of the reaction was detected by LCMS, iPrOH was spin-dried, 100mL of water was added, then the reaction solution was extracted with ethyl acetate (100 mL^{∗}3), and the extracted organic phase was spin-dried to obtain the crude product, and the crude product was passed through a fast column (120g silica gel column,ethyl acetate/petroleum ether=20/1~10/1) to obtain the product: 4-bromo-3-chloro-2-fluoro-6-(isobutylamino)benzonitrile (3.3 g, yield:33%). ES-API:[M+1]⁺= 304.9, 306.9.

Step 3: 4-bromo-3-chloro-2-fluoro-6-(isobutylamino)benzonitrile(3.3 g, 10.8 mmol) was dissolved in DMSO (50 mL), then potassium carbonate (2.8 g, 20.4 mmol) was added, hydrogen peroxide (1.3 g, 37 mmol) was slowly dropped at room temperature, the reaction solution was stirred for 3 hours at room temperature. The completion of the reaction was detected by LCMS. Sodium sulfite (5g, 39.7 mmol) was added and stirred for 30 minutes, and then water (100 mL) was added. The reaction solution was extracted with ethyl acetate (100 mL^{∗}3), the extracted organic phase was spin-dried to obtain the crude product, and the crude product was passed through a fast column (40g silica gel column, ethyl acetate/petroleum ether=10/1∼5/1) to obtain the product: 4-bromo-3-chloro-2-fluoro-6-(isobutylamino)benzamide (3.3 g, yield: 73%). ES-API:[M+1]⁺= 322.9, 324.9.

Step 4: NaH (1.6 g, 66 mmol) was dissolved in tetrahydrofuran (40 mL) and cooled to 0°C, then 4-bromo-3-chloro-2-fluoro-6-(isobutylamino)benzamide (2.1 g, 6.6 mmol) dissolved in 10 mL of tetrahydrofuran was slowly dropped to the reaction solution, stirred for 1 hour at 0°C, then N,N'-carbonyl diimidazole (1.4 g, 8.58 mmol) dissolved in 10 mL of tetrahydrofuran was slowly dropped to the reaction solution at 0°C, then naturally warmed up to room temperature, stirred for 30 minutes. The detection of LCMS showed that the product and raw materials are about 1/1, and the detection of LCMS showed no obvious change after 30 minutes. 5 mL of the saturated ammonium chloride solution was added to quench excess NaH, then 20 mL of water was added. The reaction solution was extracted with ethyl acetate (50 mL^{∗}3), the extracted organic phase was spin-dried to obtain the crude product, and the crude product (ethyl acetate/petroleum ether=3/1) was slurried to obtain the products: 7-bromo-6-chloro-5-fluoro-4-hydroxy-1-isobutylquinazolin-2(1H)-one (1.3g, yield: 92.5%) and 4-bromo-3-chloro-2-fluoro-6-(isobutylamino)benzamide (900 mg). ES-API:[M+1]⁺= 348.9, 350.9.

Step 5: 7-bromo-6-chloro-5-fluoro-4-hydroxy-1-isobutylquinazolin-2(1H)-one (1.2 g, 3.4 mmol), tert-butyl (S)-3-(hydroxymethyl)piperazin-1-carboxylate (1.1 g, 5.1 mmol) was dissolved in tetrahydrofuran (20 mL), then NaH (410 mg, 17 mmol) was slowly added to the reaction solution, stirred at room temperature for 30 minutes, the detection of LC-MS showed the completion of the reaction, 5 mL of the saturated ammonium chloride solution was added to quench the reaction, then 20 mL of water was added, extracted with ethyl acetate (30 mL^{∗}3), the extracted organic phase was spin-dried to obtain the crude product, and the crude product was passed through a fast column (20g silica gel column, dichloromethane/methanol =100/1) to obtain the product: tert-butyl (S)-3-(((7-bromo-6-chloro-4-hydroxy-1-isobutyl-2-oxo-1,2-dihydroquinazolin-5-yl)oxy)methyl)piperazin-1-carboxylate (1.3 g, yield: 72%). ES-API:[M+1]⁺= 545.1, 547.1.

Step 6: Tert-butyl (S)-3-(((7-bromo-6-chloro-4-hydroxy-1-isobutyl-2-oxo-1,2-dihydroquinazolin -5-yl)oxy)methyl)piperazin-1-carboxylate (1.2 g, 2.2 mmol) was dissolved in N,N-dimethylformamide (20 mL), 1H-benzotriazol-1-yloxytripyrrolidinyl hexafluorophosphate (3.45 g, 6.6 mmol) was added, 1,8-diazabicyclo[5.4.0]undec-7-ene(1.67 g, 11 mmol) was slowly dropped under the ice bath, then heated to room temperature and stirred for 2 hours, the detection of LC-MS showed the completion of the reaction, 30 mL of water was added, extracted with ethyl acetate (30 mL^{∗}3), the extracted organic phase was spin-dried to obtain the crude product, and the crude product was passed through a fast column (20g silica gel column, dichloromethane/methanol =20/1) to obtain the product: tert-butyl (S)-10-bromo-11-chloro-8-isobutyl-7-oxo-3,4,7,8,13,13a-hexahydropyrazino[ 2',1':3,4][1,4]oxazepine[5,6,7-de]quinazolin-2(1H)-carboxylate (1.2 g, yield:96.7%). ES-API:[M+1]⁺= 527.1, 529.1.

Step 7: tert-butyl (S)-10-bromo-11-chloro-8-isobutyl-7-oxo-3,4,7,8,13,13a-hexahydropyrazino[2' ,1':3,4][1,4]oxazepine[5,6,7-de]quinazolin-2(1H)-carboxylate (530 mg, 1.0 mmol), (2-fluoro-6-hydroxyphenyl)boronic acid (312 mg, 2.0 mmol), tripotassium phosphate (636 mg, 6.0 mmol), chloro(2-dicyclohexylphosphino-2',6'-dimethoxy-1,1'-biphenyl)(2'-amino-1,1'-b iphenyl-2-yl)palladium(II) (72 mg, 0.1 mmol), 2-bicyclohexylphosphin-2',4',6'-triisopropylbiphenyl(40 mg, 0.1 mmol) were added to dioxane (8 mL) and water (2 mL) in sequence, nitrogen was used for replacement for three times, heated to 100°C to react for 16 hours. After cooling to room temperature, the reaction solution was poured into ethyl acetate (30 mL), washed once with brine and purified by a silica gel column (12g silica gel column, methanol:dichloromethane=0~1:20)to obtain a yellow foaming solid: tert-butyl (13aS)-11-chloro-10-(2-fluoro-6-hydroxyphenyl)-8-isobutyl-7-oxo-3,4,7,8,13,1 3a-hexahydropyrazino[2',1':3,4][1,4]oxazepine[5,6,7-de]quinazolin-2(1H)-car boxylate (370 mg, yield 60%). ES-API:[M+1]⁺= 559.2.

Step 8: tert-butyl (13aS)-11-chloro-10-(2-fluoro-6-hydroxyphenyl)-8-isobutyl-7-oxo-3,4,7,8,13,1 3a-hexahydropyrazino[2',1':3,4][1,4]oxazepine[5,6,7-de]quinazolin-2(1H)-car boxylate (370 mg, 0.67 mmol) was dissolved in dichloromethane (5 mL), trifluoroacetic acid (2 mL) was added, and reacted at room temperature for 1 hour, spin-dried to obtain a yellow oily crude product: (13aS)-11-chloro-10-(2-fluoro-6-hydroxyphenyl)-8-isobutyl1,2,3,4,13,13a hexahydropyrazino[2',1':3,4][1,4]oxazepine[5,6,7-de]quinazolin-7(8H)-one (300 mg, yield 100%).

Step 9: (13aS)-11-chloro-10-(2-fluoro-6-hydroxyphenyl)-8-isobutyl1,2,3,4,13,13a hexahydropyrazino[2',1':3,4][1,4]oxazepine[5,6,7-de]quinazolin-7(8H)-one (300 mg, 0.655 mmol), and triethylamine (2 mL) was dissolved in dichloromethane (5 mL), cooled to 0°C, acrylic anhydride (75 mg, 0.59 mmol) was added, and reacted at 0°C for 0.5 hour. The reaction solution was extracted with 20 mL of water and dichloromethane (20 mL^{∗}3), the extracted organic phase was spin-dried to obtain the crude product, and the crude product was prepared and purified (Ultimate XB-C18,50^{∗}250mm,10um, acetonitrile /water=10%/90%~90%/10%, 40 minutes) and freeze-dried to obtain the product: Z32 (102 mg, yield 38%). ES-API:[M+1]⁺= 513.1. ¹H NMR (400 MHz, DMSO-*d*₆) δ 10.07 (s, 1H), 7.26 (d, J = 8.1 Hz, 1H), 7.05 (s, 1H), 6.88-6.71 (m, 3H), 6.17 (d, J = 16.6 Hz, 1H), 5.73 (d, J = 10.5 Hz, 1H), 4.60 (s, 2H), 4.52-4.23 (m, 3H), 4.10 (d, J = 12.4 Hz, 1H), 3.94 (s, 3H), 3.42 (s, 1H), 3.16 (s, 1H), 3.00 (s, 1H), 2.03 (s, 1H), 0.84 (q, *J* = 7.8 Hz, 6H).

### Example 33: Preparation of Compound Z33

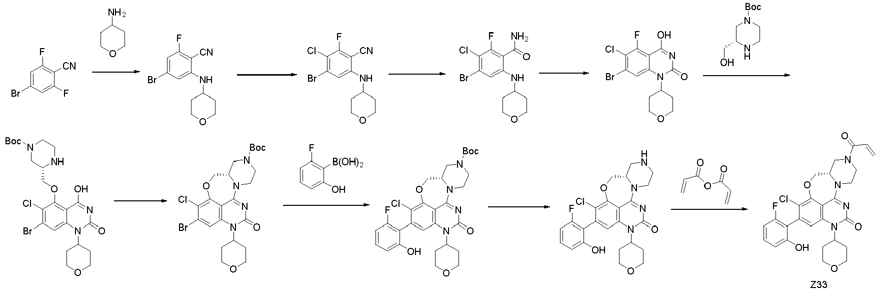

Step 1: 4-bromo-2,6-difluorobenzonitrile (10 g, 45.87 mmol) and tetrahydro-2H-pyran-4-amine (13.9 g, 137.6 mmol) was dissolved in i-PrOH (150 mL), and reacted at 70°C for 16 hours. The solvent was concentrated to obtain a crude product, the crude product was washed with water (3×100mL) to obtain 4-bromo-2-fluoro-6-((tetrahydro-2H-pyran-4-yl)amino)benzonitrile (13g, 43.48mmol, yield: 94.9%), a white solid. ES-API:[M+H]⁺= 299.0.

Step 2 : 4-bromo-2-fluoro-6-((tetrahydro-2H-pyran-4-yl)amino)benzonitrile (9 g, 30.1 mmol) was dissolved in DMF (80 mL), 1-chloropyrrolidin-2,5-dione(4.82 g, 31.62 mmol) was added in batches at 70°C. The resulting mixture reacted at 80°C for 1 hour. After coolling to room temperature, water (50mL) was added to form a solid. The solid was seperated, and washed with water (3×30 mL) to obtain 4-bromo-3-chloro-2-fluoro-6-((tetrahydro-2H-pyran-4-yl)amino)benzonitrile (7.05g, the crude product) a white solid. ES-API:[M+H]⁺= 333.1.

Step 3: 4-bromo-3-chloro-2-fluoro-6-((tetrahydro-2H-pyran-4-yl)amino)benzonitrile (7 g, 20.99 mmol) was dissolved in DMSO(100 mL), then potassium carbonate (5.82 g, 42.2 mmol) was added, and finally hydrogen peroxide (8.61 g, 75.95 mmol) was dropped at 0°C. Then, the resulting mixture was stirred at room temperature for 2 hours. The mixture was poured into ice water (50mL), and extracted with ethyl acetate (3×200mL). The organic layer was washed with brine (3×200 mL), dried with anhydrous sodium sulfate and concentrated to obtain a crude product, and the crude product was purified by a silica gel column (petroleum ether / ethyl acetate = 4:10) to obtain 4-bromo-3-chloro-2-fluoro-6-((tetrahydro-2H-pyran-4-yl)amino)benzamide(3.6 g, yield:48.78%), a white solid. ES-API:[M+H]⁺= 351.0.

Step 4: 4-bromo-3-chloro-2-fluoro-6-((tetrahydro-2H-pyran-4-yl)amino)benzamide (3.6 g, 10.24 mmol) was dissolved in tetrahydrofuran (50 mL), NaH (2.5g, 61.44mmol) was added at 0°C in batches, and reacted at 0°C for 0.5 hour. Then a solution of CDI (2.5g, 15.36mmol) in tetrahydrofuran (10mL) was dropped at 0°C, and finally the resulting mixture was stirred at 0°C for 2 hours. Ethyl acetate (100 mL) wasadded to the reaction to dilute, and the product was extracted with ethyl acetate (3×200 mL). The organic layer was washed with brine (2×100 mL), dried with anhydrous sodium sulfate and concentrated to obtain a crude product, and the crude product was washed (petroleum ether / ethyl acetate = 10:1) to obtain 7-bromo-6-chloro-5-fluoro-4-hydroxy-1-(tetrahydro-2H-pyran-4-yl)quinazolin-2(1H)-one (3.5 g, yield: 90%), a white solid. ES-API:[M+H]⁺= 377.0.

Step 5: NaH (1.8 g, 45 mmol) was added to a solution of tert-butyl (S)-3-(hydroxymethyl)piperazin-1-formate (2.92 g, 13.5 mmol) in THF (60 mL) at 0°C in batches, the resulting mixture was stirred at 0°C for 0.5 hour. Then, 7-bromo-6-chloro-5-fluoro-4-hydroxy-1-(tetrahydro-2H-pyran-4-yl)quinazolin-2(1H)-one (3.4 g, 9 mmol) was added at 0°C, and the resulting mixture was stirred at 0°C to room temperature for 2 hours. Ethyl acetate (100 mL) was added to the reaction, and the product was extracted with ethyl acetate (3×200 mL). The organic layer was washed with brine (2×100mL), dried with anhydrous sodium sulfate and concentrated to obtain a crude product, and the crude product was washed (ethyl acetate / petroleum ether = 1:3) to obtain tert-butyl (S)-3-(((7-bromo-6-chloro-4-hydroxy-2-oxo-1-(tetrahydro-2H-pyran-4-yl)-1,2-dihydroquinazolin-5-yl)oxy)methyl)piperazin-1-carboxylate (4.2g, yield= 81%), a white solid. ES-API:[M+H]⁺= 573.2.

Step 6: tert-butyl (S)-3-(((7-bromo-6-chloro-4-hydroxy-2-oxo-1-(tetrahydro-2H-pyran-4-yl)-1,2-dihydroquinazolin-5-yl)oxy)methyl)piperazin-1-carboxylate (2 g, 3.48 mmol) was dissolved in (30 mL), then carter condensing agent (3.86 g, 8.71 mmol) was added. 1, 8-diazabicycloundec-7-ene (2.65g, 17.42 mmol) was dropped at 0°C, and the resulting mixture was stirred at 0°C to room temperature for 2 hours. Ethyl acetate (100 mL) was added to the reaction, and the product was extracted with ethyl acetate (3×100mL). The organic layer was washed with brine (2×100mL), dried with anhydrous sodium sulfate and concentrated to obtain a crude product, and the crude product was purified by a silica gel column(ethyl acetate / petroleum ether = 7:3) to obtain tert-butyl (S)-10-bromo-11-chloro-7-oxo-8-(tetrahydro-2H-pyran-4-yl)-3,4,7,8,13,13a-he xahydropyrazino[2',1':3,4][1,4]oxazepine[5,6,7-de]quinazolin-2(1H)-carboxyl ate (1.68 g, yield:86.6%), a white solid. ES-API: [M+H]⁺= 555.0.

Step 7: tert-butyl (S)-10-bromo-11-chloro-7-oxo-8-(tetrahydro-2H-pyran-4-yl)-3,4,7,8,13,13a-he xahydropyrazino[2',1':3,4][1,4]oxazepine[5,6,7-de]quinazolin-2(1H)-carboxyl ate (1.3 g, 2.34 mmol), (2-fluoro-6-hydroxyphenyl)boronic acid (548 mg, 3.51 mmol), potassium phosphate (1.5 g, 7.02 mmol), SPhos (192 mg, 0.468 mmol) and SPhos-Pd-G2 (166 mg, 0.234 mmol) was dissolved in dioxane/ water (20 mL, 5:1) under nitrogen protection, and reacted at 110°C for 2 hours. After coolling to room temperature, the product was extracted with ethyl acetate (3×80 mL). The organic layer was washed with brine (3×30 mL), dried with anhydrous sodium sulfate and concentrated to obtain a crude product, and crude product was purified by rapid elution (dichloromethane / methanol = 5:1) to obtain tert-butyl (13aS)-11-chloro-10-(2-fluoro-6-hydroxyphenyl)-7-oxo-8-(tetrahydro-2H-pyra n-4-yl)-3,4,7,8,13,13a-hexahydropyrazino[2',1':3,4] [1,4]oxazepine[5,6,7-de]qu inazolin-2(1H)-carboxylate (1.37 g, yield: 99%), a white solid. ES-API:[M+H]⁺= 587.2.

Step 8: tert-butyl (13aS)-11-chloro-10-(2-fluoro-6-hydroxyphenyl)-7-oxo-8-(tetrahydro-2H-pyra n-4-yl)-3,4,7,8,13,13a-hexahydropyrazino[2',1':3,4][1,4]oxazepine[5,6,7-de]qu inazolin-2(1H)-carboxylate (500 mg, 0.85 mmol) was dissolved in dichloromethane (6.0mL) and trifluoroacetic acid (2.0 mL), and reacted at room temperature for 1 hour. The solvent was concentrated to obtain a crude product (611mg, a brown oil), it can be used in the next step without further purification. ES-API: [M+H]⁺= 487.1.

Step 9: tert-butyl (13aS)-11-chloro-10-(2-fluoro-6-hydroxyphenyl)-7-oxo-8-(tetrahydro-2H-pyra n-4-yl)-3,4,7,8,13,13a-hexahydropyrazino[2',1':3,4][1,4]oxazepine[5,6,7-de]qu inazolin-2(1H)-carboxylate (414 mg, 0.85 mmol) and triethylamine (430 mg, 4.25 mmol) was dissolved in dichloromethane (10 mL). Acrylic anhydride (107mg, 0.85mmol) in dichloromethane (1 mL) was dropped at 0°C, then the resulting mixture was stirred at 0°C for 0.5 hour. 10mL of sodium bicarbonate was added, and the product was extracted with dichloromethane (3×30mL). The organic layer was washed with sodium bicarbonate (3×10 mL), dried with anhydrous sodium sulfate and concentrated to obtain a crude product, and the crude product was purified by a reverse column (C18 spherical 20-35um 100 A) with rapid elution of acetonitrile and water (0-0, 5 min; 0-45%, 20min; 45%-45%, 7 min) to obtain Compound Z33 (127.4 mg, yield:27%), a white solid. ES-API:[M+H]⁺= 541.0. ¹H NMR (400 MHz, DMSO-*d*₆) δ 2.64-2.74 (m, 3H), 3.11 (s, 1H), 3.40-3.50 (m, 4H), 3.85-4.04 (m, 4H), 4.18-4.55 (m, 6H), 5.73 (d, *J* = 10.2 Hz, 1H), 6.16 (d, *J* = 16.6 Hz, 1H), 6.69-6.83 (m, 3H), 7.22-7.33 (m, 2H), 10.06 (s, 1H).

### Example 34: Preparation of Compound Z34

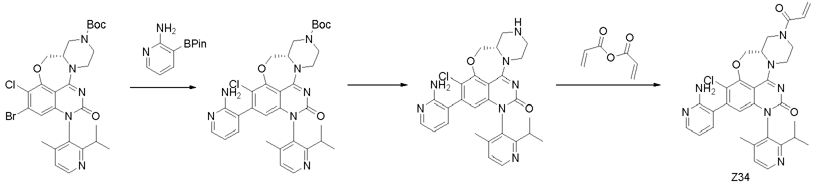

Step 1: tert-butyl (S)-10-bromo-11-chloro-8-(2-isopropyl-4-methylpyridin-3-yl)-7-oxo-3,4,7,8,13 ,13a-hexahydropyrazino[2',1':3,4][1,4]oxazepine[5,6,7-de]quinazolin-2(1H)-ca rboxylate (1.1 g, 1.82 mmol), 3-(4,4,5,5-tetramethyl-1,3,2-dioxaboran-2-yl)pyrid-2-amine (600 mg, 2.73 mmol), [1,1'-bis(diphenylphosphino)ferrocene]palladium(II) dichloride (133 mg, 0.182 mmol), potassium acetate (535 mg, 5.46 mmol) was dissolved in dioxane/water (15/3 mL), heated to 100°C and reacted for 2 hours under nitrogen protection. The reaction solution was cooled to room temperature, water, ethyl acetate were added and seperated, the organic phase was washed with brine, dried with anhydrous sodium sulfate, and spin-dried to obtain the crude product: tert-butyl (S)-10-(2-aminopyridin-3-yl)-11-chloro-8-(2-isopropyl-4-methylpyridin-3-yl)-7-oxo-3,4,7,8,13,13a-hexahydropyrazino[2',1':3,4][1,4]oxheptidine[5,6,7-de]q uinazolin-2(1H)-carboxylate (1.5 g, yield: 100%). ES-API: [M+H]⁺= 618.2.

Step 2: tert-butyl (S)-10-(2-aminopyridin-3-yl)-11-chloro-8-(2-isopropyl-4-methylpyridin-3-yl)-7-oxo-3,4,7,8,13,13a-hexahydropyrazino[2',1':3,4][1,4]oxheptidine[5,6,7-de]q uinazolin-2(1H)-carboxylate (1.5 g, 1.82 mmol) was dissolved in dichloromethane (10 mL), trifluoroacetic acid (5 mL) was added, the reaction solution was stirred at room temperature for 0.5 hour, and the solvent was concentrated to dryness under reduced pressure to obtain the crude product: (S)-10-(2-aminopyrid-3-yl)-11-chloro-8-(2-isopropyl-4-methylpyridin-3-yl)-1,2 ,3,4,13,13a-hexahydropyrazino[2',1':3,4][1,4]oxazepine[5,6,7-de]quinazolin-7( 8H)-one (1.5 g, yield: 100%). ES-API:[M+H]⁺=518.2.

Step 3: (S)-10-(2-aminopyridin-3-yl)-11-chloro-8-(2-isopropyl-4-methylpyridin-3-yl)-1 ,2,3,4,13,13a-hexahydropyrazino[2',1':3,4][1,4]oxazepine[5,6,7-de]quinazolin-7(8H)-one (1.5 g, 1.82 mmol) was dissolved in dichloromethane (100 mL), the reaction solution was cooled to 0°C, triethylamine (552 mg, 5.46 mmol) was added, acrylic anhydride (183 mg, 1.46 mmol) was dropped, and reacted at 0°C for 0.5 hour. Water was added to the reaction solution to quench the reaction, and the organic phase was washed with the saturated brine, dried with anhydrous magnesium sulfate, spin-dried, prepared by chromatography and purified, chromatographic column: Ultimate XB-C18,50^{∗}250mm,10um, mobile phase:acetonitrile /water=10/90-90/10, 40min to obtain Compound Z34 (218 mg, yield:21.0%). ES-API:[M+H]⁺= 572.2. ¹H NMR (400 MHz, DMSO-*d*₆) δ8.46 (d, J = 4.7 Hz, 1H), 7.90 (s, 1H), 7.26 (s, 1H), 6.87 (s, 1H), 6.51 (s, 1H), 6.19 (d, J = 17.1 Hz, 1H), 5.76 (d, J = 11.8 Hz, 2H), 5.60 (s, 1H), 5.53 (d, J = 10.0 Hz, 1H), 4.69 (s, 3H), 4.55-4.24 (m, 1H), 4.08 (d, J = 43.0 Hz, 1H), 3.15 (s, 1H), 2.76 (s, 1H), 1.96 (d, J = 12.4 Hz, 3H), 1.06 (d, J = 8.8 Hz, 3H), 0.98 (d, J = 6.5 Hz, 3H).

### Example 35: Preparation of Compound Z35

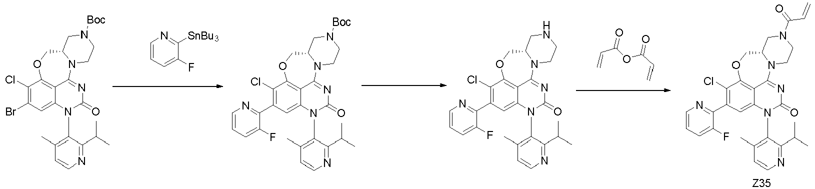

Step 1: tert-butyl (S)-10-bromo-11-chloro-8-(2-isopropyl-4-methylpyridin-3-yl)-7-oxo-3,4,7,8,13 ,13a-hexahydropyrazino[2',1':3,4][1,4]oxazepine[5,6,7-de]quinazolin-2(1H)-ca rboxylate (60 mg, 0.1 mmol), 3-fluoro-2-(tributyltinalkyl)pyridine(58 mg, 0.15 mmol), tetra(triphenylphosphine)palladium (12 mg, 0.01 mmol), lithium chloride (13 mg, 0.3 mmol), cuprous iodide (2 mg, 0.01 mmol) was dissolved in dioxane (1.5 mL), heated to 100°C and reacted for 5 hours under nitrogen protection. The reaction solution was cooled to room temperature, water, ethyl acetate was added and seperated, the organic phase was washed with brine, dried with anhydrous sodium sulfate, spin-dried, purified by thin layer chromatography (ethyl acetate: 100%) to obtain tert-butyl (S)-11-chloro-10-(3-fluoropyridin-2-yl)-8-(2-isopropyl-4-methylpyridin-3-yl)-7 -oxo-3,4,7,8,13,13a-hexahydropyrazino[2',1':3,4][1,4]oxazepine[5,6,7-de]quin azolin-2(1H)-carboxylate (30 mg, yield:48.4%). ES-API:[M+H]⁺= 621.2.

Steps 2-3: Compound Z35 was prepared according to the corresponding steps of Example 34. ES-API:[M+H]⁺= 575.2. ¹H NMR (400 MHz, DMSO-*d*₆) δ8.45 (d, J = 14.2 Hz, 2H), 7.81 (s, 1H), 7.54 (s, 1H), 7.27 (s, 1H), 6.86 (s, 0H), 6.19 (d, J = 16.5 Hz, 1H), 5.99 (s, 1H), 5.75 (d, J = 10.7 Hz, 1H), 4.74 (s, 2H), 4.33 (s, 0H), 4.12 (s, 1H), 3.54 (s, 2H), 3.14 (s, 1H), 2.76 (s, 1H), 1.95 (d, J = 10.5 Hz, 3H), 1.05 (d, J = 6.6 Hz, 3H), 0.99 (s, 3H).

### Example 36: Preparation of Compound Z36, Z36-1 and Z36-2

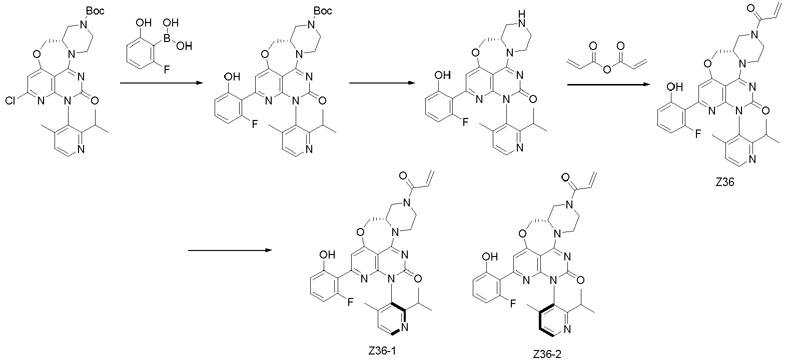

### Step 1: tert-butyl

(S)-2-chloro-12-(2-isopropyl-4-methylpyridin-3-yl)-11-oxo-5a,6,8,9,11,12-hex ahydro-4-oxo-1,7,9a,10,12-pentazabenzo[4,5]cyclohepta[1,2,3-de]naphthalen-7 (5H)-carboxylate (900 mg, 1.7 mmol), 2-fluoro-6-hydroxyphenylboronic acid(400 mg, 2.6 mmol), SPhos(53 mg, 0.13 mmol), SPhos-Pd-G2 (98 mg, 0.13 mmol), potassium phosphate (1.1 g, 5.1 mmol), 12 mL of dioxane and 3 mL of water were added to a reaction flask. The reaction was stirred under a 120°C oil bath for 1 hour under nitrogen protection. 50 mL of water was added to the reaction solution. The reaction solution was extracted with 30 mL of ethyl acetate for 3 times, and the organic phase was dried and concentrated. The crude product was purified by a fast silica gel column (methanol /dichloromethane:0-2%) to obtain tert-butyl (S)-2-(2-fluoro-6-hydroxyphenyl)-12-(2-isopropyl-4-methylpyridin-3-yl)-11-ox o-5a,6,8,9,11,12-hexahydro-4-oxo-1,7,9a,10,12-pentazabenzo[4,5]cyclohepta[1 ,2,3-de]naphthalen-7(5H)-carboxylate (1 g, purity80%). ES-API:[M+H]⁺=603.2.

Step 2: tert-butyl (S)-2-(2-fluoro-6-hydroxyphenyl)-12-(2-isopropyl-4-methylpyridin-3-yl)-11-ox o-5a,6,8,9,11,12-hexahydro-4-oxo-1,7,9a,10,12-pentazabenzo[4,5]cyclohepta[1 ,2,3-de]naphthalen-7(5H)-carboxylate (1 g, 1.66 mmol), 5 mL of methanol and 5 mL of hydrogen chloride/dioxane solution (4 *M*) were added to a round bottom flask. The reaction was stirred at room temperature for 1 hour. The completion of the reaction was detected by LC-MS. The reaction solution was concentrated to obtain the crude product: (S)-2-(2-fluoro-6-hydroxyphenyl)-12-(2-isopropyl-4-methylpyridin-3-yl)-5,5a, 6,7,8,9-hexahydro-4-oxo-1,7,9a,10,12-pentazabenzo[4,5]cyclohepta[1,2,3-de]n aphthalen-11(12H)-one (835 mg), a yellow solid. The crude product was directly used in the next step. ES-API: [M+H]⁺=503.2.

Step 3: (S)-2-(2-fluoro-6-hydroxyphenyl)-12-(2-isopropyl-4-methylpyridin-3-yl)-5,5a, 6,7,8,9-hexahydro-4-oxo-1,7,9a,10,12-pentazabenzo[4,5]cyclohepta[1,2,3-de]n aphthalen-11(12H)-one (835 mg, 1.66 mmol), 15 mL of dichloromethane and triethylamine (545 mg, 5.4 mmol) were added to a round bottom flask. The reaction was cooled to 0°C, and acrylic anhydride in dichloromethane (188 mg, 1.49 mmol) was dropped to the reaction solution. The reaction was stirred at 0°C for 10 minutes. 40 mL of the saturated sodium bicarbonate aqueous solution was added to the reaction solution, and the reaction solution was extracted with 30 mL of dichloromethane twice. The organic phase was dried and concentrated, and the crude product was purified by Preparative HPLC to obtain Z36(350 mg, 35%), a white solid. ¹H NMR (400 MHz, DMSO-*d*₆) δ 11.88-11.85 (m, 1H), 8.70 (s, 1H), 7.58-7.57 (m, 1H), 7.26-7.25 (m, 1H), 7.18-7.17 (m, 1H), 6.61-6.57 (m, 3H), 6.46-6.42 (m, 1H), 5.86-5.84 (m, 1H), 5.07-5.05 (m, 1H), 4.58-4.50 (m, 3H), 4.11-3.40 (m, 5H), 2.78-2.77 (m, 1H), 2.16-2.15 (m, 3H), 1.92-1.90(m, 3H), 1.13-1.08 (m, 6H). ES-API: [M+H]⁺=557.2.

Step 4: Compound Z36(350 mg) was resolved by a chiral column (column type: Chiralpak IB 250mm^{∗}4.6mm^{∗}5um; mobile phase: n-hexane:ethanol: ammonia methanol =50:50:0.2; flow rate:1ml/min; time=20 min) to obtain: Compound Z36-1 (121.5 mg, retention time: 8.77 min, peak 1), a white solid. HNMR (400MHz, CDCl₃) 11.84 (s, 1H), 8.72-8.71 (m, 1H), 7.57 (s, 1H), 7.27-7.20 (m, 2H), 6.69-6.43 (m, 4H), 5.87-5.84 (m, 1H), 5.02-5.00 (m, 1H), 4.57-4.50 (m, 3H), 4.13-3.67 (m, 4H), 3.40-3.38 (m, 1H), 2.85-2.83 (m, 1H), 2.16 (s, 3H), 1.28-1.17 (m, 6H). ES-API: [M+H]⁺=557.2; and Compound Z36-2 (130 mg; retention time:11.81 min, peak 2), a white solid. HNMR (400MHz, CDCl₃) 11.89 (s, 1H), 8.70-8.69 (m, 1H), 7.57 (s, 1H), 7.27-7.20 (m, 2H), 6.69-6.43 (m, 4H), 5.87-5.84 (m, 1H), 5.08-5.05 (m, 1H), 4.57-4.50 (m, 3H), 4.08-3.65 (m, 4H), 3.40-3.39 (m, 1H), 2.78-2.76 (m, 1H), 2.15 (s, 3H), 1.28-1.10 (m, 6H). ES-API: [M+H]⁺=557.2.

### Example 37: Preparation of Compound Z37, Z37-1 and Z37-2

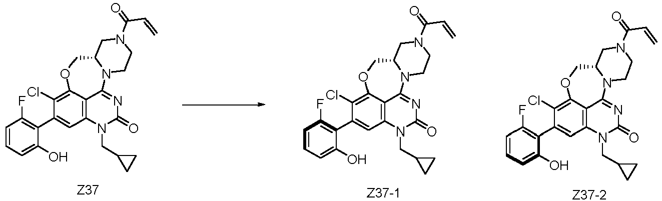

Compound Z37 was prepared with 2-cyclopropylmethyl-1-amine as the raw material according to the method of Example 32. The crude product of Compound Z37 was separated by a silica gel column (the eluent is petroleum ether/ethyl acetate/ methanol =10/20/1 ~ 5/10/1), the less polar Compound Z37-1 was collected first (TLC R_{f} value 0.32 (developing agent is petroleum ether/ethyl acetate/methanol =5/10/1), HPLC: retention time 9.686 min, 17 mg, yield 8%), ES-API:[M+H]⁺= 511.1. ¹H NMR (400 MHz, DMSO-*d*₆) δ 10.08 (s, 1H), 7.27 (d, J = 8.1 Hz, 1H),7.15 (s, 1H), 6.92-6.67 (m, 3H), 6.16 (d, J = 16.8 Hz, 1H), 5.74 (s, 1H), 4.61 (s, 2H), 4.52-4.16 (m, 3H), 4.16-3.73 (m, 4H), 3.52-3.38 (m, 2H), 1.13 (d, J = 18.8 Hz,1H), 0.38 (d, J = 7.4 Hz, 4H).

The more polar Compound Z37-2 was collected later (TLC R_{f} value 0.36 (developing agent is petroleum ether/ethyl acetate/methanol =5/10/1), HPLC: retention time 9.767 min, 18 mg, yield 8%), ES-API: [M+H]⁺= 511.1. ¹H NMR (400 MHz, DMSO-*d*₆) δ 10.08 (s, 1H), 7.27 (d, J = 8.3 Hz,1H), 7.15 (s, 1H), 6.93-6.68 (m, 3H), 6.17 (d, J = 16.4 Hz, 1H), 5.73(d, J = 9.9 Hz, 1H),4.74-4.22 (m, 5H), 4.18-3.92 (m, 4H), 3.25-2.97(m, 2H),1.10 (s, 1H), 0.38 (d, J = 7.8Hz, 4H).

### Example 38: Preparation of Compound Z38

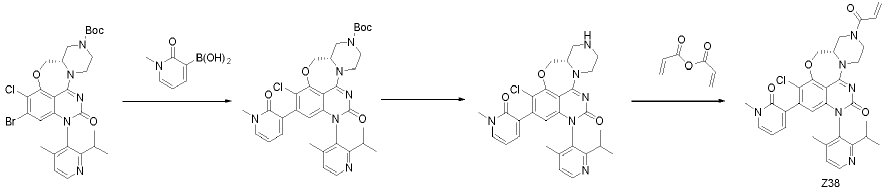

### Step 1: tert-butyl

(S)-10-bromo-11-chloro-8-(2-isopropyl-4-methylpyridin-3-yl)-7-oxo-3,4,7,8,13 ,13a-hexahydropyrazino[2'.1':3,4][1,4]oxazepine[5,6,7-de]quinazolin-2(1H)-ca rboxylate (1.1 g, 1.82 mmol), (1-methyl-2-oxo-1,2-dihydropyridin-3-yl)boronic acid (418 g, 2.73 mmol), [1,1'-bis(diphenylphosphino)ferrocene]palladium dichloride (133 mg, 0.182 mmol), potassium acetate (535 mg, 5.46 mmol) was dissolved in dioxane/water (15/5 mL), heated to 100°C and reacted for 2 hours under nitrogen protection. The reaction solution was cooled to room temperature, water, ethyl acetate were added and seperated, the organic phase was washed with brine, dried with anhydrous sodium sulfate, spin-dried to obtain the crude product: tert-butyl (S)-11-chloro-8-(2-isopropyl-4-methylpyrid-3-yl)-10-(1-methyl-2-oxo-1,2-dihy dropyrid-3-yl)-7-oxo-3,4,7,8,13,13a-hexahydropyrazino[2',1':3,4][1,4]oxazepi ne[5,6,7-de]quinazolin-2(1H)-carboxylate (1.5 g, yield: 100%). ES-API:[M+H]⁺= 633.2.

Steps 2-3: Compound Z38 was prepared according to the corresponding steps of Example 34. A chromatographic column (Ultimate XB-C18,50^{∗}250mm, 10um, mobile phase: acetonitrile /water=10/90-90/10,40min) was used for purification. ES-API:[M+H]⁺= 587.2. ¹H NMR (400 MHz, DMSO-*d*₆) δ8.50 (d, J = 4.8 Hz, 1H), 7.69 (d, J = 6.8 Hz, 1H), 7.29 (d, J = 4.7 Hz, 1H), 6.87 (q, J = 15.2 Hz, 1H), 6.18 (d, J = 16.7 Hz, 1H), 6.13-6.02 (m, 2H), 5.81-5.71 (m, 2H), 4.72 (s, 3H), 4.34 (s, 1H), 4.08 (d, J = 11.1 Hz, 1H), 3.50 (s, 1H), 3.37 (s, 3H), 3.08 (d, J = 11.8 Hz, 2H), 2.70 (dt, J = 31.2, 6.9 Hz, 1H), 1.96 (d, J = 9.0 Hz, 3H), 1.12-0.90 (m, 6H).

### Example 39: Preparation of Compound Z39

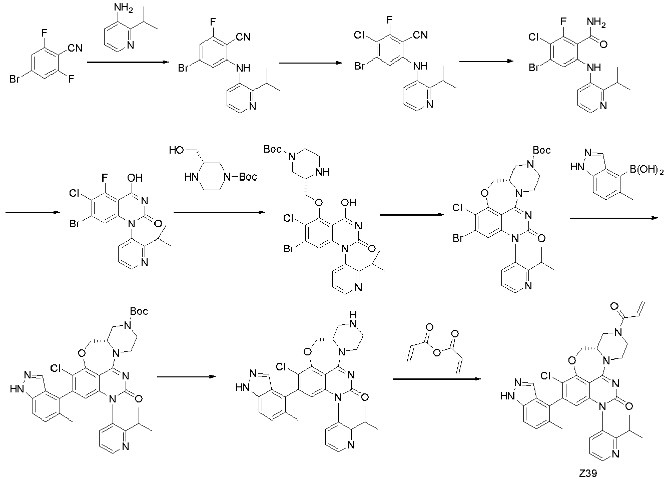

Step 1: sodium bis(trimethylsilyl)amide (42 mL) was dropped to a mixed solution of 4-bromo-2,6-difluorobenzonitrile (8.0 g, 36.70 mmol) and 2-isopropylpyridin-3-amine (5.0 g, 36.70 mmol) in tetrahydrofuran (50 mL) at -65°C, and maintained for 0.5 hour at -60°C. Tetrahydrofuran (100 mL) was added when the temperature rised to -50°C. Then the mixture slowly rised to room temperature and stirred for 3 hours. The reaction mixture was poured into ice water (20 mL) and extracted with dichloromethane (3×100 mL). the organic phases was combined, washed with the saturated brine (3×50 mL), and dried with anhydrous sodium sulfate. The solvent was spin-evaporated to be removed. The residue was purified by flash chromatography using silica gel, and eluted with a 80 g silica gel column, petroleum ether/ethyl acetate=9% to obtain 4-bromo-2-fluoro-6-((2-isopropylpyridin-3-yl)amino)benzonitrile (8.0 g, yield:65.23%), a white solid. ES-API:[M+1]⁺= 334.0.

Step 2: acetonitrile (75 mL) and deionized water (225 mL) were added to a 500 mL round bottom flask containing 4-bromo-2-fluoro-6-((2-isopropylpyridin-3-yl)amino)benzonitrile (10.0 g, 29.92 mmol), sodium chloride (3.5 g, 59.85 mmol), potassium peroxymonosulfonate (13.79 g, 22.44 mmol). The reaction mixture was heated to 70°C in an air atmosphere overnight, potassium peroxymonosulfonate (10.0 g, 16.27 mmol) was additional added, then heated to 80°C and continued to react for 6 hours. The reaction solution was extracted with dichloromethane(3×150 mL). The organic phases was combined, washed with the saturated brine (3×50 mL), and dried with anhydrous sodium sulfate. The solvent was spin-evaporated to be removed. The residue was purified by flash chromatography using silica gel, and eluted with a 120 g silica gel column, petroleum ether and ethyl acetate (3%) to obtain 4-bromo-3-chloro-2-fluoro-6-((2-isopropylpyridin-3-yl)amino)benzonitrile (2.0 g, yield: 18.13%), a white solid. ES-API:[M +1]⁺= 368.0.

Step 3: hydrogen peroxide solution (2.3 g, ω=30 %) was dropped to a mixed solution of 4-bromo-3-chloro-2-fluoro-6-((2-isopropylpyridin-3-yl)amino)benzonitrile (2.0 g, 5.43 mmol) and potassium carbonate (1.5 g, 10.85 mmol) in dimethyl sulfoxide (20 mL) at 0°C, and after the completion of the dropping, the reaction proceeded at room temperature for 2 hours. 10 % sodium thiosulfate solution (35 mL) was added at 0°C, then the mixture was poured into ice water (200 mL) and filtered with suction to obtain a filter cake, and vacuum dried to obtain a white solid of 4-bromo-3-chloro-2-fluoro-6-((2-isopropylpyridin-3-yl)amino)benzamide (1.5 g, yield:71.51 %). ES-API:[M+1]⁺= 386.0.

Step 4: sodium hydride (0.685 g, ω=60 %) was added to a mixed solution of 4-bromo-3-chloro-2-fluoro-6-((2-isopropylpyridin-3-yl)amino)benzamide(1.32 5 g, 3.43 mmol) in tetrahydrofuran (10 mL) at 0°C, and after the completion of the addition, reacted at 0°C for 1 hour. Carbonyl diimidazole (0.834 g, 5.14 mmol) dissolved in tetrahydrofuran (10 mL) was dropped to the above mixture, and maintained 0°C. After the completion of the dropping, the reaction mixture rised to room temperature and reacted for 2 hours. The mixture was poured into ice water (150 mL) and extracted with dichloromethane (3×100mL). The organic phases was combined, washed with the saturated ammonium chloride (3×50mL) and the saturated brine (3×50mL), dried with anhydrous sodium sulfate. The solvent was spin-evaporated to be removed. The residue was purified by flash chromatography using silica gel, and eluted with a 40 g silica gel column,dichloromethane and methanol (3%) to obtain 7-bromo-6-chloro-5 -fluoro-4-hydroxy-1-(2-isopropylpyridin-3-yl)quinazolin-2( 1H)-one (700 mg, 49.50 %), a white solid. ES-API:[M+1]⁺= 412.0 .

Step 5: sodium hydride (0.34 g, ω=60 %) was added to a mixed solution of 7-bromo-6-chloro-5 -fluoro-4-hydroxy-1-(2-isopropylpyridin-3-yl)quinazolin-2( 1H)-one (0.7g, 1.7 mmol) and tert-butyl (S)-3-(hydroxymethyl)piperazin-1-carboxylate (0.4 g, 1.87 mmol) in tetrahydrofuran (10 mL) at 0°C, and after the completion of the addition, reacted at 0°C for 1 hour. The mixture was poured into ice water (100 mL) and extracted with dichloromethane (3×100mL). The organic phases was combined, washed with the saturated ammonium chloride (3×30mL) and the saturated brine (3×50mL), dried with anhydrous sodium sulfate. The solvent was spin-evaporated to be removed. The residue was purified by flash chromatography using silica gel, and eluted with a 12 g silica gel column, dichloromethane and methanol (3%) to obtain tert-butyl (S)-3-(((7-bromo-6-chloro-4-hydroxy-1-(2-isopropylpyridin-3-yl)-2-oxo-1,2-di hydroquinazolin-5-yl)oxy)methyl)piperazin-1-carboxylate (0.75 g, yield: 72.61 %), a white solid. ES-API: [M +1]⁺= 608.1.

Step 6: 1H-benzotriazol-1-yloxytripyrrolidinyl hexafluorophosphate (1.54 g, 2.96 mmol) was added to a mixed solution of tert-butyl (S)-3-(((7-bromo-6-chloro-4-hydroxy-1-(2-isopropylpyridin-3-yl)-2-oxo-1,2-di hydroquinazolin-5-yl)oxy)methyl)piperazin-1-carboxylate (0.6 g, 0.985 mmol in N,N'-dimethylformamide (10 mL) at 0°C, and then 1,8-diazabicyclo[5.4.0] undec-7-ene (0.75 g, 4.93mmol) was dropped, and after addition was completed, transfered to room temperature immediately and reacted for 1 hour. The mixture was poured into ice water (100 mL) and extracted with ethyl acetate(3×30mL). The organic phases was combined, washed with the saturated ammonium chloride (3×30mL) and the saturated brine (3×50mL), dried with anhydrous sodium sulfate. The solvent was spin-evaporated to be removed. The residue was purified by flash chromatography using silica gel, and eluted with a 12 g silica gel column,dichloromethane and methanol (3%) to obtain tert-butyl (S)-10-bromo-11-chloro-8-(2-isopropylpyridin-3-yl)-7-oxo-3,4,7,8,13,13a-hexa hydropyrazino[2',1':3,4][1,4]oxazepine[5,6,7-de]quinazolin-2(1H)-carboxylate (0.4 g, yield:68.70 %), a white solid. ES-API:[M +1]⁺= 590.1 .

Step 7: tetratriphenylphosphine palladium (0.051 g, 0.044mmol) was added to a mixed solution of tert-butyl (S)-10-bromo-11-chloro-8-(2-isopropylpyridin-3-yl)-7-oxo-3,4,7,8,13,13a-hexa hydropyrazino[2',1':3,4][1,4]oxazepine[5,6,7-de]quinazolin-2(1H)-carboxylate (0.26 g,0.44mmol), (5-methyl-1H-indazol-4-yl)boronic acid (0.155 g, 0.88mmol) and sodium carbonate (0.117 g, 1.1mmol) in 1,4-dioxane(3 mL) and water (0.75mL). The resulting mixture was heated to 110°C and reacted for 1.5 hours under argon atmosphere. After coolling to room temperature, The reaction mixture was extracted with dichloromethane (3×150mL). The organic phases was combined, washed with water (1×50mL) and the saturated brine (3×50mL), dried with anhydrous sodium sulfate. The solvent was spin-evaporated to be removed. The residue was purified by flash chromatography using silica gel, and eluted with a 12 g silica gel column, dichloromethane and ethyl acetate(65%) to obtain tert-butyl (13aS)-11-chloro-8-(2-isopropylpyridin-3-yl)-10-(5-methyl-1H-indazol-4-yl)-7 -oxo-3,4,7,8,13,13a-hexahydropyrazino[2',1':3,4][1,4]oxazepine[5,6,7-de]quin azolin-2(1H)-carboxylate (200mg, yield:70.78 %), a yellow oil. ES-API:[M +1]⁺= 608.2.

Step 8: trifluoroacetic acid (1mL) was added to a solution of tert-butyl (13aS)-11-chloro-8-(2-isopropylpyridin-3-yl)-10-(5-methyl-1H-indazol-4-yl)-7 -oxo-3,4,7,8,13,13a-hexahydropyrazino[2',1':3,4][1,4]oxazepine[5,6,7-de]quin azolin-2(1H)-carboxylate (40mg, 0.062mmol) in dichloromethane (2mL). The resulting mixture was stirred at room temperature for 30 minutes. The pH of the reaction mixture was adjusted to 8 with 1M sodium bicarbonate. The reaction mixture was extracted with dichloromethane (3×20 mL). The organic phases was combined, washed with the saturated brine (3×20mL), dried with anhydrous sodium sulfate. The solvent was spin-evaporated to be removed to obtain (13aS)-11-chloro-8-(2-isopropylpyridin-3-yl)-10-(5-methyl-1H-indazol-4-yl)-1, 2,3,4,13,13a-hexahydropyrazino[2',1':3,4][1,4]oxazepine[5,6,7-de]quinazolin-7(8H)-one (30mg, yield:88.85%), a yellow oil, directly used in the next step without further purification. ES-API:[M +1]⁺= 508.1.

Step 9: triethylamine (0.056 g, 0.55 mmol) was added to a solution of (13aS)-11-chloro-8-(2-isopropylpyridin-3-yl)-10-(5-methyl-1H-indazol-4-yl)-1, 2,3,4,13,13a-hexahydropyrazino[2',1':3,4][1,4]oxazepine[5,6,7-de]quinazolin-7(8H)-one (30mg, 0.055 mmol) in dichloromethane (2 mL). The mixture was cooled to 0°C. Acrylic anhydride (6.98 mg, 0.055 mmol) was added at 0°C and stirred for 2 hours. Then the solvent was removed under reduced pressure. The residue was purified by a TLC plate to obtain Compound Z39 (2.8 mg, yield:22.71%), a white solid. ES-API:[M +1]⁺= 561.0 .¹H NMR (400 MHz, CDCl₃) δ 8.63 (s, 1H), 7.45 (d, *J* = 8.8 Hz, 3H), 6.63 (t, *J* = 13.9 Hz, 1H), 6.42 (d*, J =* 16.9 Hz, 1H), 6.12 (s, 1H), 5.83 (d, *J* = 10.8 Hz, 1H), 5.03 (d, *J* = 13.3 Hz, 1H), 4.69 (t, *J* = 17.4 Hz, 4H), 4.06 (s, 2H), 3.66-3.20 (m, 2H), 2.10 (d, *J* = 6.1 Hz, 3H), 1.09 (d, *J* = 6.6 Hz, 6H).

### Example 40: Preparation of Compound Z40

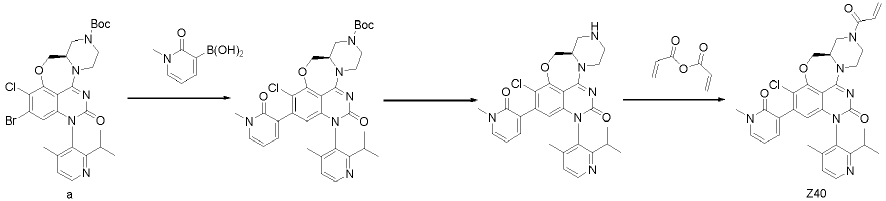

Step 1: tert-butyl (R)-10-bromo-11-chloro-8-(2-isopropyl-4-methylpyridin-3-yl)-7-oxo-3,4,7,8,1 3,13a-hexahydropyrazino[2',1':3,4][1,4]oxazepine[5,6,7-de]quinazolin-2(1H)-c arboxylate (1.0 g, 1.65 mmol), (1-methyl-2-oxo-1,2-dihydropyridin-3-yl)boronic acid (379 mg, 2.48 mmol), potassium acetate (485 mg, 4.95 mmol) and Pd (dppf)Cl₂(121 mg, 0.165 mmol) dissolved in 1,4-dioxane/water (15 mL, 5:1) was added in sequence to a 100 mL round bottom flask, and reacted at 110 °C for 1.5 hours under nitrogen protection. The product was extracted with ethyl acetate(3 * 50 mL), washed with the saturated brine (2 * 20 mL), dried with anhydrous sodium sulfate, and concentrated to obtain the crude product. The crude product was purified by a silica gel column to obtain tert-butyl (R)-11-chloro-8-(2-isopropyl-4-methylpyridin-3-yl)-10-(1-methyl-2-oxo-1,2-di hydropyridin-3-yl)-7-oxo-3,4,7,8,13,13a-hexahydropyrazino[2',1':3,4][1,4]oxa zepine[5,6,7-de]quinazolin-2(1H)-carboxylate (a brown oil, 1.0 g, yield: 94.34%). ES-API:[M+H]⁺=633, 635.

Step 2: tert-butyl (R)-11-chloro-8-(2-isopropyl-4-methylpyridin-3-yl)-10-(1-methyl-2-oxo-1,2-di hydropyridin-3-yl)-7-oxo-3,4,7,8,13,13a-hexahydropyrazino[2',1':3,4][1,4]oxa zepine[5,6,7-de]quinazolin-2(1H)-carboxylate (1.0 g, 1.58 mmol was dissolved in dichloromethane (12.0 mL), then trifluoroacetic acid (4.0 mL) was added, and reacted at room temperature for 1 hour. The reaction solution was concentrated to obtain (R)-11-chloro-8-(2-isopropyl-4-methylpyridin-3-yl)-10-(1-methyl-2-oxo-1,2-di hydropyridin-3-yl)-1,2,3,4,13,13a-hexahydropyrazino[2',1':3,4][1,4]oxazepine [5,6,7-de]quinazolin-7(8H)-1-one (a brown oil, 921 mg, the crude product). ES-API:[M+H]⁺=533.

Step 3: (R)-11-chloro-8-(2-isopropyl-4-methylpyridin-3-yl)-10-(1-methyl-2-oxo-1,2-di hydropyridin-3-yl)-1,2,3,4,13,13a-hexahydropyrazino[2',1':3,4][1,4]oxazepine [5,6,7-de]quinazolin-7(8H)-1-one (842 mg, 1.58 mmol) was dissolved in dichloromethane (10 mL), and triethylamine (479 mg, 4.74 mmol) was added at 0°C. After stirring for 2min, acrylic anhydride (199 mg, 1.58 mmol, dissolved in 2.0 mL of dichloromethane) was dropped. After the dropping, the reaction proceeded at 0°C for 1 hour. The saturated sodium bicarbonate (10 mL) was added to the reaction solution. The reaction solution was extracted with dichloromethane (3 * 20 mL), washed with the saturated sodium bicarbonate (2 * 20 mL) and the saturated brine (2 * 20 mL), dried with anhydrous sodium sulfate, and concentrated to obtain the crude product. Product Z40 was purified by a reversed-phase column (a 20G C18 reversed-phase column, acetonitrile /water=36/100) (a white solid, 122.5 mg, yield: 13.21%). ES-API:[M+H]⁺=587. ¹H NMR (400 MHz, DMSO-*d*₆) δ 0.96-1.08 (m, 6H), 1.96 (d, *J* = 9.0 Hz, 3H), 2.70-2.78 (m, 1H), 3.09 (d, *J* = 13.2 Hz, 1H), 3.23 (d, *J* = 18.6 Hz, 1H), 3.37 (s, 3H), 3.50 (s, 1H), 4.06-4.12 (m, 2H), 4.34 (d, *J* = 12.9 Hz, 1H), 4.45 (d, *J* = 13.3 Hz, 1H), 4.71 (s, 3H), 5.76 (d, *J* = 21.1 Hz, 2H), 6.02-6.13 (m, 2H), 6.19 (d, *J* = 16.6 Hz, 1H), 6.79-6.90 (m, 1H), 7.29 (d, *J* = 4.7 Hz, 1H), 7.68 (d, *J* = 6.9 Hz, 1H), 8.50 (d, *J* = 4.6 Hz, 1H).

### Example 41: Preparation of Compound Z41

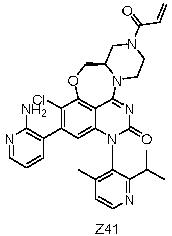

Compound Z41 was prepared with Intermediate a as the raw material according to the method of Example 34. ES-API:[M+H]⁺=572. ¹H NMR (400 MHz, DMSO-*d*₆) δ 1.02 (dd, *J* = 26.5, 6.3 Hz, 6H), 1.96 (d, *J* = 11.4 Hz, 3H), 2.76 (s, 1H), 3.17 (s, 1H), 3.25 (s, 1H), 3.39-3.49 (m, 2H), 4.03 (s, 1H), 4.14 (s, 1H), 4.32-4.48 (m, 2H), 4.69 (s, 3H), 5.53 (d, *J* = 9.5 Hz, 1H), 5.60 (s, 1H), 5.74 (d, *J* = 10.9 Hz, 2H), 6.19 (d, *J* = 16.7 Hz, 1H), 6.51 (t, *J* = 6.0, 6.0 Hz, 1H), 6.87-7.00 (m, 1H), 7.09 (s, 1H), 7.26 (s, 1H), 7.90 (s, 1H), 8.46 (d, *J* = 4.5 Hz, 1H).

### Example 42: Preparation of Compound Z42

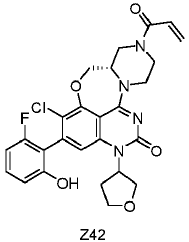

Compound Z42 was prepared with tetrahydrofuran-3-amine as the raw material according to the method of Example 33. ES-API:[M/2+1]⁺= 527.2. ¹H NMR (400 MHz, CDCl₃) δ 7.43 (s, 1H), 6.84 (s, 1H), 6.75 (s, 1H), 6.57 (s, 1H), 6.42 (d, J = 16.3 Hz, 1H), 5.95 (s, 1H), 5.82 (s, 1H), 4.73 (s, 1H), 4.47 (d, J = 21.3 Hz, 4H), 4.29 (s, 1H), 4.08 (s, 1H), 3.93 (d, J = 8.5 Hz, 3H), 3.71 (s, 1H), 3.61-3.15 (m, 3H), 2.26 (s, 2H).

### Example 43: Preparation of Compound Z43

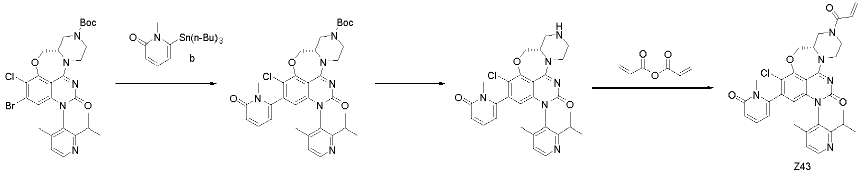

### Step 1: tert-butyl

(S)-10-bromo-11-chloro-8-(2-isopropyl-4-methylpyridin-3-yl)-7-oxo-3,4,7,8,13 ,13a-hexahydropyrazino[2',1':3,4][1,4]oxazepine[5,6,7-de]quinazolin-2(1H)-ca rboxylate (800 mg, 1.32 mmol) was dissolved in toluene (10 mL), 1-methyl-6-(tributyltinalkyl)pyridin-2(1H)-one (631 mg, 1.59 mmol) and tetra(triphenylphosphine)palladium (114 mg, 0.132 mmol) were added, and reacted at 100°C for 16 hours under nitrogen protection. The reaction solution was extracted with ethyl acetate, concentrated under reduced pressure, and the crude product was purified by column chromatography (dichloromethane/methanol =20/1) to obtain tert-butyl (S)-11-chloro-8-(2-isopropyl-4-methylpyridin-3-yl)-10-(1-methyl-6-oxo-1,6-di hydropyridin-2-yl)-7-oxo-3,4,7,8,13,13a-hexahydropyrazino[2',1':3,4][1,4]oxa zepine[5,6,7-de]quinazolin-2(1H)-carboxylate (brown liquid, 630 mg, yield: 75%). ES-API:[M+H]⁺=633.3.

Steps 2-3: Compound Z43 was prepared according to the corresponding steps of Example 34. ES-API:[M+H]⁺=587.3. ¹H NMR (400 MHz, DMSO-*d*₆) δ 8.48 (d, *J* = 4.7 Hz, 1H), 7.47-7.19 (m, 2H), 6.85 (s, 1H), 6.40 (d, *J* = 9.2 Hz, 1H), 6.19 (d, *J* = 16.7 Hz, 1H), 6.07 (d, *J* = 6.1 Hz, 1H), 5.93 (d, *J* = 52.9 Hz, 1H), 5.75 (d, *J* = 10.5 Hz, 1H), 4.74 (s, 3H), 4.52-3.98 (m, 3H), 3.52 (d, *J =* 15.5 Hz, 2H), 3.25-3.11 (m, 1H), 3.02 (d, *J* = 9.9 Hz, 3H), 2.73 (dd, *J* = 25.7, 17.4 Hz, 1H), 1.96 (dd, *J* = 10.5, 6.8 Hz, 3H), 1.13-0.93 (m, 6H).

### Example 44: Preparation of Compound Z44

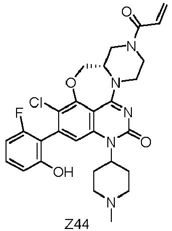

Compound Z44 was prepared with 1-methylpiperidine-4-amine as the raw material according to the method of Example 33. ES-API:[M +1]⁺= 554.2.

¹H NMR (400 MHz, DMSO-*d*₆) δ 10.13 (s, 1H), 7.27 (d, *J* = 7.5 Hz, 2H), 6.91-6.68 (m, 3H), 6.16 (d, *J* = 16.7 Hz, 1H), 5.73 (d, *J* = 10.2 Hz, 1H), 4.56 (s, 4H), 4.28-3.93 (m, 3H), 3.44 (d, *J* = 52.7 Hz, 3H), 3.15-2.77 (m, 6H), 2.56 (s, 3H), 1.71 (d, *J* = 12.2 Hz, 2H).

### Example 45: Preparation of Compound Z45-1, Z45-2, Z45-3 and Z45-4

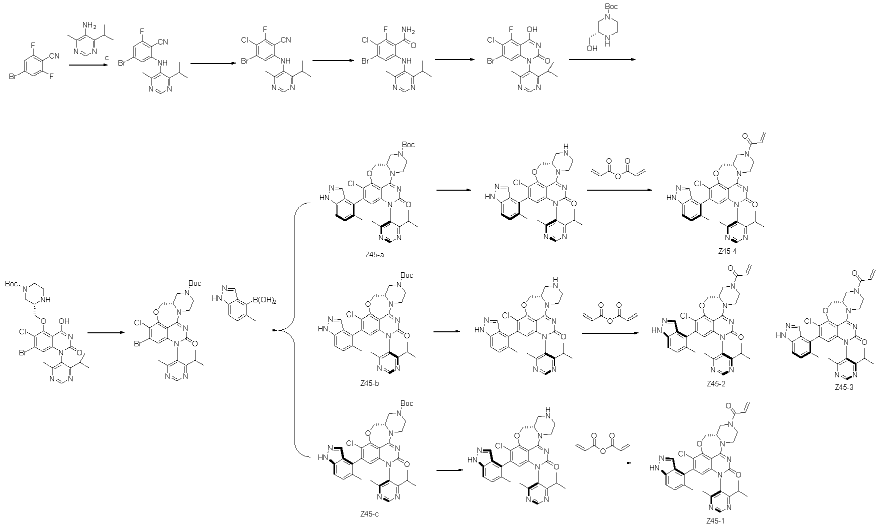

Step 1: 4-bromo-2,6-difluorobenzonitrile (540 mg, 2.48 mmol) was dissolved in tetrahydrofuran (4 mL), and cooled to -70°C, sodium bis(trimethylsilyl)amide (2.3 mL, 4.5 mmol) was dropped, and after stirring for 30 minutes at -70°C, a solution of 4-isopropyl-6-methylpyrimidin-5-amine in tetrahydrofuran (4 mL) was dropped. The reaction solution gradually rised to room temperature, and stirred overnight. The reaction was quenched with ammonium chloride aqueous solution. The reaction solution was extracted with ethyl acetate, the organic phase was dried, spin-dried, and passed the column machine (12 g column) to purify (petroleum ether/ethyl acetate=3/1) to obtain the product (380 mg, yield:44%). ES-API:[M+1]⁺= 349.0.

Step 2: 4-bromo-2-fluoro-6-((4-isopropyl-6-methylpyrimidin-5-yl)amino)benzonitrile (1.1 g, 3.15 mmol) was dissolved in acetonitrile (20 mL), NCS(419 mg, 3.15 mmol) was added, heated to 80°C, and stirred for 3 hours. The reaction solution was extracted with ethyl acetate, the organic phase was dried, spin-dried, and passed the column machine (12 g column) to purify (petroleum ether/ethyl acetate=4/1) to obtain the product (670 mg, yield:65%). ES-API:[M+1]⁺= 385.0.

Step 3: 4-bromo-3-chloro-2-fluoro-6-((4-isopropyl-6-methylpyrimidin-5-yl)amino)ben zonitrile (620 mg, 1.61 mmol) was dissolved in dimethyl sulfoxide (8 mL), potassium carbonate (1.1 g, 8.07 mmol) was added, cooled to 0°C, hydrogen peroxide was dropped (659 mg, 5.81 mmol). The reaction rised to room temperature, and stirred for 5 hours. The reaction solution was quenched with sodium sulfite aqueous solution. The reaction solution was extracted with ethyl acetate, the organic phase was dried, spin-dried, and passed the column machine (12 g column) to purify (petroleum ether/ethyl acetate=2/1) to obtain the product (400 mg, yield: 55%). ES-API:[M+1]⁺= 403.0.

Step 4: sodium hydride (497 mg, 12.4 mmol) was suspended in tetrahydrofuran (5 mL), cooled to -10°C, a solution of 4-bromo-3-chloro-2-fluoro-6-((4-isopropyl-6-methylpyrimidin-5-yl)amino)ben zamide (500 mg, 1.24 mmol) in tetrahydrofuran (5 mL) was dropped, and after stirring for 20 minutes, a solution of carbonyl diimidazole in tetrahydrofuran (5 mL) was dropped. The reaction solution was stirred at -10°C for1 hour. The reaction solution cooled to -30°C, and the pH was adjusted to 2-3 with 0.5M hydrochloric acid solution. The reaction solution was extracted with ethyl acetate, the organic phase was dried, spin-dried, purified by reversed-phase column chromatography (40g column) (water/acetonitrile =1/1) to obtain the product (240 mg, yield:45%). ES-API:[M+1]⁺= 429.0.

Step 5: 7-bromo-6-chloro-5-fluoro-4-hydroxy-1-(4-isopropyl-6-methylpyrimidin-5-yl) quinazolin-2(1H)-one (240 mg, 0.56 mmol) and tert-butyl (S)-3-(hydroxymethyl)piperazin-1-carboxylate (182 mg, 0.84 mmol) was dissolved in tetrahydrofuran (4 mL), cooled to 0°C, sodium hydride (112 mg, 2.8 mmol) was added, rised to room temperature, and stirred for 1 hour. The reaction solution was quenched with water. The reaction solution was extracted with ethyl acetate, the organic phase was dried, spin-dried, and passed the column machine (12 g column) to purify (dichloromethane/methanol =10/1) to obtain the product (260 mg, yield:74%). ES-API:[M+1]⁺= 625.2.

Step 6: tert-butyl (S)-3-(((7-bromo-6-chloro-4-hydroxy-1-(4-isopropyl-6-methylpyrimidin-5-yl)-2-oxo-1,2-dihydroquinazolin-5-yl)oxy)methyl)piperazin-1-carboxylate (180 mg, 0.29 mmol) was dissolved in acetonitrile (9 mL), N,N-diisopropylethylamine(112 mg, 0.86 mmol) and phosphorus oxychloride (66 mg, 0.43 mmol) were added, heated to 80°C, and stirred for 2 hours. The reaction solution was quenched with sodium bicarbonate aqueous solution. The reaction solution was extracted with ethyl acetate, the organic phase was dried, spin-dried, passed the column machine (12 g column) (ethyl acetate) to obtain the product (80 mg, yield:45%). ES-API:[M+1]⁺= 607.1.

Step 7: tert-butyl (S)-10-bromo-11-chloro-8-(4-isopropyl-6-methylpyrimidin-5-yl)-7-oxo-3,4,7,8, 13,13a-hexahydropyrazino[2',1':3,4][1,4]oxazepine[5,6,7-de]quinazolin-2(1H) -carboxylate (150 mg, 0.25 mmol) was dissolved in dioxane(2 mL) and water (0.4 mL), (5-methyl-1H-indazol-4-yl)boronic acid (87 mg, 0.49 mmol), 2-dicyclohexylphosphino-2',6'-dimethoxy-biphenyl (10 mg, 0.025 mmol), tris(dibenzylideneacetone)dipalladium (23 mg, 0.025 mmol) and potassium phosphate (157 mg, 0.74 mmol) were added, and reacted under a 110°C microwave for 1.33 hours. The reaction solution was extracted with ethyl acetate, the organic phase was dried, spin-dried, column chromatography (ethyl acetate/petroleum ether=0-100%, in the order of polarity from small to large) to obtain the following products, respectively:
tert-butyl (13aS)-11-chloro-8-(4-isopropyl-6-methylpyrimidin-5-yl)-10-(5-methyl-1H-ind azol-4-yl)-7-oxo-3,4,7,8,13,13a-hexahydropyrazino[2',1':3,4][1,4]oxazepine[5, 6,7-de]quinazolin-2(1H)-carboxylate Z45-a (19 mg), TLC R_{f} value: 0.30 (ethyl acetate), ES-API:[M+1]⁺= 657.3;
tert-butyl (13aS)-11-chloro-8-(4-isopropyl-6-methylpyrimidin-5-yl)-10-(5-methyl-1H-ind azol-4-yl)-7-oxo-3,4,7,8,13,13a-hexahydropyrazino[2',1':3,4][1,4]oxazepine[5, 6,7-de]quinazolin-2(1H)-carboxylate Z45-b (30 mg), TLC R_{f} value: 0.25 (ethyl acetate), ES-API:[M+1]⁺= 657.3;
tert-butyl (13aS)-11-chloro-8-(4-isopropyl-6-methylpyrimidin-5-yl)-10-(5-methyl-1H-ind azol-4-yl)-7-oxo-3,4,7,8,13,13a-hexahydropyrazino[2',1':3,4][1,4]oxazepine[5, 6,7-de]quinazolin-2(1H)-carboxylate Z45-c (20 mg), TLC R_{f} value: 0.10 (ethyl acetate), ES-API:[M+1]⁺= 657.3.

Step 8: Compound Z45-c (20 mg, 0.03 mmol) was dissolved in dichloromethane(2 mL), trifluoroacetic acid(0.5 mL) was added and reacted at room temperature for 1 hour, spin-dried to obtain a yellow oily crude product: (13aS)-11-chloro-8-(4-isopropyl-6-methylpyrimidin-5-yl)-10-(5-methyl-1H-ind azol-4-yl)-1,2,3,4,13, 13a-hexahydropyrazine[2',1':3,4][1,4]oxazepine[5,6,7-de]quinazolin-7(8H)-on e, a crude product (17 mg, yield: 100%).

Step 9: (13aS)-11-chloro-8-(4-isopropyl-6-methylpyrimidin-5-yl)-10-(5-methyl-1H-ind azol-4-yl)-1,2,3,4,13, 13a-hexahydropyrazine[2',1':3,4][1,4]oxazepine[5,6,7-de]quinazolin-7(8H)-on e (17 mg, 0.03 mmol), and triethylamine (0.5 mL) was dissolved in dichloromethane (2 mL), cooled to 0°C, acrylic anhydride (3 mg, 0.027 mmol) was added, and reacted at 0°C for 0.5 hour. The reaction solution was extracted with 10 mL of water and dichloromethane (10 mL^{∗}3), the extracted organic phase was spin-dried to obtain the crude product, the crude product was prepared and purified (chromatographic column: Ultimate XB-C18,50^{∗}250mm,10um; elution system: acetonitrile /water, 40 minutes from 10% to 90%; monitoring wavelength 210nm; flow rate 80mL/min), freeze-dried to obtain Compound Z45-1 (4 mg, yield: 20%). ES-API:[M+1]⁺= 611.2. ¹H NMR (400 MHz, CDCl₃) δ 9.01 (s, 1H), 7.45 (d, *J* = 9.5 Hz, 1H), 6.70-6.56 (m, 1H), 6.42 (d, *J* = 16.6 Hz, 1H), 6.04 (s, 1H), 5.84 (d, *J* = 10.4 Hz, 1H), 5.08 (d, *J* = 13.2 Hz, 1H), 4.69 (s, 2H), 4.12 (d, *J* = 7.2 Hz, 1H), 3.69 (d, *J* = 43.3 Hz, 1H), 3.51-3.28 (m, 2H), 2.84-2.77 (m, 1H), 2.31 (s, 3H), 2.11 (s, 3H), 2.04 (s, 1H), 1.41 (s, 1H), 1.33 (s, 2H), 1.25 (s, 6H), 1.06 (d, *J* = 6.7 Hz, 2H).

Steps 10-11: With Compound Z45-b as raw material, the method of steps 8-9 in Example 45 was referred to finally purify by column chromatography (methanol /dichloromethane = 0-10%)(polarity from small to large) to obtain:
Compound Z45-2 (a white solid, 3.8 mg); LC-MS: retention time:1.597 min; ES-API:[M+1]⁺= 611.2. ¹H NMR (400 MHz, CDCl₃) δ 9.01 (s, 1H), 7.45 (d, J = 9.5 Hz, 1H), 6.70-6.56 (m, 1H), 6.42 (d, J = 16.6 Hz, 1H), 6.04 (s, 1H), 5.84 (d, J = 10.4 Hz, 1H), 5.08 (d, J = 13.2 Hz, 1H), 4.69 (s, 2H), 4.12 (d, J = 7.2 Hz, 1H), 3.69 (d, J = 43.3 Hz, 1H), 3.51-3.28 (m, 2H), 2.84-2.77 (m, 1H), 2.31 (s, 3H), 2.11 (s, 3H), 2.04 (s, 1H), 1.41 (s, 1H), 1.33 (s, 2H), 1.25 (s, 6H), 1.06 (d, J = 6.7 Hz, 2H).
and Compound Z45-3 (a white solid, 4.0 mg); LC-MS: retention time:1.648 min; ES-API:[M+1]⁺= 611.2. ¹H NMR (400 MHz, CDCl₃) δ 9.01 (s, 1H), 7.32 (s, 1H), 6.64 (dd, J = 16.7, 10.5 Hz, 1H), 6.43 (d, J = 16.7 Hz, 1H), 6.03 (s, 1H), 5.84 (d, J = 10.4 Hz, 1H), 5.08 (d, J = 13.1 Hz, 1H), 4.69 (d, J = 14.7 Hz, 3H), 4.08 (s, 2H), 3.72-3.30 (m, 3H), 2.87-2.76 (m, 1H), 2.34 (s, 3H), 2.09 (s, 3H), 1.43-1.37 (m, 1H), 1.26 (d, J = 8.1 Hz, 6H), 1.05 (d, J = 6.6 Hz, 2H).

Steps 12-13: With Compound Z45-a as raw material, the method of steps 8-9 in Example 45 was referred to obtain Compound Z45-4 (a white solid, 4.2 mg). ES-API:[M+1]⁺= 611.2.¹H NMR (400 MHz, CDCl₃) δ 9.01 (s, 1H), 7.45 (d, J = 9.5 Hz, 1H), 6.70-6.56 (m, 1H), 6.42 (d, J = 16.6 Hz, 1H), 6.04 (s, 1H), 5.84 (d, J = 10.4 Hz, 1H), 5.08 (d, J = 13.2 Hz, 1H), 4.69 (s, 2H), 4.12 (d, J = 7.2 Hz, 1H), 3.69 (d, J = 43.3 Hz, 1H), 3.51-3.28 (m, 2H), 2.84-2.77 (m, 1H), 2.31 (s, 3H), 2.11 (s, 3H), 2.04 (s, 1H), 1.41 (s, 1H), 1.33 (s, 2H), 1.25 (s, 6H), 1.06 (d, J = 6.7 Hz, 2H).

### Example 46: Preparation of Compound Z46, Z46-1 and Z46-2

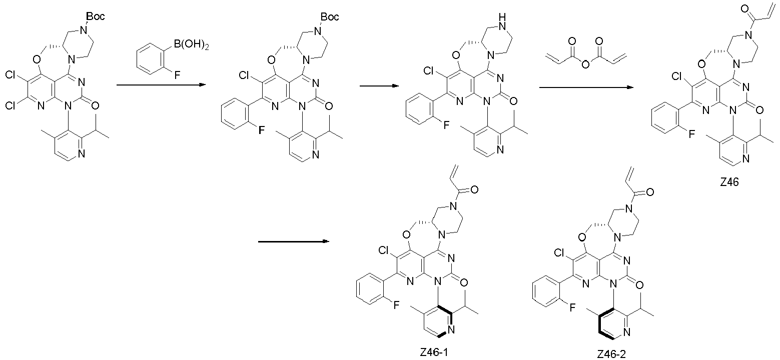

Step 1: tert-butyl (S)-2,3-dichloro-12-(2-isopropyl-4-methylpyridin-3-yl)-11-oxo-5α,6,8,9,11,12h exahydro-4-oxo-1,7,9a,10,12-pentazabenzo[4,5]cyclohepta[1,2,3-de]naphthale n-7(5H)-carboxylate (300 mg, 0.54mmol), (2-fluorophenyl)boronic acid (227mg, 1.62 mmol), tetratriphenylphosphine palladium (62mg, 0.054mmol), sodium carbonate (172 mg, 1.62 mmol), 50 mL of dioxane and 10 mL of water were added to a 100mL reaction flask. The reaction was stirred at 65°C for 3 hours under nitrogen protection, and the reaction stopped. 30 mL of water was added to the reaction solution. The reaction solution was extracted with 30 mL of ethyl acetate for 3 times, and the organic phase was dried and concentrated. The crude product was purified by a fast silica gel column (ethyl acetate/petroleum ether:0-100%) to obtain the product: tert-butyl (S)-3-chloro-2-(2-fluorophenyl)-12-(2-isopropyl-4-methylpyridin-3-yl)-11-oxo-5a,6,8,9,11,12-hexahydro-4-oxo-1,7,9a,10,12-pentazabenzo[4,5]cyclohepta[1,2 ,3-de]naphthalen-7(5H)-carboxylate (211mg, 63%), a yellow solid. ES-API:[M+H]⁺= 622.3.

Steps 2-3: Compound Z46 was prepared according to the corresponding steps of Example 34. ES-API: [M+H]⁺=576.2. ¹H NMR (500 MHz, DMSO-*d*₆) δ 8.35 (d, *J* = 4.8 Hz, 1H), 7.47 (dd, *J* = 13.4, 6.2 Hz, 1H), 7.31-7.21 (m, 2H), 7.15 (d, *J* = 4.8 Hz, 2H), 6.95-6.75 (m, 1H), 6.21 (dd, *J* = 16.6, 2.2 Hz, 1H), 5.77 (dd, *J* = 10.4, 2.2 Hz, 1H), 4.98-4.78 (m, 2H), 4.65-4.53 (m, 1H), 4.51-4.32 (m, 1H), 4.26 (d, *J* = 20.2 Hz, 1H), 4.18-3.94 (m, 1H), 3.82-3.56 (m, 2H), 2.83-2.66 (m, 1H), 1.94 (d, *J* = 11.5 Hz, 3H), 1.06 (d, *J* = 6.8 Hz, 3H), 0.93 (d, *J* = 6.6 Hz, 3H).

Step 4: Compound Z46 was prepared and resolved chirally (column type: IG, 250mm^{∗}4.6mm^{∗}5um, mobile phase: n-hexane:ethanol=40:60, flow rate: 1ml/min, column temperature=30°C) to obtain:
Compound Z46-1 (79.8mg, peak 1, retention time 8.093 min, yield:30.2%), a white solid. ¹H NMR (400 MHz, DMSO-*d*₆) δ 8.36 (d, *J* = 4.8 Hz, 1H), 7.48 (ddd, *J* = 15.6, 5.4, 1.8 Hz, 1H), 7.35-7.07 (m, 4H), 6.96-6.77 (m, 1H), 6.00 (ddd, *J* = 13.5, 12.7, 2.3 Hz, 2H), 5.01-4.79 (m, 2H), 4.69-4.25 (m, 3H), 4.07 (d, *J* = 31.0 Hz, 1H), 3.82-3.57 (m, 2H), 3.38 (s, 1H), 2.78 (dt, *J* = 13.4, 6.7 Hz, 1H), 1.94 (s, 3H), 1.06 (d, J = 6.7 Hz, 3H), 0.93 (d, J = 6.7 Hz, 3H).
and Compound Z46-2 (84.48mg, peak 2, retention time 11.618 min, yield:31.9%), a white solid. ¹H NMR (400 MHz, DMSO-*d*₆) δ 8.36 (d, *J* = 4.8 Hz, 1H), 7.48 (dd, *J* = 13.3, 6.1 Hz, 1H), 7.34-7.10 (m, 4H), 6.95-6.77 (m, 1H), 6.22 (d, *J* = 16.7 Hz, 1H), 5.78 (d, *J* = 12.2 Hz, 1H), 4.99-4.81 (m, 2H), 4.68-4.22 (m, 3H), 4.07 (d, *J* = 14.4 Hz, 1H), 3.83-3.58 (m, 2H), 3.37 (s, 1H), 2.78-2.62 (m, 1H), 1.96 (s, 3H),1.06 (d, *J* = 6.7 Hz, 3H), 0.94 (d, *J* = 6.7 Hz, 3H).

### Example 47: Preparation of Compound Z47, Z47-1 and Z47-2

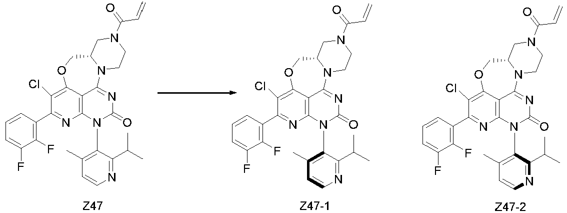

Compound Z47 was prepared with (2,3-difluorophenyl)boronic acid as the raw material according to the method of Example 46. ES-API: [M+H]⁺=594.2. Compound Z47 was resolved chirally (mobile phase :acetonitrile: isopropanol=60:40); column type: IC (250mm^{∗}4.6mm^{∗} 5um); flow rate: 1.0 ml/min; column temperature: 30°C) to obtain:Compound Z47-1 (23mg, retention time: 5.176min, purity:100 %, de value: 100 %). ES-API:[M+H]⁺= 594.2;
and Compound Z47-2 ( 29mg, retention time: 9.285min, purity: 100%, de value: 99%). ES-API:[M+H]⁺= 594.2. ¹H NMR (500 MHz, DMSO-*d*₆) δ 8.36 (d, *J* = 4.8 Hz, 1H), 7.52 (dd, *J* = 18.0, 8.6 Hz, 1H), 7.26 (dd, *J* = 12.8, 8.0 Hz, 1H), 7.20-7.13 (m, 1H), 6.99 (d, *J* = 6.4 Hz, 1H), 6.94-6.75 (m, 1H), 6.21 (dd, *J* = 16.8, 2.0 Hz, 1H), 5.78 (dd, *J* = 10.4, 2.0 Hz, 1H), 5.04-4.81 (m, 2H), 4.56 (s, 1H), 4.42 (dd, *J* = 53.8, 12.1 Hz, 1H), 4.30 (s, 1H), 4.06 (d, *J* = 34.1 Hz, 1H), 3.85-3.61 (m, 2H), 3.41-3.30 (m, 1 H), 2.78-2.63 (m, 1H), 1.94 (d, *J* = 12.3 Hz, 3H), 1.06 (d, *J* = 6.8 Hz, 3H), 0.94 (d, *J* = 6.6 Hz, 3H).

### Example 48: Preparation of Compound Z48

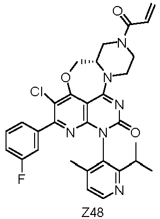

Compound Z48 was prepared with (3-fluorophenyl)boronic acid as the raw material according to the method of Example 46. ES-API: [M+H]⁺=576.2. ¹H NMR (500 MHz, DMSO-*d*₆) δ 8.42 (d, *J* = 4.8 Hz, 1H), 7.45 (dd, *J* = 14.0, 8.0 Hz, 1H), 7.40-7.27 (m, 2H), 7.25-7.08 (m, 2H), 7.00-6.72 (m, 1H), 6.22 (d, *J* = 16.6 Hz, 1H), 5.78 (d, *J* = 11.6 Hz, 1H), 4.87 (ddd, *J* = 19.0, 17.8, 8.2 Hz, 2H), 4.59 (s, 1H), 4.52-4.34 (m, 1H), 4.27 (s, 1H), 4.08 (d, *J* = 40.8 Hz, 1H), 3.71 (d, *J* = 44.8 Hz, 2H), 3.40-3.25 (m, 1 H), 2.87-2.67 (m, 1H), 1.95 (d, *J* = 10.1 Hz, 3H), 1.08 (d, *J* = 6.8 Hz, 3H), 0.95 (d, *J* = 6.4 Hz, 3H).

### Example 49: Preparation of Compound Z49, Z49-1 and Z49-2

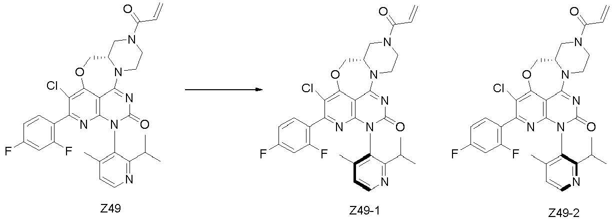

Compound Z49 was prepared with (2,4-difluorophenyl)boronic acid as the raw material according to the method of Example 46. ES-API: [M+H]⁺=594.2. Compound Z49(75mg, 0.12mmol) was resolved chirally (mobile phase: acetonitrile :isopropanol=60:40; column type: IC (250mm^{∗}4.6mm 5um) ; flow rate: 1.0 ml/min; column temperature: 30°C) to obtain:Compound Z49-1 (27mg, retention time: 5.318min, purity:100 %, de value: 100 %). ES-API:[M+H]⁺= 594.1.
and Compound Z49-2 (28mg, retention time: 8.840min, purity:99%, de value: 99%). ES-API:[M+H]⁺= 594.1. ¹H NMR (500 MHz, DMSO-*d*₆) δ 8.37 (d, *J* = 4.8 Hz, 1H), 7.35 (t, *J* = 9.8 Hz, 1H), 7.29-7.10 (m, 3H), 6.98-6.73 (m, 1H), 6.22 (dd, *J =* 16.8, 2.2 Hz, 1H), 5.78 (dd, *J* = 10.4, 2.2 Hz, 1H), 5.05-4.80 (m, 2H), 4.57 (s, 1H), 4.42 (dd, *J* = 54.5, 12.0Hz, 1H), 4.29 (s, 1H), 4.06 (d*, J* = 33.4 Hz, 1H), 3.73 (d, *J* = 54.0 Hz, 2H), 2.87-2.65 (m, 1H), 1.94 (d, *J* = 12.0 Hz, 3H), 1.07 (d, *J* = 6.8 Hz, 3H), 0.93 (d, *J* = 6.8 Hz, 3H).

### Example 50: Preparation of Compound Z50

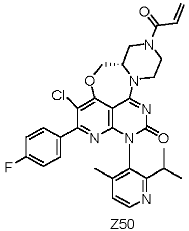

Compound Z50 was prepared with (4-fluorophenyl)boronic acid as the raw material according to the method of Example 46. ES-API: [M+H]⁺=576.2. ¹H NMR (500 MHz, DMSO-*d*₆) δ 8.42 (d, *J* = 5.0 Hz, 1H), 7.48 (dd, *J* = 8.8, 5.6 Hz, 2H), 7.24 (t, *J* = 8.8 Hz, 2H), 7.20 (t, *J* = 4.2 Hz, 1H), 6.86 (m, 1H), 6.21 (m, 1H), 5.78 (d, *J* = 12.0Hz, 1H), 4.87 (m, 2H), 4.60 (s, 1H), 4.43 (dd, *J* = 54.2, 12.8 Hz, 1H), 4.26 (s, 1H), 4.09 (d, *J* = 43.2Hz, 1H), 3.70 (d, *J* = 52.2 Hz, 2H), 2.74 (m, 1H), 1.95 (d, *J* = 9.8Hz, 3H), 1.07 (d, *J* = 6.8 Hz, 3H), 0.94 (d, *J* = 6.6 Hz, 3H).

### Example 51: Preparation of Compound Z51

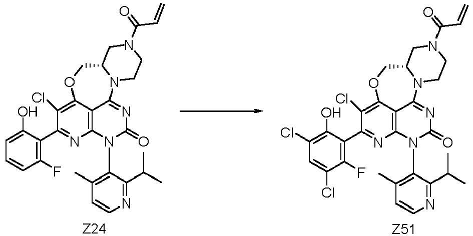

(5aS)-7-acryloyl-3-chloro-2-(2-fluoro-6-hydroxyphenyl)-12-(2-isopropyl-4-methylpyridin-3-yl)-5,5a,6,7,8,9-hexahydro-4-oxal,7,9a,10,12-pentazabenzo[4, 5]cyclohepta[1,2,3-de]naphthalene11(12H)-one (200mg, 0.34 mmol), N-chlorosuccinimide (137mg, 1.02 mmol) and acetic acid (30 mL) were added to a round bottom flask. The reaction was stirred at 75°C for 2 hours. concentrated, and the crude product was seperated by Preparative HPLC to obtain the product: (5aS)-7-acryloyl-3-chloro-2-(3,5-dichloro-2-fluoro-6-hydroxyphenyl)-12-(2-iso propyl-4-methylpyridin-3-yl)-5,5a,6,7,8,9-hexahydro-4-oxo-1,7,9a,10,12-penta zabenzo[4,5]cyclohepta[1,2,3-de]naphthalen-11(12H)-one (176mg, 56%), a yellow solid. ES-API: [M+H]⁺=659.6. ¹H NMR (500 MHz, DMSO-*d*₆) δ 10.25 (dd, *J* = 23.0, 10.0 Hz, 1H), 8.29 (d, *J* = 4.8 Hz, 1H), 7.70 (d, *J* = 7.8 Hz, 1H), 7.10 (s, 1H), 6.78 (s, 1H), 6.14 (d, *J* = 16.5 Hz, 1H), 5.71 (d, *J* = 10.2Hz, 1H), 4.93-4.72 (m, 2H), 4.59-4.35 (m, 2H), 4.28-4.11 (m, 1H), 4.10-3.89 (m, 1H), 3.77-3.48 (m, 3H), 2.81-2.56 (m, 1H), 1.85 (dd, *J* = 19.6, 12.4 Hz, 3H), 0.99 (d, *J* = 6.2 Hz, 3H), 0.89-0.81 (m, 3H).

### Example 52: Preparation of Compound Z52

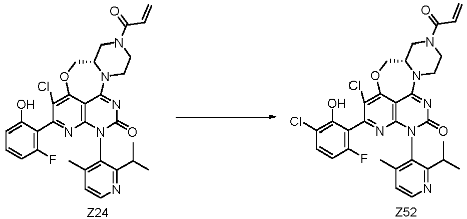

(5aS)-7-acryloyl-3-chloro-2-(2-fluoro-6-hydroxyphenyl)-12-(2-isopropyl-4-methylpyridin-3-yl)-5,5a,6,7,8,9-hexahydro-4-oxo-1,7,9a,10,12-pentazabenz o[4,5]cyclohepta[1,2,3-de]naphthalen-11(12H)-one (300 mg, 0.5085mmol) was added to 30mL of acetonitrile, finally N-chlorosuccinimide (135mg, 1.0169mmol) was added, and reacted at 75°C for 1 hour. 80mL of ethyl acetate was added, the reaction solution was washed with the saturated brine for 3 times (3^{∗}60mL). The organic phase was dried with anhydrous sodium sulfate, filtered, and the filtrate was spin-dried and prepared to obtain (5aS)- 7 -acryloyl-3-chloro-2-(3-chloro-6- fluoro-2-hydroxyphenyl)-12-(2- isopro pyl-4-methylpyridin-3-yl)-5,5a,6,7,8,9-hexahydro-4-oxo-1,7,9a,10,12-pentazab enzo[4,5]cyclohepta[1,2,3-de]naphthalen-1 1(12H)-one (9.2mg, yield:3%). ES-API: [M+H]⁺=625.2.

### Example 53: Preparation of Compound Z53

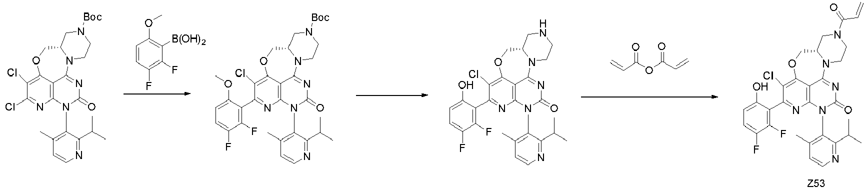

Step 1: tert-butyl (S)-2,3-dichloro-12-(2-isopropyl-4-methylpyridin-3-yl)-11-oxo-5a,6,8,9, 11,12-hexahydro-4-oxo-1,7,9a,10,12-pentazabenzo[4,5]cyclohepta[1,2,3-de]naphthal en-7(5H)-carboxylate (200 mg, 0.36 mmol), (2,3-difluoro-6-methoxyphenyl)boronic acid (200 mg, 1.07mmol), chloro(2-dicyclohexylphosphino-2',6'-dimethoxy-1,1'-biphenyl)(2'-amino-1,1'-b iphenyl-2-yl)palladium(II) (28 mg, 0.04 mmol), 2-bicyclohexylphosphin-2',6'-dimethoxybiphenyl (16 mg, 0.04 mmol), a mixed solution of potassium phosphate (227 mg, 1.07 mmol) in 1,4-dioxane(2 mL) and water (0.4 mL) reacted under a 100°C microwave for 1 hour under nitrogen protection. The reaction solution was filtered, washed with ethyl acetate (30 mL), the filtrate was washed with the saturated brine (10 mL^{∗}3), the obtained organic phase was dried and concentrated, purified with a fast silica column (0-10% methanol /dichloromethane) to obtain a yellow solid: tert-butyl (S)-3-chloro-2-(2,3-difluoro-6-methoxyphenyl)-12-(2-isopropyl-4-methylpyridi n-3-yl)-11-oxo-5a,6,8,9,11,12-hexahydro-4-oxo-1,7,9a,10,12-pentazabenzo[4,5 ]cyclohepta[1,2,3-de]naphthalen-7(5H)-carboxylate (100 mg, yield:23%). ES-API: [M+H]⁺=669.1.

Step 2: 17% boron tribromide in dichloromethane solution (2 mL) was added to a solution of tert-butyl (S)-3-chloro-2-(2,3-difluoro-6-methoxyphenyl)-12-(2-isopropyl-4-methylpyridi n-3-yl)-11-oxo-5a,6,8,9,11,12-hexahydro-4-oxo-1,7,9a,10,12-pentazabenzo[4,5 ]cyclohepta[1,2,3-de]naphthalen-7(5H)-carboxylate (100 mg, 0.15 mmol) in dichloromethane (2 mL) solution under the ice bath, and stirred at room temperature for 3 hours. The reaction solution was quenched with the saturated sodium bicarbonate solution (20 mL), extracted with dichloromethane, and concentrated to obtain a yellow solid: (S)-3-chloro-2-(2,3-difluoro-6-hydroxyphenyl)-12-(2-isopropyl-4-methylpyridi n-3-yl)-5,5a,6,7,8,9-hexahydro-4-oxo-1,7,9a,10,12-pentazabenzo[4,5]cyclohept a[1,2,3-de]naphthalen-11(12H)-one (83 mg). ES-API:[M+H]⁺=555.1.

Step 3: *N*,*N*-diisopropylethylamine (58 mg, 0.45 mmol) was added to a solution of (S)-3-chloro-2-(2,3-difluoro-6-hydroxyphenyl)-12-(2-isopropyl-4-methylpyridi n-3-yl)-5,5a,6,7,8,9-hexahydro-4-oxo-1,7,9a,10,12-pentazabenzo[4,5]cyclohept a[1,2,3-de]naphthalen-11(12H)-one (83 mg, 0.15 mmol) in dichloromethane (2 mL) under the ice bath. After the reaction solution was clarified, acrylic anhydride (11 mg, 0.09 mmol) was added, and stirred for 5 minutes. The reaction solution was concentrated, and then purified by Preparative HPLC (ammonium bicarbonate system) to obtain a white solid: Z53 **(7.7**6 mg, purity: 90%, yield: 9%). ¹H NMR (500 MHz, DMSO-*d*₆) δ 10.04 (t, *J* = 15.7 Hz, 1H), 8.37 (d, *J* = 4.7 Hz, 1H), 7.32-7.23 (m, 1H), 7.21-7.14 (m 1H), 6.92-6.78 (m, 1H), 6.67-6.61 (m, 1H), 6.21 (dd, *J* = 16.7, 2.2 Hz, 1H), 5.78 (dd, *J* = 10.5, 2.1 Hz, 1H), 4.96-4.82 (m, 2H), 4.63-4.52 (m, 1H), 4.49-4.34 (m, 1H), 4.29 (s, 1H), 4.11-3.99 (m, 1H), 3.73-3.59 (m, 1H), 3.56-3.47 (m, 1H), 2.80-2.62 (m, 1H), 1.93 (d, *J* = 12.2 Hz, 3H), 1.08 (d, *J* = 6.7 Hz, 3H), 0.94 (d, *J* = 4.7 Hz, 3H). ES-API: [M+H]⁺=609.1.

### Example 54: Preparation of Compound Z54

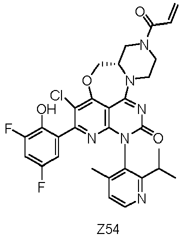

Compound Z54 was prepared with (3,5-difluoro-2-methoxyphenyl)boronic acid as the raw material according to the method of Example 53. ¹H NMR (500 MHz, DMSO-*d*₆) δ 9.83 (d, *J* = 5.1 Hz, 1H), 8.37 (d, *J* = 4.8 Hz, 1H), 7.35-7.24 (m, 1H), 7.17 (d, *J* = 4.6 Hz, 1H), 6.95-6.78 (m, 1H), 6.61 (d*, J* = 8.5 Hz, 1H), 6.22 (d*, J* = 16.6 Hz, 1H), 5.78 (d, *J* = 12.2 Hz, 1H), 4.99-4.81 (m, 2H), 4.60 (d, *J* = 16.8 Hz, 1H), 4.43 (dd, *J* = 55.1, 12.0 Hz, 1H), 4.27 (s, 1H), 4.08 (d, *J* = 42.2 Hz, 1H), 3.69 (d, *J* = 41.3 Hz, 2H), 3.37 (d*, J* = 11.3 Hz, 1H), 2.74 (ddd, *J* = 26.9, 13.3, 6.7 Hz, 1H), 1.94 (d, *J* = 10.1 Hz, 3H), 1.07 (d, *J* = 6.7 Hz, 3H), 0.96 (dd, *J* = 6.6, 1.7 Hz, 3H). ES-API: [M+H]⁺=609.1.

### Example 55: Preparation of Compound Z55

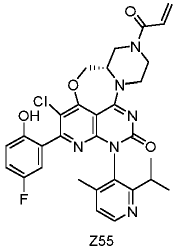

Compound Z55 was prepared with (5-fluoro-2-hydroxyphenyl)boronic acid as the raw material according to the method of Example 46. ES-API: [M+H]+=591.1. ¹H NMR (500 MHz, DMSO-*d*₆) δ 9.72 (s, 1H), 8.37 (d, *J* = 4.8 Hz, 1H), 7.17 (d*, J* = 3.8 Hz, 1H), 7.05 (td*, J* = 8.7, 3.2 Hz, 1H), 6.86 (ddd, *J =* 13.7, 12.7, 7.5 Hz, 2H), 6.75-6.66 (m, 1H), 6.22 (dd, *J* = 16.7, 2.1 Hz, 1H), 5.78 (dd, *J* = 10.5, 2.0 Hz, 1H), 5.04-4.81 (m, 2H), 4.58 (s, 1H), 4.43 (dd, *J* = 56.0, 12.8 Hz, 1H), 4.25 (s, 1H), 4.10 (d*, J* = 52.6 Hz, 1H), 3.77-3.57 (m, 2H), 3.38 (s, 1H), 2.74 (ddt, *J* = 33.6, 13.1, 6.6 Hz, 1H), 1.94 (d, *J* = 9.7 Hz, 3H), 1.07 (d, *J* = 6.7 Hz, 3H), 0.97 (dd, *J* = 6.7,3.5 Hz, 3H).

### Example 56: Preparation of Compound Z56

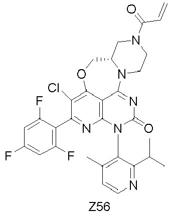

Compound Z56 was prepared with (2,4,6-trifluorophenyl)boronic acid as the raw material according to the method of Example 46. ES-API: [M+H]+=612.2. ¹H NMR (500 MHz, DMSO-*d*₆) δ 8.36 (d, *J* = 4.8 Hz, 1H), 7.32 (s, 2H), 7.16 (d, *J* = 4.8 Hz, 1H), 6.96-6.69 (m, 1H), 6.21 (dd, *J* = 16.8, 2.2 Hz, 1H), 5.77 (dd*, J* = 10.4, 2.2 Hz, 1H), 5.08-4.80 (m, 2H), 4.63-4.27(m, 3H), 4.03 (s, 1H), 3.91-3.58 (m, 2H), 3.54-3.41 (m, 1H), 2.83-2.67 (m, 1H), 1.91 (d, *J* = 17.8 Hz, 3H), 1.05 (d, *J* = 6.8 Hz, 3H), 0.91 (t, *J* = 6.3 Hz, 3H).

### Example 57: Preparation of Compound Z57, Z57-1 and Z57-2

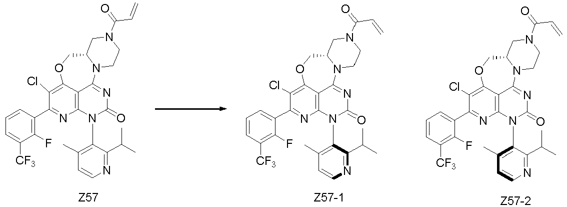

Compound Z57 was prepared with (2-fluoro-3-(trifluoromethyl)phenyl)boronic acid as the raw material according to the method of Example 46. ES-API: [M+H]⁺=644.2. Compound Z57(40mg) was resolved chirally (mobile phase: n-hexane:ethanol =50:50; column type: IG (250mm^{∗}4.6mm ^{∗}5um) ; flow rate: 1.0 ml/min; column temperature: 30 °C) to obtain:Compound Z57-1 (16mg, retention time:7.222min, purity:100 %, de value: 99.5 %). ES-API:[M+H]⁺= 644.2.
and Compound Z57-2 (12mg, retention time: 10.152min, purity: 100%, de value: 99.3%). ES-API:[M+H]⁺= 644.2. ¹H NMR (500 MHz, DMSO-*d*₆) δ 8.36 (d, *J* = 4.8 Hz, 1H), 7.88 (t, *J* = 7.1 Hz, 1H), 7.56-7.43 (m, 2H), 7.16 (d, *J* = 2.9 Hz, 1H), 6.95-6.74 (m, 1H), 6.22 (dd, *J* = 16.7, 2.2 Hz, 1H), 5.78 (dd, *J* = 10.4, 2.2 Hz, 1H), 5.02-4.82 (m, 2H), 4.64-4.54 (m, 1H), 4.43 (dd, *J* = 52.2, 12.1 Hz, 1H), 4.31 (s, 1H), 4.07 (d, *J* = 27.8 Hz, 1H), 3.87-3.57 (m, 2H), 2.83-2.66 (m, 1H), 2.53-2.46 (m, 1 H), 1.95 (d, *J* = 11.2 Hz, 3H), 1.07 (d, *J* = 6.8 Hz, 3H), 0.94 (d, *J* = 6.6 Hz, 3H).

### Example 58: Preparation of Compound Z58

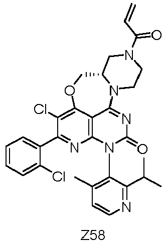

Compound Z58 was prepared with 2-chlorophenylboronic acid as the raw material according to the method of Example 46. ES-API: [M+H]⁺ =591.1. ¹H NMR (500 MHz, DMSO-*d*₆) δ 8.34 (d, *J* = 4.8 Hz, 1H), 7.50 (d, *J* = 7.9 Hz, 1H), 7.43 (td, *J* = 7.7, 1.8 Hz, 1H), 7.41-7.36 (m, 1H), 7.16 (dd, *J* = 13.4, 3.5 Hz, 2H), 6.86 (m, 1H), 6.22 (dd, *J* = 16.7, 2.4 Hz, 1H), 5.78 (dd, *J* = 10.4, 2.4 Hz, 1H), 4.90 (dd, *J* = 26.6, 14.0 Hz, 2H), 4.57 (d, *J* = 28.2 Hz, 1H), 4.42 (dd, *J* = 52.6, 13.4 Hz, 1H), 4.30 (s, 1H), 4.07 (d, *J* = 30.3 Hz, 1H), 3.67 (s, 2H), 3.38 (dd, *J* = 10.2, 5.2 Hz, 1H), 2.84-2.63 (m, 1H), 1.94 (d, *J* = 10.6 Hz, 3H), 1.00 (m, 6.6 Hz, 6H).

### Example 59: Preparation of Compound Z59

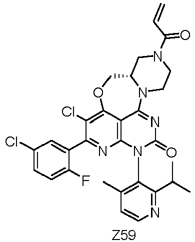

Compound Z59 was prepared with (5-chloro-2-fluorophenyl)boronic acid as the raw material according to the method of Example 46. ES-API:[M+H]⁺ =609.0. ¹H NMR (500 MHz, DMSO-*d*₆) δ 8.38 (d, *J* = 4.8 Hz, 1H), 7.60-7.53 (m, 1H), 7.41-7.33 (m, 1H), 7.23 (td*, J* = 5.9, 2.7 Hz, 1H), 7.18 (dd, *J* = 5.0, 2.1 Hz, 1H), 6.94-6.78 (m, 1H), 6.22 (dd, *J* = 16.6, 2.4 Hz, 1H), 5.78 (dd, *J* = 10.4, 2.3 Hz, 1H), 4.99-4.82 (m, 2H), 4.59 (s, 1H), 4.42 (dd, *J* = 52.5, 13.2 Hz, 1H), 4.29 (s, 1H), 4.07 (d, *J* = 32.5 Hz, 1H), 3.73 (d, *J* = 55.8 Hz, 2H), 3.38 (s, 1H), 2.82-2.68 (m, 1H), 1.95 (d*, J* = 11.7 Hz, 3H), 1.13-0.90 (m, 6H).

### Example 60: Preparation of Compound Z60

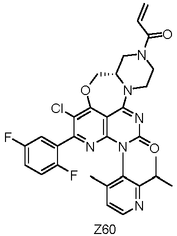

Compound Z60 was prepared with (2, 5-difluorophenyl)boronic acid as the raw material according to the method of Example 46. ES-API:[M+H]⁺ =593.2. ¹H NMR (500 MHz, DMSO-*d*₆) δ 8.37 (d, *J* = 4.9 Hz, 1H), 7.42-7.31 (m, 2H), 7.18 (dd, *J* = 5.0, 2.6 Hz, 1H), 7.03 (q, *J* = 7.1 Hz, 1H), 6.86 (m, 1H), 6.22 (m, 1H), 5.78 (dd, *J* = 10.4, 2.4 Hz, 1H), 5.02-4.80 (m, 2H), 4.66-4.52 (m, 1H), 4.43 (m, H), 4.29 (s, 1H), 4.19-4.00 (m, 1H), 3.74 (d, *J* = 60.6 Hz, 2H), 3.37 (s, 1H), 2.74 (m, 1H), 1.95 (d*, J* = 12.1 Hz, 3H), 1.00 (dd, *J* = 64.3, 6.7 Hz, 6H).

### Example 61: Preparation of Compound Z61

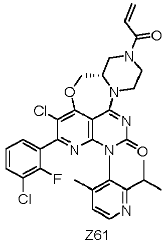

Compound Z61 was prepared with (3-chloro-2-fluorophenyl)boronic acid as the raw material according to the method of Example 46. ES-API:[M+H]⁺ =609.1. ¹H NMR (500 MHz, DMSO-*d*₆) δ 8.36 (d, *J* = 4.9 Hz, 1H), 7.74-7.63 (m, 1H), 7.29 (t, *J* = 7.9 Hz, 1H), 7.20-7.11 (m, 2H), 6.86 (m, 1H), 6.22 (dd, *J* = 16.7, 2.4 Hz, 1H), 5.78 (dd, *J* = 10.3, 2.4 Hz, 1H), 5.02-4.79 (m, 2H), 4.66-4.52 (m, 1H), 4.50-4.34 (m, 1H), 4.30 (s, 1H), 4.07 (d*, J* = 32.6 Hz, 1H), 3.74 (d, *J* = 56.3 Hz, 2H), 3.39 (s, 1H), 2.74 (m, 1H), 1.95 (d*, J* = 12.1 Hz, 3H), 1.01 (dd, *J* = 62.4, 6.7 Hz, 6H).

### Example 62: Preparation of Compound Z62

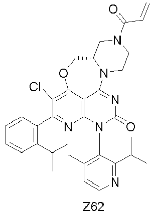

Compound Z62 was prepared with 2-isopropylbenzeneboronic acid as the raw material according to the method of Example 46. ¹H NMR (500 MHz, DMSO-*d*₆) δ 8.32 (d, *J* = 4.2, 1H), 7.34 (d*, J* = 3.1 Hz, 2H), 7.24-7.15 (m, 1H), 7.15-7.08 (m, 1H), 6.96-6.69 (m, 2H), 6.22 (dd, J = 16.7, 2.1 Hz, 1H), 5.78 (d, J = 12.5 Hz, 1H), 4.98-4.80 (m, 2H), 4.67-4.52 (m, 1H), 4.51-4.34 (m, 1H), 4.29 (s, 1H), 4.19-3.99 (m, 1H), 3.88-3.62 (m, 2H), 3.42-3.25 (m, 1H), 2.79-2.64 (m, 1H), 1.92 (d, J = 11.6 Hz, 3H), 1.06 (s, 6H), 0.96-0.86 (m, 3H), 0.83-0.70 (s, 3H).ES-API:[M+H]⁺=599.2.

### Example 63: Preparation of Compound Z63

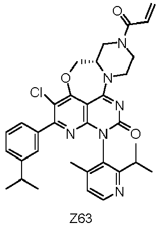

Compound Z63 was prepared with 3-isopropylbenzeneboronic acid as the raw material according to the method of Example 46. ¹H NMR (500 MHz, DMSO-*d*₆) δ 8.41 (dd, *J* = 4.7, 2.5 Hz, 1H), 7.44 (d, *J* = 7.1 Hz, 1H), 7.32-7.26 (m, 2H), 7.24 (s, 1H), 7.18 (t, J = 4.7 Hz, 1H), 6.95-6.77 (m, 1H), 6.22 (d, J = 17.0 Hz, 1H), 5.78 (d, J = 11.7 Hz, 1H), 4.95-4.81 (m, 2H), 4.70-4.55 (m, 1H), 4.50-4.35 (m, 1H), 4.31-4.22 (m, 1H), 4.17-4.00 (m, 1H), 3.79-3.57 (m, 2H), 3.37-3.28 (m, 1H), 2.85-2.71 (m, 2H), 1.94 (d, *J* = 7.5 Hz, 3H), 1.12-1.05 (m, 9H), 1.00 (t, *J* = 7.1 Hz, 3H). ES-API:[M+H]⁺=599.2.

### Example 64: Preparation of Compound Z64

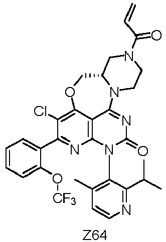

Compound Z64 was prepared with (2-(trifluoromethoxy)phenyl)boronic acid as the raw material according to the method of Example 46. ES-API: [M+H]+=641.1. ¹H NMR (500 MHz, DMSO-*d*₆) δ 8.34 (d, *J* = 4.8 Hz, 1H), 7.59-7.54 (m, 1H), 7.44 (t, *J* = 7.4 Hz, 2H), 7.27-7.12 (m, 2H), 6.87 (d*, J* = 10.2 Hz, 1H), 6.22 (dd, *J* = 16.7, 2.2 Hz, 1H), 5.78 (dd, *J* = 10.4, 2.2 Hz, 1H), 4.88 (d, *J* = 21.2 Hz, 2H), 4.37 (dd, *J* = 53.4, 41.9 Hz, 3H), 4.05 (s, 1H), 3.69 (s, 2H), 3.37 (d, *J* = 5.7 Hz, 1H), 2.72 (s, 1H), 2.05-1.83 (m, 3H), 1.07 (d, *J* = 6.0 Hz, 3H), 0.99-0.78 (m, 3H).

### Example 65: Preparation of Compound Z65

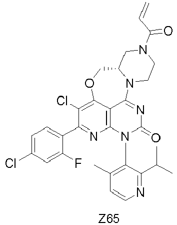

Compound Z65 was prepared with (4-chloro-2-fluorophenyl)boronic acid as the raw material according to the method of Example 46. ES-API: [M+H]+=609.1. ¹H NMR (500 MHz, DMSO-*d*₆) δ 8.37 (d, *J* = 4.8 Hz, 1H), 7.55 (d, *J* = 9.8 Hz, 1H), 7.36 (d, *J* = 8.2 Hz, 1H), 7.25-7.12 (m, 2H), 6.94-6.79 (m, 1H), 6.22 (dd, *J* = 16.7, 2.2 Hz, 1H), 5.78 (dd, *J* = 10.4, 2.1 Hz, 1H), 4.99-4.82 (m, 2H), 4.58 (d*, J* = 15.7 Hz, 1H), 4.42 (dd, *J* = 54.0, 13.6 Hz, 1H), 4.29 (s, 1H), 4.06 (d, *J* = 29.2 Hz, 1H), 3.72 (d, *J* = 48.9 Hz, 2H), 3.41-3.34 (m, 1H), 2.74 (ddd, *J* = 30.8, 13.4, 6.7 Hz, 1H), 1.94 (d*, J* = 12.5 Hz, 3H), 1.07 (d*, J* = 6.7 Hz, 3H), 0.93 (d, *J* = 6.6 Hz, 3H).

### Example 66: Preparation of Compound Z66

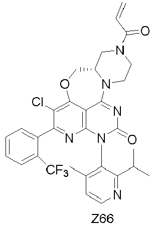

Compound Z66 was prepared with (2-(trifluoromethyl)phenyl)boronic acid as the raw material according to the method of Example 46. ES-API: [M+H]⁺=625.2. ¹H NMR (500 MHz, DMSO-*d*₆) δ 8.31 (d, *J* = 4.6 Hz, 1H), 7.75 (dd, *J* = 27.0, 7.7 Hz, 2H), 7.64 (t, *J* = 7.2 Hz, 1H), 7.29 (dd, *J* = 24.8, 7.5 Hz, 1H), 7.16-7.11 (m, 1H), 6.92-6.79 (m, 1H), 6.22 (dd, *J* = 16.7, 2.2 Hz, 1H), 5.78 (dd, *J* = 10.4, 2.2 Hz, 1H), 4.97-4.78 (m, 2H), 4.63-4.28 (m, 3H), 4.05 (s, 1H), 3.75 (d, *J* = 63.0 Hz, 2H), 3.37 (s, 1H), 2.78-2.66 (m, 1H), 1.91 (t, *J* = 10.0 Hz, 3H), 1.06 (dd, *J* = 11.8, 5.4 Hz, 3H), 0.96-0.84 (m, 3H).

### Example 67: Preparation of Compound Z67

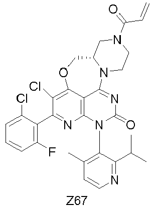

Compound Z67 was prepared with 2-chloro-6-fluorophenylboronic acid as the raw material according to the method of Example 46. ¹H NMR (500 MHz, DMSO-*d*₆) δ 8.35 (d, *J* = 5.0 Hz, 1H), 7.55-7.47 (m, 1H), 7.44-7.39 (m, 1H), 7.37-7.29 (m, 1H), 7.19-7.14 (m, 1H), 6.93-6.77 (m, 1H), 6.22 (d*, J* = 16.6 Hz, 1H), 5.78 (d*, J* = 10.9 Hz, 1H), 5.02-4.83 (m, 2H), 4.65-4.38 (m, 2H), 4.37-4.28 (m, 1H), 4.14-3.99 (m, 1H), 3.90-3.65 (m, 2H), 2.81-2.62 (m, 2H), 1.92 (t, *J* = 14.8 Hz, 3H), 1.08 (d, *J* = 6.6 Hz, 3H), 0.98-0.87 (m, 3H). ES-API:[M+H]⁺=609.1.

### Example 68: Preparation of Compound Z68

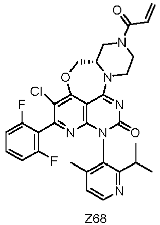

Compound Z68 was prepared with (2,6-difluorophenyl)boronic acid as the raw material according to the method of Example 46. ES-API:[M+H]⁺=594.2. ¹H NMR (500 MHz, DMSO-*d*₆) δ 8.35 (d, *J* = 4.8 Hz, 1H), 7.60-7.49 (m, 1H), 7.30-7.11 (m, 3H), 6.96-6.73 (m, 1H), 6.21 (dd, *J* = 16.7, 2.2 Hz, 1H), 5.78 (dd, *J* = 10.4, 2.2 Hz, 1H), 5.01-4.80 (m, 2H), 4.66-4.24(m, 3H), 4.33-4.25(m, 1H), 4.15-3.95 (m, 1H), 3.81-3.62 (m, 2H), 3.42-3.16 (m, 1 H), 2.85-2.62 (m, 1H), 1.92 (d*, J* = 16.8 Hz, 3H), 1.07 (d, *J* = 6.8Hz, 3H), 0.92 (t, *J* = 6.0 Hz, 3H).

### Example 69: Preparation of Compound Z69

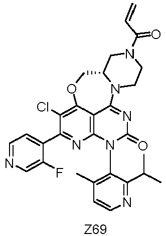

Compound Z69 was prepared with (3-fluoropyrid-4-yl)boronic acid as the raw material according to the method of Example 46. ¹H NMR (500 MHz, DMSO-*d*₆) δ 8.62 (s, 1H), 8.44 (d, *J* = 4.8 Hz, 1H), 8.29 (d, *J* = 4.9 Hz, 1H), 7.24-7.05 (m, 2H), 6.88-6.69 (m, 1H), 6.14 (dd, *J* = 16.7, 2.3 Hz, 1H), 5.71 (dd, *J* = 10.4, 2.3 Hz, 1H), 4.95-4.83 (m, 2H), 4.59-4.18 (m, 3H), 3.98 (d*, J* = 28.4 Hz, 1H), 3.67 (d*, J* = 56.4 Hz, 2H), 3.30 (s, 1H), 2.75-2.59 (m, 1H), 1.88 (d, *J* = 13.2 Hz, 3H), 0.99 (d, *J* = 6.7 Hz, 3H), 0.86 (d, *J* = 6.7 Hz, 3H). ES-API: [M+H]⁺=643.1.

### Example 70: Preparation of Compound Z70

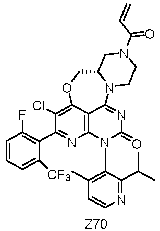

Compound Z70 was prepared with (2-fluoro-6-(trifluoromethyl)phenyl)boronic acid as the raw material according to the method of Example 46. ES-API: [M+H]⁺=643.1. ¹H NMR (500 MHz, DMSO-*d*₆) δ 8.36 (d, *J* = 4.8 Hz, 1H), 7.82 (d*, J* = 9.7 Hz, 1H), 7.67 (d*, J* = 8.0 Hz, 1H), 7.43 (dd, *J* = 14.0, 7.0 Hz, 1H), 7.17 (s, 1H), 6.84 (dd, *J* = 25.9, 14.1 Hz, 1H), 6.22 (dd, *J* = 16.7, 2.2 Hz, 1H), 5.78 (dd, *J* = 10.5, 2.2 Hz, 1H), 5.01-4.82 (m, 2H), 4.64-4.25 (m, 3H), 4.07 (d, *J* = 29.3 Hz, 1H), 3.74 (d, *J* = 56.6 Hz, 2H), 3.37 (s, 1H), 2.83-2.66 (m, 1H), 1.95 (d, *J* = 12.1 Hz, 3H), 1.07 (d, *J* = 6.7 Hz, 3H), 0.94 (d, *J* = 6.6 Hz, 3H).

### Example 71: Preparation of Compound Z71

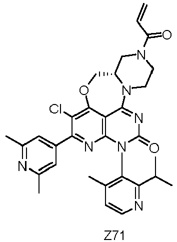

Compound Z71 was prepared with (2,6-dimethylpyridin-4-yl)boronic acid as the raw material according to the method of Example 46. ES-API: [M+H]⁺=586.2. ¹H NMR (500 MHz, DMSO-*d*₆) δ 8.41 (d, *J* = 4.5 Hz, 1H), 7.20 (s, 1H), 7.00 (s, 2H), 6.84 (dd, *J* = 27.0, 14.4 Hz, 1H), 6.22 (d*, J* = 16.8 Hz, 1H), 5.78 (d*, J* = 10.3 Hz, 1H), 5.01-4.77 (m, 2H), 4.66-4.24 (m, 3H), 4.07 (d*, J* = 33.5 Hz, 1H), 3.72 (d*, J* = 53.7 Hz, 2H), 3.37 (s, 1H), 2.87-2.67 (m, 1H), 2.38 (s, 6H), 1.93 (d, *J* = 9.7 Hz, 3H), 1.12-0.91 (m, 6H).

### Example 72: Preparation of Compound Z72

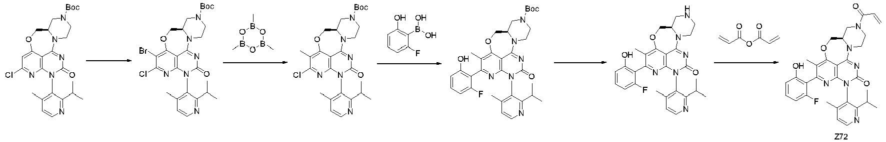

Step 1: tert-butyl (R)-2-chloro-12-(2-isopropyl-4-methylpyridin-3-yl)-11-oxo5α,6,8,9,11,12hexa hydro-4-oxo-1,7,9a,10,12-pentazabenzo[4,5]cyclohepta[1,2,3-de]naphthalen-7( 5H)-carboxylate (0.43 g, 0.82 mmol), N-bromosuccinimide (292 mg, 1.64 mmol) and acetonitrile (5 mL) were added to a round bottom flask. The reaction was stirred at 70°C for 2 hours. Sodium thiosulfate aqueous solution was added to the reaction solution. The reaction solution was extracted with ethyl acetate. The organic phase was dried and concentrated. The crude product was purified by a fast silica gel column (ethyl acetate/petroleum ether:0-50%) to obtain tert-butyl (R)-3-bromo-2-chloro-12-(2-isopropyl-4-methylpyridin-3-yl)-11-oxo-5a,6,8,9, 1 1,12-hexahydro-4-oxo-1,7,9a,10,12-pentazabenzo[4,5]cyclohepta[1,2,3-de]nap hthalen-7(5H)-carboxylate (440 mg), yield of 88%, a yellow solid. ES-API: [M+H]+=605.2.

Step 2: tert-butyl (R)-3-bromo-2-chloro-12-(2-isopropyl-4-methylpyridin-3-yl)-11-oxo-5a,6,8,9, 1 1,12-hexahydro-4-oxo-1,7,9a,10,12-pentazabenzo[4,5]cyclohepta[1,2,3-de]nap hthalen-7(5H)-carboxylate (180 mg, 0.30 mmol), 2,4,6-trimethyl-1,3,5,2,4,6-trioxytriborane (113 mg, 0.90 mmol), Pd(dppf)Cl₂ (22 mg, 0.03 mmol), potassium phosphate (191 mg, 0.9 mmol), 5 mL of dioxane and 1 mL of water were added to a reaction flask. The reaction was stirred under a 70°C oil bath for 12 hours, and the reaction stopped. 20mL of water was added to the reaction solution. The reaction solution was extracted with 20mL of ethyl acetate for 3 times, and the organic phase was dried and concentrated. The crude product was purified by a fast silica gel column (ethyl acetate/petroleum ether:0-60%) to obtain the target product: tert-butyl (R)-2-chloro-12-(2-isopropyl-4-methylpyridin-3-yl)-3-methyl-11-oxo-5a,6,8,9, 11,12-hexahydro-4-oxo-1,7,9a,10,12-pentazabenzo[4,5]cyclohepta[1,2,3-de]na phthalen-7(5H)-carboxylate (110mg, 50% purity), a yellow solid. ES-API:[M+H]⁺ =541.2.

Step 3: tert-butyl (R)-2-chloro-12-(2-isopropyl-4-methylpyridin-3-yl)-3-methyl-11-oxo-5a,6,8,9, 11,12-hexahydro-4-oxo-1,7,9a,10,12-pentazabenzo[4,5]cyclohepta[1,2,3-de]na phthalen-7(5H)-carboxylate (110 mg, 0.20 mmol), 2-fluoro-6-hydroxyphenylboronic acid (156 mg, 1.0 mmol), SPhos (8 mg, 0.02 mmol), SPhos-Pd-G2 (14 mg, 0.02 mmol), potassium phosphate (127 mg, 0.6 mmol), 5 mL of dioxane and 1 mL of water were added to a reaction flask. The reaction was stirred under a 80°C oil bath for 6 hours, and the reaction stopped. 20 mL of water was added to the reaction solution. The reaction solution was extracted with 20 mL of ethyl acetate for 3 times, and the organic phase was dried and concentrated. The crude product was purified by a fast silica gel column (ethyl acetate/petroleum ether:0-60%) to obtain the target product: tert-butyl (5aR)-2-(2-fluoro-6-hydroxyphenyl)-12-(2-isopropyl-4-methylpyridin-3-yl)-3-methyl-11-oxo5α,6,8,9,11,12-hexahydro-4-oxo-1,7,9a,10,12-pentazabenzo[4,5] cyclohepta[1,2,3-de]naphthalen-7(5H)-carboxylate (55 mg, yield of two steps: 30%), a yellow solid. ES-API:[M+H]⁺=617.2.

Step 4: tert-butyl (5aR)-2-(2-fluoro-6-hydroxyphenyl)-12-(2-isopropyl-4-methylpyridin-3-yl)-3-methyl-11-oxo5α,6,8,9,11,12-hexahydro-4-oxo-1,7,9a,10,12-pentazabenzo[4,5] cyclohepta[1,2,3-de]naphthalen-7(5H)-carboxylate (55 mg, 0.089 mmol), 1 mL of methanol and 4 M hydrogen chloride/dioxane solution (3 mL) were added to a round bottom flask. The reaction was stirred at room temperature for 1 hour. The completion of the reaction was detected by LC-MS. The reaction solution was concentrated to obtain (5aR)-2-(2-fluoro-6-hydroxyphenyl)-12-(2-isopropyl-4-methylpyridin-3-yl)-3-methyl-5,5a,6,7,8,9-hexahydro-4-oxo-1,7,9a,10,12-pentazabenzo[4,5]cyclohept an [1,2,3-de]naphthalen-11(12H)-one (46 mg, the crude product), a yellow solid. ES-API:[M+H]⁺=517.2.

Step 5: (5aR)-2-(2-fluoro-6-hydroxyphenyl)-12-(2-isopropyl-4-methylpyridin-3-yl)-3-methyl-5,5a,6,7,8,9-hexahydro-4-oxo-l,7,9a,10,12-pentazabenzo[4,5]cyclohept an [1,2,3-de]naphthalen-11(12H)-one (46 mg, 0.089 mmol), 3 mL of dichloromethane and triethylamine (27 mg, 0.27 mmol) were added to a 50 mL round bottom flask. The reaction was cooled to 0°C, and acrylic anhydride in dichloromethane (31 mg, 0.25 mmol, 0.5 mL) was dropped to the reaction solution. The reaction was stirred at 0°C for 10 minutes. 20 mL of the saturated sodium bicarbonate aqueous solution was added to the reaction solution, and the reaction solution was extracted with 10 mL of dichloromethane for 3 times. The organic phase was dried and concentrated. The crude product was purified by Preparative HPLC to obtain Z72 (17.88 mg, yield of two steps: 35%), a white solid. ¹H NMR (500MHz, CDCl₃) 8.69 (brs, 1H), 8.50-8.49 (m, 1H), 7.19-7.13 (m, 2H), 6.61-6.53 (m, 3H), 6.35-6.31 (m, 1H), 5.78 (d, *J*=8 Hz, 1H), 5.01-4.51 (m, 4H), 4.02-3.31 (m, 5H), 2.78-2.76 (m, 1H), 2.05-2.04 (m, 6H), 1.22-1.20 (m, 3H), 1.03-1.00 (m, 3H). ES-API: [M+H]+=571.2.

### Example 73: Preparation of Compound Z73, Z73-1 and Z73-2

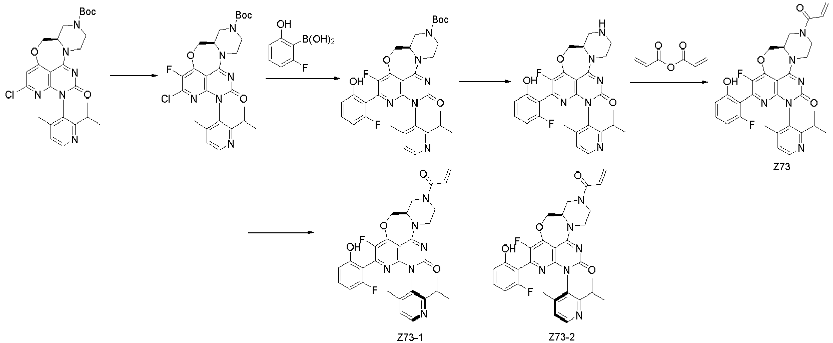

Step 1: tert-butyl (R)-2-chloro-12-(2-isopropyl-4-methylpyridin-3-yl)-11-oxo5α,6,8,9,11,12hexa hydro-4-oxo-1,7,9a,10,12-pentazabenzo[4,5]cyclohepta[1,2,3-de]naphthalen-7( 5H)-carboxylate (1.1 g, 2.09 mmol), Selectfluor (3.69 g, 10.4 mmol) and 50 mL of acetonitrile were added to a round bottom flask. The reaction was stirred at 80°C for 2 hours. The reaction solution was poured into ice water. The reaction solution was extracted with ethyl acetate. The organic phase was dried with sodium sulfate and concentrated. The crude product was purified by a fast silica gel column (methanol /dichloromethane:0-3%) to obtain tert-butyl (R)-2-chloro-3-fluoro-12-(2-isopropyl-4-methylpyridin-3-yl)-11-oxo-5a,6,8,9,1 1,12-hexahydro-4-oxo-1,7,9a,10,12-pentazabenzo[4,5]cyclohepta[1,2,3-de]nap hthalen-7(5H)-carboxylate (0.6 g, purity8%). The crude product was directly used in the next step. ES-API: [M+H]⁺=545.2.

Steps 2-4: Compound Z73 was prepared according to the method of steps 3-5 in Example 72. ¹H NMR (400 MHz, DMSO-*d*₆) δ 10.25 (s, 1H), 8.36 (d, *J*=3.8 Hz, 1H), 7.23-7.22 (m, 1H), 7.15 (d, *J*=3.8 Hz, 1H), 6.82-6.70 (m, 1H), 6.63-6.61 (m, 2H), 6.22-6.17 (m, 1H), 5.78-5.77 (m, 1H), 4.88-4.80 (m, 2H), 4.46-4.02 (m, 4H), 3.82-3.72 (m, 2H), 3.34-3.32 (m, 1H), 2.77-2.66 (m, 1H), 1.92-1.90(m, 3H), 1.07-1.06 (m, 3H), 0.94-0.92 (m, 3H). ES-API: [M+H]⁺=575.2.

Step 5: Compound Z73 (15mg) was resolved chirally (mobile phase: n-hexane:ethanol =60:40; column type: IG (250mm^{∗}4.6mm ^{∗}5um); flow rate: 1.0 ml/min; column temperature: 30 °C) to obtain:Compound Z73-1 (5.6mg, retention time: 10.149 min, purity: 100 %, de value: 100 %). ES-API:[M+H]⁺= 575.2. and Compound Z73-2 (6.5mg, retention time: 13.445min, purity:100%, de value: 99%). ES-API:[M+H]⁺= 575.2.

### Example 74: Preparation of Compound Z74

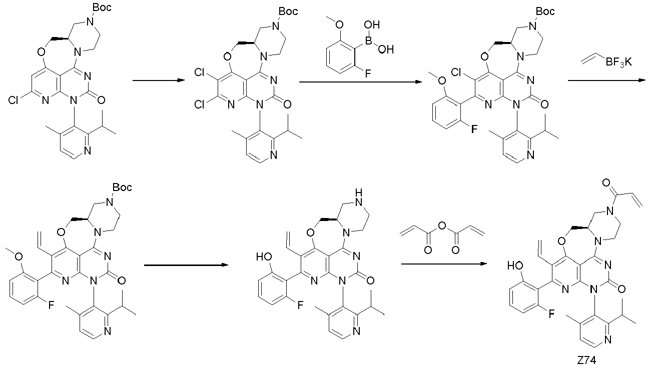

Step 1: tert-butyl (R)-2-chloro-12-(2-isopropyl-4-methylpyridin-3-yl)-11-oxo-5a,6,8,9,11,12-hex ahydro-4-oxo-1,7,9a,10,12-pentazabenzo[4,5]cyclohepta[1,2,3-de]naphthalen-7 (5H)-carboxylate (1 g, 1.9 mmol) and a solution of N-chlorosuccinimide (507 mg, 3.8 mmol) in acetonitrile (20 mL) were stirred at 80°C for 3 hours under nitrogen protection. The reaction solution was filtered, concentrated and purified by a fast silica gel column (0-10%methanol /dichloromethane) to obtain a white solid: tert-butyl (R)-2,3-dichloro-12-(2-isopropyl-4-methylpyridin-3-yl)-11-oxo-5a,6,8,9, 11,12-hexahydro-4-oxo-1,7,9a,10,12-pentazabenzo[4,5]cyclohepta[1,2,3-de]naphthal en-7(5H)-carboxylate (1 g, yield: 94%). ES-API:[M+H]⁺=561.1.

Step 2: tert-butyl (R)-2,3-dichloro-12-(2-isopropyl-4-methylpyridin-3-yl)-11-oxo-5a,6,8,9, 11,12-hexahydro-4-oxo-1,7,9a,10,12-pentazabenzo[4,5]cyclohepta[1,2,3-de]naphthal en-7(5H)-carboxylate (300 mg, 0.53 mmol), 2-fluoro-6-methoxyphenylboronic acid (270 mg, 1.60 mmol), [1,1'-bis(diphenylphosphino)ferrocene]palladium dichloride (39 mg, 0.05 mmol) and a mixed solution of potassium carbonate (220 mg, 1.60 mmol) in 1,4-dioxane (5 mL) and water (1 mL) reacted under a 120°C microwave for 2 hours under nitrogen protection. The reaction solution was filtered, washed with ethyl acetate (30 mL), the filtrate was washed with the saturated brine (10 mL^{∗}3), the obtained organic phase was dried and concentrated, purified with a fast silica column (0-10%methanol /dichloromethane) to obtain a yellow solid: tert-butyl (5aR)-3-chloro-2-(2-fluoro-6-methoxyphenyl)-12-(2-isopropyl-4-methylpyridin -3-yl)-11-oxo-5a,6,8,9,11,12-hexahydro-4-oxo-1,7,9a,10,12-pentazabenzo[4,5] cyclohepta[1,2,3-de]naphthalen-7(5H)-carboxylate (220 mg, purity: 63%). ES-API: [M+H]⁺=651.2.

Step 3: tert-butyl (5aR)-3-chloro-2-(2-fluoro-6-methoxyphenyl)-12-(2-isopropyl-4-methylpyridin -3-yl)-11-oxo-5a,6,8,9,11,12-hexahydro-4-oxo-1,7,9a,10,12-pentazabenzo[4,5] cyclohepta[1,2,3-de]naphthalen-7(5H)-carboxylate (200 mg, 0.3 mmol), potassium vinyltrifluoroborate (120 mg, 0.9 mmol), chloro(2-dicyclohexylphosphino-2',6'-dimethoxy-1,1'-biphenyl)(2'-amino-1,1'-b iphenyl-2-yl)palladium(II) (20 mg, 0.03 mmol), 2-bicyclohexylphosphin-2',6'-dimethoxybiphenyl (12 mg, 0.03 mmol) and a mixed solution of potassium phosphate (190 mg, 0.9 mmol) in 1,4-dioxane (2 mL) and water (0.4 mL) reacted under a 150°C microwave for 2 hours under nitrogen protection. The reaction solution was filtered, washed with ethyl acetate (30 mL), the filtrate was washed with the saturated brine (10 mL^{∗}3), the obtained organic phase was dried and concentrated, purified with a fast silica column (0-10%methanol /dichloromethane) to obtain a yellow solid: tert-butyl (5aR)-2-(2-fluoro-6-methoxyphenyl)-12-(2-isopropyl-4-methylpyridin-3-yl)-11 -oxo-3-vinyl-5a,6,8,9,11,12-hexahydro-4-oxo-1,7,9a,10,12-pentazabenzo[4,5]c yclohepta[1,2,3-de]naphthalen-7(5H)-carboxylate (120 mg, purity:35%). ES-API:[M+H]⁺=643.3.

Step 4: 17% boron tribromide in dichloromethane solution(2 mL) was added to a solution of tert-butyl (5aR)-2-(2-fluoro-6-methoxyphenyl)-12-(2-isopropyl-4-methylpyridin-3-yl)-11 -oxo-3-vinyl-5a,6,8,9,11,12-hexahydro-4-oxo-1,7,9a,10,12-pentazabenzo[4,5]c yclohepta[1,2,3-de]naphthalen-7(5H)-carboxylate (120 mg, 0.19 mmol) in dichloromethane(2 mL) under the ice bath, and stirred at room temperature for 3 hours. The reaction solution was quenched with methanol (5 mL), poured into the saturated sodium bicarbonate solution (20 mL), and extracted with dichloromethane (20 mL^{∗}3), and the organic phase was dried and concentrated to obtain a yellow solid: (5aR)-2-(2-fluoro-6-hydroxyphenyl)-12-(2-isopropyl-4-methylpyridin-3-yl)-3-vinyl-5,5a,6,7,8,9-hexahydro-4-oxo-1,7,9a,10,12-pentazabenzo[4,5]cyclohepta[ 1,2,3-de]naphthalen-11(12H)-one (100 mg). ES-API:[M+H]⁺=529.2.

Step 5: *N*,*N*-diisopropylethylamine(74 mg, 0.57 mmol) was added to a solution of (5aR)-2-(2-fluoro-6-hydroxyphenyl)-12-(2-isopropyl-4-methylpyridin-3-yl)-3-vinyl-5,5a,6,7,8,9-hexahydro-4-oxo-1,7,9a,10,12-pentazabenzo[4,5]cyclohepta[ 1,2,3-de]naphthalen-11(12H)-one (100 mg, 0.19 mmol) in dichloromethane (2 mL) under the ice bath. After the reaction solution was clarified, acrylic anhydride (17 mg, 0.19 mmol) was dropped, and stirred for 5 minutes. The reaction solution was washed with the saturated sodium bicarbonate solution (5 mL). The organic phase was dried and concentrated, then purified by Preparative HPLC (ammonium bicarbonate system) to obtain a white solid: Z74 (2.4 mg, purity: 94%, yield:10%). ES-API:[M+H]⁺=583.2.

### Example 75: Preparation of Compound Z75

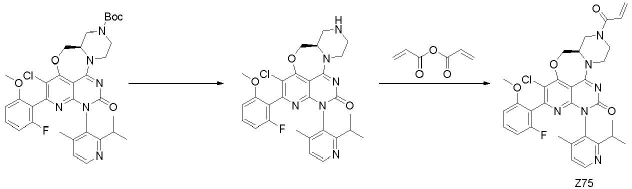

Compound Z75 was prepared according to the method of steps 2-3 in Example 34. ES-API: [M+H]⁺=605.2.

### Example 76: Preparation of Compound Z76, Z76-1 and Z76-2

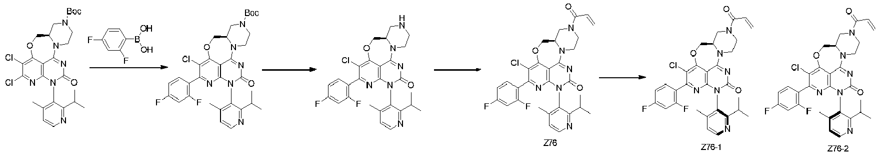

Compound Z76 was prepared with (2,4-difluorophenyl)boronic acid as the raw material according to the method of Example 46. ES-API: [M+H]⁺= 593.1.

Compound Z76 was prepared and resolved chirally (column type: IG, 250mm^{∗}4.6mm^{∗}5um, mobile phase: n-hexane:ethanol=40:60,flow rate:1ml/min, column temperature=30°C) to obtain:Compound Z76-1 (50.7mg, peak 1, retention time 8.084 min, yield: 18%), a white solid. ES-API: [M+H]+=593.1. ¹H NMR (500 MHz, DMSO-*d*₆) δ 8.37 (d, *J* = 4.8 Hz, 1H), 7.35 (td*, J* = 10.1, 2.4 Hz, 1H), 7.28-7.11 (m, 3H), 6.96-6.76 (m, 1H), 6.22 (dd, *J* = 16.7, 2.3 Hz, 1H), 5.78 (dd, *J* = 10.4, 2.2 Hz, 1H), 4.97-4.82 (m, 2H), 4.65-4.23 (m, 3H), 4.07 (d, *J* = 20.5 Hz, 1H), 3.71 (dd, *J* = 35.3, 22.8 Hz, 2H), 3.37 (s, 1H), 2.70 (dt, *J* = 13.4, 6.7 Hz, 1H), 1.96 (s, 3H), 1.06 (d, *J* = 6.7 Hz, 3H), 0.93 (d, *J*= 6.7 Hz, 3H).
and Compound Z76-2 (53.4mg, peak 2, retention time 11.484 min, yield: 19%), a white solid. ES-API: [M+H]⁺=593.1. ¹H NMR (500 MHz, DMSO-*d*₆) δ 8.37 (d, *J* = 4.8 Hz, 1H), 7.35 (td, *J* = 10.2, 2.4 Hz, 1H), 7.16 (dd, *J* = 9.7, 3.5 Hz, 3H), 6.95-6.76 (m, 1H), 6.22 (dd, *J* = 16.7, 2.3 Hz, 1H), 5.78 (dd, *J* = 10.4, 2.3 Hz, 1H), 5.00-4.79 (m, 2H), 4.64-4.26 (m, 3H), 4.07 (d, *J* = 31.9 Hz, 1H), 3.70 (dd, *J* = 40.4, 28.8 Hz, 2H), 3.38 (s, 1H), 2.77 (dt, *J* = 13.5, 6.8 Hz, 1H), 1.93 (s, 3H),1.06 (d, *J* = 6.7 Hz, 3H), 0.93 (d, *J* = 6.7 Hz, 3H).

### Example 77: Preparation of Compound Z77, Z77-1 and Z77-2

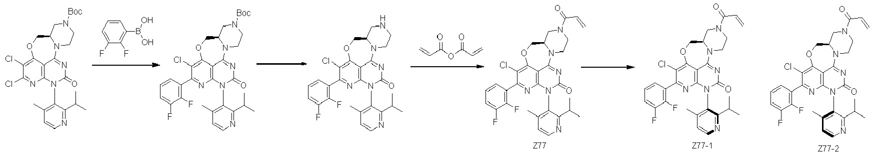

Compound Z77 was prepared with (2,3-difluorophenyl)boronic acid as the raw material according to the method of Example 46. ES-API: [M+H]⁺=593.1. Compound Z77 was prepared and resolved chirally (column type: IG, 250mm^{∗}4.6mm^{∗}5um, mobile phase: n-hexane:ethanol=40:60,flow rate:1ml/min, column temperature=30°C) to obtain:Compound Z77-1 (45.3mg,peak 1, retention time 7.568 min, yield:24%), a white solid. ¹H NMR (500 MHz, DMSO-*d*₆) δ 8.37 (d, *J* = 4.8 Hz, 1H), 7.54 (dt, *J* = 17.2, 5.6 Hz, 1H), 7.27 (dd, *J* = 12.5, 7.7 Hz, 1H), 7.17 (d, *J* = 4.9 Hz, 1H), 7.05-6.77 (m, 2H), 6.24 (s, 1H), 5.78 (dd, *J* = 10.4, 2.2 Hz, 1H), 4.90 (d, *J* = 4.6 Hz, 2H), 4.65-4.26 (m, 3H), 4.06 (d, *J* = 29.1 Hz, 1H), 3.72 (dd, *J* = 36.9, 24.2 Hz, 2H), 3.41-3.36 (m, 1H), 2.70 (dt*, J* = 13.4, 6.6 Hz, 1H), 1.96 (s, 3H), 1.06 (d*, J* = 6.7 Hz, 3H), 0.94 (d, J = 6.7 Hz, 3H).
and Compound Z77-2 (37.8mg, peak 2, retention time 9.781 min, yield:20%), a white solid. ¹H NMR (500 MHz, DMSO-*d*₆) δ 8.37 (d, *J* = 4.8 Hz, 1H), 7.52 (dd, *J* = 17.1, 8.3 Hz, 1H), 7.17 (d, *J* = 5.2 Hz, 2H), 7.07-6.75 (m, 2H), 6.22 (dd, *J* = 16.7, 2.3 Hz, 1H), 5.85-5.72 (m, 1H), 5.03-4.81 (m, 2H), 4.69-4.26 (m, 3H), 4.07 (d, *J* = 32.9 Hz, 1H), 3.71 (dd, *J* = 40.6, 28.9 Hz, 2H), 3.36 (d, *J* = 10.8 Hz, 1H), 2.78 (dt*, J* = 13.3, 6.6 Hz, 1H), 1.93 (s, 3H), 1.06 (d, J = 6.7 Hz, 3H), 0.93 (d, J = 6.7 Hz, 3H).

### Example 78: Preparation of Compound Z78

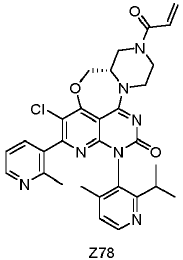

Compound Z78 was prepared with 2-methyl-3-(4,4,5,5-tetramethyl-1,3,2-dioxaboran-2-yl)pyridine as the raw material according to the method of Example 46. ES-API: [M+H]⁺=572.2. ¹H NMR (500 MHz, DMSO-*d*₆) δ 8.46 (d, J = 3.6 Hz, 1H), 8.35 (d, J = 4.8 Hz, 1H), 7.53 - 7.46 (m, 1H), 7.27 (dd, J = 7.7, 4.9 Hz, 1H), 7.17 (d, J = 4.9 Hz, 1H), 6.92 - 6.80 (m, 1H), 6.22 (dd, J = 16.7, 2.3 Hz, 1H), 5.79 (dd, J = 10.4, 2.3 Hz, 1H), 4.99 - 4.79 (m, 2H), 4.71 - 4.24 (m, 3H), 4.08 (d, J = 32.5 Hz, 1H), 3.88 - 3.62 (m, 2H), 3.33-3.29 (m, 1H), 2.78 - 2.65 (m, 1H), 2.10 (s, 3H), 1.95 (d, J = 12.3 Hz, 3H), 1.10 - 1.00 (m, 3H), 0.89 (dd, J = 6.6, 3.6 Hz, 3H).

### Example 79: Preparation of Compound Z79

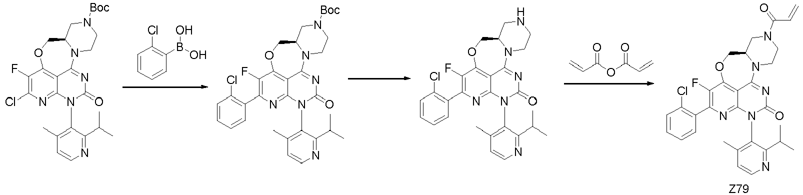

Compound Z79 was prepared with 2-chloro-phenylboronic acid as the raw material according to the method of steps 3-5 in Example 72. ES-API: [M+H]⁺=576.3. ¹H NMR (500 MHz, DMSO-*d*₆) δ 8.36 (d, *J* = 4.8 Hz, 1H), 7.54 (d*, J* = 8.0 Hz, 1H), 7.47 (td*, J* = 7.8, 1.6 Hz, 1H), 7.40 (dd, *J* = 7.5, 6.6 Hz, 1H), 7.27-7.21 (m, 1H), 7.17 (d, *J* = 4.8 Hz, 1H), 6.92-6.77 (m, 1H), 6.22 (dd, *J* = 16.7, 2.1 Hz, 1H), 5.78 (dd, *J* = 10.4, 2.3 Hz, 1H), 4.97-4.79 (m, 2H), 4.58 (s, 1H), 4.51-4.26(m, 2H), 4.05 (s, 1H), 3.93-3.66 (m, 2H), 2.78 (dt, *J* = 13.6, 6.8 Hz, 1H), 2.73-2.60 (m, 1H), 1.95 (d, *J* = 17.0Hz, 3H), 1.07 (d, *J*= 6.8 Hz, 3H), 0.94 (dd, *J* = 6.4, 4.8 Hz, 3H).

### Example 80: Preparation of Compound Z80

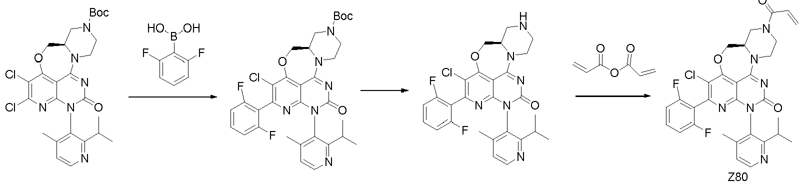

Compound Z80 was prepared according to the method of Example 46. ES-API: [M+H]⁺=594.2. ¹H NMR (500 MHz, DMSO-*d*₆) δ 8.35 (d, *J* = 4.8 Hz, 1H), 7.62-7.47 (m, 1H), 7.30-7.08 (m, 3H), 6.94-6.75 (m, 1H), 6.22 (dd, *J* = 16.7, 2.3 Hz, 1H), 5.78 (dd, *J* = 10.4, 2.2 Hz, 1H), 4.99-4.78 (m, 2H), 4.49-4.43 (m, 2H), 4.33-4.26 (m, 2H), 4.16-3.95(m, 1H), 3.82-3.61 (m, 2H), 2.88-2.63 (m, 1H), 1.92 (d, *J* = 17.0 Hz, 3H), 1.07 (d, *J* = 6.8 Hz, 3H), 0.91 (t, *J* = 6.0 Hz, 3H).

### Example 81: Preparation of Compound Z81

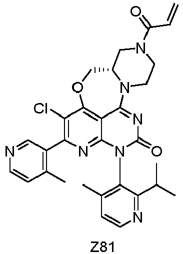

Compound Z81 was prepared with 4-methyl-3-(4,4,5,5-tetramethyl-1,3,2-dioxaboran-2-yl)pyridine as the raw material according to the method of Example 46. ES-API: [M+H]⁺=572.2.

### Example 82: Preparation of Compound Z82, Z82-1 and Z82-2

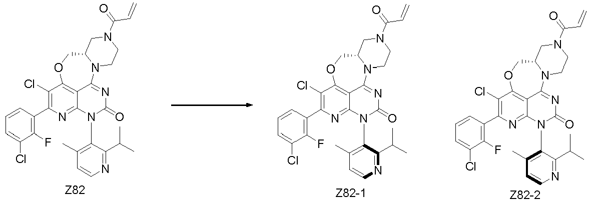

Compound Z82 was prepared with (3-chloro-2-fluorophenyl)boronic acid as the raw material according to the method of Example 46, resolved chirally (column type: Chiralpak IG 250mm^{∗}4.6mm^{∗}5um; mobile phase: n-hexane: ethanol=50:50; flow rate:1ml/min; column temperature=30°C) to obtain: Compound Z82-1(13mg, retention time:9.805 min; peak 1, yield4.1%). ¹H NMR (400 MHz, DMSO-*d*₆) δ 8.37 (d, *J* = 4.8 Hz, 1H), 7.68 (t*, J* = 6.8 Hz, 1H), 7.28 (d*, J* = 8.2 Hz, 1H), 7.16 (dd, *J* = 13.4, 6.5 Hz, 2H), 6.84 (d, *J* = 14.1 Hz, 1H), 6.22 (dd, *J* = 16.4, 2.1 Hz, 1H), 5.80-5.77 (m, 1H), 4.99-4.81 (m, 2H), 4.65-4.25 (m, 3H), 4.15-3.97 (m, 1H), 3.75-3.48 (m, 2H), 3.32 (s, 1H), 2.82-2.75 (m, 1H), 1.93 (s, 3H), , 1.07 (d*, J* = 6.7 Hz, 3H), 0.94 (d*, J* = 6.7 Hz, 3H).
and Compound Z82-2 (8 mg, retention time: 13.724 min; peak 2, yield2.5%).

### Example 83: Preparation of Compound Z83, Z83-1 and Z83-2

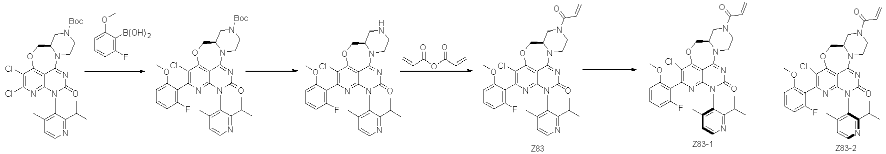

Compound Z83 was prepared with (2-fluoro-6-methoxyphenyl)boronic acid as the raw material according to the method of steps 3-5 in Example 72, resolved chirally (column type: Chiralpak IC 250mm^{∗}4.6mm^{∗}5um mobile phase: acetonitrile :isopropanol=60:40; flow rate:1ml/min; column temperature=30°C) to obtain Compound Z83-1 (8mg, retention time:5.005min;peak 1, yield2.6%)and Compound Z83-2 (8 mg, retention time:6.658 min; peak 2, yield2.6%).

### Example 84: Preparation of Compound Z84

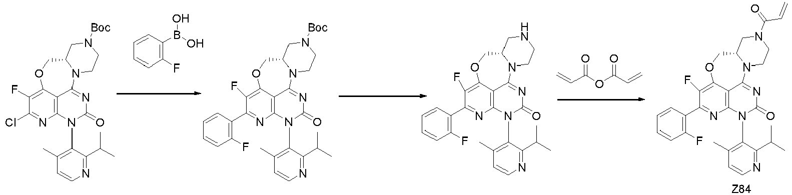

Compound Z84 was prepared according to the method of steps 3-5 in Example 72 . ES-API: [M+H]⁺=559.2.

### Example 85: Preparation of Compound Z85

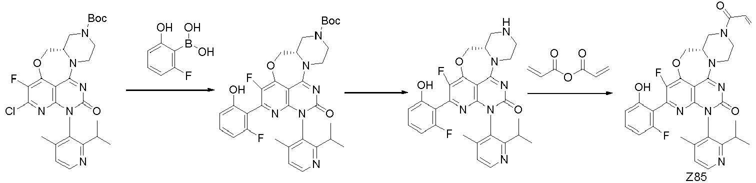

Compound Z85 was prepared according to the method of steps 3-5 in Example 72 . ES-API: [M+H]⁺=575.2. ¹H NMR (500 MHz, DMSO-*d*₆) δ 10.16 (s, 1H), 8.36 (d, *J* = 4.8 Hz, 1H), 7.25 (dd, *J* = 15.4, 8.2 Hz, 1H), 7.16 (d, *J =* 4.9 Hz, 1H), 6.99-6.78 (m, 1H), 6.74-6.63 (m, 2H), 6.21 (dd, *J* = 16.7, 2.3 Hz, 1H), 5.78 (dd, *J* = 10.4, 2.3 Hz, 1H), 4.96-4.77 (m, 2H), 4.60 (s, 1H), 4.39 (dd, *J* = 50.2, 19.6 Hz, 2H), 4.05 (s, 1H), 3.75 (dd, *J* = 38.5, 27.0 Hz, 2H), 3.38 (s, 1H), 2.83-2.63 (m, 1H), 1.92 (d, *J* = 14.2 Hz, 3H), 1.07 (d, *J* = 6.7 Hz, 3H), 0.94 (t, *J* = 7.1 Hz, 3H).

### Example 86: Preparation of Compound Z86

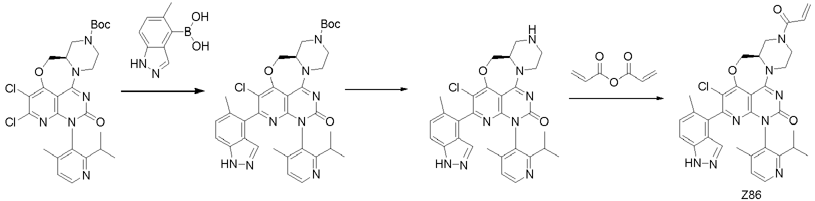

Compound Z86 was prepared with (5-methyl-1H-indazol-4-yl)boronic acid as the raw material according to the method of Example 46. ES-API: [M+H]+=611.2. ¹H NMR (400 MHz, DMSO-*d*₆) δ 13.08 (s, 1H), 8.28 (t, *J* = 4.5 Hz, 1H), 7.45 (t, *J* = 7.8 Hz, 2H), 7.25-7.01 (m, 2H), 6.90 (d*, J* = 26.1 Hz, 1H), 6.23 (dd, *J* = 16.7, 2.1 Hz, 1H), 5.79 (dd, *J* = 10.5, 2.2 Hz, 1H), 5.00-4.81 (m, 2H), 4.67-4.29 (m, 3H), 4.08 (s, 1H), 3.71 (s, 2H), 3.38 (d, *J* = 10.1 Hz, 1H), 2.84-2.70 (m, 1H), 1.96 (dd, *J* = 17.1, 12.1 Hz, 6H), 1.10-0.72 (m, 6H).

### Example 87: Preparation of Compound Z87, Z87-1 and Z87-2

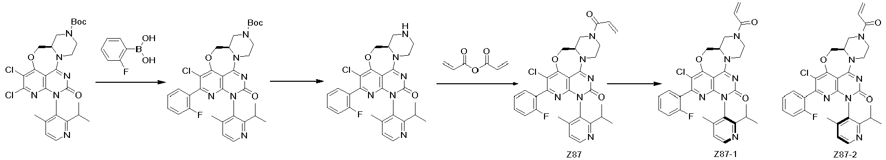

Compound Z87 was prepared with 2-fluorophenylboronic acid as the raw material according to the method of Example 46. ES-API: [M+H]⁺=575.2. 290mg Compound Z87 was prepared and resolved chirally (column type: IG, 250mm^{∗}4.6mm^{∗}5um, mobile phase: n-hexane:ethanol=40:60,flow rate:1ml/min, column temperature=30°C) to obtain: Compound Z87-1 (93.98mg,peak 1, retention time8.384 min, yield:32.4%), a white solid. ¹H NMR (500 MHz, DMSO-*d*₆) δ 8.36 (d, *J* = 4.9 Hz, 1H), 7.70 - 7.42 (m, 2H), 7.37 - 7.12 (m, 2H), 7.05 - 6.76 (m, 2H), 6.21 (dd, *J* = 16.6, 2.4 Hz, 1H), 5.77 (dd, *J* = 10.4, 2.4 Hz, 1H), 5.03 - 4.83 (m, 2H), 4.69 - 4.22 (m, 3H), 4.05 (d, *J* = 29.7 Hz, 1H), 3.87 - 3.60 (m, 2H), 3.39-3.34 (m, 1H), 2.70 (p, *J* = 6.7 Hz, 1H), 1.95 (s, 3H), 1.06 (d, *J* = 6.7 Hz, 3H), 0.94 (d, *J* = 6.7 Hz, 3H).
and Compound Z87-2(95.37mg, peak 2, retention time11.618 min, yield:32.7%), a white solid. ¹H NMR (400 MHz, DMSO-*d*₆) δ 8.29 (d, *J* = 4.8 Hz, 1H), 7.46-7.35 (m, 1H), 7.26-7.04 (m, 4H), 6.89-6.69 (m, 1H), 6.14 (dd, *J* = 16.7, 2.3 Hz, 1H), 5.71 (dd, *J* = 10.4, 2.2 Hz, 1H), 4.94-4.73 (m, 2H), 4.60-4.15 (m, 3H), 4.11-3.92 (m, 1H), 3.76-3.50 (m, 2H), 3.39-3.34 (m, 1H), 2.71 (dt*, J* = 13.5, 6.8 Hz, 1H), 1.86 (s, 3H), 1.06 (d*, J* = 6.7 Hz, 3H), 0.93 (d, *J* = 6.7 Hz, 3H).

### Example 88: Preparation of Compound Z88, Z88-1 and Z88-2

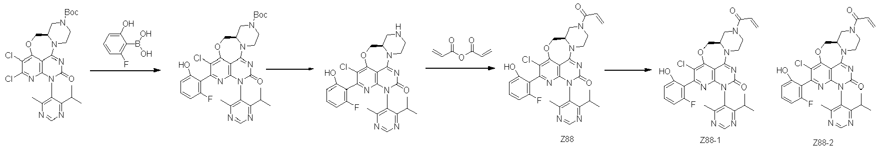

Compound Z88 was prepared with (2-fluoro-6-hydroxyphenyl)boronic acid as the raw material according to the method of Example 46. ES-API: [M+H]⁺=592.2. Compound Z88 was prepared and resolved chirally (column type: IG, 250mm^{∗}4.6mm^{∗}5um, mobile phase: n-hexane:ethanol=65:35,flow rate:1ml/min, column temperature=30°C) to obtain Compound Z88-1(4.83mg,peak 1, retention time 10.605 min, yield: 10.7%), a white solid. and Compound Z88-2(13.64mg, peak 2, retention time14.649 min, yield:30.3%), a white solid.

### Example 89: Preparation of Compound Z89

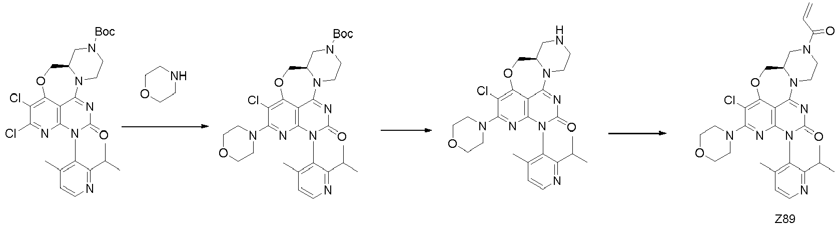

Step 1: morpholine (17.4 mg, 0.2mmol) and 60% NaH (12mg, 0.3mmol) was dissolved in dry DMF (3ml) under the ice water bath, stirred for 10 minutes, tert-butyl (R)-2,3-dichloro-12-(2-isopropyl-4-methylpyridin-3-yl)-11-oxo-5a,6,8,9,11,12-hexahydro-4-oxo-1,7,9a,10,12-pentazabenzo[4,5]cyclohepta[1,2,3-de]naphthal en-7(5H)-carboxylate (56mg, 0.1mmoL) was slowly added, the reaction rised to room temperature and proceeded for 1 hour. The reaction solution was poured into ice water, washed with water and the saturated brine, concentrated, and purified by column chromatography to obtain tert-butyl (R)3-chloro-2-(morpholin)-12-(2-isopropyl-4-methylpyridin-3-yl)-11-oxo-5a,6, 8,9,11,12-hexahydro-4-oxo-1,7,9a,10,12-pentazabenzo[4,5]cyclohepta[1,2,3-de ]naphthalen-7(5H)-carboxylate (30mg, yield49%). ES-API:[M+H]⁺ =612.3.

Steps 2-3: Compound Z89 was prepared according to the corresponding steps of Example 34. ES-API:[M+H]⁺ =566.2.

### Example 90: Preparation of Compound Z90

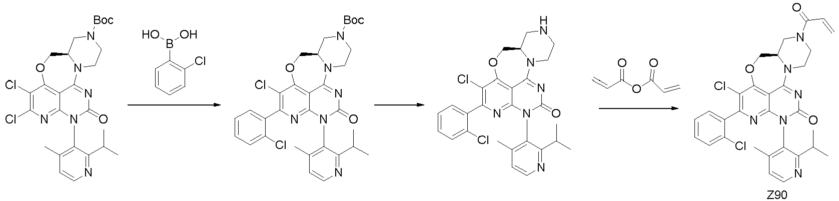

Compound Z90 was prepared with (2-chlorophenyl)boronic acid as the raw material according to the method of Example 46. ES-API: [M+H]⁺=592.2. ¹H NMR (500 MHz, DMSO-*d*₆) δ 8.27 (d, *J* = 4.8 Hz, 1H), 7.44 (s, 0H), 7.41-7.26 (m, 2H), 7.09 (t, *J* = 6.7 Hz, 2H), 6.89-6.64 (m, 1H), 6.14 (dd, *J* = 16.8, 2.3 Hz, 1H), 5.71 (dd, *J* = 10.4, 2.3 Hz, 1H), 4.83 (dd, *J* = 23.8, 12.6 Hz, 2H), 4.61-4.35 (m, 2H), 4.33-4.13 (m, 1H), 3.97 (s, 1H), 3.59 (s, 2H), 3.34-3.21 (m, 1 H), 2.67 (d, *J* = 31.0 Hz, 1H), 1.87 (d, *J* = 10.4 Hz, 3H), 1.00 (d*, J* = 6.6 Hz, 3H), 0.86 (d, *J* = 6.4 Hz, 3H).

### Example 91: Preparation of Compound Z91, Z91-1 and Z91-2

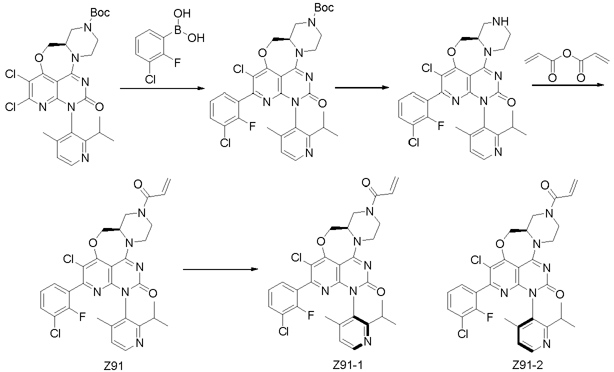

Compound Z91 was prepared with (3-chloro-2-fluorophenyl)boronic acid as the raw material according to the method of Example 46. ES-API: [M+H]⁺=610.3. Compound Z91 (274mg, 0.45mmol) was resolved chirally (mobile phase: n-hexane-ethanol-40-60); column type: IG (250mm^{∗}4.6mm 5um) ; flow rate: 1.0 ml/min; column temperature: 30 °C) to obtain:Compound Z91-1 (103mg, retention time: 8.228min, purity:99 %, de value: 99 %). ES-API:[M+H]⁺= 610.3.
and Compound Z91-2 (109mg, retention time: 10.820min, purity:99 %, de value: 99%). ES-API:[M+H]⁺= 610.3. ¹H NMR (500 MHz, DMSO-*d*₆) δ 8.36 (d, *J* = 4.8 Hz, 1H), 7.68 (t, *J* = 7.4 Hz, 1H), 7.29 (t, *J* = 8.0Hz, 1H), 7.24-7.10 (m, 2H), 7.06-6.71 (m, 1H), 6.22 (dd, *J* = 16.8, 2.0 Hz, 1H), 5.78 (dd, *J* = 10.4, 2.0 Hz, 1H), 5.07-4.75 (m, 2H), 4.70-4.22 (m, 3H), 4.16-3.98 (m, 1H), 3.84-3.57 (m, 2H), 3.40-3.29 (m, 1H), 2.89-2.63 (m, 1H), 1.94 (d*, J* = 12.0 Hz, 3H), 1.06 (d*, J* = 6.7 Hz, 3H), 0.94 (d*, J* = 6.6 Hz, 3H).

### Example 92: Preparation of Compound Z92

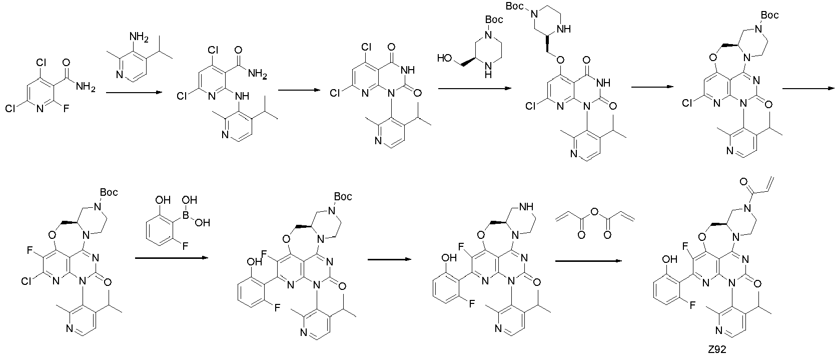

Step 1: 4-isopropyl-2-methylpyridin-3-amine (1 g, 6.7 mmol) and 30 mL of tetrahydrofuran were added to a 250 mL round bottom flask. After the system cooling to 0°C, sodium bis(trimethylsilyl)amide (8.3mL, 2 M in tetrahydrofuran, 16.5 mmol) was dropped to the reaction solution. After the completion of the dropping, the reaction was stirred at 0°C for 10 minutes. and then a solution of 4,6-dichloro-2-fluoronicotinamide(1.4g, 6.7 mmol) in tetrahydrofuran (30 mL) was dropped to the reaction solution. After the completion of the dropping, the reaction was stirred at 0°C for 20 minutes. After the completion of the reaction, the reaction solution was poured into ice water. The reaction solution was extracted with ethyl acetate. The organic phase was washed with 1M hydrochloric acid, saturated aqueous sodium bicarbonate solution, and saturated brine. The organic phase was dried with anhydrous sodium sulfate, filtered, and the filtrate was concentrated and dried to obtain the product: 4,6-dichloro-2-(((4-isopropyl-2-methylpyridin-3-yl)amino)nicotinamide (2.1g, 90%), a yellow solid. ES-API: [M+H]⁺=340.1.

Step 2: 4,6-dichloro-2-(((4-isopropyl-2-methylpyridin-3-yl)amino)nicotinamide (2.1 g, 6.0 mmol) and 80 mL of dry tetrahydrofuran were added to a 250 mL three-necked round bottom flask, cooled to 0-5°C under the ice bath, sodium hydride (1.2g, 30 mmol) was added in batches, and the reaction proceeded at this temperature for 10 minutes. A suspension of CDI (1.9g, 12 mmol) in tetrahydrofuran (40mL) was dropped to the above solution, and then the reaction proceeded at this temperature for 20 minutes. The completion of the reaction was detected by LCMS. The reaction solution was poured into about 150 mL of ice water, and the pH was adjusted to about 4 with 3 M hydrochloric acid. The reaction solution was extracted with ethyl acetate. The organic phase was washed with the saturated brine, dried with anhydrous sodium sulfate, filtered. The filtrate was concentrated and dried to obtain 5,7-dichloro-1-(4-isopropyl-2-methylpyridin-3-yl)pyridine[2,3-d]pyrimidin-2,4 (1H,3H)-dione (1.49g), a yellow solid, the crude product was directly used in the next step. ES-API:[M+H]⁺=366.2.

Step 3: tert-butyl (R)-3-(hydroxymethyl)piperazin-1-carboxylate (885mg, 4.1mmol) was added to a suspension of 60% sodium hydride (676mg, 16.9 mmol) in tetrahydrofuran (20 mL) at 0°C. a solution of 5,7-dichloro-1-(4-isopropyl-2-methylpyridin-3-yl)pyridine[2,3-d]pyrimidin-2,4 (1H,3H)-dione (1.49g, 4.1 mmol) in tetrahydrofuran (20 mL) was dropped thereto. The reaction was stirred at 0°C for 30 minutes. The completion of the reaction was detected by LC-MS. The reaction solution was poured into 100mL of ice water. The reaction solution was extracted with ethyl acetate for 3 times. The organic phase was dried and concentrated. The crude product was purified by a fast silica gel column (ethyl acetate/petroleum ether:20-100%) to obtain tert-butyl (S)-3-(((7-chloro-1-(4-isopropyl-2-methylpyridin-3-yl)-2,4-dioxo-1 ,2,3,4-tetrah ydropyrido[2,3-d]pyrimidin-5-yl)oxy)methylpiperazin-1-formate (1.34g, 60%), a yellow solid. ES-API: [M+H]⁺=546.3.

Step 4: tert-butyl (S)-3-(((7-chloro-1-(4-isopropyl-2-methylpyridin-3-yl)-2,4-dioxo-1 ,2,3,4-tetrah ydropyrido[2,3-d]pyrimidin-5-yl)oxy)methylpiperazin-1-formate (1.34 g, 2.45 mmol), diisopropylethylamine(20 mL), dichloromethane(30 mL) were added to a round bottom flask. propylphosphonic anhydride solution (20 mL, 50%w/wethyl acetate solution was dropped thereto. The reaction was stirred at room temperature for 30 minutes. The completion of the reaction was detected by LC-MS. 50 mL of dichloromethane was added to the reaction, and the organic phase was washed with 30 mL of hydrochloric acid (1M) and 100 mL of the saturated sodium bicarbonate aqueous solution. The organic phase was dried and concentrated. The crude product was purified by a fast silica gel column (methanol /dichloromethane:0-3%) to obtain tert-butyl (R)-2-chloro-12-(4-isopropyl-2-methylpyridin-3-yl)-11-oxo-5a,6,8,9,11,12-hex ahydro-4-oxo-1,7,9a,10,12-pentazabenzo[4,5]cyclohepta[1,2,3-de]naphthalen-7 (5H)-carboxylate (903mg, 70%). ES-API: [M+H]⁺=527.3.

Steps 5-8: Compound Z92 was prepared according to the method of Example 73. ES-API: [M+H]⁺=575.2.

### Example 93: Preparation of Compound Z93

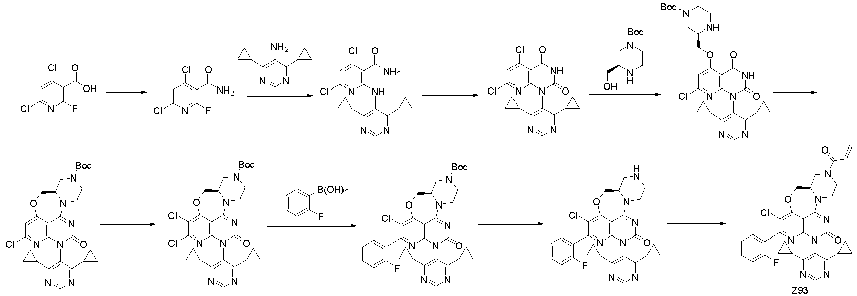

Step 1: 2,4-dichloro-6-fluorobenzoic acid (15g, 71.4mmoL) was dissolved in 200mL of dichloromethane and cooled to 0°C, oxalyl chloride (12ml, 142.9mmol) was added. After 30 minutes for the completion of the dropping, DMF (0.5ml) was dropped, the reaction rised to room temperature and proceeded for 1 hour. After the completion of the reaction, the reaction solution was concentrated and diluted with 150mL of dichloromethane, cooled to 0°C, slowly dropped to 20mL of ammonia water, and stirred for 2 hours. After the completion of the reaction, the reaction solution was concentrated and slurried with ethyl acetate, filtered to obtain the crude product: 2,4-dichloro-6-fluorobenzamide (13.5g, yield 90%). ES-API:[M+H]⁺= 208.9.

Step 2: 4,6-dicyclopropylpyrimidin-5-amine (3.56 g, 20.3mmol) was dissolved in 60mL of tetrahydrofuran, LiHMDS (38.2ml, 38.2 mmol) was dropped under the ice water bath under nitrogen protection, stirred for 30 minutes, 2,4,6-nicotinamide (4 g, 19.1 mmol) dissolved in 30mL of tetrahydrofuran was added to the above reaction solution, slowly rised to room temperature, reacted for 2 hours, cooled under the ice water bath, and dilute hydrochloric acid was dropped to pH of 7~8. The reaction solution was extracted with ethyl acetate, concentrated to obtain a gray solid, slurried with ethyl acetate, and filtered to obtain the crude product: 4,6-dichloro-2-(((4,6-dicyclopropylpyrimidin-5-yl)amino)nicotinamide (6.5 g,yield 85%). ES-API:[M+H]⁺= 364.0.

Step 3: 4,6-dichloro-2-(((4,6-dicyclopropylpyrimidin-5-yl)amino)nicotinamide (6.5g,17.9mmoL) was dissolved in 120ml of tetrahydrofuran, 60% NaH (3.6g, 89.5moml) was added under the ice water bath, stirred for 15 minutes, then CDI (4.35g, 26.6mmol) was added, the reaction proceeded under the ice water bath for 1 hour, raw material disappeared, the reaction solution was poured into ice water, and the pH was adjusted to 7~8 with dilute hydrochloric acid. The reaction solution was extracted with ethyl acetate, washed with water and the saturated brine in sequence, concentrated under reduced pressure to obtain 5,7-dichloro-1-(4,6-dicyclopropylpyrimidin-5-yl)pyrido[2,3-d]pyrimidin-2,4(1 H, 3H)-dione (5.2g,yield74%). ES-API:[M+H]⁺=390.0.

Step 4: tert-butyl (R)-3-(hydroxymethyl)piperazin-1-carboxylate (2.83g, 8.48mmoL) was dissolved in tetrahydrofuran, 60% NaH (1.02g, 25.4mml) was added under the ice water bath, and stirred for 10 minutes. 5,7-dichloro-1-(4,6-dicyclopropylpyrimidin-5-yl)pyrido[2,3-d]pyrimidin-2,4(1 H, 3H)-dione (4.3g, 8.48mmoL) was added, and stirred for 20 minutes. After the completion of the reaction, the reaction solution was poured into ice water, extracted with ethyl acetate for 3 times, the organic phases was combined, dried and concentrated under reduced pressure, purified by column chromatography (methanol /dichloromethane:0~10%) to obtain tert-butyl (R)-3-((((7-chloro-1-(4,6-dicyclopropylpyrimidin-5-yl)-2,4-dioxo-1,2,3,4-tetrah ydropyridine [2,3-d]pyrimidin-5-yl)oxy)methyl)piperazin-1-formate (5.7g, yield92%). ES-API:[M+H]⁺=570.2.

Step 5: tert-butyl (R)-3-((((7-chloro-1-(4,6-dicyclopropylpyrimidin-5-yl)-2,4-dioxo-1,2,3,4-tetrah ydropyridine [2,3-d]pyrimidin-5-yl)oxy)methyl)piperazin-1-formate (5.7g, 10.0mmoL) was dissolved in dichloromethane, diisopropylethylamine (15.5g, 120.0mml) was added at room temperature, and stirred for 5 minutes. 1-propylphosphonic anhydride (19g, 30.0mmoL) was added, stirred fors 20 minutes. After the completion of the reaction, the reaction solution was poured into ice water, extracted with ethyl acetate for 3 times, the organic phases was combined, washed with the saturated brine, dried and concentrated under reduced pressure, and purified by column chromatography (methanol /dichloromethane: 0-10%) to obtain tert-butyl (R)2-chloro-12-(4,6-dicyclopropylpyrimidin-5-yl)-11-oxo-5a,6,8,9,11,12-hexah ydro-4-oxo-1,7,9a,10,12-pentazabenzo[4,5]cyclohepta[1,2,3-de]naphthalen-7(5 H)-carboxylate (3.8g, yield69%). ES-API:[M+H]⁺=552.2.

Step 6: tert-butyl (R)-2-chloro-12-(4,6-dicyclopropylpyrimidin-5-yl)-11-oxo-5a,6,8,9,11,12-hexa ydro-4-oxo-1,7,9a,10,12-pentazabenzo[4,5]cyclohepta[1,2,3-de]naphthalen-7( 5H)-carboxylate (550mg, 1.0mmoL) was dissolved in 10mL of acetonitrile, N-chlorosuccinimide(266mg, 2.0mmol) was added, heated to 80°C and reacted for 1 hour, cooled to room temperature, filtered, washed with water and the saturated brine, concentrated, and purified by column chromatography to obtain tert-butyl (R)-2,3-dichloro-12-(4,6-dicyclopropylpyrimidin-5-yl)-11-oxo-5a,6,8,9,11,12-h exahydro-4-oxo-1,7,9a,10,12-pentazabenzo[4,5]cyclohepta[1,2,3-de]naphthale n-7(5H)-carboxylate (530mg, yield90%). ES-API:[M+H]⁺ =586.2.

Step 7: tert-butyl (R)-2,3-dichloro-12-(4,6-dicyclopropylpyrimidin-5-yl)-11-oxo-5a,6,8,9, 11,12-h exahydro-4-oxo-1,7,9a,10,12-pentazabenzo[4,5]cyclohepta[1,2,3-de]naphthale n-7(5H)-carboxylate (180mg, 0.307mmoL), (2-fluorophenyl)boronic acid (215 mg, 1.54mmol), SPhos-Pd-G2 (82mg, 0.114mmol) and potassium carbonate (472mg, 3.42mmol) was dissolved in 8mL of dioxane and 2mL of water, nitrogen was used for replacement, the reaction proceeded at 80°C for 1 hour, cooled to room temperature, filtered, diluted with ethyl acetate, washed with water and the saturated brine, concentrated, and purified by column chromatography to obtain tert-butyl (R)3-chloro-12-(4,6-dicyclopropylpyrimidin-5-yl)-2-(2-fluorophenyl)-11-oxo-5 a,6,8,9,11,12-hexahydro-4-oxo-1,7,9a,10,12-pentazabenzo[4,5]cyclohepta[1,2, 3-de]naphthalen-7(5H)-carboxylate (120mg, yield: 60%). ES-API:[M+H]⁺ =646.2.

Step 8: tert-butyl (R)-3-chloro-12-(4,6-dicyclopropylpyrimidin-5-yl)-2-(2-fluorophenyl)-11-oxo-5a,6,8,9,11,12-hexahydro-4-oxo-1,7,9a,10,12-pentazabenzo[4,5]cyclohepta[1,2 ,3-de]naphthalen-7(5H)-carboxylate (120mg, 0.186mmoL) was dissolved in 10mL of dichloromethane, 5mL of trifluoroacetic acid was slowly dropped at room temperature, reacted for 5 hours, then 10mL of dichloromethane was added. The reaction solution was concentrated under reduced pressure to obtain (R)-3-chloro-12-(4,6-dicyclopropylpyrimidin-5-yl)-2-(2-fluorophenyl)-5,5a,6,7 ,8,9-hexahydro-4-oxa-1,7,9a,10,12-pentazabenzo[4,5]cyclohepta[1,2,3-de]naph thalen-11(12H)-1-one (180mg, the crude product), directly used in the next step. ES-API: [M+H]⁺ =546.2

Step 9: (R)-3-chloro-12-(4,6-dicyclopropylpyrimidin-5-yl)-2-(2-fluorophenyl)-5,5a,6,7 ,8,9-hexahydro-4-oxa-1,7,9a,10,12-pentazabenzo[4,5]cyclohepta[1,2,3-de]naph thalen-11(12H)-1-one (180mg, the crude product) was dissolved in dichloromethane, diisopropylethylamine (255mg, 1.98mmol) and acrylic anhydride (83mg, 0.66mmol) was dropped under the ice water bath, and reacted under the ice bath for 10 minutes. After the completion of the reaction, the reaction solution was concentrated at room temperature under reduced pressure, and purified by preparative HPLC to obtain Compound Z93(40mg, yield27%), ES-API:[M+H]⁺ =600.2. ¹H NMR (500 MHz, DMSO-*d*₆) δ 8.62 (s, 1H), 7.48-7.41 (m, 1H), 7.27-7.19 (m, 2H), 7.16 (m, 1H), 6.79 (m, 1H), 6.14 (dd, J = 16.7, 2.4 Hz, 1H), 5.71 (dd, J = 10.4, 2.3 Hz, 1H), 4.85 (t, J = 13.3 Hz, 1H), 4.77 (dd, J = 12.8, 5.2 Hz, 1H), 4.52 (d, J = 23.8 Hz, 1H), 4.42 (d, J = 13.1 Hz, 1H), 4.22 (s, 1H), 3.99 (d, J = 31.2 Hz, 1H), 3.78-3.62 (m, 1H), 3.62-3.52 (m, 1H), 3.25 (d, J = 5.8 Hz, 1H), 1.73 (m, 1H), 1.65 (s, 1H), 1.05-0.65 (m, 8H).

### Example 94: Preparation of Compound Z94

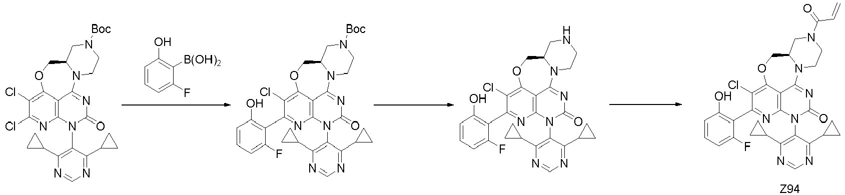

Compound Z94 was prepared with (2-hydroxy-6-fluorophenyl)boronic acid as the raw material according to the method of steps 7-9 in Example 93. ES-API:[M+H]⁺ =617.1. 1H NMR (500 MHz, DMSO-*d*₆) δ 10.06 (d, J = 4.7 Hz, 1H), 8.67 (s, 1H), 7.25-7.21 (m, 1H), 6.92-6.80 (m, 1H), 6.71 (dd, J = 8.3, 4.3 Hz, 1H), 6.66 (m, 1H), 6.21 (dd, J = 16.7, 2.4 Hz, 1H), 5.78 (dd, J = 10.3, 2.4 Hz, 1H), 4.96-4.80 (m, 2H), 4.60 (d, J = 20.4 Hz, 1H), 4.43 (d, J = 59.7 Hz, 1H), 4.27 (s, 1H), 4.04 (s, 1H), 3.83-3.59 (m, 2H), 3.33-3.31 (m, 1H), 1.73 (d, J = 48.5 Hz, 2H), 1.02-0.73 (m, 8H).

### Example 95: Preparation of Compound Z95, Z95-1 and Z95-2

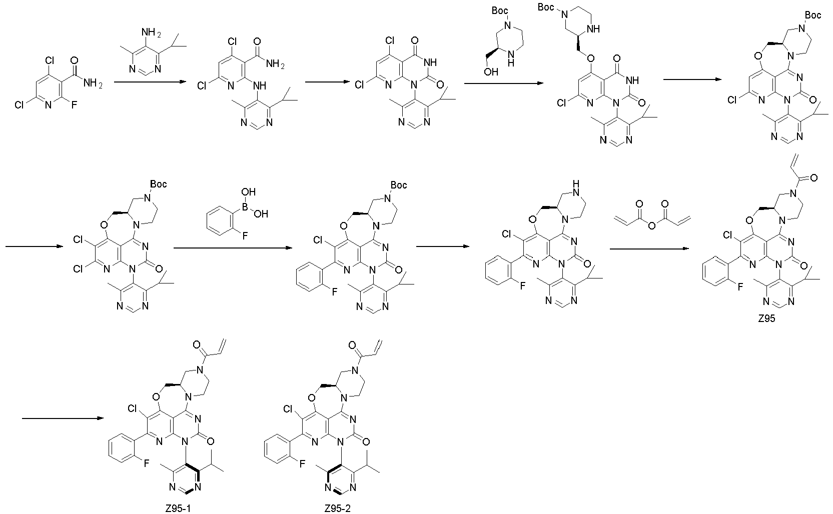

Step 1: 4-isopropyl-6-methylpyrimidin-5-amine (2.8 g, 19.1 mmol) and 45 mL of tetrahydrofuran were added to a 250 mL round bottom flask. After the system cooling to 0°C, sodium bis(trimethylsilyl)amide (28.5 mL, 2 M in tetrahydrofuran, 57.3 mmol) was dropped to the reaction solution. After the completion of the dropping, the reaction was stirred at 0°C for 10 minutes. and then 4,6-dichloro-2-fluoronicotinamide(4 g, 19.1 mmol) in tetrahydrofuran (15 mL) was dropped to the reaction solution. After the completion of the dropping, the reaction was stirred at 0°C for 20 minutes. After the completion of the reaction, the reaction solution was poured into ice water. The reaction solution was extracted with ethyl acetate. The organic phase was washed with 1M hydrochloric acid, saturated aqueous sodium bicarbonate solution, and saturated brine. The organic phase was dried with anhydrous sodium sulfate, filtered, and the filtrate was concentrated and dried to obtain the product: 4,6-dichloro-2-(((4-isopropyl-6-methylpyrimidin-5-yl)amino)nicotinamide (5.7 g, 87%), a yellow solid. ES-API: [M+H]⁺=340.1.

Step 2: 4,6-dichloro-2-(((4-isopropyl-6-methylpyrimidin-5-yl)amino)nicotinamide (5.7 g, 16.7 mmol) and 80 mL of dry tetrahydrofuran were added to a 250 mL three-necked round bottom flask, cooled to 0-5°C under the ice bath, sodium hydride (3 g, 75.1 mmol) was added in batches, and the reaction proceeded at this temperature for 5 minutes. A suspension of CDI (4 g, 24.7 mmol) in tetrahydrofuran (40mL) was dropped to the above solution, and then the reaction proceeded at this temperature for 10 minutes. The completion of the reaction was detected by LCMS. The reaction solution was poured into about 150 mL of ice water, and the pH was adjusted to about 4 with 3 M hydrochloric acid. The reaction solution was extracted with ethyl acetate. The organic phase was washed with the saturated brine, dried with anhydrous sodium sulfate, filtered. The filtrate was concentrated and dried to obtain 5,7-dichloro-1-(4-isopropyl-6-methylpyrimidin-5-yl)pyrido[2,3-d]pyrimidin-2, 4(1H,3H)-dione (4.5 g, purity 70%), a yellow solid, the crude product was directly used in the next step. ES-API:[M+H]⁺=366.1

Step 3: tert-butyl (R)-3-(hydroxymethyl)piperazin-1-carboxylate (3.2 g, 14.8 mmol) was added to a suspension of 60% sodium hydride (1.5 g, 36.9 mmol) in tetrahydrofuran (60 mL) at 0°C. A solution of 5,7-dichloro-1-(4-isopropyl-6-methylpyrimidin-5-yl)pyrido[2,3-d]pyrimidin-2, 4(1H,3H)-dione (4.5 g,12.3 mmol) in tetrahydrofuran (20 mL) was dropped thereto. The reaction was stirred at 0°C for 30 minutes. The completion of the reaction was detected by LC-MS. The reaction solution was poured into 100mL of ice water. The reaction solution was extracted with ethyl acetate for 3 times. The organic phase was dried and concentrated. The crude product was purified by a fast silica gel column (ethyl acetate/petroleum ether:20-100%) to obtain tert-butyl (R)-3-((((7-chloro-1-(4-isopropyl-6-methylpyrimidin-5-yl)-2,4-dioxo-1 ,2,3,4-te trahydropyridinyl[2,3-d]pyrimidin-5-yl)oxy)methylpiperazin-1-formate (3 g, 44%), a yellow solid. ES-API: [M+H]⁺=546.3

Step 4: tert-butyl (R)-3-((((7-chloro-1-(4-isopropyl-6-methylpyrimidin-5-yl)-2,4-dioxo-1 ,2,3,4-te trahydropyridinyl[2,3-d]pyrimidin-5-yl)oxy)methylpiperazin-1-formate (3 g, 5.5 mmol), diisopropylethylamine (10 mL), dichloromethane (30 mL) were added to a round bottom flask. 1-propylphosphonic anhydride solution (10 mL, 50%w/wethyl acetate solution) was dropped thereto. The reaction was stirred at room temperature for 30 minutes. The completion of the reaction was detected by LC-MS. 50 mL of dichloromethane was added to the reaction, and the organic phase was washed with 30 mL of hydrochloric acid (1*M*) and 100 mL of the saturated sodium bicarbonate aqueous solution. The organic phase was dried and concentrated. The crude product was purified by a fast silica gel column (methanol /dichloromethane:0-3%) to obtain tert-butyl (R)-2-chloro-12-(4-isopropyl-6-methylpyrimidin-5-yl)-11-oxo-5a,6,8,9,11,12-h exahydro-4-oxo-1,7,9a,10,12-pentazabenzo[4,5]cycloheptan[1,2,3-de]-naphthal en-7(5H)-carboxylate (2.3 g, 79%). ES-API: [M+H]⁺=528.3.

Step 5: tert-butyl (R)-2-chloro-12-(4-isopropyl-6-methylpyrimidin-5-yl)-11-oxo-5a,6,8,9,11,12-h exahydro-4-oxo-1,7,9a,10,12-pentazabenzo[4,5]cycloheptan[1,2,3-de]-naphthal en-7(5H)-carboxylate (500 mg, 0.9 mmol), N-chlorosuccinimide(252 mg, 1.9 mmol) and acetonitrile (10 mL) were added to a round bottom flask. The reaction was stirred at 80°C for 2 hours. Sodium thiosulfate aqueous solution was added to the reaction solution. The reaction solution was extracted with ethyl acetate. The organic phase was dried and concentrated. The crude product was purified by a fast silica gel column (methanol /dichloromethane:0-3%) to obtain the product: tert-butyl (R)-2,3-dichloro-12-(4-isopropyl-6-methylpyrimidin-5-yl)-11-oxo-5a,6,8,9, 11,1 2-hexahydro-4-oxo-1,7,9a,10,12-pentazabenzo[4,5]cyclohepta[1,2,3-de]naphth alen-7(5H)-carboxylate (470 mg, 88%), a yellow solid. ES-API: [M+H]+=562.2.

Step 6: tert-butyl (R)-2,3-dichloro-12-(4-isopropyl-6-methylpyrimidin-5-yl)-11-oxo-5a,6,8,9, 11,1 2-hexahydro-4-oxo-1,7,9a,10,12-pentazabenzo[4,5]cyclohepta[1,2,3-de]naphth alen-7(5H)-carboxylate (200 mg, 0.36 mmol), 2-fluoro-phenylboronic acid (124mg, 0.89 mmol), Pd(PPh₃)₄(42 mg, 0.036 mmol), sodium carbonate (114 mg, 1.08 mmol), 18 mL of dioxane and 2 mL of water were added to a 100mL reaction flask. The reaction was stirred at 80°C for 2.5 hours under nitrogen protection, and the reaction stopped. 20 mL of water was added to the reaction solution. The reaction solution was extracted with 20mL of ethyl acetate for 3 times, and the organic phase was dried and concentrated. The crude product was purified by a fast silica gel column (ethyl acetate/petroleum ether:0-100%) to obtain the product: tert-butyl (R)-3-chloro-2-(2-fluorophenyl)-12-(4-isopropyl-6-methylpyrimidin-5-yl)-11-o xo-5a,6,8,9,11,12-hexahydro-4-oxo-1,7,9a,10,12-pentazabenzo[4,5]cyclohepta[ 1,2,3-de]naphthalen-7(5H)-carboxylate (200 mg, 89%), a yellow solid. ES-API: [M+H]⁺= 622.2.

Step 7: tert-butyl (R)-3-chloro-2-(2-fluorophenyl)-12-(4-isopropyl-6-methylpyrimidin-5-yl)-11-o xo-5a,6,8,9,11,12-hexahydro-4-oxo-1,7,9a,10,12-pentazabenzo[4,5]cyclohepta[ 1,2,3-de]naphthalen-7(5H)-carboxylate (170 mg, 0.27 mmol), 1 mL of methanol and 3 mL of hydrogen chloride/dioxane solution (4 *M*) were added to a round bottom flask. The reaction was stirred at room temperature for 1 hour. The completion of the reaction was detected by LC-MS. The reaction solution was concentrated to obtain (R)-3-chloro-2-(2-fluorophenyl)-12-(4-isopropyl-6-methylpyrimidin-5-yl)-5,5a, 6,7,8,9-hexahydro-4-oxo-1,7,9a,10,12-pentazabenzo[4,5]cycloheptan[1,2,3-de] -naphthalen-11(12H)-one (167 mg), a yellow solid. the crude product was directly used in the next step. ES-API: [M+H]⁺=522.2.

Step 8: (R)-3-chloro-2-(2-fluorophenyl)-12-(4-isopropyl-6-methylpyrimidin-5-yl)-5,5a, 6,7,8,9-hexahydro-4-oxo-1,7,9a,10,12-pentazabenzo[4,5]cycloheptan[1,2,3-de] -naphthalen-11(12H)-one (167 mg, 0.32 mmol), 3 mL of dichloromethane and triethylamine (97 mg, 0.96 mmol) were added to a round bottom flask. The reaction was cooled to 0°C, and acrylic anhydride in dichloromethane (40 mg, 0.32 mmol, 0.5 mL) was dropped to the reaction solution. The reaction was stirred at 0°C for 10 minutes. 40 mL of the saturated sodium bicarbonate aqueous solution was added to the reaction solution, and the reaction solution was extracted with 20 mL of dichloromethane for 3 times . The organic phase was dried and concentrated. The crude product was purified by Preparative HPLC to obtain a racemate Z95.

Step 9: Compound Z95 was resolved chirally (column type: Chiralpak IF 250mm^{∗}4.6mm^{∗}5um; mobile phase: n-hexane: ethanol=40:60; flow rate:1ml/min) to obtain:Compound Z95-1(22.3 mg, retention time: 12.6 min; peak 1), a white solid. ES-API: [M+H]⁺=576.2.
and Compound Z95-2(31.2 mg, retention time: 17.3 min;peak 2), a white solid. ES-API: [M+H]⁺=576.2. ¹HNMR (500MHz, DMSO-*d₆*) 8.93 (s,1H), 7.51-7.50 (m, 1H), 7.48-7.17 (m, 3H), 6.88-6.80 (m, 1H), 6.23-6.20 (m, 1H), 5.77 (dd, *J*=10.5, 2 Hz, 1H), 4.87-4.84 (m, 2H), 4.47-4.31 (m, 3H), 4.05-4.02 (m, 1H), 3.68-3.36 (m, 3H), 2.82-2.80 (m, 1H), 2.13 (s, 3H), 1.08 (d, *J*=6.5 Hz, 3H), 0.95 (d, *J*=6.5 Hz, 3H).

### Example 96: Preparation of Compound Z96, Z96-1 and Z96-2

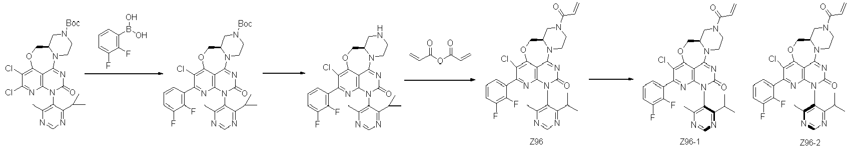

Compound Z96 was prepared with (2,3-difluorophenyl)boronic acid as the raw material according to the method of steps 6-8 in Example 95, resolved chirally (column type: Chiralpak IF 250mm^{∗}4.6mm^{∗}5um; mobile phase: n-hexane:ethanol=40:60; flow rate:1ml/min) to obtain:Compound Z96-1 (25 mg, retention time: 12.02 min; peak 1), a white solid. ES-API: [M+H]⁺=594.1.
and Compound Z96-2 (37 mg, retention time: 17.03 min;peak 2), a white solid. ES-API: [M+H]⁺=594.1. ¹HNMR (500MHz, DMSO-*d₆*) 8.94 (s,1H), 7.56-7.54 (m, 1H), 7.53-7.52 (m, 1H), 7.26-7.24 (m, 1H), 6.86-6.80 (m, 1H), 6.23-6.20 (m, 1H), 5.79-5.77 (m, 1H), 4.88-4.84 (m, 2H), 4.47-4.32 (m, 3H), 4.02-4.01 (m, 1H), 3.72-3.66 (m, 2H), 3.34-3.33 (m, 1H), 2.82-2.80 (m, 1H), 2.12 (s, 3H), 1.07 (d, *J*=6.5 Hz, 3H), 0.95 (d, *J*=6.5 Hz, 3H).

### Example 97: Preparation of Compound Z97

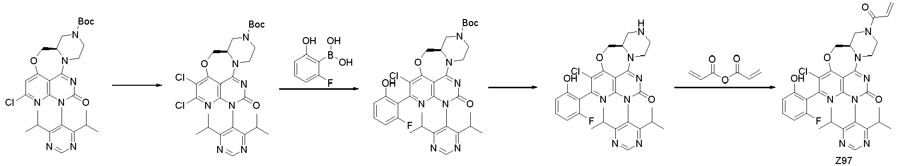

Step 1: tert-butyl (R)-2-chloro-12-(4,6-diisopropylpyrimidin-5-yl)-11-oxo-5a,6,8,9,11,12-hexahy dro-4-oxo-1,7,9a,10,12-pentazabenzo[4,5]cyclohepta[1,2,3-de]naphthalen-7(5 *H*)-carboxylate (450 mg, 0.81 mmol) and a solution of N-chlorosuccinimide (216 mg, 1.62 mmol) in acetonitrile (20 mL) was stirred at 80°C for 2 hours under nitrogen protection. The reaction solution was dissolved in ethyl acetate (50 mL), washed with water (30 mL^{∗}2). The organic phase was dried and concentrated, and purified by a fast silica column (0-10%methanol /dichloromethane) to obtain a yellow solid: tert-butyl (R)-2,3-dichloro-12-(4,6-diisopropylpyrimidin-5-yl)-11-oxo-Sa,6,8,9,11,12-hex ahydro-4-oxo-1,7,9a,10,12-pentazabenzo[4,5]cyclohepta[1,2,3-de]naphthalen-7 (5*H*)-carboxylate (350 mg, yield:73%). ES-API:[M+H]⁺=590.2.

Steps 2-4: Compound Z97 was prepared according to the method of Example 46. ¹H NMR (500 MHz, DMSO-*d₆*) δ 10.04 (s, 1H), 9.01 (s, 1H), 7.21 (dd, *J =* 15.6, 8.1 Hz, 1H), 6.99-6.78 (m, 1H), 6.68 (dd, *J =* 8.3, 3.3 Hz, 1H), 6.66-6.61 (m, 1H), 6.22 (dd, *J* = 16.7, 2.1 Hz, 1H), 5.78 (dd, *J* = 10.5, 2.2 Hz, 1H), 4.96-4.85 (m, 2H), 4.66-4.44 (m, 2H), 4.41-4.25 (m, 2H), 4.13-3.99 (m, 1H), 3.84-3.65 (m, 2H), 2.83-2.74 (m, 1H), 2.73-2.63 (m, 1H), 1.08 (d, *J* = 6.7 Hz, 6H), 0.97-0.78 (m, 6H).ES-API:[M+H]⁺=\620.2.

### Example 98: Preparation of Compound Z98

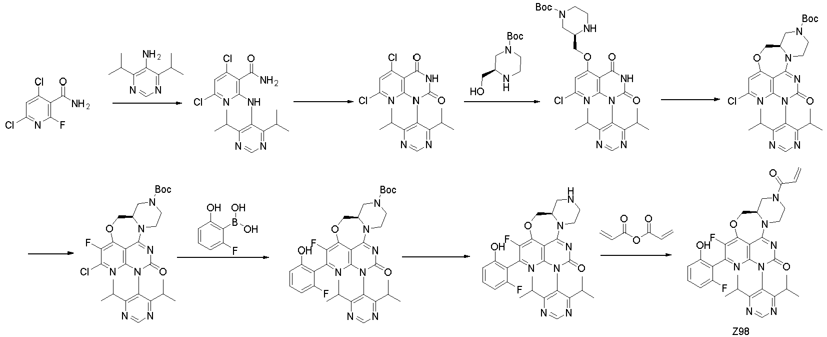

Compound Z98 was prepared with 4,6-diisopropylpyrimidin-5-amine as the raw material according to the method of Example 92. ¹H NMR (500 MHz, DMSO-*d₆*) δ 10.15 (s, 1H), 9.02 (s, 1H), 7.24 (dd, *J* = 15.3, 8.2 Hz, 1H), 6.94-6.76 (m, 1H), 6.71 (d, *J* = 8.3 Hz, 1H), 6.66 (t, *J* = 8.9 Hz, 1H), 6.22 (d, *J* = 16.8 Hz, 1H), 5.83-5.75 (m, 1H), 4.91 (t, *J =* 11.0 Hz, 1H), 4.84 (dd, *J* = 12.7, 5.2 Hz, 1H), 4.57 (s, 1H), 4.49-4.28 (m, 2H), 4.08-3.98 (m, 1H), 3.94-3.66 (m, 2H), 3.50-3.38 (m, 1H), 2.78 (dt, *J* = 13.4, 6.9 Hz, 1H), 2.67 (dd, *J* = 13.4, 6.6 Hz, 1H), 1.11-1.05 (m, 6H), 0.94 (dd, *J* = 18.8, 6.7 Hz, 6H).ES-API:[M+H]⁺=604.2.

### Example 99: Preparation of Compound Z99

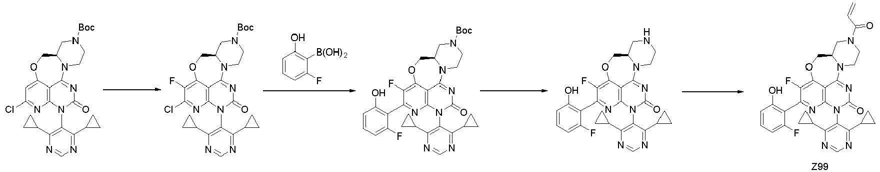

Step 1: tert-butyl (R)2-chloro-12-(4,6-dicyclopropylpyrimidin-5-yl)-11-oxo-5a,6,8,9,11,12-hexah ydro-4-oxo-1,7,9a,10,12-pentazabenzo[4,5]cyclohepta[1,2,3-de]naphthalen-7(5 H)-carboxylate (2.0g, 3.63mmoL) was dissolved in 100mL of acetonitrile, Selectfluor (6.64g, 18.2mmol) was added, heated to 55°C and reacted for 18 hours, then cooled to room temperature, filtered, washed with water and the saturated brine, concentrated, and purified by column chromatography to obtain tert-butyl (R)-2,3-dichloro-12-(4,6-dicyclopropylpyrimidin-5-yl)-11-oxo-5a,6,8,9,11,12-h exahydro-4-oxo-1,7,9a,10,12-pentazabenzo[4,5]cyclohepta[1,2,3-de]naphthale n-7(5H)-carboxylate (2.0g,the crude product). ES-API:[M+H]⁺ =570.2.

Steps 2-4: Compound Z99 was prepared according to the method of steps 7-9 in Example 93. ES-API:[M+H]⁺ =600.1. 1H NMR (500 MHz, DMSO-*d₆*) δ 10.17 (s, 1H), 8.69 (s, 1H), 7.27 (m, 1H), 6.85 (m, 1H), 6.77-6.65 (m, 2H), 6.21 (dd, J = 16.7, 2.4 Hz, 1H), 5.78 (dd, J = 10.3, 2.4 Hz, 1H), 4.89 (t, J = 12.2 Hz, 1H), 4.82 (dd, J = 12.8, 5.2 Hz, 1H), 4.59 (s, 1H), 4.41 (dd, J = 56.1, 13.1 Hz, 1H), 4.29 (s, 1H), 4.02 (s, 1H), 3.83 (d, J = 41.6 Hz, 1H), 3.69 (dd, J = 25.9, 13.6 Hz, 1H), 3.42 (d, J = 12.9 Hz, 1H), 1.77 (m, 1H), 1.70-1.62 (m, 1H), 1.04-0.84 (m, 6H), 0.79 (q, J = 8.1 Hz, 2H).

### Example 100: Preparation of Compound Z100, Z100-1 and Z100-2

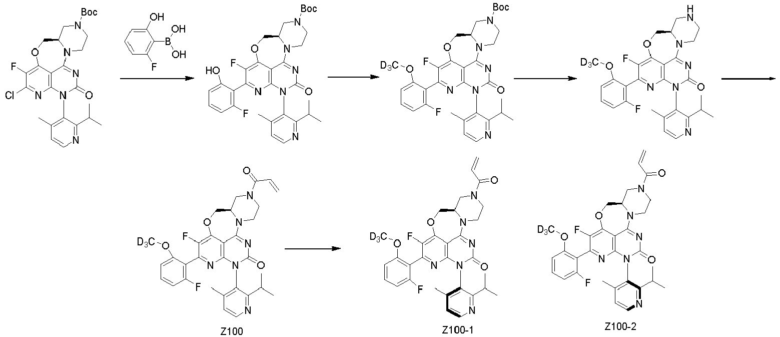

Step 1: tert-butyl (R)-2-chloro-3-fluoro-12-(2-isopropyl-4-methylpyrid-3-yl)-11-oxo-5a,6,8,9,11, 12-hexahydro-4-oxo-1,7,9a,10,12-pentazabenzo[4,5]cyclohepta[1,2,3-de]napht halen-7(5H)-carboxylate (3.2g, 5.87mmol), (2-fluoro-6-hydroxyphenyl)boronic acid (5.5g, 35.25mmol), SPhos-pd-G2 (1.0g, 1.1mmol), potassium phosphate (2.5g, 11.79mmol), 100mL of dioxane and 20mL of water were added to a 250 mL three-necked round bottom flask. The system was replaced with the nitrogen for three times, and then protected with a nitrogen ball. The reaction proceeded at 120°C for 3 hours. 100 mL of ethyl acetate was added to the reaction solution, the reaction solution was washed with the saturated brine for 3 times, dried and concentrated. The crude product was purified by a fast silica gel column to obtain the target product: tert-butyl (5aR)-3-fluoro-2-(2-fluoro-6-hydroxyphenyl)-12-(2-isopropyl-4-methylpyridin -3-yl)-11-oxo-5a,6,8,9,11,12-hexahydro-4-oxo-1,7,9a,10,12-pentazabenzo[4,5] cyclohepta[1,2,3-de]naphthalen-7(5H)-carboxylate (350mg, yield: 3%). ES-API: [M+H]+=621.3.

Step 2: tert-butyl (5aR)-3-fluoro-2-(2-fluoro-6-hydroxyphenyl)-12-(2-isopropyl-4-methylpyridin -3-yl)-11-oxo-5a,6,8,9,11,12-hexahydro-4-oxo-1,7,9a,10,12-pentazabenzo[4,5] cyclohepta[1,2,3-de]naphthalen-7(5H)-carboxylate (150 mg,0.24mol) was dissolved in acetonitrile (8 mL), deuterated methyl iodide (300mg, 2.11mmol) was added, and the reaction proceeded at 80°C for 1 hour. 30 mL of ethyl acetate was added to the reaction solution, the reaction solution was washed with 30 mL of the saturated brine for 3 times, dried and concentrated. The crude product was purified by a fast silica gel column to obtain the target product: tert-butyl (5aR)-3-fluoro-2-(2-fluoro-6-(methoxy-d3)phenyl)-12-(2-isopropyl-4-methylpy ridin-3-yl)-11-oxo-5a,6,8,9,11,12-hexahydro-4-oxo-1,7,9a,10,12-pentazabenzo[ 4,5]cyclohepta[1,2,3-de]naphthalen-7(5H)-carboxylate (120mg, yield: 78%)ES-API: [M+H]+=638.3.

Step 3: tert-butyl (5aR)-3-fluoro-2-(2-fluoro-6-(methoxy-d3)phenyl)-12-(2-isopropyl-4-methylpy ridin-3-yl)-11-oxo-5a,6,8,9,11,12-hexahydro-4-oxo-1,7,9a,10,12-pentazabenzo[ 4,5]cyclohepta[1,2,3-de]naphthalen-7(5H)-carboxylate was dissolved in dichloromethane (8 mL), and trifluoroacetic acid (2 mL) was added. The reaction was stirred at room temperature for 2 hours. The reaction solution was concentrated to obtain the target intermediate .

Step 4: the intermediate was dissolved in dichloromethane (15 mL), and triethylamine (1g, 10mmol) was added. The reaction was cooled to 0°C, and acrylic anhydride (120 mg, 0.95mmol) was dropped to the reaction solution. The reaction was stirred at 0°C for 15 minutes. 40 mL of dichloromethane was added to the reaction solution, the reaction solution was washed with 50mL of the saturated NaHCO₃ aqueous solution, 40mL of the saturated brine, dried and concentrated, the crude product was purified by preparative liquid chromatography to obtain the target product: Z100(55mg,yield: 48%). ES-API: [M+H]⁺=592.2.

Step 5: Compound Z100 was prepared and resolved chirally (column type: IC, 250mm^{∗}4.6mm^{∗}5um, mobile phase: acetonitrile :isopropanol=60:40,flow rate:1ml/min, column temperature=30°C) to obtain:Compound Z100-1(13mg, peak 1, retention time5.065 min, yield:23.6%), a white solid; and Compound Z100-2(7mg, peak 2, retention time 6.804 min, yield: 12.7%), a white solid.

### Example 101: Preparation of Compound Z101, Z101-1 and Z101-2

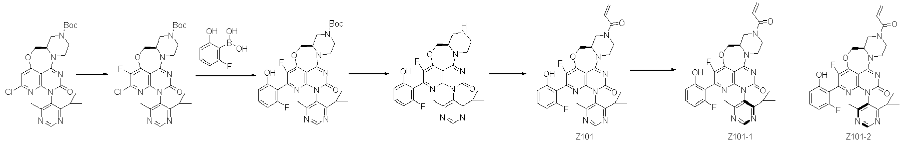

Compound Z101 was prepared according to the method of Example 99, and resolved chirally (column type: Chiralpak IF 250mm^{∗}4.6mm^{∗}5um; mobile phase: n-hexane:ethanol=40:60; flow rate:1ml/min; column temperature=30 °C) to obtain:Compound Z101-1 (8.7 mg, retention time: 7.10 min; peak 1), a white solid. ES-API: [M+H]⁺=576.2.
and Compound Z101-2(15.2 mg, retention time: 9.68 min; peak 2), a white solid. ES-API: [M+H]⁺=576.2.

### Example 102: Preparation of Compound Z102, Z102-1 and Z102-2

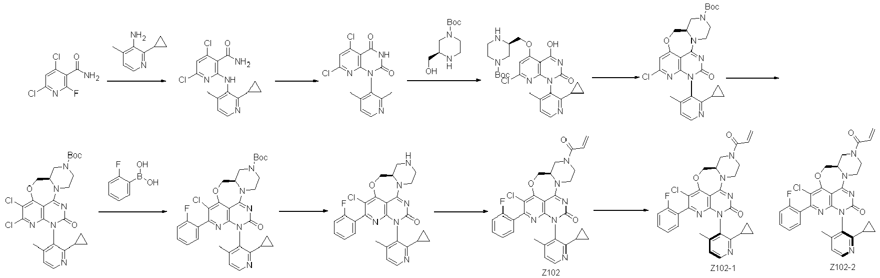

Compound Z102 was prepared with 2-cyclopropyl-4-methylpyridin-3-amine as the raw material according to the method of Example 95. was purified by Preparative HPLC(ammonium bicarbonate system, column type: Chiralpak IC 250mm^{∗}4.6mm^{∗}5um; mobile phase: acetonitrile : isopropanol =70:30; flow rate:1ml/min; column temperature=30°C) to obtain:Compound Z102-1(85 mg, retention time:8.467 min;peak 1, yield:27%). ¹H NMR (500 MHz, DMSO-*d₆*) δ 8.22 (d, *J =* 4.9 Hz, 1H), 7.49 (td, *J* = 7.3, 1.8 Hz, 1H), 7.31-7.19 (m, 3H), 7.09 (d, *J* = 5.0 Hz, 1H), 6.93-6.79 (m, 1H), 6.21 (dd, *J* = 16.7, 2.3 Hz, 1H), 5.78 (dd, *J* = 10.4, 2.2 Hz, 1H), 4.96-4.83 (m, 2H), 4.66-4.54 (m, 1H), 4.50-4.26 (m, 2H), 4.07 (d, *J =* 40.0 Hz, 1H), 3.79-3.62 (m, 2H), 3.31 (d, *J* = 9.5 Hz, 1H), 1.96 (s, 3H), 1.75 ~ 1.68 (m, 1H), 0.89 ~ 0.66 (m, 4H).
and Compound Z102-2(80 mg, retention time:9.990 min;peak 2, yield:25%). ¹H NMR (500 MHz, DMSO-*d₆*) δ 8.21 (d, *J* = 4.9 Hz, 1H), 7.50 (dd, *J* = 13.6, 5.5 Hz, 1H), 7.31-7.19 (m, 3H), 7.08 (d, *J* = 4.9 Hz, 1H), 6.93-6.79 (m, 1H), 6.22 (dd, *J* = 16.7, 2.2 Hz, 1H), 5.78 (dd, *J* = 10.5, 2.1 Hz, 1H), 4.96-4.81 (m, 2H), 4.58 (d, *J* = 13.5 Hz, 1H), 4.49-4.23 (m, 2H), 4.06 *(d, J* = 29.1 Hz, 1H), 3.69 (dd, *J* = 41.5, 29.9 Hz, 2H), 3.32 (s, 1H), 1.98 (s, 3H), 1.65 (s, 1H), 0.92 (dd, *J* = 11.5, 5.9 Hz, 1H), 0.77~0.67 (m, 3H).

### Example 103: Preparation of Compound Z103

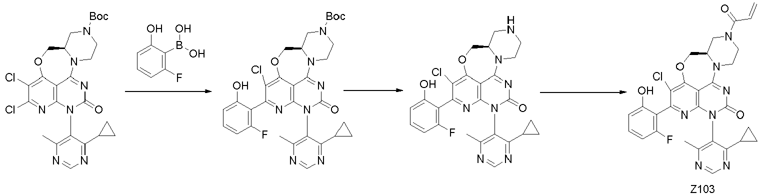

Compound Z103 was prepared with (2-fluoro-6-hydroxyphenyl)boronic acid as the raw material according to the method of Example 53. ES-API: [M+H]+=590.2. ¹H NMR (500 MHz, DMSO-*d*₆)δ 10.04 (dd, *J =* 12.5, 4.9 Hz, 1H), 8.68 (s, 1H), 7.16 (dd, *J* = 15.4, 7.3 Hz, 1H), 6.80 (dd, *J* = 27.5, 16.5 Hz, 1H), 6.69-6.53 (m, 2H), 6.14 (dd, *J* = 16.7, 2.2 Hz, 1H), 5.71 (dd, *J* = 10.5, 2.1 Hz, 1H), 4.96-4.73 (m, 2H), 4.59-4.26 (m, 2H), 4.21 (s, 1H), 3.97 (s, 1H), 3.57 (s, 2H), 2.05 (t, *J* = 15.6 Hz, 3H), 1.68 (d, *J* = 48.3 Hz, 1H), 0.95-0.69 (m, 4H).

### Example 104: Preparation of Compound Z104, Z104-1 and Z104-2

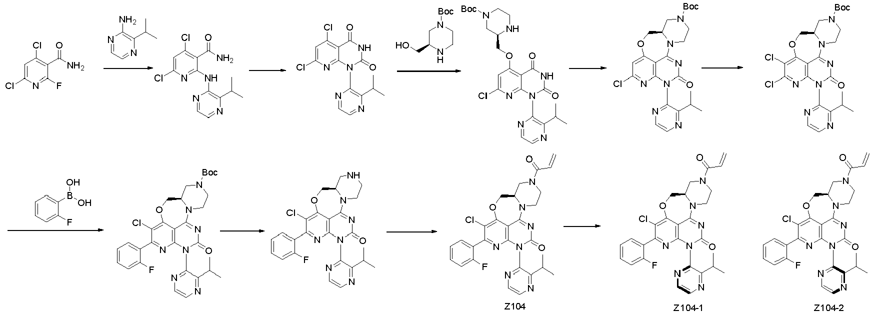

Compound Z104 was prepared with 3-isopropylpyrazine-2-amine as the raw material according to the method of Example 93. ES-API: [M+H]⁺=562.2. Compound Z104(191mg, 0.34mmol) was resolved chirally (mobile phase: n-hexane-ethanol-40-60; column type: IC (250mm^{∗}4.6mm^{∗}5um) ; flow rate: 1.0 ml/min; column temperature: 30 °C) to obtain:Compound Z104-1 (86mg, retention time: 8.554min, purity:99.2 %, de value: 99 %). ES-API:[M+H]⁺= 562.2.
and Compound Z104-2 (81mg, retention time: 15.317min, purity:100 %, de value: 99%). ES-API:[M+H]⁺= 562.2.

### Example 105: Preparation of Compound Z105, Z105-1 and Z105-2

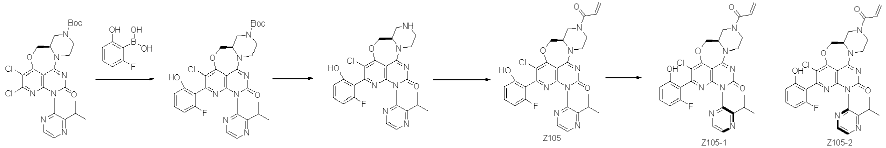

Compound Z105 was prepared with (2-fluoro-6-hydroxyphenyl)boronic acid as the raw material according to the method of steps 6-8 in Example 104. ES-API: [M+H]⁺=579.2. Compound Z105(182mg, 0.31mmol) was resolved chirally (mobile phase: n-hexane-ethanol-40-60); column type: IC (250mm^{∗}4.6mm^{∗}5um; flow rate: 1.0 ml/min; T: 30 °C) to obtain:Compound Z105-1 (78mg, retention time9.559min, purity:99.5 %, de value: 99 %). ES-API: [M+H]⁺= 579.2.
and Compound Z105-2 (81mg, retention time16.267min, purity: 100 %, de value: 99%). ES-API:[M+H]⁺= 579.2.

### Example 106: Preparation of Compound Z106

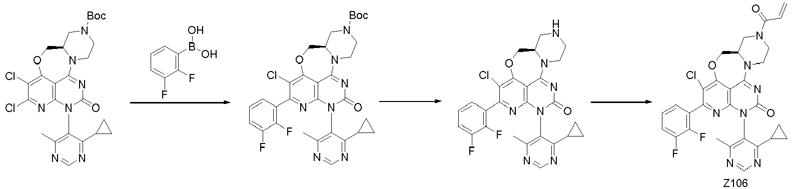

Compound Z106 was prepared with (2,3-difluorophenyl)boronic acid as the raw material according to the method of Example 46. ES-API: [M+H]⁺=592.2. ¹H NMR (500 MHz, DMSO-*d*₆)δ 8.77 (s, 1H), 7.56 (dd, *J =* 17.7, 8.8 Hz, 1H), 7.31 (dd, *J* = 12.8, 8.1 Hz, 1H), 7.07 (t, *J* = 6.7 Hz, 1H), 6.86 (ddd, *J* = 33.1, 16.8, 10.3 Hz, 1H), 6.22 (dd, *J* = 16.7, 2.2 Hz, 1H), 5.78 (dd, *J* = 10.4, 2.1 Hz, 1H), 5.01-4.81 (m, 2H), 4.67-4.37 (m, 2H), 4.31 (s, 1H), 4.05 (d, *J* = 27.3 Hz, 1H), 3.89-3.62 (m, 2H), 2.15 (d, *J* = 12.2 Hz, 3H), 1.87-1.71 (m, 1H), 1.08-0.76 (m, 4H).

### Example 107: Preparation of Compound Z107, Z107-1 and Z107-2

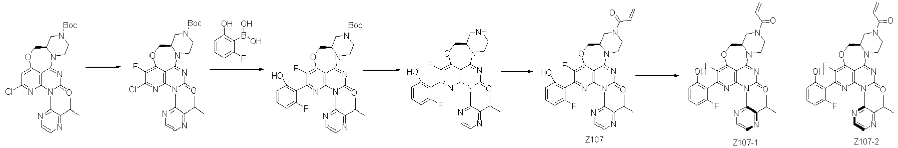

Compound Z107 was prepared according to the method of Example 99. ES-API: [M+H]⁺=562.1. Compound Z107(207mg, 0.37mmol) was resolved chirally (mobile phase: n-hexane-ethanol-40-60; column type: IB (250mm^{∗}4.6mm^{∗} 5um; flow rate: 1.0 ml/min; column temperature: 30 °C) to obtain:Compound Z107-1 (98mg, retention time 9.02min, purity:99.2 %, de value: 99 %). ES-API:[M+H]⁺= 562.1.
and Compound Z107-2 (95mg, retention time14.76min, purity: 100 %, de value: 99%). ES-API:[M+H]⁺= 562.1.

### Example 108: Preparation of Compound Z108

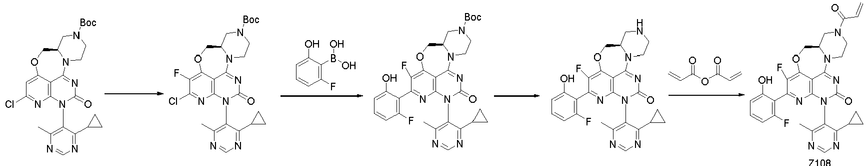

Compound Z108 was prepared according to the method of Example 99. ES-API: [M+H]⁺=574.2. ¹H NMR (500 MHz, DMSO-*d*₆)δ 10.21 (s, 1H), 8.77 (s, 1H), 7.27 (dd, *J* = 15.4, 8.0 Hz, 1H), 6.84 (ddd, *J =* 27.0, 17.0, 10.6 Hz, 1H), 6.77-6.65 (m, 2H), 6.21 (dd, *J* = 16.7, 1.9 Hz, 1H), 5.80-5.73 (m, 1H), 4.85 (ddd, *J* = 18.0, 15.2, 5.7 Hz, 2H), 4.57 (s, 1H), 4.51-4.24 (m, 2H), 4.02 (s, 1H), 3.88 (d, *J* = 39.9 Hz, 1H), 3.70 (d, *J* = 38.6 Hz, 1H), 3.49-3.35 (m, 1H), 2.12 (d, *J* = 18.0 Hz, 3H), 1.83-1.67 (m, 1H), 1.02-0.87 (m, 3H), 0.86-0.76 (m, 1H).

### Example 109: Preparation of Compound Z109, Z109-1 and Z109-2

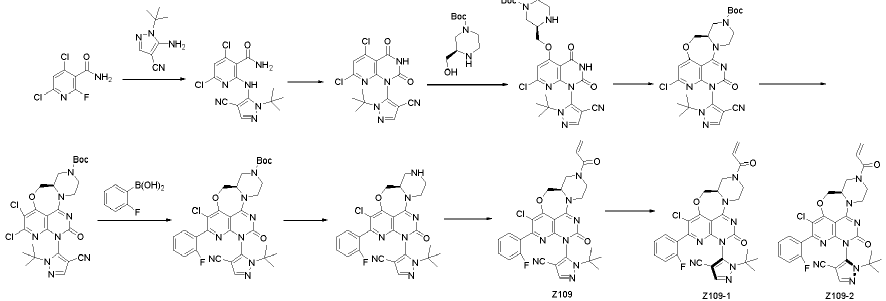

Compound Z109 was prepared with 5-amino-1-(tert-butyl)-1H-pyrazole-4-nitrile as the raw material according to the method of Example 93. ES-API: [M+H]⁺=589.1, resolved chirally (column type: Chiralpak IA 250mm^{∗}4.6mm^{∗}5um; mobile phase: n-hexane: ethanol=60:40; flow rate:1ml/min; column temperature=30°C) to obtain:Compound Z109-1(17 mg, retention time7.25 min, peak 1), a yellow solid, ES-API: [M+H]⁺=589.1;
and Compound Z109-2(17 mg, retention time9.22 min, peak 2), a yellow solid. ES-API: [M+H]⁺=589.2. ¹HNMR (500MHz, DMSO-*d*₆): 7.87 (s,1H), 7.26-7.25 (m, 1H), 7.20-7.23 (m, 2H), 7.14-7.10 (m, 1H), 6.62-6.59 (m, 1H), 6.48-6.45 (m, 1H), 5.89-5.87 (m, 1H), 4.94-4.71 (m, 4H), 4.15-4.12 (m, 1H), 4.02-3.44 (m, 4H), 1.31 (s, 9H).

### Example 110: Preparation of Compound Z110

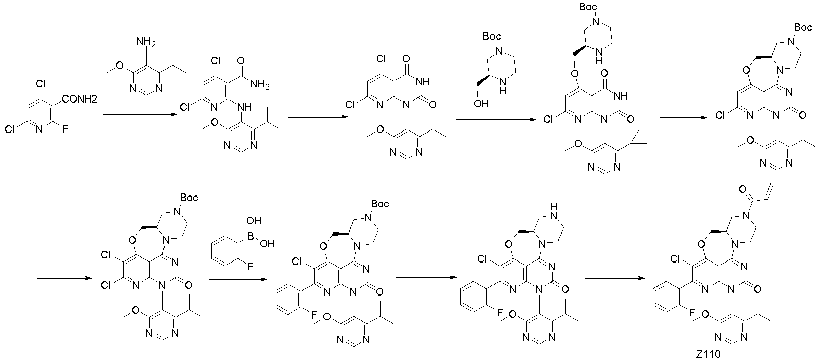

Compound Z110 was prepared with 4-isopropyl-6-methoxypyrimidin-5-amine as the raw material according to the method of Example 93. ES-API: [M+H]⁺=592.2. ¹H NMR (500 MHz, DMSO-*d₆*) δ 8.72 (s, 1H), 7.57-7.46 (m, 1H), 7.37-7.23 (m, 2H), 7.18 (t, *J* = 7.4 Hz, 1H), 6.84 (dd, *J* = 30.7, 13.7 Hz, 1H), 6.21 (d, *J =* 16.7 Hz, 1H), 5.78 (d, *J* = 12.2 Hz, 1H), 5.01-4.80 (m, 2H), 4.68-4.22 (m, 3H), 4.06 (d, *J* = 32.5 Hz, 1H), 3.68 (s, 5H), 3.38 (d, *J* = 11.8 Hz, 1H), 2.83 (dd, *J* = 27.6, 21.0 Hz, 1H), 1.13-0.88 (m, 7H).

### Example 111: Preparation of Compound Z111, Z111-1 and Z111-2

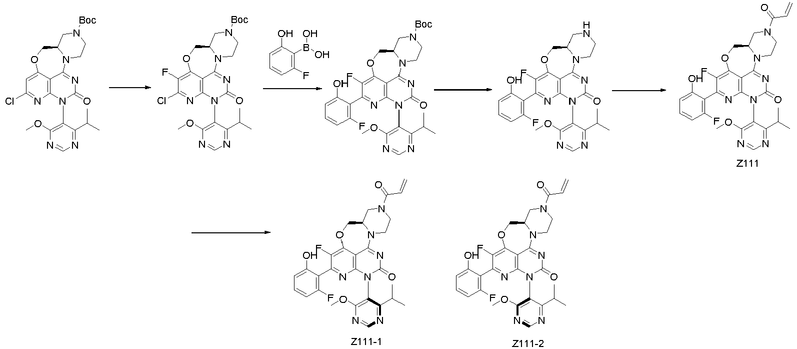

Compound Z111 was prepared according to the method of Example 99. ES-API: [M+H]⁺=592.2. ¹H NMR (500 MHz, DMSO-*d₆*) δ 10.18 (s, 1H), 8.71 (s, 1H), 7.26 (dd, *J* = 15.3, 8.2 Hz, 1H), 6.95-6.77 (m, 1H), 6.75-6.62 (m, 2H), 6.21 (d, *J* = 15.5 Hz, 1H), 5.84-5.69 (m, 1H), 4.84 (ddd, *J* = 19.9, 18.1, 8.9 Hz, 2H), 4.68-4.22 (m, 3H), 4.02 (s, 1H), 3.94-3.62 (m, 5H), 2.79 (ddd, *J* = 45.6, 13.2, 6.6 Hz, 1H), 1.11-0.95 (m, 6H). Compound Z111(75mg,0.1268mmol) was resolved chirally (column type: IF, 250mm^{∗}4.6mm^{∗}5um, mobile phase: n-hexane:ethanol=50:50,flow rate:1ml/min, column temperature=30°C) to obtain:Compound Z111-1 (25mg,peak 1, retention time:7.33 min, yield:34%), a light yellow solid. and Compound Z111-2(26mg, peak 2, retention time: 1.42min, yield:34.6%), a light yellow solid. ES-API: [M+H]⁺=592.2.

### Example 112: Preparation of Compound Z112, Z112-1 and Z112-2

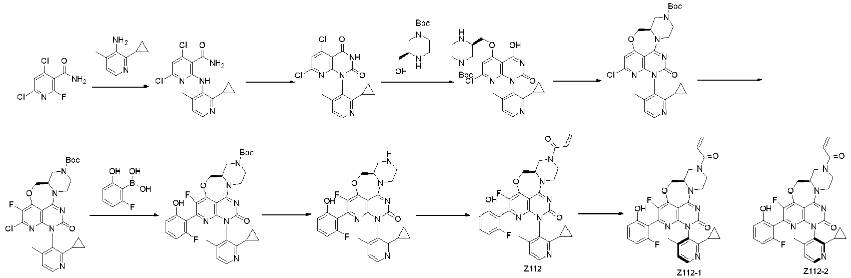

Compound Z112 was prepared with 2-cyclopropyl-4-methylpyridin-3-amine as the raw material according to the method of Example 92. was purified by Preparative HPLC(ammonium bicarbonate system, column type: Chiralpak IC 250mm^{∗}4.6mm^{∗}5um; mobile phase: acetonitrile : isopropanol: ammonia methanol =85:15:0.2; flow rate:1ml/min; column temperature=30°C) to obtain a yellow solid: Compound Z112-1(60 mg, retention time: 9.201 min; peak 1, yield:22%). ¹H NMR (500 MHz, DMSO-*d₆*) δ 8.22 (d, *J* = 4.9 Hz, 1H), 7.23 (dd, *J* = 15.2, 7.8 Hz, 1H), 7.08 (d, *J* = 5.2 Hz, 1H), 6.91-6.79 (m, 1H), 6.73-6.59 (m, 2H), 6.21 (d, *J =* 17.2 Hz, 1H), 5.78 (dd, *J* = 10.4, 2.3 Hz, 1H), 4.91-4.80 (m, 2H), 4.55 (s, 1H), 4.47-4.34 (m, 1H), 4.28 (s, 1H), 4.03 (d, *J* = 29.0 Hz, 1H), 3.74 (dd, *J* = 55.0, 44.2 Hz, 4H), 1.93 (s, 3H), 1.70 (td, *J* = 8.1, 4.1 Hz, 1H), 0.82 (d, *J* = 4.0 Hz, 2H), 0.77-0.71 (m, 1H), 0.65 (d, *J* = 7.9 Hz, 1H).
and Compound Z112-2(12 mg, retention time: 12.307 min;peak 2, yield:4.4%). ¹H NMR (500 MHz, DMSO-*d₆*) δ 10.15 (s, 1H), 8.21 (d, *J* = 4.9 Hz, 1H), 7.25 (dd, *J* = 15.3, 8.3 Hz, 1H), 7.08 (d, *J* = 4.9 Hz, 1H), 6.85 (s, 1H), 6.73-6.65 (m, 2H), 6.21 (dd, *J* = 16.7, 2.3 Hz, 1H), 5.77 (dd, *J* = 10.4, 2.2 Hz, 1H), 4.90-4.79 (m, 2H), 4.59 (s, 1H), 4.49-4.27 (m, 2H), 4.02 (s, 1H), 3.79-3.58 (m, 2H), 3.30 (s, 1H), 1.96 (s, 3H), 1.61 (d, *J* = 4.6 Hz, 1H), 0.84 (s, 1H), 0.75 (dd, *J =* 15.9, 7.2 Hz, 2H), 0.61 (d, *J =* 8.3 Hz, 1H).

### Example 113: Preparation of Compound Z113

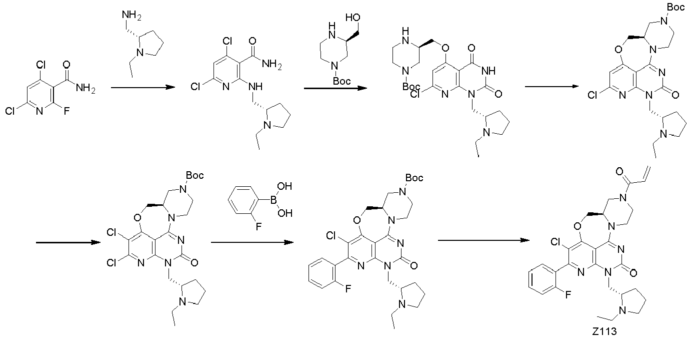

Step 1: 4,6-dichloro-2-fluoronicotinamide (10.5g,50mmol), (S)-(1-ethylpyrrolidin-2-yl)(7.68g, 60mmol), potassium carbonate (20.7g, 150mmol) and 200mL of dimethylformamide were added to a 500 mL three-necked round bottom flask, the reaction proceeded at 90°C for 2 hours. 300 mL of ethyl acetate was added to the reaction solution, the reaction solution was washed with 300 mL of the saturated brine for 3 times, dried and concentrated to obtain the target crude product: (S)-4,6-dichloro-2-(((1-ethylpyrrolidin-2-yl)methyl)amino)nicotinamide (12.3g, yield: 77%). ES-API: [M+H]⁺=317.1.

Step 2: (S)-4,6-dichloro-2-(((1-ethylpyrrolidin-2-yl)methyl)amino)nicotinamide (5.5g,17.3mmol) and 100mL of anhydrous dimethylformamide were added to a 250 mL three-necked round bottom flask, NaH (7.0g, 17.5mmol) was added under the ice water bath, stirred for 30 minutes with heat preserved, N,N-carbonyldiimidazole (5.2g, 32mmol) was added to the solution, and after five minutes, tert-butyl (R)-3-(hydroxymethyl)piperazin-1-carboxylate(3.8g, 17.6mmol) was added under the ice water bath, reacted for 20 minutes with heat preserved. 300 mL of ethyl acetate was added to the reaction solution, the reaction solution was washed with 300 mL of the saturated brine for 3 times, dried, the filtrate was concentrated and passed through the column to obtain the target product: tert-butyl (R)-3-(((7-chloro-1-(((S)-1-ethylpyrrolidin-2-yl)methyl)-2,4-dioxo-1,2,3,4-tetra hydropyrido[2,3-d]pyrimidin-5-yl)oxy)methylpiperazin-1-formate (4.5g, yield: 49%). ES-API: [M+H]⁺=523.3.

Step 3: tert-butyl (R)-3-(((7-chloro-1-(((S)-1-ethylpyrrolidin-2-yl)methyl)-2,4-dioxo-1,2,3,4-tetra hydropyrido[2,3-d]pyrimidin-5-yl)oxy)methylpiperazin-1-formate (5.2g,9.9mmol), DIEA (23g,178mmol), T₃P (23g,36mmol) and 50mL of dichloromethane were added to a 100 mL three-necked round bottom flask, and stirred at room temperature for 2 hours. 80 mL of ethyl acetate was added to the reaction solution, the reaction solution was washed with 80 mL of the saturated brine for three times. The phase in ethyl acetate was dried and concentrated. The crude product was purified by a fast silica gel column to obtain the target product: tert-butyl (R)-2-chloro-12-(((S)-1-ethylpyrrolidin-2-yl)methyl)-11-oxo5a,6,8,9,11,12hexa hydro-4-oxo-1,7,9a,10,12-pentazabenzo[4,5]cyclohepta[1,2,3-de]naphthalen-7( 5H)-carboxylate (750mg, yield: 14.7%). ES-API: [M+H]⁺=505.3.

Step 4: tert-butyl (R)-2-chloro-12-(((S)-1-ethylpyrrolidin-2-yl)methyl)-11-oxo5a,6,8,9,11,12hexa hydro-4-oxo-1,7,9a,10,12-pentazabenzo[4,5]cyclohepta[1,2,3-de]naphthalen-7( 5H)-carboxylate (400mg, 0.79mmol), NCS (300mg, 2.25mmol) were added to a 100 mL three-necked round bottom flask, and the reaction proceeded at 80°C for 2 hours. 30 mL of ethyl acetate was added to the reaction solution, the reaction solution was washed with 30 mL of the saturated brine for 3 times, dried and concentrated. The crude product was purified by a fast silica gel column to obtain the target product: tert-butyl (R)-2,3-dichloro-12-(((S)-1-ethylpyrrolidin-2-yl)methyl)-11-oxo-5α,6,8,9,11,12 hexahydro 4-oxo-1,7,9a,10,12-pentazabenzo[4,5]cyclohepta[1,2,3-de]naphthalen-7(5H)-ca rboxylate (400mg, yield: 94%). ES-API: [M+H]+=539.2.

Step 5: tert-butyl (R)-2,3-dichloro-12-(((S)-1-ethylpyrrolidin-2-yl)methyl)-11-oxo-5a,6,8,9,11,12 hexahydro 4-oxo-1,7,9a,10,12-pentazabenzo[4,5]cyclohepta[1,2,3-de]naphthalen-7(5H)-ca rboxylate (370mg, 0.68mmol), (2-fluorophenyl)boronic acid (380mg,2.7mmol), tetratriphenylphosphine palladium (150mg, 0.13mmol), sodium carbonate (209mg, 1.97mmol), 10mL of dioxane and 2mL of water were added to a 100 mL three-necked round bottom flask. The system was replaced with the nitrogen for three times, and then protected with a nitrogen ball. The reaction proceeded at 120°C for 1 hour. 30 mL of ethyl acetate was added to the reaction solution, the reaction solution was washed with 30 mL of the saturated brine for 3 times, dried and concentrated. The crude product was purified by a fast silica gel column to obtain the target product: tert-butyl (R)-3-chloro-12-(((S)-1-ethylpyrrolidin-2-yl)methyl)-2-(2-fluorophenyl)-11-ox o-5a,6,8,9,11,12-hexahydro-4-oxo-1,7,9a,10,12-pentazabenzo[4,5]cyclohepta[1 ,2,3-de]naphthalen-7(5H)-carboxylate (199mg, yield: 96%). ES-API: [M+H]⁺=599.2.

Step 6: tert-butyl (R)-3-chloro-12-(((S)-1-ethylpyrrolidin-2-yl)methyl)-2-(2-fluorophenyl)-11-ox o-5a,6,8,9,11,12-hexahydro-4-oxo-1,7,9a,10,12-pentazabenzo[4,5]cyclohepta[1 ,2,3-de]naphthalen-7(5H)-carboxylate (199 mg,0.33mmol) was dissolved in dichloromethane (8 mL), and trifluoroacetic acid (2 mL) was added. The reaction was stirred at room temperature for 2 hours. The reaction solution was concentrated to obtain the target intermediate. The intermediate was dissolved in dichloromethane(15 mL), and triethylamine(1g, 10mmol) was added. The reaction was cooled to 0°C, and acrylic anhydride (50mg, 0.39mmol) was dropped to the reaction solution. The reaction was stirred at 0°C for 15 minutes. 40 mL of dichloromethane was added to the reaction solution, the reaction solution was washed with 50mL of the saturated NaHCO₃ aqueous solution, 40mL of the saturated brine, dried and concentrated. The crude product was purified by preparative liquid chromatography to obtain the target product: Z113(52mg,yield: 28%). ES-API: [M+H]⁺=553.2. ¹H NMR (500 MHz, DMSO-*d₆*) δ 7.65-7.50 (m, 2H), 7.38 (dd, *J* = 12.9, 5.9 Hz, 2H), 6.93-6.76 (m, 1H), 6.28-6.16 (m, 1H), 5.76 (d, *J* = 12.0 Hz, 1H), 4.88-4.73 (m, 2H), 4.53-3.98 (m, 6H), 3.55 (t, *J* = 19.2 Hz, 2H), 3.20 (s, 1H), 2.99-2.71 (m, 3H), 2.22-2.06 (m, 2H), 1.75-1.52 (m, 4H), 0.84 (t, *J* = 7.1 Hz, 3H).

### Example 114: Preparation of Compound Z114

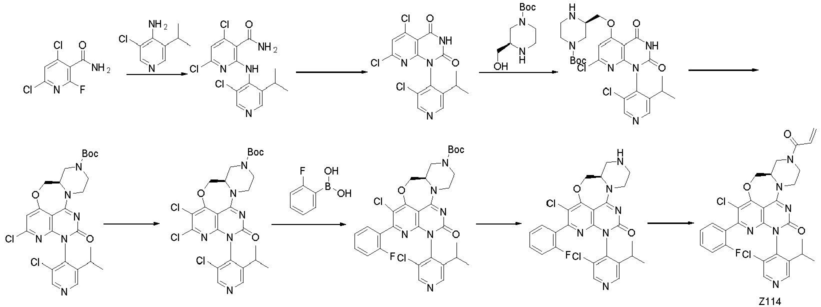

Compound Z114 was prepared with 3-chloro-5-isopropylpyridin-4-amine as the raw material according to the method of Example 93. ¹H NMR (500 MHz, DMSO-*d₆*) δ 8.65 (d, *J* = 4.2 Hz, 1H), 8.59 (d, *J* = 3.4 Hz, 1H), 7.52-7.46 (m, 1H), 7.30-7.23 (m, 2H), 7.15 (t, *J =* 7.3 Hz, 1H), 6.90-6.78 (m, 1H), 6.21 (d, *J* = 16.5 Hz, 1H), 5.80-5.75 (m, 1H), 4.89 (dd, *J* = 19.2, 13.8 Hz, 2H), 4.51 (d, *J* = 32.0 Hz, 2H), 4.32 (d, *J* = 28.9 Hz, 2H), 4.03 (s, 1H), 3.75 (dd, *J* = 41.6, 27.4 Hz, 2H), 3.36 (s, 1H), 3.30 (s, 1H), 2.78-2.65 (m, 1H), 1.16 (d, *J =* 6.8 Hz, 3H), 1.07-1.02 (m, 3H).

### Example 115: Preparation of Compound Z115

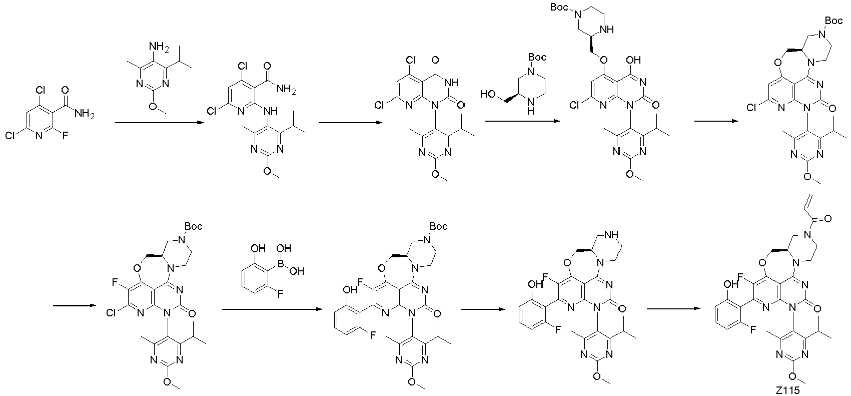

Compound Z115 was prepared with 4-isopropyl-2-methoxy-6-methylpyrimidin-5-amine as the raw material according to the method of Example 92. ES-API: [M+H]⁺=606.2. ¹H NMR (500 MHz, DMSO-*d₆*) δ 10.19 (d, *J* = 4.2 Hz, 1H), 7.27 (dd, *J* = 15.5, 8.0 Hz, 1H), 6.98-6.78 (m, 1H), 6.76-6.56 (m, 2H), 6.21 (d, *J* = 16.6 Hz, 1H), 5.78 (dd, *J* = 10.4, 2.2 Hz, 1H), 4.98-4.74 (m, 2H), 4.57 (s, 1H), 4.46 (d, *J* = 12.8 Hz, 1H), 4.33 (d, *J =* 4.4 Hz, 2H), 4.04 (s, 1H), 3.75 (s, 2H), 2.86-2.57 (m, 1H), 2.04 (d, *J* = 18.0 Hz, 3H), 1.06 (d, *J* = 6.8Hz, 3H), 0.93 (dd, *J* = 10.0, 6.8Hz, 3H).

### Example 116: Preparation of Compound Z116

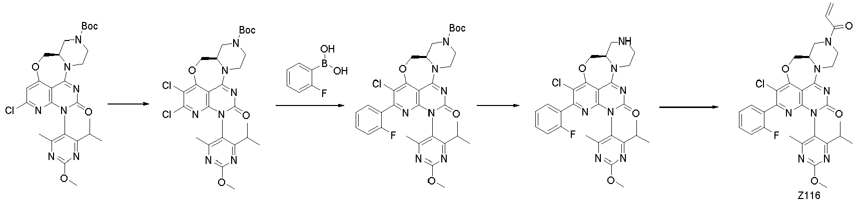

Compound Z116 was prepared according to the method of steps 6-9 in Example 93. ES-API: [M+H]⁺=606.2. ¹H NMR (500 MHz, DMSO-*d₆*) δ 7.51 (dd, *J* = 13.6, 5.7 Hz, 1H), 7.29 (dd, *J =* 18.2, 9.0 Hz, 2H), 7.25-7.17 (m, 1H), 6.97-6.72 (m, 1H), 6.21 (dd, *J* = 16.7, 2.3 Hz, 1H), 5.78 (dd, *J* = 10.4, 2.2 Hz, 1H), 4.88 (ddd, *J* = 19.6, 17.8, 8.6 Hz, 2H), 4.68-4.52 (m, 1H), 4.43 (dd, *J* = 54.5, 13.2 Hz, 1H), 4.30 (s, 1H), 4.06 (d, *J* = 27.0 Hz, 1H), 3.89 (s, 3H), 3.84-3.58 (m, 2H), 2.82-2.60 (m, 1H), 2.06 (d, *J* = 13.0 Hz, 3H), 1.06 (d, *J* = 6.8Hz, 3H), 0.93 (d, *J* = 6.8 Hz, 3H).

### Example 117: Preparation of Compound Z117

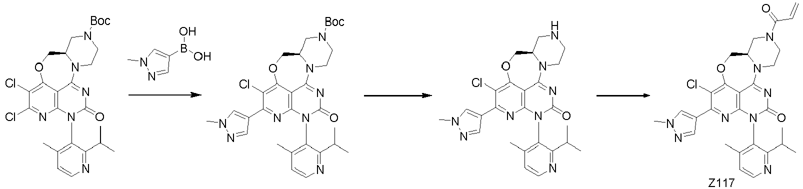

Compound Z117 was prepared with (1-methyl-1H-pyrazole-4-yl)boronic acid as the raw material according to the method of Example 46. ES-API: [M+H]+=561.2. ¹H NMR (500 MHz, DMSO-*d₆*) δ 8.44 (dd, *J* = 69.0, 4.5 Hz, 2H), 7.34-7.19 (m, 2H), 6.95-6.76 (m, 1H), 6.21 (d, *J* = 16.6 Hz, 1H), 5.77 (d, *J* = 10.4 Hz, 1H), 4.83 (s, 2H), 4.70-4.01 (m, 4H), 3.83 (s, 3H), 3.64 (t, *J =* 20.8 Hz, 2H), 3.32 (s, 1H), 2.82-2.63 (m, 1H), 1.93 (d, *J =* 16.8 Hz, 3H), 1.00 (ddd, *J =* 17.9, 8.9, 4.8 Hz, 6H).

### Example 118: Preparation of Compound Z118

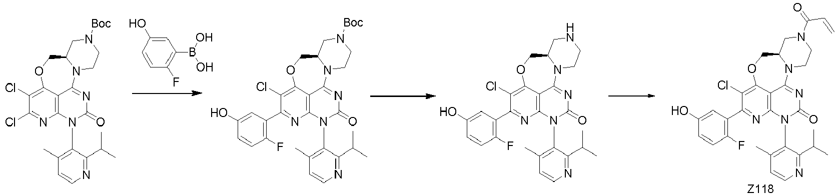

Compound Z118 was prepared with (2-fluoro-5-hydroxyphenyl)boronic acid as the raw material according to the method of Example 46. ES-API: [M+H]+=591.2. ¹H NMR (500 MHz, DMSO-*d₆*) δ 9.62 (s, 1H), 8.37 (d, *J* = 4.8 Hz, 1H), 7.20-7.01 (m, 2H), 6.79 (d, *J* = 8.6 Hz, 2H), 6.48-6.39 (m, 1H), 6.21 (dd, *J* = 16.7, 2.0 Hz, 1H), 5.78 (d, *J* = 12.4 Hz, 1H), 4.86 (dd, *J* = 20.3, 9.2 Hz, 2H), 4.28 (s, 3H), 4.07 (d, *J* = 37.9 Hz, 1H), 3.66 (s, 2H), 3.34 (s, 1H), 2.79-2.65 (m, 1H), 1.93 (d, *J* = 12.4 Hz, 3H), 1.00 (dd, *J* = 59.9, 6.6 Hz, 6H).

### Example 119: Preparation of Compound Z119

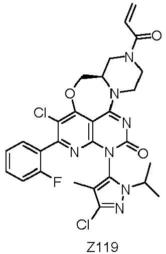

Compound Z119 was prepared with 3-chloro-1-isopropyl-4-methyl-1H-pyrazole-5-amine and 4,6-dichloro-2-fluoronicotinamide as the raw material according to the method of Example 93.

### Example 120: Preparation of Compound Z120

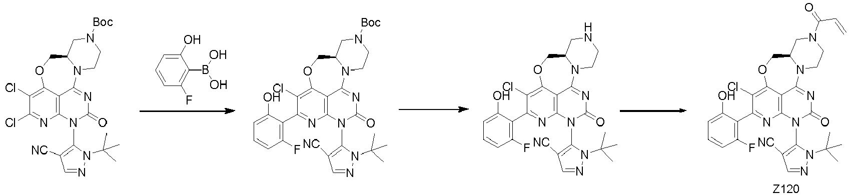

Step 1: tert-butyl (R)-12-(1-(tert-butyl)-4-cyano-1H-pyrazole-5-yl)-2,3-dichloro-11-oxo-5a,6,8,9, 11,12-hexahydro-4-oxo-1,7,9a,10,12-pentazabenzo[4,5]cyclohepta[1,2,3-de]na phthalen-7(5H)-carboxylate (550mg, 0.95mmol), (2-fluoro-6-hydroxyphenyl)boronic acid (447mg, 2.86 mmol), SPhos-Pd-G2 (102mg, 0.14mmol), sodium carbonate (304mg, 2.87mmol), 10mL of dioxane and 2mL of water were added to a 100 mL three-necked round bottom flask. The system was replaced with the nitrogen for three times, and then protected with a nitrogen ball. The reaction proceeded at 110°C for 2 hours. 30 mL of ethyl acetate was added to the reaction solution, the reaction solution was washed with 30 mL of the saturated brine for 3 times, dried and concentrated. The crude product was purified by a fast silica gel column to obtain the target product: tert-butyl (5aR)-12-(1-(tert-butyl)-4-cyano-1H-pyrazole-5-yl)-3-chloro-2-(2-fluoro-6-hyd roxyphenyl)-11-oxo-5a,6,8,9,11,12-hexahydro-4-oxo-1,7,9a,10,12-pentazabenz o[4,5]cyclohepta[1,2,3-de]naphthalen-7(5H)-carboxylate (350mg, yield: 56%). ES-API: [M+H]+=651.2.

Step 2: tert-butyl (SaR)-12-(1-(tert-butyl)-4-cyano-1H-pyrazole-5-yl)-3-chloro-2-(2-fluoro-6-hyd roxyphenyl)-11-oxo-5a,6,8,9,11,12-hexahydro-4-oxo-1,7,9a,10,12-pentazabenz o[4,5]cyclohepta[1,2,3-de]naphthalen-7(5H)-carboxylate (350 mg,0.53mmol) was dissolved in dichloromethane (8 mL), and trifluoroacetic acid (2 mL) was added. The reaction was stirred at room temperature for 2 hours. The reaction solution was concentrated to obtain the target intermediate.

Step 3: the intermediate was dissolved in dichloromethane (15 mL), and triethylamine (1g, 9.9mmol) was added. The reaction was cooled to 0°C, and acrylic anhydride (14 mg, 0.11mmol) was dropped to the reaction solution. The reaction was stirred at 0°C for 15 minutes. 40 mL of dichloromethane was added to the reaction solution, the reaction solution was washed with 50mL of the saturated NaHCO₃ aqueous solution, 40mL of the saturated brine, dried and concentrated. The crude product was purified by preparative liquid chromatography to obtain Compound Z120 (64mg,yield:44%). ES-API: [M+H]⁺=605.1. ¹H NMR (400 MHz, DMSO-*d₆*) δ 10.17 (s, 1H), 8.16-8.09 (m, 1H), 7.26 (d, *J* = 7.2 Hz, 1H), 6.90-6.66 (m, 3H), 6.20 (dd, *J* = 16.7, 2.2 Hz, 1H), 5.77 (dd, *J* = 10.4, 2.2 Hz, 1H), 4.91 (s, 2H), 4.63-4.27 (m, 4H), 3.86 (d, *J* = 102.5 Hz, 3H), 3.55-3.37 (m, 1H), 1.40 (dd, *J* = 5.0, 3.2 Hz, 9H).

### Example 121: Preparation of Compound Z121, Z121-1 and Z121-2

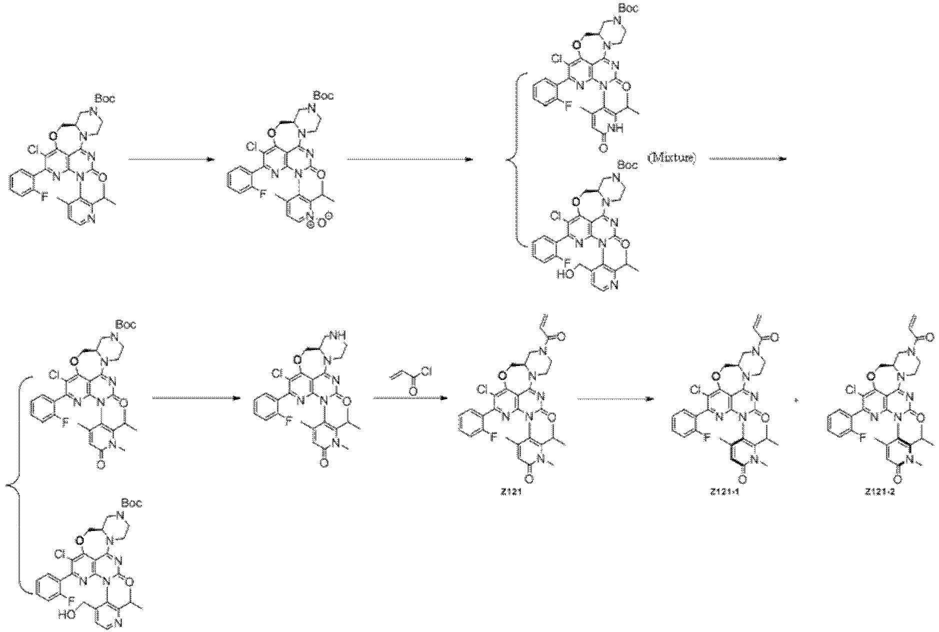

Step 1: m-chloroperoxybenzoic acid (2.78 g, 16.10 mmol) was added to a solution of tert-butyl (R)-3-chloro-2-(2-fluorophenyl)-12-(2-isopropyl-4-methylpyridin-3-yl)-11-oxo -5a,6,8,9,11,12-hexahydro-4-oxo-1,7,9a,10,12-pentazabenzo[4,5]cyclohepta[1, 2,3-de]naphthalen-7(5H)-carboxylate (5 g, 8.05 mmol) in dichloromethane (70 mL). The reaction solution was stirred at room temperature overnight. After the completion of the reaction, the reaction solution was diluted with dichloromethane(100 mL), washed with water (50 mL^{∗}3), the organic phase was dried and concentrated, and purified by a fast silica column (0-10% methanol /dichloromethane) to obtain a yellow solid: (R)-3-(7-(tert-butoxycarbonyl)-3-chloro-2-(2-fluorophenyl)-11-oxo-5,5a,6,7,8,9 -hexahydro-4-oxo-1,7,9a,10,12-pentazabenzo[4,5]cyclohepta[1,2,3-de]naphthal en-12(11H)-yl)-2-isopropyl-4-methylpyridine1-oxide (2.5 g, yield=49%). ES-API: [M+H]+=637.2.

Step 2: A solution of Compound (R)-3-(7-(tert-butoxycarbonyl)-3-chloro-2-(2-fluorophenyl)-11-oxo-5,5a,6,7,8,9 -hexahydro-4-oxo-1,7,9a,10,12-pentazabenzo[4,5]cyclohepta[1,2,3-de]naphthal en-12(11H)-yl)-2-isopropyl-4-methylpyridine1-oxide (2.5 g, 3.92 mmol) in acetic anhydride (25 mL) was stirred at 80°C for 1 hour. When the raw material was completely converted to the intermediate state, it was concentrated in vacuum to remove acetic anhydride, re-dissolved in methanol (12 mL), and potassium carbonate was added (8 g). The mixture was stirred at room temperature for 30 minutes. After concentrating to remove methanol, 30 mL of water and 30 mL of dichloromethane were added, separated. The organic phase was dried and concentrated to obtain a mixture of tert-butyl (R)-3-chloro-2-(2-fluorophenyl)-12-(2-isopropyl-4-methyl-6-oxo-1,6-dihydrop yridin-3-yl)-11-oxo-5a,6,8,9,11,12-hexahydro-4-oxo-1,7,9a,10,12-pentazabenz o[4,5]cyclohepta[1,2,3-de]naphthalen-7(5H)-carboxylate and tert-butyl (R)-3-chloro-2-(2-fluorophenyl)-12-(4-(hydroxymethyl)-2-isopropylpyridin-3-yl)-11-oxo-5a,6,8,9,11,12-hexahydro-4-oxo-1,7,9a,10,12-pentazabenzo[4,5]cyc lohepta[1,2,3-de]naphthalen-7(5H)-carboxylate (2.3 g). ES-API: [M+H]+=637.2.

Step 3: methyl iodide (5.12 g, 36.10 mmol) and potassium carbonate (1.50 g, 10.83 mmol) was added to a solution of the mixture of tert-butyl (R)-3-chloro-2-(2-fluorophenyl)-12-(2-isopropyl-4-methyl-6-oxo-1,6-dihydrop yridin-3-yl)-11-oxo-5a,6,8,9,11,12-hexahydro-4-oxo-1,7,9a,10,12-pentazabenz o[4,5]cyclohepta[1,2,3-de]naphthalen-7(5H)-carboxylate and tert-butyl (R)-3-chloro-2-(2-fluorophenyl)-12-(4-(hydroxymethyl)-2-isopropylpyridin-3-yl)-11-oxo-5a,6,8,9,11,12-hexahydro-4-oxo-1,7,9a,10,12-pentazabenzo[4,5]cyc lohepta[1,2,3-de]naphthalen-7(5H)-carboxylate (2.3 g, 3.61 mmol) in acetonitrile (20 mL), the tube was sealed, and stirred at 65°C for 6 hours. The reaction solution was concentrated, and purified by a fast silica column (0-5%methanol /dichloromethane) to obtain two compounds: tert-butyl (R)-3-chloro-2-(2-fluorophenyl)-12-(2-isopropyl-1,4-dimethyl-6-oxo-1,6-dihyd ropyridin-3-yl)-11-oxo-5a,6,8,9,11,12-hexahydro-4-oxo-1,7,9a,10,12-pentazabe nzo[4,5]cyclohepta[1,2,3-de]naphthalen-7(5H)-carboxylate (600 mg, yield=15%). ES-API: [M+H]⁺=651.2. Compound tert-butyl (R)-3-chloro-2-(2-fluorophenyl)-12-(4-(hydroxymethyl)-2-isopropylpyridin-3-yl)-11-oxo-5a,6,8,9,11,12-hexahydro-4-oxo-1,7,9a,10,12-pentazabenzo[4,5]cyc lohepta[1,2,3-de]naphthalen-7(5H)-carboxylate (750 mg, yield=31%). ES-API: [M+H]+=637.2.

Step 4: tert-butyl (R)-3-chloro-2-(2-fluorophenyl)-12-(2-isopropyl-1,4-dimethyl-6-oxo-1,6-dihyd ropyridin-3-yl)-11-oxo-5a,6,8,9,11,12-hexahydro-4-oxo-1,7,9a,10,12-pentazabe nzo[4,5]cyclohepta[1,2,3-de]naphthalen-7(5H)-carboxylate (600 mg, 0.92 mmol) was dissolved in dichloromethane(8 mL), and trifluoroacetic acid (2 mL) was added. The reaction was stirred at room temperature for 1 hour. The reaction solution was concentrated to obtain the crude product: (R)-3-chloro-2-(2-fluorophenyl)-12-(2-isopropyl-1,4-dimethyl-6-oxo-1,6-dihyd ropyridin-3-yl)-5,5a,6,7,8,9-hexahydro-4-oxo-1,7,9a,10,12-pentazabenzo[4,5]c yclohepta[1,2,3-de]naphthalen-11(12H)-one (508 mg), directly used in the next step. ES-API: [M+H]⁺=551.2.

Step 5: (R)-3-chloro-2-(2-fluorophenyl)-12-(2-isopropyl-1,4-dimethyl-6-oxo-1,6-dihyd ropyridin-3-yl)-5,5a,6,7,8,9-hexahydro-4-oxo-1,7,9a,10,12-pentazabenzo[4,5]c yclohepta[1,2,3-de]naphthalen-11(12H)-one (508 mg, 0.92 mmol) was dissolved in dichloromethane(6 mL), and N,N-diisopropylethylamine(596 mg, 4.61 mmol) was added. The reaction solution was cooled to 0°C, and acrylic chloride (167 mg, 1.84 mmol) was dropped. The reaction solution was stirred at 0°C for 10 minutes, concentrated, and the crude product was purified by Preparative HPLC to obtain Compound Z121 (160 mg, purity: 100%, yield:29%), a white solid. ¹H NMR (500 MHz, DMSO-*d₆*) δ 8.22 (s, 1H), 7.53-7.44 (m, 1H), 7.31-7.22 (m, 2H), 7.18-7.11 (m, 1H), 6.94-6.75 (m, 1H), 6.21 (dd, *J =* 16.5, 2.0 Hz, 1H), 5.77 (dd, *J* = 10.5, 2.0 Hz, 1H), 4.94-4.83 (m, 2H), 4.70-4.53 (m, 1H), 4.50-4.33 (m, 1H), 4.28 (s, 1H), 4.13-4.01 (m, 1H), 3.88 (d, *J =* 1.5 Hz, 3H), 3.77-3.60 (m, 2H), 3.29 (s, 1H), 2.70-2.58 (m, 1H), 1.78 (d, *J* = 13.5 Hz, 3H), 1.04 (d, *J* = 6.5 Hz, 3H), 0.95-0.90 (m, 3H).ES-API: [M+H]+=605.2.

Step 6: Compound Z121(160 mg, 0.26 mmol) was resolved by a chiral chromatographic column (column type: IC 250mm^{∗}4.6mm^{∗}5um; mobile phase: acetonitrile :isopropanol=70:30; flow rate:1mL/min; column temperature 30 °C) to obtain:Compound Z121-1(72 mg, peak 1, retention time 4.07 min, purity: 100%, de value: 100%). ¹H NMR (500 MHz, DMSO-*d₆*) δ 8.22 (s, 1H), 7.53-7.44 (m, 1H), 7.32-7.22 (m, 2H), 7.17-7.12 (m, 1H), 6.93-6.78 (m, 1H), 6.21 (dd, J = 16.5, 2.0 Hz, 1H), 5.81-5.75 (m, 1H), 4.95-4.84 (m, 2H), 4.62-4.51 (m, 1H), 4.42 (dd, J = 56.6, 11.7 Hz, 1H), 4.27 (s, 1H), 4.05 (d, J = 23.5 Hz, 1H), 3.88 (s, 3H), 3.80-3.63 (m, 2H), 3.33 (s, 1H), 2.65-2.59 (m, 1H), 1.80 (s, 3H), 1.04 (d, J = 7.0 Hz, 3H), 0.94 (d, J = 6.5 Hz, 3H).ES-API: [M+H]⁺=605.2.
and Compound Z121-2(46 mg, peak 2, retention time 5.51 min, purity:96%, de value: 97.3%). ¹H NMR (500 MHz, DMSO-*d₆*) δ8.23 (s, 1H), 7.53-7.45 (m, 1H), 7.32-7.22 (m, 2H), 7.19-7.13 (m, 1H), 6.95-6.78 (m, 1H), 6.21 (dd, J = 17.0, 2.0 Hz, 1H), 5.78 (dd, J = 10.5, 2 Hz, 1H), 4.94 (t, J = 12.0 Hz, 1H), 4.88-4.81 (m, 1H), 4.67-4.53 (m, 1H), 4.52-4.34 (m, 1H), 4.28 (s, 1H), 4.18-4.00 (m, 1H), 3.89 (s, 3H), 3.77-3.59 (m, 2H), 3.31 (s, 1H), 2.75-2.66 (m, 1H), 1.78 (s, 3H), 1.06-1.02 (m, 3H), 0.94 (d, J = 6.5 Hz, 3H).ES-API:[M+H]⁺=605.2.

### Example 122: Preparation of Compound Z122, Z122-1 and Z122-2

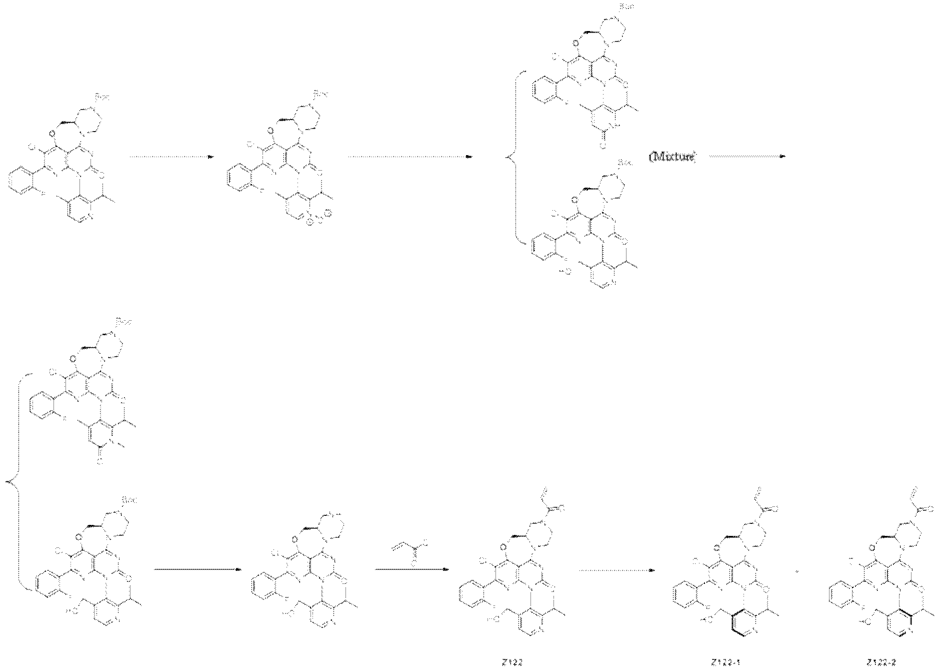

Step 1, Step 2 and Step 3 were the same as Step 1, Step 2 and Step 3 of Example 121;

Step 4: tert-butyl (R)-3-chloro-2-(2-fluorophenyl)-12-(4-(hydroxymethyl)-2-isopropylpyridin-3-yl)-11-oxo-5a,6,8,9,11,12-hexahydro-4-oxo-1,7,9a,10,12-pentazabenzo[4,5]cyc lohepta[1,2,3-de]naphthalen-7(5H)-carboxylate (750 mg, 1.18 mmol) was dissolved in dichloromethane (8 mL), and trifluoroacetic acid (2 mL) was added. The reaction was stirred at room temperature for 1 hour. The reaction solution was concentrated to obtain the crude product: (R)-3-chloro-2-(2-fluorophenyl)-12-(4-(hydroxymethyl)-2-isopropylpyridin-3-yl)-5,5a,6,7,8,9-hexahydro-4-oxo-1,7,9a,10,12-pentazabenzo[4,5]cyclohepta[1, 2,3-de]naphthalen-11(12H)-one (632 mg), directly used in the next step. ES-API: [M+H]⁺=537.2.

Step 5: (R)-3-chloro-2-(2-fluorophenyl)-12-(4-(hydroxymethyl)-2-isopropylpyridin-3-yl)-5,5a,6,7,8,9-hexahydro-4-oxo-1,7,9a,10,12-pentazabenzo[4,5]cyclohepta[1, 2,3-de]naphthalen-11(12H)-one (632 mg, 1.18 mmol) was dissolved in dichloromethane (6 mL), and N,N-diisopropylethylamine (760 mg, 5.88 mmol) was added. The reaction solution was cooled to 0°C, and acrylic chloride (85 mg, 0.94 mmol) was dropped. The reaction solution was stirred at 0°C for 10 minutes, concentrated, and the crude product was purified by Preparative HPLC to obtain Compound Z122 (470 mg, purity: 100%, yield:67%), a white solid. ¹H NMR (500 MHz, DMSO-*d₆*) δ 8.49 (d, J = 5.0 Hz, 1H), 7.53-7.43 (m, 1H), 7.39-7.34 (m, 1H), 7.30-7.20 (m, 2H), 7.17-7.11 (m, 1H), 6.96-6.76 (m, 1H), 6.21 (dd, J = 16.5, 2.5 Hz, 1H), 5.78 (dd, J = 10.5, 2.0 Hz, 1H), 5.37-5.29 (m, 1H), 4.97-4.80 (m, 2H), 4.63-4.50 (m, 1H), 4.49-4.34 (m, 1H), 4.32-4.21 (m, 2H), 4.18-3.99 (m, 2H), 3.82-3.63 (m, 2H), 3.29 (s, 1H), 2.81-2.62 (m, 1H), 1.06 (d, J = 6.5 Hz, 3H), 0.95 (dd, J = 6.5, 2.5 Hz, 3H).ES-API: [M+H]⁺=591.2.

Step 6: Compound Z122(470 mg, 0.79 mmol) was resolved by a chiral chromatographic column (column type: IC 250mm^{∗}4.6mm^{∗}5um; mobile phase: acetonitrile :isopropanol=70:30; flow rate:1mL/min; column temperature= 30 °C) to obtain:Compound Z122-1(177 mg, peak 1, retention time4.34 min, purity: 100%, de value: 100%). ¹H NMR (500 MHz, DMSO-*d₆*) δ 8.50 (d, J = 5.0 Hz, 1H), 7.51-7.44 (m, 1H), 7.38 (d, J = 4.5 Hz, 1H), 7.33-7.20 (m, 2H), 7.18-7.11 (m, 1H), 6.94-6.78 (m, 1H), 6.21 (dd, J = 16.5, 2.5 Hz, 1H), 5.78 (dd, J = 10.5, 2.0 Hz, 1H), 5.35 (s, 1H), 4.93 (s, 1H), 4.84 (dd, J = 13.0, 5.0 Hz, 1H), 4.68-4.52 (m, 1H), 4.51-4.33 (m, 1H), 4.33-4.22 (m, 2H), 4.16-3.99 (m, 2H), 3.81-3.59 (m, 2H), 3.33 (s, 1H), 2.84-2.71 (m, 1H), 1.07 (d, J = 6.5 Hz, 3H), 0.95 (d, J = 6.5 Hz, 3H). ES-API: [M+H]⁺=591.2.
and Compound Z122-2 (266 mg, peak 2, retention time7.32 min, purity:97.55%, de value: 100%). ¹H NMR (500 MHz, DMSO-*d₆*) δ 8.50 (d, J = 5.0 Hz, 1H), 7.51-7.44 (m, 1H), 7.38 (d, J = 4.0 Hz, 1H), 7.32-7.19 (m, 2H), 7.16-7.11 (m, 1H), 6.96-6.76 (m, 1H), 6.21 (dd, J = 17.0, 2.5 Hz, 1H), 5.78 (dd, J = 10.5, 2.5 Hz, 1H), 5.36 (s, 1H), 5.00-4.79 (m, 2H), 4.63-4.50 (m, 1H), 4.49-4.34 (m, 1H), 4.32-4.21 (m, 2H), 4.18-3.99 (m, 2H), 3.82-3.63 (m, 2H), 3.36 (s, 1H), 2.75-2.65 (m, 1H), 1.07 (d, J = 6.7 Hz, 3H), 0.96 (d, J = 6.7 Hz, 3H). ES-API:[M+H]⁺=591.2.

### Example 123: Preparation of Compound Z123

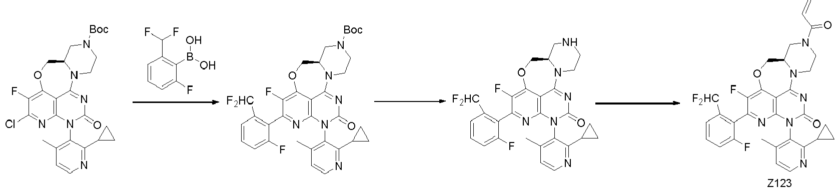

Compound Z123 was prepared with (2-(difluoromethyl)-6-fluorophenyl)boronic acid as the raw material according to the method of steps 3-5 in Example 72. ES-API: [M+H]⁺=607.2. ¹H NMR (500 MHz, DMSO-*d₆*) δ 8.22 (d, *J* = 4.8 Hz, 1H), 7.70 (dd, *J* = 13.6, 8.0 Hz, 1H), 7.55 (dd, *J =* 18.2, 8.6 Hz, 2H), 7.07 (s, 1H), 6.92-6.60 (m, 1H), 6.58 (d, *J* = 47.9 Hz, 1H), 6.21 (d, *J* = 17.0 Hz, 1H), 5.78 (dd, *J* = 10.4, 2.1 Hz, 1H), 4.71-4.03 (m, 2H), 4.46 (s, 2H), 4.35 (s, 2H), 4.01 (s, 1H), 3.73 (s, 1H), 3.43 (s, 1H), 1.94 (s, 3H), 1.73 (s, 1H), 1.02-0.78(m, 4H).

### Example 124: Preparation of Compound Z124

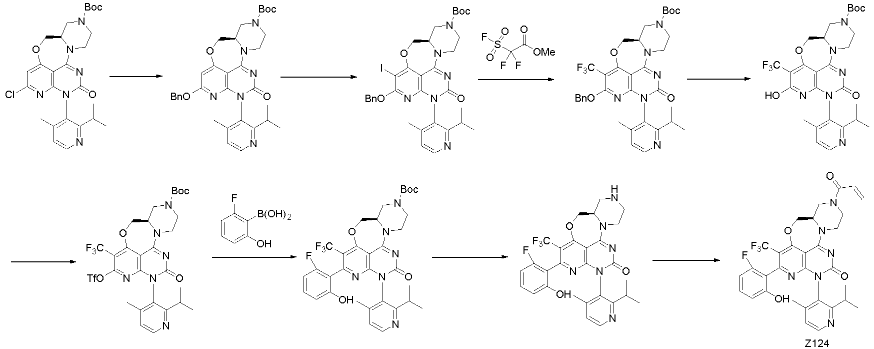

Step 1: tert-butyl (R)-2-chloro-12-(2-isopropyl-4-methylpyridm-3-yl)-11-oxo-5a,6,8,9,11,12-hex ahydro-4-oxo-1,7,9a,10,12-pentazabenzo[4,5]cycloheptan[1,2,3-de]-naphthalen -7(5H)-carboxylate (1.26 g, 2.4 mmol), benzyl alcohol (2.6 g, 24.0 mmol), XantPhos-Pd-G2 (192 mg, 0.22 mmol), xantphos (127 mg, 0.22 mmol), cesium carbonate (1.56 g, 4.8 mmol) and dioxane (10 mL) were added to the microwave tube. After nitrogen bubbling for 5 minutes in the system, the reaction proceeded under a 80°C microwave for 3.5 hours. After the completion of the reaction, filtered. The filtrate was concentrated, and the crude product was purified by a fast silica gel column (ethyl acetate/petroleum ether: 0-100%) to obtain the target product: tert-butyl (R)-2-(benzyloxy)-12-(2-isopropyl-4-methylpyridin-3-yl)-11-oxo-5a,6,8,9,11,1 2-hexahydro-4-oxo-1,7,9a,10,12-pentazabenzo[4,5]cyclohepta[1,2,3-de]naphth alen-7(5H)-carboxylate (866 mg, 59%), a yellow solid. ES-API:[M+H]⁺=599.3.

Step 2: tert-butyl (R)-2-(benzyloxy)-12-(2-isopropyl-4-methylpyridin-3-yl)-11-oxo-5a,6,8,9,11,1 2-hexahydro-4-oxo-1,7,9a,10,12-pentazabenzo[4,5]cyclohepta[1,2,3-de]naphth alen-7(5H)-carboxylate (2.6 g, 4.3 mmol), N-iodosuccinimide (2.4 g, 10.8 mmol) and acetonitrile (30 mL) were added to a round bottom flask. The reaction was stirred at 80°C for 16 hours. Sodium thiosulfate aqueous solution was added to the reaction solution. The reaction solution was extracted with ethyl acetate. The organic phase was dried and concentrated. The crude product was purified by a fast silica gel column (ethyl acetate/petroleum ether:0-100%) to obtain tert-butyl (R)-2-(benzyloxy)-3-iodo-12-(2-isopropyl-4-methylpyridin-3-yl)-11-oxo-5a,6,8 ,9,11,12-hexahydro-4-oxa-1,7,9a,10,12-pentazabenzo[4,5]cyclohepta[1,2,3-de] naphthalen-7(5H)-carboxylate (2.2 g), yield of 70%. ES-API:[M+H]⁺=725.2.

Step 3: tert-butyl (R)-2-(benzyloxy)-3-iodo-12-(2-isopropyl-4-methylpyridin-3-yl)-11-oxo-5a,6,8 ,9,11,12-hexahydro-4-oxa-1,7,9a,10,12-pentazabenzo[4,5]cyclohepta[1,2,3-de] naphthalen-7(5H)-carboxylate (1.9 g, 2.62 mmol), methyl 2,2-difluoro-2-(fluorosulfonyl)acetate (1.5 g, 7.86 mmol), cuprous iodide (1.5 g, 7.86 mmol), N,N-dimethylformamide (15 mL) were added to the microwave tube. The system was heated in a sealed tube under a 90°C oil bath for 2 hours. Water was added to the reaction solution. The reaction solution was extracted with ethyl acetate. The organic phase was dried and concentrated. The crude product was purified by a fast silica gel column (ethyl acetate/petroleum ether: 0-100%) to obtain the product: tert-butyl (R)-2-(benzyloxy)-12-(2-isopropyl-4-methylpyridin-3-yl)-11-oxo-3-(trifluorom ethyl)-5a,6,8,9,11,12-hexahydro-4-oxo-1,7,9a,10,12-pentazabenzo-[4,5]cyclohe pta[1,2,3-de]naphthalen-7(5H)-carboxylate (1 g, 57%), a yellow solid. ES-API:[M+H]⁺=667.2.

Step 4: tert-butyl (R)-2-(benzyloxy)-12-(2-isopropyl-4-methylpyridin-3-yl)-11-oxo-3-(trifluorom ethyl)-5a,6,8,9,11,12-hexahydro-4-oxo-1,7,9a,10,12-pentazabenzo-[4,5]cyclohe pta[1,2,3-de]naphthalen-7(5H)-carboxylate (1 g, 1.5 mmol), Pd/C (1 g,10% purity) and methanol (10 mL) were added to a round bottom flask. The reaction was stirred at room temperature for 16 hours. The completion of the reaction was detected by LC-MS. The reaction was filtered with diatomaceous earth, and the filtrate was concentrated to obtain the target product: tert-butyl (R)-2-hydroxy-12-(2-isopropyl-4-methylpyridin-3-yl)-11-oxo-3-(trifluorometh yl)-5a,6,8,9,11,12-hexahydro-4-oxo-1,7,9a,10,12-pentazabenzo[4,5]cyclohepta[ 1,2,3-de]naphthalen-7(5H)-carboxylate (800 mg, 93%). ES-API: [M+H]+=577.2.

Step 5: tert-butyl (R)-2-hydroxy-12-(2-isopropyl-4-methylpyridin-3-yl)-11-oxo-3-(trifluorometh yl)-5a,6,8,9,11,12-hexahydro-4-oxo-1,7,9a,10,12-pentazabenzo[4,5]cyclohepta[ 1,2,3-de]naphthalen-7(5H)-carboxylate (800 mg, 1.4 mmol), triethylamine (1.1 g, 11.2 mmol) and 10 mL of dichloromethane were added to a reaction flask. The reaction solution was cooled to 0°C, trifluoromethanesulfonic anhydride (1.56 g, 5.5 mmol) was dropped. The reaction was stirred at 0°C for 10 minutes. The saturated sodium bicarbonate aqueous solution was added to the reaction solution, the reaction solution was extracted with dichloromethane for 3 times, and the organic phase was dried and concentrated. The crude product was purified by a fast silica gel column (ethyl acetate/petroleum ether:0-80%) to obtain the target product: tert-butyl (R)-12-(2-isopropyl-4-methylpyridin-3-yl)-11-oxo-3-(trifluoromethyl)-2-(((trifl uoromethyl)sulfonyl)oxy)-5a,6,8,9,11,12hexahydro-4-oxo-1,7,9a,10,12-pentaza benzo[4,5]cyclohepta[1,2,3-de]naphthalen-7(5H)-carboxylate (500 mg), a yellow solid, yield of 50%. ES-API: [M+H]⁺=709.2 .

Step 6: tert-butyl (R)-12-(2-isopropyl-4-methylpyridin-3-yl)-11-oxo-3-(trifluoromethyl)-2-(((trifl uoromethyl)sulfonyl)oxy)-5a,6,8,9,11,12hexahydro-4-oxo-1,7,9a,10,12-pentaza benzo[4,5]cyclohepta[1,2,3-de]naphthalen-7(5H)-carboxylate (500 mg, 0.70 mmol), 2-fluoro-6-hydroxyphenylboronic acid (330 mg, 2.11 mmol), tetratriphenylphosphine palladium (81 mg, 0.07 mmol), sodium phosphate (224 mg, 2.11 mmol), 10 mL of dioxane and 2 mL of water were added to a round bottom flask. The reaction was stirred under a 100°C oil bath for 1 hour, and the reaction stopped. 50 mL of water was added to the reaction solution. The reaction solution was extracted with 50 mL of ethyl acetate for 3 times, and the organic phase was dried and concentrated. The crude product was purified by a fast silica gel column (ethyl acetate/petroleum ether:0-100%) to obtain the target product: tert-butyl (5aR)-2-(2-fluoro-6-hydroxyphenyl)-12-(2-isopropyl-4-methylpyridin-3-yl)-11-oxo-3-(trifluoromethyl)-5a,6,8,9,11,12hexahydro-4-oxo-1,7,9a,10,12-pentazabe nzo[4,5]cyclohepta[1,2,3-de]-naphthalene7(5H)-carboxylate (150 mg), a yellow solid, yield of 31%. ES-API:[M+H]⁺=671.2.

Step 7: tert-butyl (5aR)-2-(2-fluoro-6-hydroxyphenyl)-12-(2-isopropyl-4-methylpyridin-3-yl)-11-oxo-3-(trifluoromethyl)-5a,6,8,9,11,12hexahydro-4-oxo-1,7,9a,10,12-pentazabe nzo[4,5]cyclohepta[1,2,3-de]-naphthalene7(5H)-carboxylate (150 mg, 0.22 mmol), 1 mL of methanol and 4 M hydrogen chloride/dioxane solution (3 mL) were added to a round bottom flask. The reaction was stirred at room temperature for 1 hour. The completion of the reaction was detected by LC-MS. The reaction solution was concentrated to obtain the crude product: (5aR)-2-(2-fluoro-6-hydroxyphenyl)-12-(2-isopropyl-4-methylpyridin-3--yl)-3-( trifluoromethyl)-5,5a,6,7,8,9-hexahydro-4-oxo-1,7,9a,10,12-pentazabenzo[4,5] cyclohepta[1,2,3-de]naphthalen-11(12H)-one (125 mg), a yellow solid. ES-API: [M+H]⁺=571.2.

Step 8: (5aR)-2-(2-fluoro-6-hydroxyphenyl)-12-(2-isopropyl-4-methylpyridin-3-yl)-3-( trifluoromethyl)-5,5a,6,7,8,9-hexahydro-4-oxo-1,7,9a,10,12-pentazabenzo[4,5] cyclohepta[1,2,3-de]naphthalen-11(12H)-one (125 mg, 0.22 mmol), 5 mL of dichloromethane and triethylamine (111 mg, 1.1 mmol) were added to a round bottom flask. The reaction was cooled to 0°C, and acrylic anhydride in dichloromethane (23 mg, 0.18 mmol, 0.5 mL) was dropped to the reaction solution. The reaction was stirred at 0°C for 10 minutes. 20 mL of the saturated sodium bicarbonate aqueous solution was added to the reaction solution, and the reaction solution was extracted with 20 mL of dichloromethane for 3 times. The organic phase was dried and concentrated. The crude product was purified by Preparative HPLC to obtain Z124(46 mg), a white solid, yield of two steps is 33%. ¹HNMR (500MHz, CDCl₃) 8.57-8.54 (m, 1H), 8.53-8. 41 (m, 1H), 7.26-7.12 (m, 2H), 6.61-6.55 (m, 3H), 6.41-6.40 (m, 1H), 5.86-5.84 (m, 1H), 5.19-4.63 (m, 4H), 4.29-3.29 (m, 5H), 2.77-2.69 (m, 1H), 2.05-2.01 (m, 3H), 1.21-1.19 (m, 3H), 0.94-0.88 (m, 3H). ES-API: [M+H]⁺=625.1.

### Example 125 to Example 236

Compound Z125-236 was prepared according to the synthesis methods of the above Examples.

| Example No. | Compound Structure and No. | ES-API: [M+H]⁺ | Example No. | Compound Structure and No. | ES-API: [M+H]⁺ |
|---|---|---|---|---|---|
| 125 | 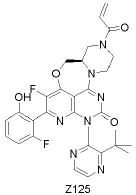 | 576.2 | 126 | 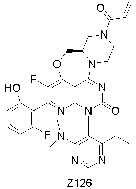 | 605.2 |
| 127 | 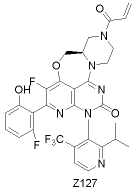 | 629.2 | 128 | 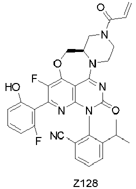 | 585.2 |
| 129 | 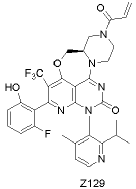 | 625.2 | 130 | 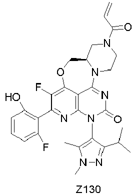 | 578.2 |
| 131 | 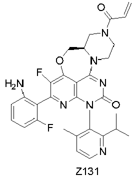 | 574.2 | 133 | 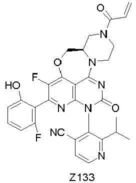 | 586.2 |
| 134 | 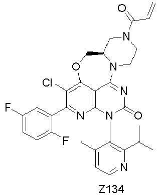 | 593.2 | 135 | 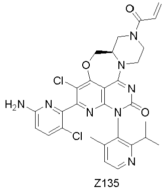 | 607.2 |
| 136 | 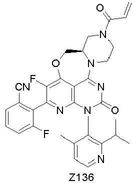 | 584.2 | 137 | 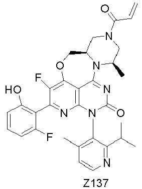 | 589.2 |
| 138 | 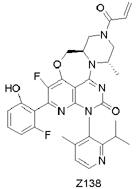 | 589.2 | 139 | 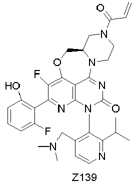 | 618.2 |
| 140 | 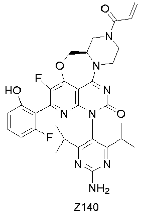 | 619.2 | 141 | 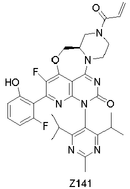 | 618.2 |
| 142 | 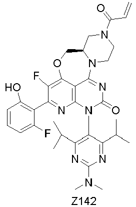 | 647.2 | 143 | 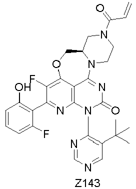 | 576.2 |
| 144 | 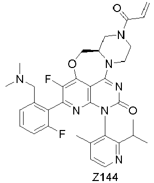 | 616.2 | 145 | 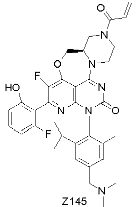 | 631.2 |
| 146 | 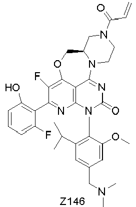 | 647.2 | 147 | 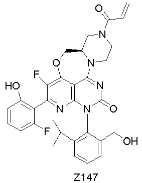 | 590.2 |
| 148 | 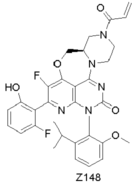 | 590.2 | 149 | 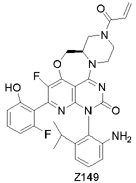 | 575.2 |
| 150 | 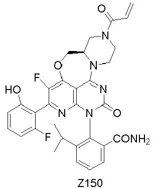 | 603.2 | 151 | 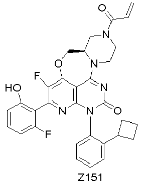 | 572.2 |
| 152 | 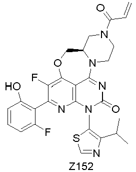 | 567.2 | 153 | 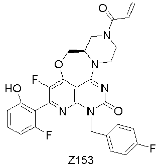 | 550.2 |
| 154 | 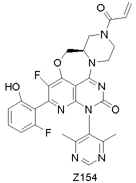 | 548.2 | 155 | 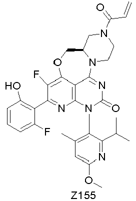 | 605.2 |
| 156 | 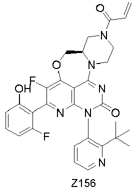 | 575.2 | 157 | 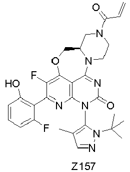 | 578.2 |
| 158 | 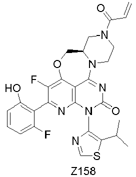 | 567.2 | 159 | 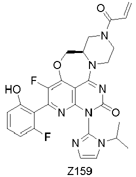 | 550.2 |
| 160 | 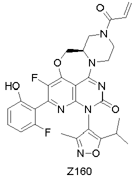 | 565.2 | 161 | 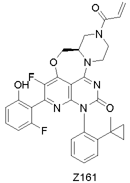 | 572.2 |
| 162 | 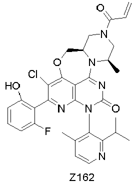 | 605.2 | 163 | 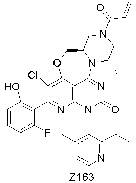 | 605.2 |
| 164 | 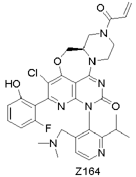 | 634.2 | 165 | 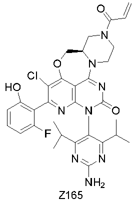 | 635.2 |
| 166 | 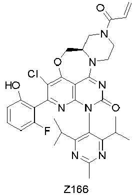 | 634.2 | 167 | 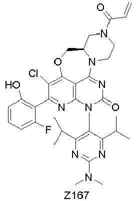 | 663.2 |
| 168 | 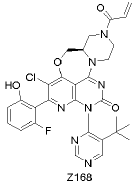 | 592.2 | 169 | 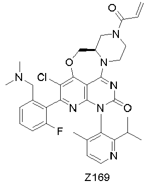 | 632.2 |
| 170 | 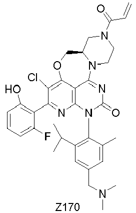 | 647.2 | 171 | 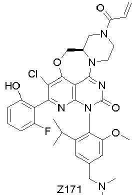 | 663.2 |
| 172 | 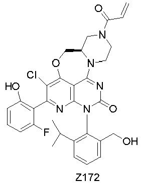 | 606.2 | 173 | 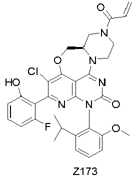 | 606.2 |
| 174 | 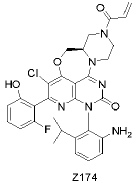 | 591.2 | 175 | 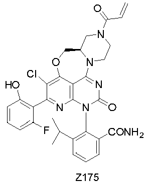 | 619.2 |
| 176 | 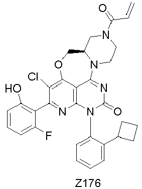 | 588.2 | 177 | 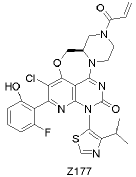 | 583.1 |
| 178 | 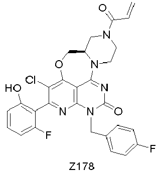 | 566.2 | 179 | 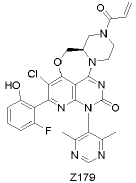 | 564.2 |
| 180 | 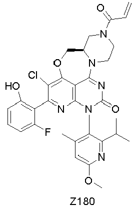 | 621.2 | 181 | 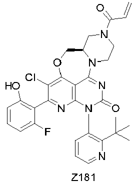 | 591.2 |
| 182 | 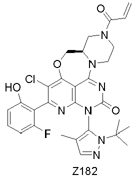 | 594.2 | 183 | 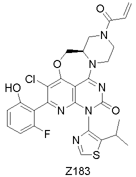 | 583.2 |
| 184 | 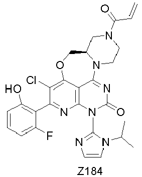 | 566.2 | 185 | 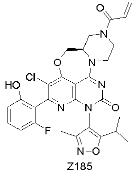 | 581.2 |
| 186 | 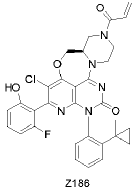 | 588.2 | 187 | 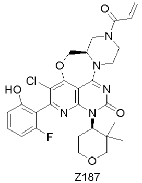 | 570.2 |
| 188 | 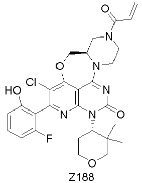 | 570.2 | 189 | 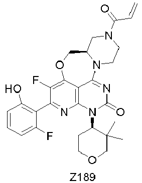 | 554.2 |
| 190 | 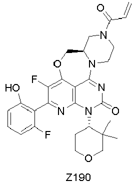 | 554.2 | 191 | 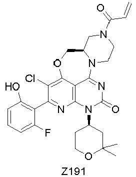 | 570.2 |
| 192 | 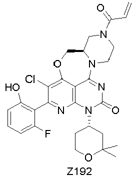 | 570.2 | 193 | 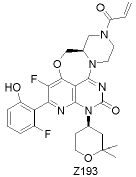 | 554.2 |
| 194 | 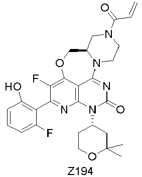 | 554.2 | 195 | 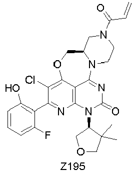 | 556.2 |
| 196 | 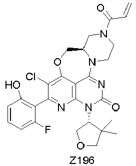 | 556.2 | 197 | 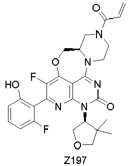 | 540.2 |
| 198 | 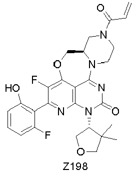 | 540.2 | 199 | 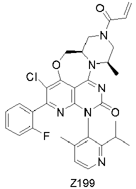 | 589.2 |
| 200 | 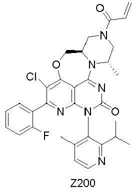 | 589.2 | 201 | 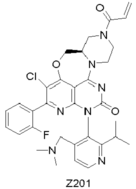 | 618.2 |
| 202 | 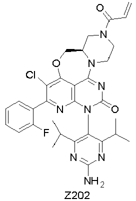 | 619.2 | 203 | 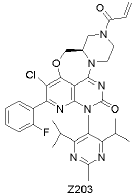 | 618.2 |
| 204 | 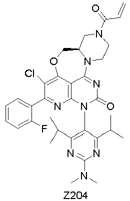 | 647.2 | 205 | 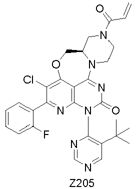 | 576.2 |
| 206 | 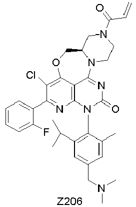 | 631.2 | 207 | 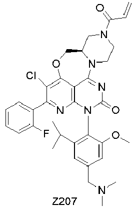 | 647.2 |
| 208 | 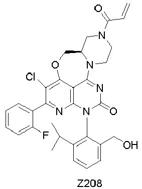 | 590.2 | 209 | 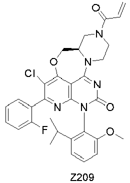 | 590.2 |
| 210 | 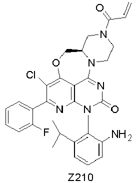 | 575.2 | 211 | 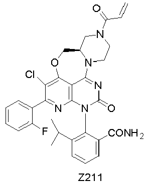 | 603.2 |
| 212 | 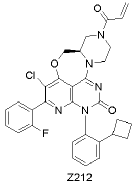 | 572.2 | 213 | 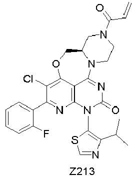 | 567.2 |
| 214 | 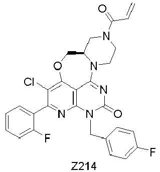 | 550.2 | 215 | 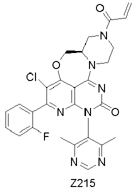 | 548.2 |
| 216 | 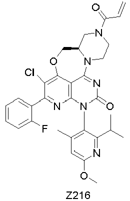 | 605.2 | 217 | 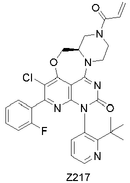 | 575.2 |
| 218 | 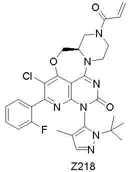 | 578.2 | 219 | 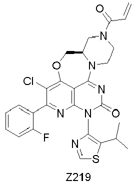 | 567.2 |
| 220 | 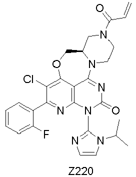 | 550.2 | 221 | 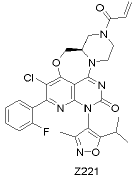 | 565.2 |
| 222 | 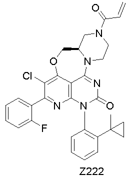 | 572.2 | 223 | 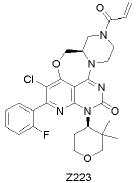 | 554.2 |
| 224 | 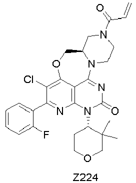 | 554.2 | 225 | 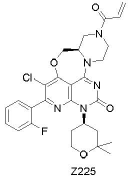 | 554.2 |
| 226 | 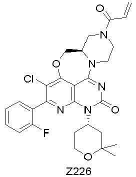 | 554.2 | 227 | 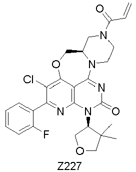 | 540.2 |
| 228 | 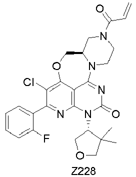 | 540.2 | 229 | 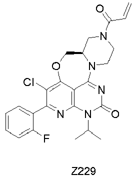 | 484.1 |
| 230 | 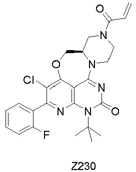 | 498.2 | 231 | 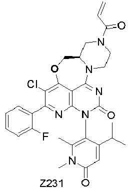 | 605.2 |
| 232 | 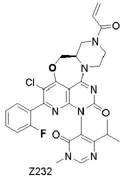 | 592.2 | 233 | 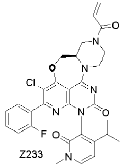 | 591.2 |
| 234 | 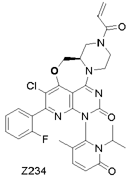 | 591.2 | 235 | 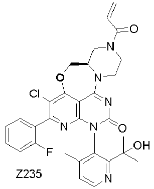 | 591.2 |
| 236 | 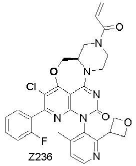 | 589.2 | | | |

### Example 237 Preparation of Compound Z237-1 and Z237-2

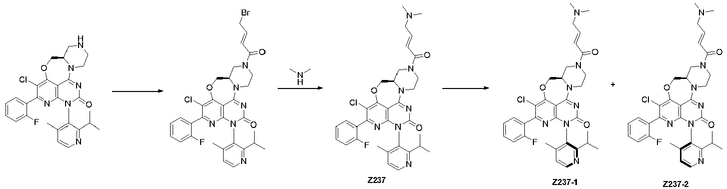

Step 1: N,N-diisopropylethylamine(182 mg, 1.44 mmol) was added to a solution of (R)-3-chloro-2-(2-fluorophenyl)-12-(2-isopropyl-4-methylpyridin-3-yl)-5,5a,6, 7,8,9-hexahydro-4-oxa-1,7,9a,10,12-pentazabenzo[4,5]cycloheptan[1,2,3-dinap hthalene] 11(12H)-one (250 mg, 0.48 mmol) in dichloromethane (4 mL) under the ice bath, then (E)-4-bromobut-2-enoyl chloride (88 mg, 0.48 mmol) was added, and stirred for 10 minutes. The reaction solution was dissolved in 10 mL of water, extracted with 10 mL of dichloromethane, the organic phase was dried and concentrated, and purified by a fast silica column (0-10%methanol /dichloromethane) to obtain a white solid: (R,E)-7-(4-bromobut-2-enoyl)-3-chloro-2-(2-fluorophenyl)-12-(2-isopropyl-4-methylpyridin-3-yl)-5,5a,6,7,8,9-hexahydro-4-oxa-1,7,9a,10,12-pentazabenzo[ 4,5]cyclohepta[1,2,3-de]naphthalen-11(12H)-one (300 mg), yield 93%.

Step 2: 1 M dimethylamine in tetrahydrofuran solution (1.35 mL, 1.35 mmol) was dropped into a solution of (R,E)-7-(4-bromobut-2-enoyl)-3-chloro-2-(2-fluorophenyl)-12-(2-isopropyl-4-methylpyridin-3-yl)-5,5a,6,7,8,9-hexahydro-4-oxa-1,7,9a,10,12-pentazabenzo[ 4,5]cyclohepta[1,2,3-de]naphthalen-11(12H)-one (300 mg, 0.45 mmol) in tetrahydrofuran under the ice bath, and stirred for 1 hour. The reaction solution was concentrated, and purified by Preparative HPLC(ammonium bicarbonate) to obtain (R,E)-3-chloro-7-(4-(dimethylamino)but-2-enoyl)-2-(2-fluorophenyl)-12-(2-iso propyl-4-methylpyridin-3-yl)-5,5a,6,7,8,9-hexahydro-4-oxa-1,7,9a,10,12-penta zabenzo[4,5]cyclohepta[1,2,3-de]naphthalen-11(12H)-one (120 mg), yield 42%.

Step 3: the above-obtained Compound (R,E)-3-chloro-7-(4-(dimethylamino)but-2-enoyl)-2-(2-fluorophenyl)-12-(2-iso propyl-4-methylpyridin-3-yl)-5,5a,6,7,8,9-hexahydro-4-oxa-1,7,9a,10,12-penta zabenzo[4,5]cyclohepta[1,2,3-de]naphthalen-11(12H)-one (120 mg, 0.19 mmol) was resolved chirally (column type: IG 250mm^{∗}4.6mm^{∗}5um; mobile phase: n-hexane:ethanol (0.2% ammonia methanol) = 40:60; flow rate:1mL/min; column temperature 30 °C) to obtain:Z237-1 (65 mg, peak 1, retention time 11.060 min, purity: 100%, de value: 100%). ¹H NMR (500 MHz, DMSO-d6) δ 8.35 (d, J = 5 Hz, 1H), 7.52 - 7.44 (m, 1H), 7.30 - 7.21 (m, 2H), 7.17 - 7.11 (m, 2H), 6.75 - 6.58 (m, 2H), 4.95 - 4.83 (m, 2H), 4.64 - 4.51 (m, 1H), 4.51 - 4.32 (m, 1H), 4.32 - 4.23 (m, 1H), 4.13 - 3.96 (m, 1H), 3.84 - 3.58 (m, 2H), 3.41 - 3.36 (m, 1H), 3.07 (d, J = 4.9 Hz, 2H), 2.75 - 2.63 (m, 1H), 2.18 (s, 6H), 1.95 (s, 3H), 1.05 (d, J = 6.5 Hz, 3H), 0.94 (d, J = 7.0 Hz, 3H). ES-API:[M+H]⁺ = 632.2; and Z237-2(45 mg, peak 2, retention time 14.48 min, purity: 100%, de value:99.68%). ¹H NMR (500 MHz, DMSO-d6) δ 8.35 (d, J = 5 Hz, 1H), 7.51 - 7.44 (m, 1H), 7.31 - 7.20 (m, 2H), 7.18 - 7.12 (m, 2H), 6.74 - 6.58 (m, 2H), 4.98 - 4.80 (m, 2H), 4.63 - 4.51 (m, 1H), 4.50 - 4.33 (m, 1H), 4.32 - 4.24 (m, 1H), 4.14 - 3.97 (m, 1H), 3.84 - 3.56 (m, 2H), 3.41 - 3.36 (m, 1H), 3.08 (d, J = 4.6 Hz, 2H), 2.84 - 2.69 (m, 1H), 2.18 (s, 6H), 1.93 (s, 3H), 1.06 (d, J = 6.7 Hz, 3H), 0.93 (d, J = 6.7 Hz, 3H).ES-API:[M+H]⁺ = 632.2.

### Test Example 1. Cell Proliferation Inhibition Experiment

NCI-H358 was a human non-small cell lung cancer cell line with Kras G12C mutation, cultured in 10% FBS RPMI-1640 medium; A549 was a human lung adenocarcinoma cell line with Kras G12S mutation, cultured in 10% FBS F-12K medium. The cells in the logarithmic growth phase was taken, digested with trypsin EDTA, collected and counted, and H358 was adjusted to 1.8E4 cells/ml using 2% FBS RPMI-1640 medium, and A549 was adjusted to 8.9E3 cells/ml using 2% FBS F-12K medium; 800 (45µl) H358 or 400 (45µl) A549 cells were seeded in a 384-well sphere plate respectively, cultured overnight to establish a 3D cell model. DMSO was used to prepare 1000X compound 3.16-fold gradient concentration stock solution, and 2% FBS medium was used to dilute 100-fold to 10X compound stock solution. On the second day after cell seeding, 5µl 10X compound stock solution was added to each cell culture well, the final concentration was 1X, and DMSO content was 0.1%. DMSO was used as the experimental control (control) and 2% FBS medium as the blank control (blank). After the compound and cells were added and cultured for 5 days, 25µl CellTiter-Glo working solution was added to each well, mixed at 400rpm and incubated for 30 minutes. After 30 minutes at room temperature, 40µl of the mixture was transfered to a 384-well plate with a non-transparent white bottom, the luminescence chemiluminescence value was read, and the cells proliferation inhibition rate IR (%) = (RLU control-RLU compound) / (RLU control-RLU blank) × 100% was calculated, Prism 6 four-parameter method was used to fit the compound's gradient dilution concentration and the corresponding cell proliferation inhibition rate, and the IC₅₀ value was calculate.

It can be seen from the results that the exemplary compounds of the present invention had high inhibitory activity against Kras G12C mutant NCI-H358 cells, with IC₅₀ of less than 500 nM, or less than 100 nM, or less than 10 nM, while their inhibitory activity against A549 cells was low, with both IC₅₀ more than 1000 nM, having obvious selective inhibitory activity.

**Table 1 Inhibitory activity of compounds on H358 and A549 cells**

| Compound No. | H358 IC₅₀(µM) | A549 IC₅₀(µM) | Compound No. | H358 IC₅₀(µM) | A549 IC₅₀(µM) |
|---|---|---|---|---|---|
| Z1 | 0.002 | 7.448 | Z68 | 0.003 | >10 |
| Z15 | 0.036 | >10 | Z69 | 0.095 | >10 |
| Z15-1 | 0.017 | 22.189 | Z70 | 0.042 | >10 |
| Z15-2 | 0.149 | 29.763 | Z72 | 0.005 | >10 |
| Z16 | 0.041 | >10 | Z73 | 0.002 | >10 |
| Z17 | 0.063 | 22.931 | Z73-1 | 0.009 | >10 |
| Z17-2 | 0.037 | 12.833 | Z73-2 | 0.002 | >10 |
| Z18 | 0.107 | 12.414 | Z74 | 0.016 | >10 |
| Z19 | 0.068 | 46.373 | Z75 | 0.002 | >10 |
| Z19-1 | 0.321 | 6.986 | Z76-1 | 0.040 | >10 |
| Z19-2 | 0.025 | 12.027 | Z76-2 | 0.001 | >10 |
| Z20 | 0.005 | 3.760 | Z77-1 | 0.026 | >10 |
| Z20-1 | 0.010 | 12.213 | Z77-2 | 0.001 | >10 |
| Z20-2 | 0.046 | 5.952 | Z78 | 0.254 | >10 |
| Z20-3 | 0.0006 | >10 | Z79 | 0.004 | >10 |
| Z20-4 | 0.026 | 2.670 | Z80 | 0.003 | >10 |
| Z21 | 0.283 | >10 | Z81 | 0.204 | >10 |
| Z22 | 0.118 | >10 | Z82-1 | 0.023 | 3.565 |
| Z23 | 0.002 | >10 | Z82-2 | 0.002 | 8.298 |
| Z23-1 | 0.001 | >10 | Z83-1 | 0.001 | 12.081 |
| Z23-2 | 0.021 | >10 | Z83-2 | 0.015 | 9.771 |
| Z24 | 0.008 | 10.437 | Z84 | 0.013 | >10 |
| Z24-1 | 0.016 | >10 | Z85 | 0.003 | 18.709 |
| Z24-2 | 0.003 | >10 | Z86 | 0.013 | >10 |
| Z25 | 0.094 | 24.199 | Z87-1 | 0.047 | 10.222 |
| Z25-1 | 0.046 | >10 | Z87-2 | 0.001 | 5.555 |
| Z25-2 | 0.281 | >10 | Z88 | 0.004 | >10 |
| Z26-1 | 0.031 | 13.826 | Z88-1 | 0.100 | >10 |
| Z26-2 | 0.001 | >10 | Z88-2 | 0.003 | >10 |
| Z26-2-2 | 0.002 | >10 | Z90 | 0.004 | 7.775 |
| Z28 | 0.184 | >10 | Z91 | 0.002 | 4.222 |
| Z29 | 0.007 | 12.572 | Z91-1 | 0.030 | 10.147 |
| Z29-1 | 0.003 | >10 | Z91-2 | 0.003 | >30 |
| Z29-2 | 0.005 | >10 | Z92 | 0.003 | >10 |
| Z29-3 | 0.165 | >10 | Z93 | 0.001 | 12.777 |
| Z29-4 | 0.044 | >10 | Z94 | 0.002 | >10 |
| Z30-1 | 0.065 | 25.009 | Z95-1 | 0.052 | 11.283 |
| Z30-2-2 | 0.003 | >10 | Z95-2 | 0.001 | >10 |
| Z30-2 | 0.002 | 20.726 | Z96-1 | 0.059 | >10 |
| Z31 | 0.047 | 12.311 | Z96-2 | 0.002 | >10 |
| Z32 | 0.308 | 23.532 | Z97 | 0.001 | 26.840 |
| Z35 | 0.164 | >10 | Z98 | 0.001 | >10 |
| Z36-2 | 0.068 | 14.393 | Z99 | 0.002 | >10 |
| Z37-2 | 0.276 | 29.490 | Z100-1 | 0.002 | 19.664 |
| Z41 | 0.318 | >10 | Z100-2 | 0.031 | >10 |
| Z45-1 | 0.003 | >10 | Z101-1 | 0.042 | >10 |
| Z45-4 | 0.065 | >10 | Z101-2 | 0.003 | >10 |
| Z46 | 0.005 | >10 | Z102-1 | 0.004 | 13.252 |
| Z46-1 | 0.089 | 8.365 | Z102-2 | 0.027 | 12.181 |
| Z46-2 | 0.003 | 5.476 | Z103 | 0.007 | >30 |
| Z47 | 0.003 | >10 | Z104-1 | 0.008 | 11.257 |
| Z47-1 | 0.001 | >10 | Z104-2 | 0.028 | >10 |
| Z47-2 | 0.042 | >10 | Z105-1 | 0.038 | >30 |
| Z48 | 0.022 | >10 | Z105-2 | 0.005 | >30 |
| Z49 | 0.012 | >10 | Z106 | 0.004 | >30 |
| Z49-1 | 0.006 | >10 | Z107-1 | 0.019 | >30 |
| Z49-2 | 0.113 | >10 | Z107-2 | 0.007 | >30 |
| Z50 | 0.050 | >10 | Z108 | 0.009 | >30 |
| Z52 | 0.004 | >10 | Z109-1 | 0.005 | 8.479 |
| Z53 | 0.004 | >10 | Z109-2 | 0.106 | 9.775 |
| Z54 | 0.066 | >10 | Z110 | 0.004 | 14.689 |
| Z55 | 0.009 | >10 | Z111 | 0.004 | >30 |
| Z56 | 0.011 | >10 | Z111-1 | 0.034 | >30 |
| Z57 | 0.005 | >10 | Z111-2 | 0.003 | >30 |
| Z57-1 | 0.111 | >10 | Z112-1 | 0.003 | >30 |
| Z57-2 | 0.003 | >10 | Z112-2 | 0.094 | >30 |
| Z58 | 0.001 | >10 | Z114 | 0.008 | 4.629 |
| Z59 | 0.006 | >10 | Z115 | 0.005 | 18.748 |
| Z60 | 0.007 | >10 | Z116 | 0.003 | 8.891 |
| Z61 | 0.001 | >10 | Z118 | 0.019 | >30 |
| Z62 | 0.006 | >10 | Z119 | 0.012 | 7.903 |
| Z64 | 0.007 | >10 | Z120 | 0.017 | 27.662 |
| Z65 | 0.009 | >10 | Z121 | 0.003 | >10 |
| Z66 | 0.003 | >10 | Z122 | 0.007 | >30 |
| Z67 | 0.003 | >10 | Z237-2 | 0.047 | >30 |

It can be seen from Table 1 that the exemplary compounds of the present invention had higher inhibitory activity against Kras G12C mutant NCI-H358 cells, but had lower inhibitory activity against A549 cells, and had obvious selective inhibitory activity.

### Test Example 2 Cell p-ERK detection experiment

MIA PaCa2 was a human pancreatic cancer cell line with Kras G12C mutation, cultured in 10% FBS + 2.5% Horse serum DMEM medium. The cells in the logarithmic growth phase were taken, digested with enzyme EDTA, collected, counted and 2.5E4 cells were seeded in a 96-well cell culture plate, cultured overnight. DMSO was used to prepare 1000X compound 3.16-fold gradient concentration stock solution, and medium was used to dilute 200-fold to 5X compound stock solution. On the second day after cell seeding, 5X compound stock solution was added to each cell culture well, the final concentration was 1X, and DMSO content was 0.1%. DMSO was used as an experimental control (control). After the compound was added and cultured for two hours, the remaining medium was removed. 50ul cell lysis buffer was added to each well, mixed and incubated for 30 minutes, then 16ul of the mixture was transfered to a 96-well plate with a non-transparent white bottom, and 16ul cell lysis buffer was added to blank wells. After the completion of the transfer, 4ul p-ERK HTRF antibody mixture was added to each well, incubated for 4 hours and the fluorescence value was read. The compound inhibition rate IR (%) = (RLU control-RLU compound) / (RLU control-RLU blank) × 100% was calculated, Prism 8 four-parameter method was used to fit the compound's gradient dilution concentration and the corresponding cell proliferation inhibition rate, and the IC₅₀ value was calculate. It can be seen from the results that the exemplary compounds of the present invention had a good inhibitory activity on the level of phosphorylated ERK downstream of the cell pathway with Kras G12C protein mutation, with IC₅₀ lower than 10 µM; or lower than 1000 nM, or lower than 500 nM, or lower than 100nM. The results of the example compounds were shown in Table 2 below.

**Table 2 Inhibitory activity of compounds on p-ERK**

| Compound No. | p-ERK IC50(µM) | Compound No. | p-ERK IC50(µM) |
|---|---|---|---|
| Z23-1 | 0.013 | Z88-1 | 0.356 |
| Z24-1 | 0.053 | Z88-2 | 0.027 |
| Z24-2 | 0.025 | Z90 | 0.019 |
| Z46-2 | 0.020 | Z91 | 0.016 |
| Z47-1 | 0.015 | Z91-1 | 0.093 |
| Z47-2 | 0.314 | Z91-2 | 0.018 |
| Z49 | 0.128 | Z92 | 0.025 |
| Z49-1 | 0.064 | Z93 | 0.009 |
| Z53 | 0.048 | Z94 | 0.007 |
| Z57-2 | 0.027 | Z95-1 | 0.248 |
| Z58 | 0.014 | Z95-2 | 0.005 |
| Z60 | 0.047 | Z96-1 | 0.195 |
| Z61 | 0.050 | Z96-2 | 0.007 |
| Z65 | 0.182 | Z97 | 0.007 |
| Z66 | 0.039 | Z99 | 0.012 |
| Z67 | 0.021 | Z104-1 | 0.014 |
| Z72 | 0.063 | Z104-2 | 0.185 |
| Z73 | 0.022 | Z105-1 | 0.395 |
| Z73-1 | 0.116 | Z105-2 | 0.020 |
| Z73-2 | 0.020 | Z107-1 | 0.226 |
| Z74 | 0.128 | Z107-2 | 0.035 |
| Z75 | 0.112 | Z108 | 0.049 |
| Z76-1 | 0.109 | Z109-1 | 0.018 |
| Z76-2 | 0.008 | Z110 | 0.043 |
| Z77-1 | 0.058 | Z111 | 0.047 |
| Z77-2 | 0.008 | Z111-1 | 0.186 |
| Z79 | 0.045 | Z111-2 | 0.034 |
| Z80 | 0.019 | Z112-1 | 0.024 |
| Z82-1 | 0.407 | Z112-2 | 0.502 |
| Z82-2 | 0.023 | Z114 | 0.039 |
| Z83-1 | 0.009 | Z115 | 0.031 |
| Z83-2 | 0.067 | Z116 | 0.026 |
| Z84 | 0.105 | Z118 | 0.166 |
| Z85 | 0.054 | Z119 | 0.059 |
| Z86 | 0.101 | Z120 | 0.193 |
| Z87-1 | 0.158 | Z121 | 0.016 |
| Z87-2 | 0.009 | Z122 | 0.101 |
| Z88 | 0.086 | | |

### Test Example 3 Cell Proliferation Inhibition Experiment

MIA PaCa2 was a human pancreatic cancer cell line with Kras G12C mutation, cultured in 10% FBS + 2.5% Horse serum DMEM medium; A549 was a human lung adenocarcinoma cell line with Kras G12S mutation, cultured in 10% FBS F-12K medium. The cells in the logarithmic growth phase were taken, digested with enzyme EDTA, collected, counted and 200 MIA PaCa-2 or 400 A549 cells were seeded in a 384-well sphere plate respectively, cultured overnight to establish a 3D cell model. DMSO was used to prepare 1000X compound 3.16-fold gradient concentration stock solution, and medium was used to dilute 100-fold to 10X compound stock solution. On the second day after cell seeding, 10X compound stock solution was added to each cell culture well, the final concentration was IX, and DMSO content was 0.1%. DMSO was used as an experimental control (control), medium as the blank control (blank). After the compound and cells were added and cultured for 5 days, 30µl CellTiter-Glo working solution was added to each well, mixed and incubated for 30 minutes. After 30 minutes at room temperature, 40µl of the mixture was transfered to a 384-well plate with a non-transparent white bottom, the luminescence chemiluminescence value was read, and the cells proliferation inhibition rate IR (%) = (RLU control-RLU compound) / (RLU control-RLU blank) × 100% was calculated, XLFit four-parameter method was used to fit the compound's gradient dilution concentration and the corresponding cell proliferation inhibition rate, and the IC₅₀ value was calculate. It can be seen from the results that the exemplary compounds of the present invention had high inhibitory activity against Kras G12C mutant MIA PaCa-2 cells, with IC₅₀ of less than 1000 nM, or less than 500 nM, or less than 100 nM, or less than 10 nM. The results of the example compounds were shown in Table 3 below.

**Table 3 Inhibitory activity of compounds on MIA-PaCa2**

| Compound No. | MIA-PaCa2 IC50(µM) | Compound No. | MIA-PaCa2 IC50(µM) |
|---|---|---|---|
| Z15-1 | 0.020 | Z67 | 0.006 |
| Z15-2 | 0.261 | Z68 | 0.009 |
| Z16 | 0.029 | Z73 | 0.003 |
| Z17 | 0.055 | Z73-2 | 0.005 |
| Z17-2 | 0.033 | Z76-2 | 0.002 |
| Z19-2 | 0.024 | Z77-2 | 0.002 |
| Z20 | 0.015 | Z79 | 0.006 |
| Z20-1 | 0.017 | Z84 | 0.022 |
| Z20-2 | 0.173 | Z85 | 0.006 |
| Z20-3 | 0.0005 | Z86 | 0.024 |
| Z20-4 | 0.051 | Z87-2 | 0.002 |
| Z23-1 | 0.003 | Z91-2 | 0.015 |
| Z24 | 0.009 | Z92 | 0.002 |
| Z24-2 | 0.008 | Z93 | 0.002 |
| Z26-1 | 0.074 | Z94 | 0.002 |
| Z26-2 | 0.004 | Z95-2 | 0.001 |
| Z26-2-2 | 0.003 | Z96-2 | 0.002 |
| Z29 | 0.014 | Z97 | 0.001 |
| Z29-1 | 0.008 | Z98 | 0.002 |
| Z29-2 | 0.006 | Z99 | 0.003 |
| Z29-4 | 0.358 | Z100-1 | 0.004 |
| Z30-1 | 0.078 | Z101-1 | 0.056 |
| Z30-2-2 | 0.004 | Z101-2 | 0.003 |
| Z30-2 | 0.005 | Z102-1 | 0.004 |
| Z31 | 0.025 | Z102-2 | 0.083 |
| Z36-2 | 0.121 | Z103 | 0.018 |
| Z39 | 0.444 | Z104-1 | 0.012 |
| Z41 | 0.344 | Z105-2 | 0.011 |
| Z46 | 0.020 | Z106 | 0.007 |
| Z46-2 | 0.008 | Z107-2 | 0.019 |
| Z47 | 0.009 | Z108 | 0.017 |
| Z47-1 | 0.003 | Z109-1 | 0.012 |
| Z49 | 0.022 | Z110 | 0.011 |
| Z49-1 | 0.010 | Z111 | 0.007 |
| Z52 | 0.023 | Z111-2 | 0.013 |
| Z53 | 0.010 | Z112-1 | 0.004 |
| Z55 | 0.026 | Z112-2 | 0.126 |
| Z57 | 0.022 | Z114 | 0.074 |
| Z58 | 0.003 | Z115 | 0.021 |
| Z59 | 0.026 | Z116 | 0.026 |
| Z60 | 0.019 | Z119 | 0.113 |
| Z61 | 0.008 | Z120 | 0.085 |
| Z64 | 0.009 | Z121 | 0.005 |
| Z65 | 0.034 | Z122 | 0.011 |
| Z66 | 0.007 | Z237-2 | 0.048 |

All documents mentioned in the present invention are cited as references in this application, as if each document is individually cited as a reference. In addition, it should be understood that after reading the above teaching content of the present invention, those skilled in the art may make various changes or modifications to the present invention, and these equivalent forms also fall within the scope defined by the appended claims of the present application.

## Claims

1. A compound or a pharmaceutically acceptable salt, stereoisomer, solvate or prodrug thereof, the compound has a structure as represented by formula (I), 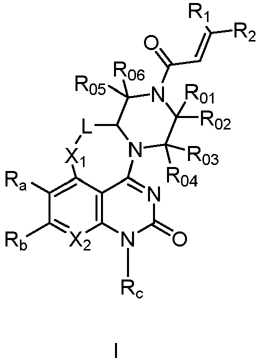 wherein,
R₁, R₂ are each independently hydrogen, cyano, C₁₋₃ alkyl, or -C₁₋₃ alkyl-NR^{a}R^{b};
R₀₁, R₀₂, R₀₃, R₀₄, R₀₅, R₀₆ are each independently hydrogen, C₁₋₆ alkyl, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
or R₀₁, R₀₂ together with the carbon atom attached thereto form C₃₋₆ monocyclic cycloalkyl;
or R₀₃, R₀₄ together with the carbon atom attached thereto form C₃₋₆ monocyclic cycloalkyl;
or R₀₅, R₀₆ together with the carbon atom attached thereto form C₃₋₆ monocyclic cycloalkyl;
L is a bond, (CR_{L1}R_{L2})ₙ, C(O), C(O)C(R_{L1}R_{L2}), or C(R_{L1}R_{L2})C(O); wherein R_{L1}, R_{L2} are each independently hydrogen, halo, or C₁₋₆ alkyl;
n is 1 or 2;
X₁ is NRₓ₁, O, or CRₓ₂Rₓ₃; wherein Rₓ₁ is hydrogen, or C₁₋₆ alkyl; Rₓ₂, Rₓ₃ are each independently hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{g}R^{h}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
X₂ is N, or CRₓ₄; wherein Rₓ₄ is hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{g}R^{h}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
Rₐ is hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{c}R^{d}, C₂₋₄ alkenyl, C₂₋₄ alkynyl, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
R_{b} is C₆₋₁₀ aryl, or C₅₋₁₀ heteroaryl; the C₆₋₁₀ aryl, C₅₋₁₀ heteroaryl are unsubstituted or substituted by 1, 2, 3, or 4 substituent(s) independently selected from the group S1, the substituents of the group S1 are halo, cyano, nitro, hydroxy, C₁₋₆ alkyl, C₁₋₆ alkoxy, halo C₁₋₆ alkyl, halo C₁₋₆ alkoxy, C₃₋₆ monocyclic cycloalkyl, NRⁱR^{j}, C(O)NR^{e}R^{f}, -SO₂C₁₋₃ alkyl, -SO₂halo C₁₋₃ alkyl, -SO₂NR^{e}R^{f}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-NR^{e}R^{f}, -C₁₋₄ alkyl-C(O)NR^{e}R^{f}, -C₁₋₄ alkyl-SO₂C₁₋₃ alkyl, or C₂₋₄ alkynyl;
R_{c} is C₁₋₆ alkyl, C₆₋₁₀ aryl, C₅₋₁₀ heteroaryl, C₃₋₆ monocyclic cycloalkyl, C₃₋₆ monocyclic heterocyclyl, 7- to 11-membered spirocycloalkyl, -C₁₋₄ alkyl-C₆₋₁₀ aryl, -C₁₋₄ alkyl-C₅₋₁₀ heteroaryl, -NR^{e}-C₆₋₁₀ aryl, -O-C₆₋₁₀ aryl, -C₁₋₄ alkyl-C₃₋₆ monocyclic heterocyclyl, -C₁₋₄ alkyl-C₃₋₆ monocyclic cycloalkyl; wherein
the C₃₋₆ monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione;
the C₃₋₆ monocyclic heterocyclyl is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one;
the -C₁₋₄ alkyl- is unsubstituted or substituted by 1, 2, 3, or 4 substituent(s) independently selected from C₁₋₃ alkyl;
the C₁₋₆ alkyl, C₆₋₁₀ aryl, C₅₋₁₀ heteroaryl, 7- to 11-membered spirocycloalkyl, C₃₋₆ monocyclic cycloalkyl, C₃₋₆ monocyclic heterocyclyl are unsubstituted or substituted by 1, 2, 3, or 4 substituent(s) independently selected from the group S2, the substituents of the group S2 are halo, cyano, hydroxy, C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic heterocyclyl, NRⁱR^{j}, C(O)NR^{e}R^{f}, -SO₂C₁₋₃ alkyl, -SO₂halo C₁₋₃ alkyl, -SO₂NR^{e}R^{f}, -C₁₋₄ alkyl-hydroxy, -C₁₋₄ alkyl-C₂₋₄ alkynyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-C₃₋₆ monocyclic cycloalkyl, -C₁₋₄ alkyl-NR^{e}R^{f}, -C₁₋₄ alkyl-C(O)NR^{e}R^{f}, -C₁₋₄ alkyl-SO₂C₁₋₃ alkyl, or C₂₋₄ alkynyl; wherein the C₃₋₆ monocyclic cycloalkyl in the substituents of the group S2 is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; and the C₁₋₆ alkyl, C₁₋₆ alkoxy, -C₁₋₄ alkyl-, C₃₋₆ monocyclic cycloalkyl, C₃₋₆ monocyclic heterocyclyl in the substituents of the group S2 are optionally substituted by 1, 2, or 3 substituent(s) independently selected from the group consisting of halo, methyl, ethyl, propyl, isopropyl, trifluoromethyl, amino, N(CH₃)₂, hydroxy, carboxyl; wherein the C₃₋₆ monocyclic cycloalkyl is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1 -dioxide, tetrahydropyran;
R^{a}, R^{b}, R^{c}, R^{d}, R^{e}, R^{f}, R^{g}, R^{h} are each independently hydrogen, or C₁₋₃ alkyl;
Rⁱ, R^{j} are each independently hydrogen, C₁₋₃ alkyl, -C(O)C₁₋₃ alkyl, -CO₂C₁₋₃ alkyl.

2. The compound or a pharmaceutically acceptable salt, stereoisomer, solvate or prodrug thereof according to claim 1, **characterized in that**, R₁, R₂ are each independently hydrogen, cyano, C₁₋₃ alkyl, -CH₂NH₂, -CH₂NHCH₃, or -CH₂N(CH₃)₂.

3. The compound or a pharmaceutically acceptable salt, stereoisomer, solvate or prodrug thereof according to claim 1, **characterized in that**, R₀₁, R₀₂, R₀₃, R₀₄, R₀₅, R₀₆ are each independently hydrogen, C₁₋₃ alkyl, -C₁₋₂ alkyl-hydroxy, -C₁₋₂ alkyl-cyano, -C₁₋₂ alkyl-C₁₋₃ alkoxy, -C₁₋₂ alkyl-halo C₁₋₃ alkyl, -C₁₋₂ alkyl-halo C₁₋₃ alkoxy;
or R₀₁, R₀₂ together with the carbon atom attached thereto form C₃₋₆ monocyclic cycloalkyl;
or R₀₃, R₀₄ together with the carbon atom attached thereto form C₃₋₆ monocyclic cycloalkyl;
or R₀₅, R₀₆ together with the carbon atom attached thereto form C₃₋₆ monocyclic cycloalkyl.

4. The compound or a pharmaceutically acceptable salt, stereoisomer, solvate or prodrug thereof according to claim 1, **characterized in that**, R₀₂, R₀₄ are each independently hydrogen, CH₃, -CH₂-hydroxy, or -CH₂-cyano; R₀₁, R₀₃, R₀₅, R₀₆ are hydrogen.

5. The compound or a pharmaceutically acceptable salt, stereoisomer, solvate or prodrug thereof according to claim 1, **characterized in that**, L is a bond, or (CR_{L1}R_{L2})ₙ; wherein R_{L1}, R_{L2} are each independently hydrogen, halo, or C₁₋₆ alkyl; n is 1 or 2.

6. The compound or a pharmaceutically acceptable salt, stereoisomer, solvate or prodrug thereof according to claim 1, **characterized in that**, X₁ is NRₓ₁ or O; wherein Rₓ₁ is hydrogen, or C₁₋₆ alkyl.

7. The compound or a pharmaceutically acceptable salt, stereoisomer, solvate or prodrug thereof according to claim 1, **characterized in that**, X₂ is N or CRₓ₄; wherein Rₓ₄ is hydrogen, halo, C₁₋₆ alkyl, C₁₋₆ alkoxy, or halo C₁₋₆ alkyl.

8. The compound or a pharmaceutically acceptable salt, stereoisomer, solvate or prodrug thereof according to claim 1, **characterized in that**, Rₐ is hydrogen, halo, cyano, C₁₋₄ alkyl, C₁₋₄ alkoxy, halo C₁₋₃ alkyl, halo C₁₋₃ alkoxy, C₃₋₆ monocyclic cycloalkyl, NR^{c}R^{d}, C₂₋₄ alkenyl, C₂₋₄ alkynyl, -C₁₋₂ alkyl-hydroxy, -C₁₋₂ alkyl-cyano, -C₁₋₂ alkyl-C₁₋₃ alkoxy, -C₁₋₂ alkyl-halo C₁₋₃ alkyl, or -C₁₋₂ alkyl-halo C₁₋₃ alkoxy; wherein R^{c}, R^{d} are each independently hydrogen, or C₁₋₃ alkyl.

9. The compound or a pharmaceutically acceptable salt, stereoisomer, solvate or prodrug thereof according to claim 1, **characterized in that**, the 7- to 11-membered spirocycloalkyl in R_{c} is a monospirocycloalkyl containing one spiro atom formed by any two monocyclic cycloalkyl rings selected from cyclopropyl ring, cyclobutyl ring, cyclopentyl ring, and cyclohexyl ring.

10. The compound or a pharmaceutically acceptable salt, stereoisomer, solvate or prodrug thereof according to claim 1, **characterized in that**, the C₆₋₁₀ aryl in R_{b}, R_{c} are each independently phenyl, naphthyl, a 9- or 10-membered aromatic fused bicyclic ring formed by fusing a phenyl to one C₅₋₆ monocyclic heterocyclyl, or a 9- or 10-membered aromatic fused bicyclic ring formed by fusing a phenyl to one C₅₋₆ monocyclic cycloalkyl.

11. The compound or a pharmaceutically acceptable salt, stereoisomer, solvate or prodrug thereof according to claim 10, **characterized in that**, the C₅₋₆ monocyclic heterocyclyl in the 9- or 10-membered aromatic fused bicyclic ring formed by fusing a phenyl to one C₅₋₆ monocyclic heterocyclyl is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahyro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one.

12. The compound or a pharmaceutically acceptable salt, stereoisomer, solvate or prodrug thereof according to claim 10, **characterized in that**, the C₅₋₆ monocyclic cycloalkyl in the 9- or 10-membered aromatic fused bicyclic ring formed by fusing a phenyl to one C₅₋₆ monocyclic cycloalkyl is selected from the group consisting of: cyclopentyl, cyclopentenyl, cyclohexyl, cyclohexenyl, cyclohexdienyl, cyclopentanone, cyclopentan-1,3-dione, cyclohexanone, cyclohexan-1,3-dione.

13. The compound or a pharmaceutically acceptable salt, stereoisomer, solvate or prodrug thereof according to claim 1, **characterized in that**, the C₅₋₁₀ heteroaryl in R_{b}, R_{c} are each independently a 5- or 6-membered monoheteroaryl, a 9- or 10-membered biheteroaryl formed by fusing a phenyl to a 5- or 6-membered monoheteroaryl, a 8- to 10-membered biheteroaryl formed by fusing a 5- or 6-membered monoheteroaryl to a 5- or 6-membered monoheteroaryl, a 8- to 10-membered biheteroaryl formed by fusing a 5- or 6-membered monoheteroaryl to one C₅₋₆ monocyclic heterocyclyl, or a 8- to 10-membered biheteroaryl formed by fusing a 5- or 6-membered monoheteroaryl to one C₅₋₆ monocyclic cycloalkyl.

14. The compound or a pharmaceutically acceptable salt, stereoisomer, solvate or prodrug thereof according to claim 13, **characterized in that**, when the C₅₋₁₀ heteroaryl in R_{b}, R_{c} are 5- or 6-membered monoheteroaryl, the 5- or 6-membered monoheteroaryl are each independently selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine.

15. The compound or a pharmaceutically acceptable salt, stereoisomer, solvate or prodrug thereof according to claim 13, **characterized in that**, the 5- or 6-membered monoheteroaryl in the 9- or 10-membered biheteroaryl formed by fusing a phenyl to a 5- or 6-membered monoheteroaryl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine.

16. The compound or a pharmaceutically acceptable salt, stereoisomer, solvate or prodrug thereof according to claim 13, **characterized in that**, the 5- or 6-membered monoheteroaryl in the 8- to 10-membered biheteroaryl formed by fusing a 5- or 6-membered monoheteroaryl to a 5- or 6-membered monoheteroaryl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine.

17. The compound or a pharmaceutically acceptable salt, stereoisomer, solvate or prodrug thereof according to claim 13, **characterized in that**, the 5- or 6-membered monoheteroaryl in the 8- to 10-membered biheteroaryl formed by fusing a 5- or 6-membered monoheteroaryl to one C₅₋₆ monocyclic heterocyclyl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine;
the C₅₋₆ monocyclic heterocyclyl is selected from the group consisting of: oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahyro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one.

18. The compound or a pharmaceutically acceptable salt, stereoisomer, solvate or prodrug thereof according to claim 13, **characterized in that**, the 5- or 6-membered monoheteroaryl in the 8- to 10-membered biheteroaryl formed by fusing a 5- or 6-membered monoheteroaryl to one C₅₋₆ monocyclic cycloalkyl is selected from the group consisting of: thiophene, N-alkylcyclopyrrole, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, or pyrazine;
the C₅₋₆ monocyclic cycloalkyl is selected from the group consisting of: cyclopentyl, cyclopentenyl, cyclohexyl, cyclohexenyl, cyclohexdienyl, cyclopentanone, cyclopentan-1,3-dione, cyclohexanone, cyclohexan-1,3-dione.

19. The compound or a pharmaceutically acceptable salt, stereoisomer, solvate or prodrug thereof according to claim 10, **characterized in that**, the 9- or 10-membered aromatic fused bicyclic ring formed by fusing a phenyl to one C₅₋₆ monocyclic heterocyclyl has a structure selected from the group consisting of:

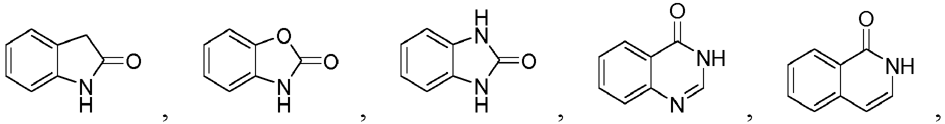

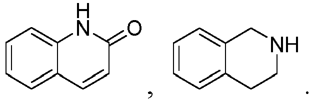

20. The compound or a pharmaceutically acceptable salt, stereoisomer, solvate or prodrug thereof according to claim 13, **characterized in that**, the 9- or 10-membered biheteroaryl formed by fusing a phenyl to a 5- or 6-membered monoheteroaryl has a structure selected from the group consisting of:

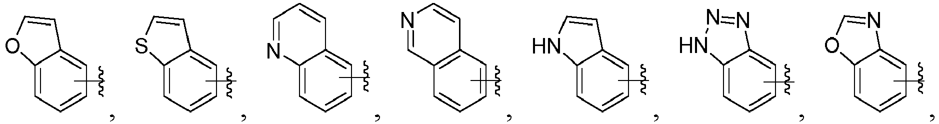

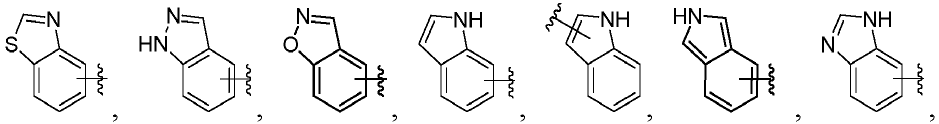

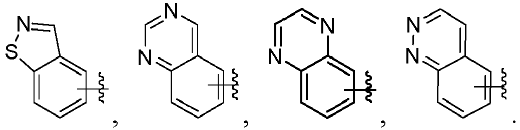

21. The compound or a pharmaceutically acceptable salt, stereoisomer, solvate or prodrug thereof according to claim 13, **characterized in that**, the 8- to 10-membered biheteroaryl formed by fusing a 5- or 6-membered monoheteroaryl to a 5- or 6-membered monoheteroaryl has a structure selected from the group consisting of:

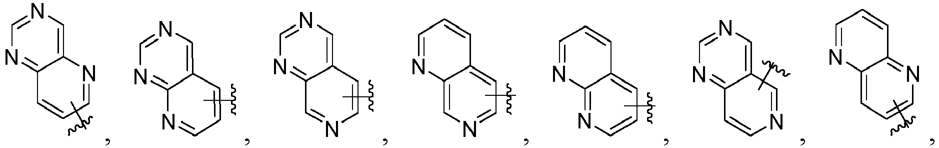

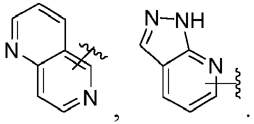

22. The compound or a pharmaceutically acceptable salt, stereoisomer, solvate or prodrug thereof according to claim 13, **characterized in that**, the 9- or 10-membered biheteroaryl formed by fusing a phenyl to a 5- or 6-membered monoheteroaryl has a structure selected from the group consisting of:

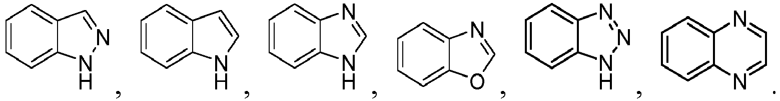

23. The compound or a pharmaceutically acceptable salt, stereoisomer, solvate or prodrug thereof according to claim 13, **characterized in that**, the 8- to 10-membered biheteroaryl formed by fusing a 5- or 6-membered monoheteroaryl to a 5- or 6-membered monoheteroaryl has a structure selected from the group consisting of:

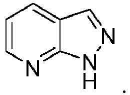

24. The compound or a pharmaceutically acceptable salt, stereoisomer, solvate or prodrug thereof according to claim 13, **characterized in that**, the 9- or 10-membered biheteroaryl formed by fusing a phenyl to a 5- or 6-membered monoheteroaryl or the 8- to 10-membered biheteroaryl formed by fusing a 5- or 6-membered monoheteroaryl to a 5- or 6-membered monoheteroaryl has a structure selected from the group consisting of:

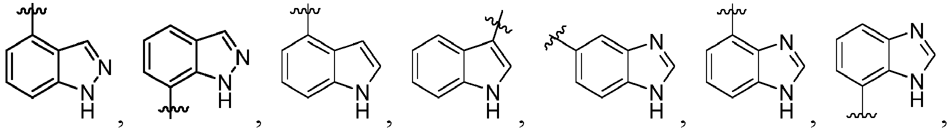

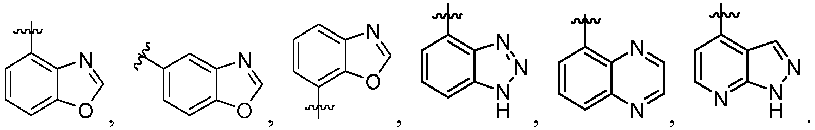

25. The compound or a pharmaceutically acceptable salt, stereoisomer, solvate or prodrug thereof according to claim 10, **characterized in that**, the 9- or 10-membered aromatic fused bicyclic ring formed by fusing a phenyl to one C₅₋₆ monocyclic heterocyclyl has a structure selected from the group consisting of:

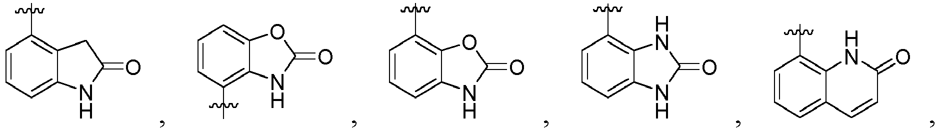

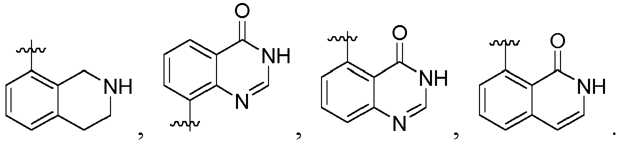

26. The compound or a pharmaceutically acceptable salt, stereoisomer, solvate or prodrug thereof according to claim 1, **characterized in that**, the R_{b} has a structure selected from the group consisting of:

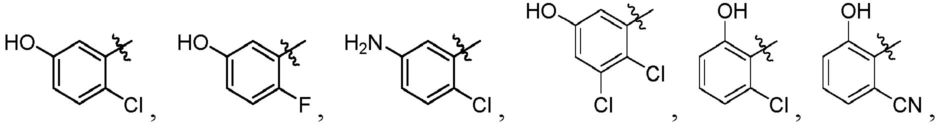

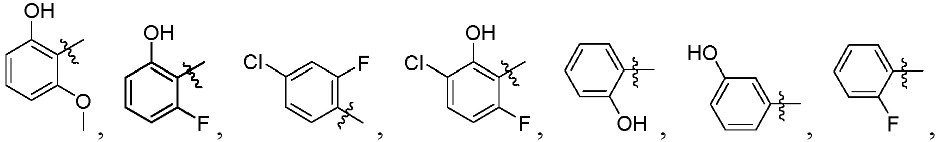

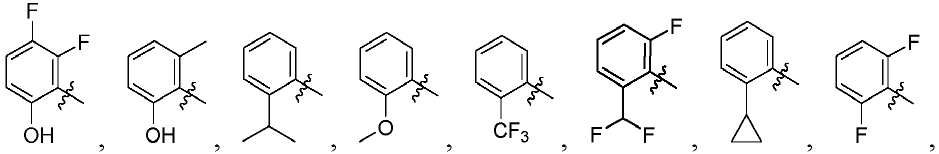

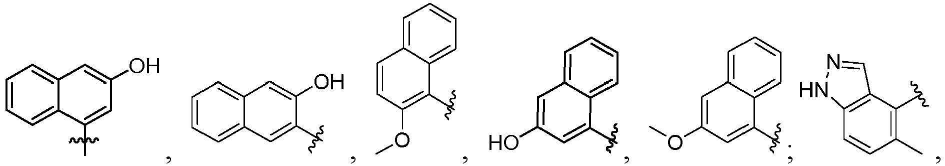

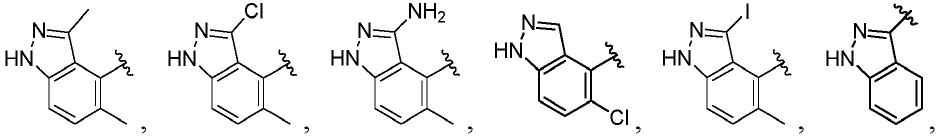

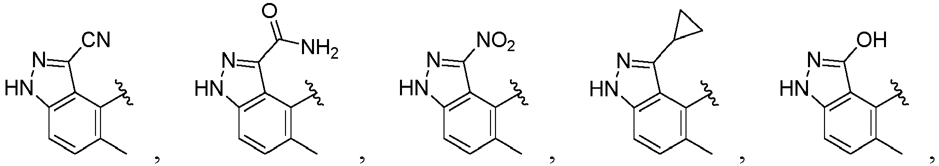

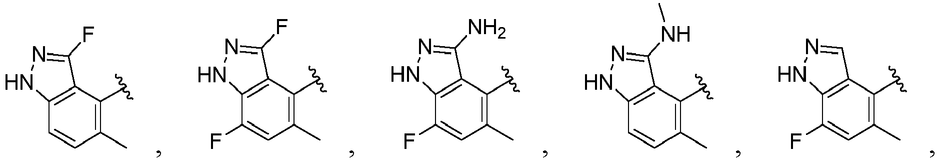

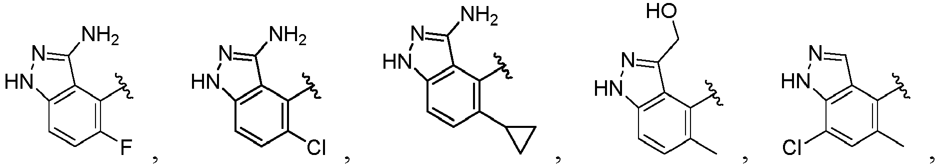

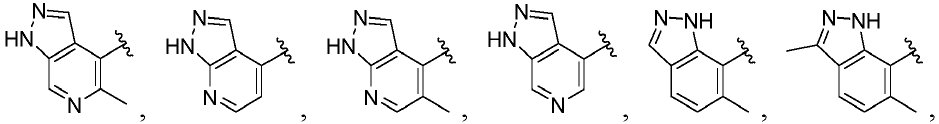

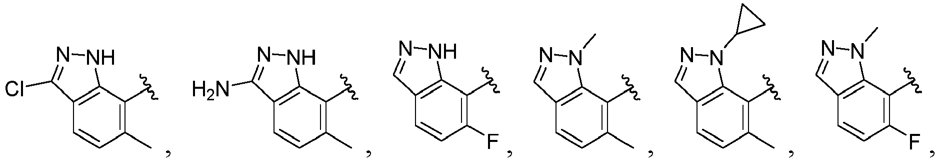

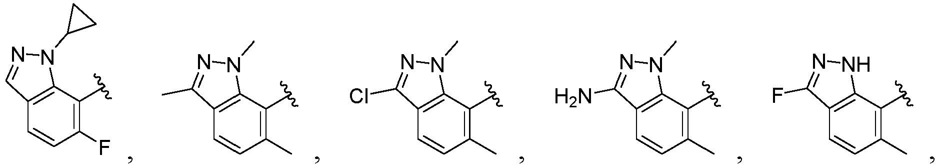

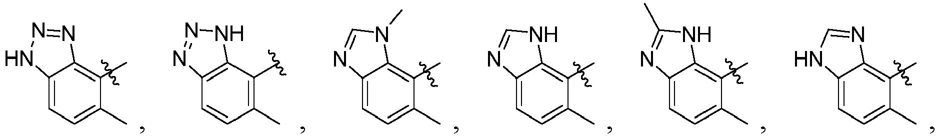

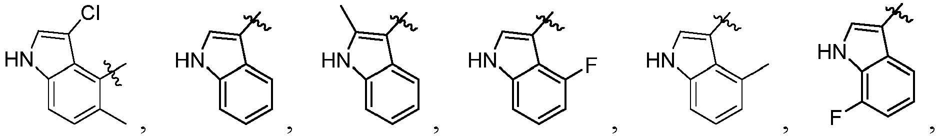

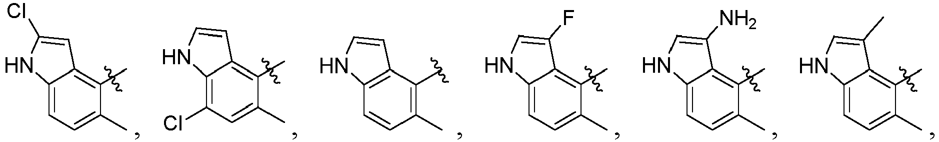

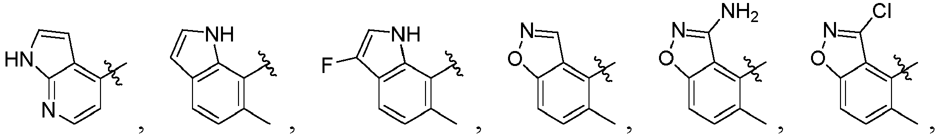

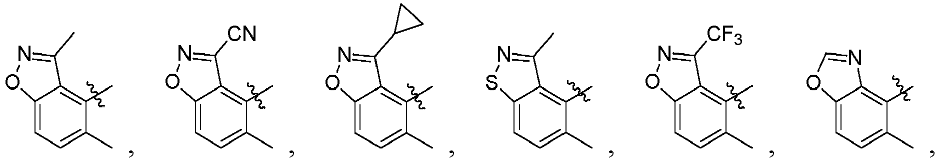

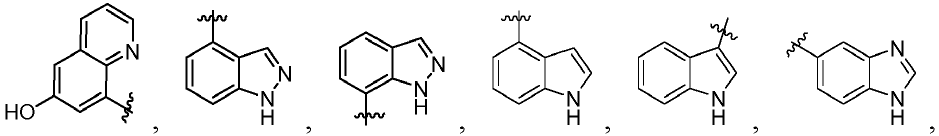

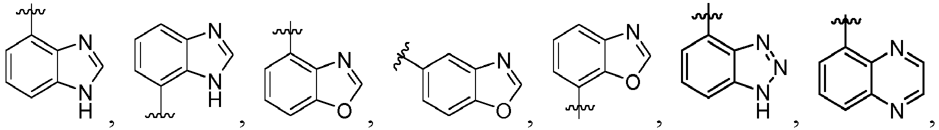

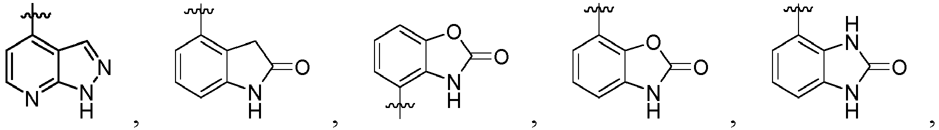

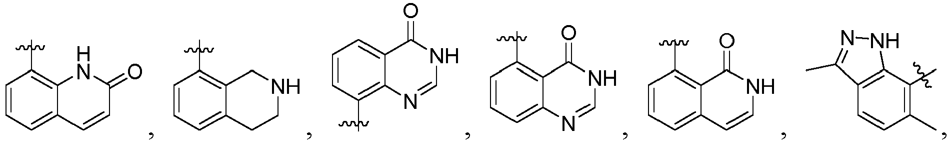

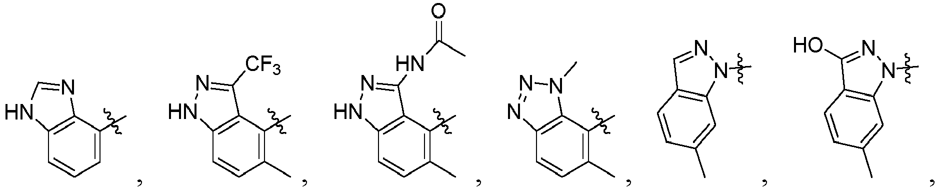

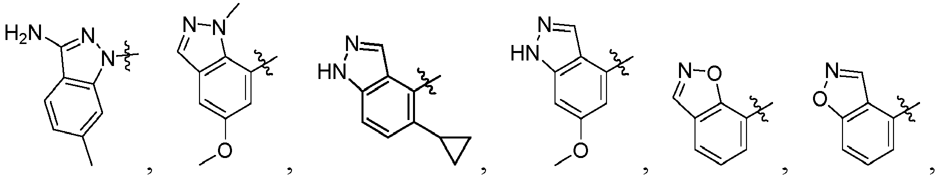

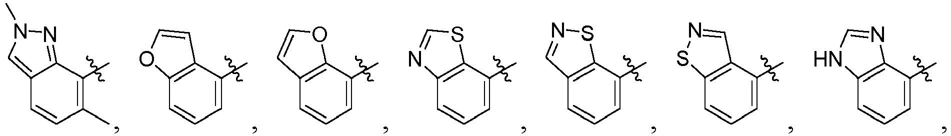

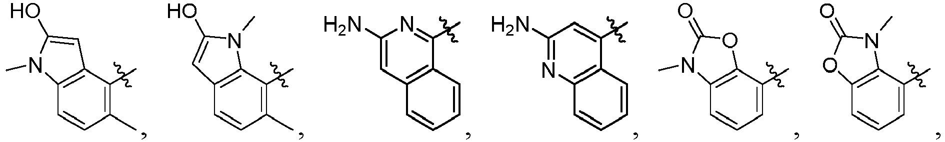

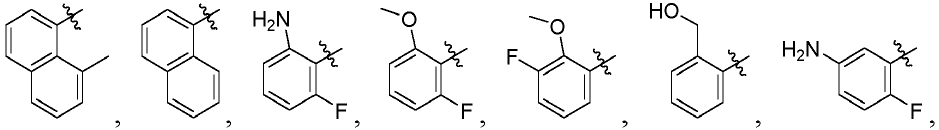

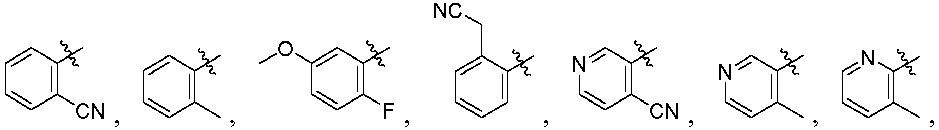

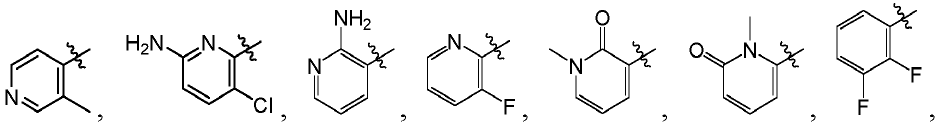

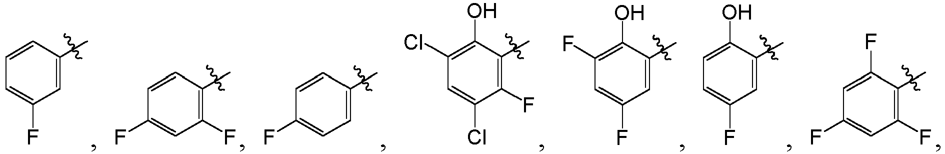

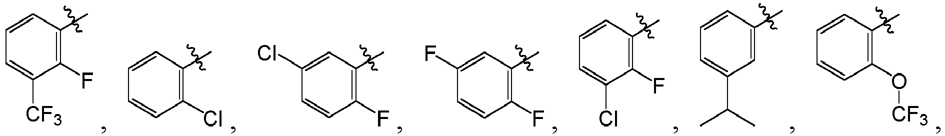

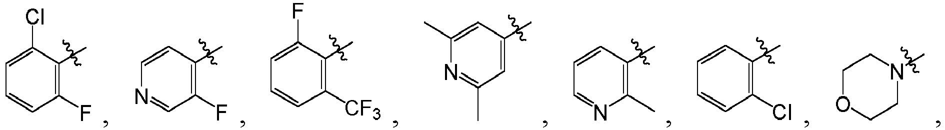

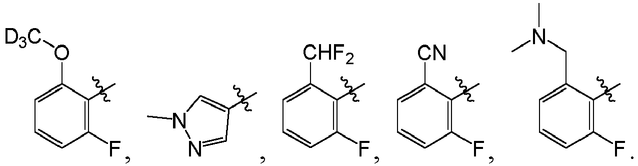

27. The compound or a pharmaceutically acceptable salt, stereoisomer, solvate or prodrug thereof according to claim 1, **characterized in that**, the R_{c} has a structure selected from the group consisting of:

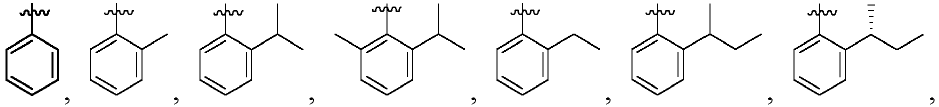

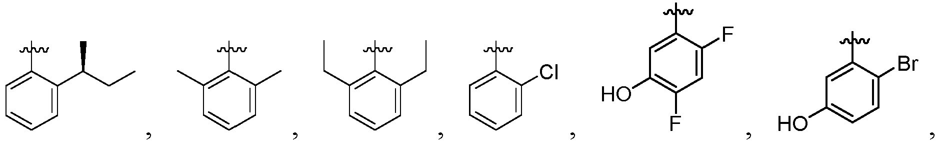

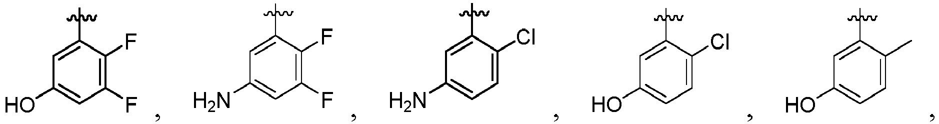

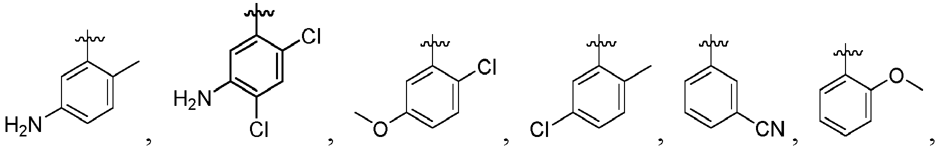

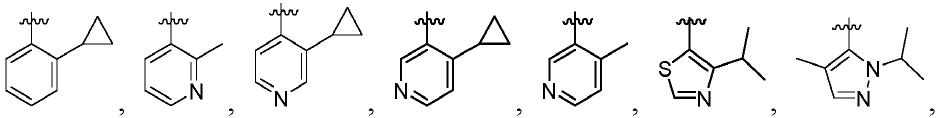

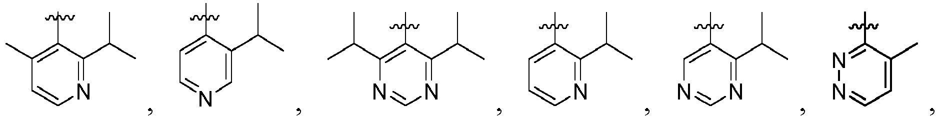

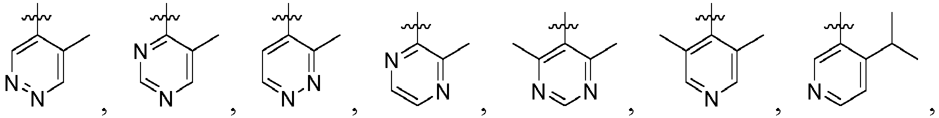

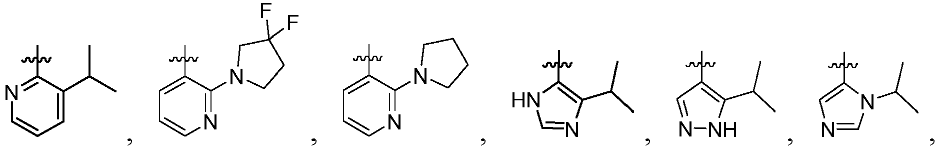

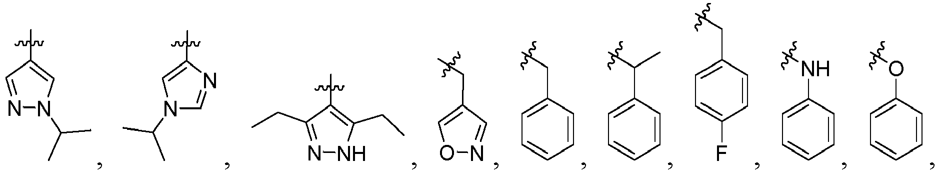

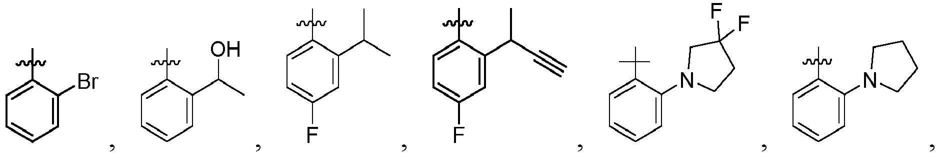

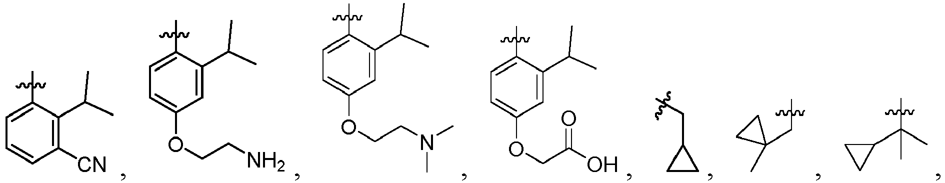

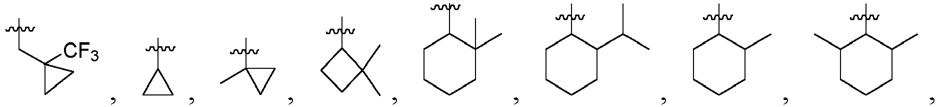

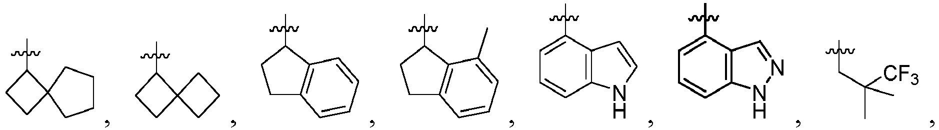

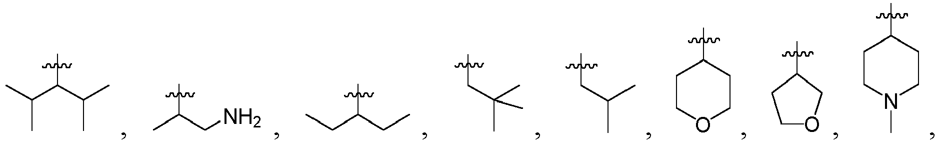

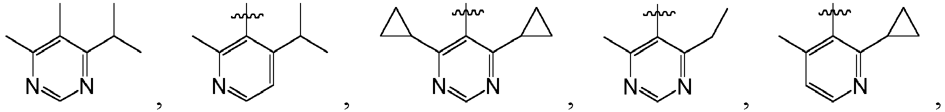

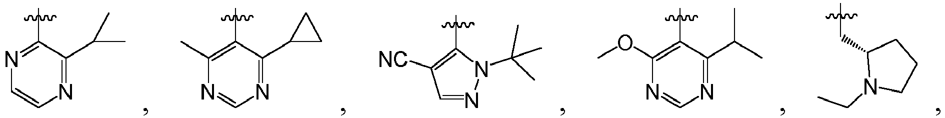

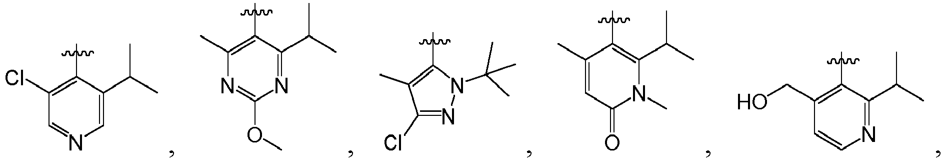

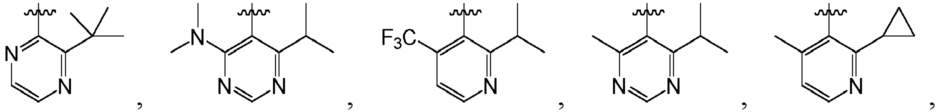

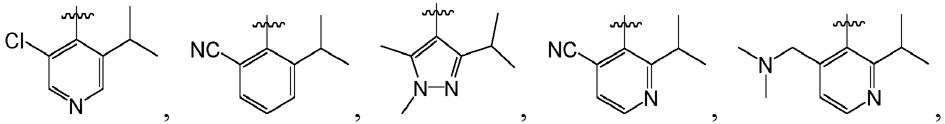

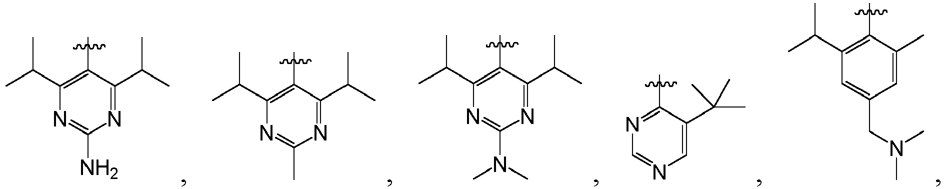

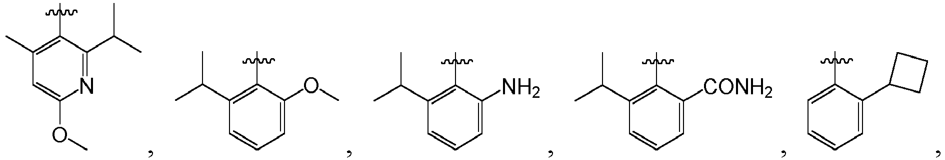

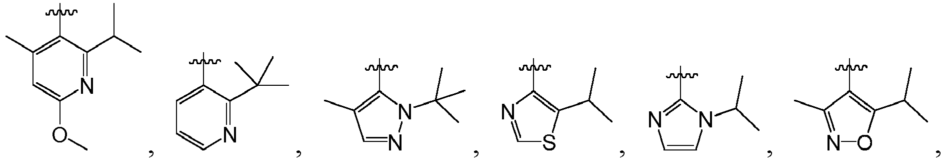

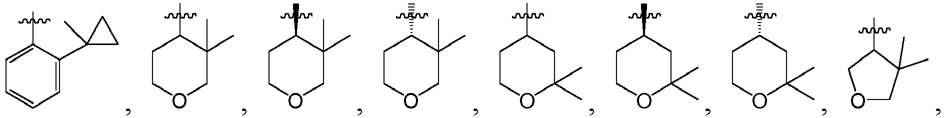

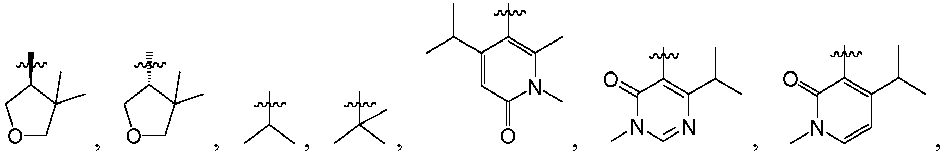

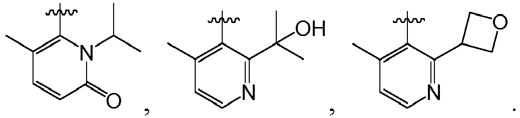

28. The compound or a pharmaceutically acceptable salt, stereoisomer, solvate or prodrug thereof according to claim 1, **characterized in that**, the compound of formula (I) is selected from each specific compound noted in the Examples.

29. The compound or a pharmaceutically acceptable salt, stereoisomer, solvate or prodrug thereof according to claim 1, **characterized in that**, the compound of formula (I) has a structure as shown in formula (IA) or formula (IB): 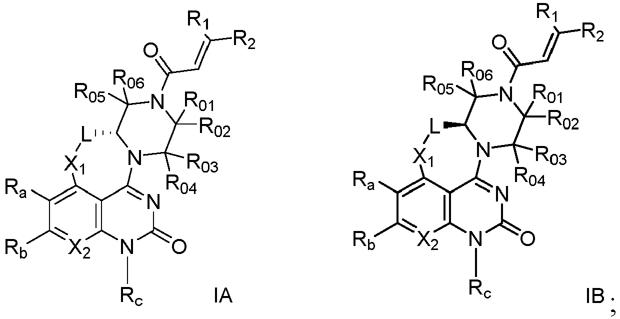 wherein each group is as defined in claim 1.

30. A pharmaceutical composition, comprising the compound or a pharmaceutically acceptable salt, stereoisomer, solvate or prodrug thereof according to any one of claims 1-28 and 29; and a pharmaceutically acceptable carrier.

31. Use of the compound or a pharmaceutically acceptable salt, stereoisomer, solvate or prodrug thereof according to any one of claims 1-28 and 29, or the pharmaceutical composition according to claim 30 in the preparation of a medicament for preventing and/or treating cancer.

32. Use of the compound or a pharmaceutically acceptable salt, stereoisomer, solvate or prodrug thereof according to any one of claims 1-28 and 29, or the pharmaceutical composition according to claim 30 in the preparation of an inhibitor of KRAS mutation (preferably, the KRAS mutation is KRAS G12C mutation).

33. An oxaazaquinazolin-7(8H)-one compound or a pharmaceutically acceptable salt, stereoisomer, solvate or prodrug thereof, the compound has a structure as represented by formula (II): 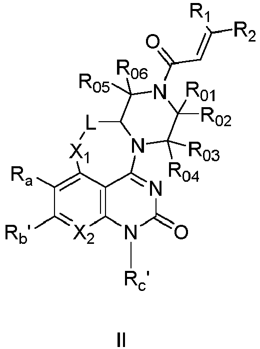 wherein
R₁, R₂ are each independently hydrogen, cyano, C₁₋₃ alkyl, or -C₁₋₃ alkyl-NR^{a}R^{b};
R₀₁, R₀₂, R₀₃, R₀₄, R₀₅, R₀₆ are each independently hydrogen, C₁₋₆ alkyl, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
or R₀₁, R₀₂ together with the carbon atom attached thereto form C₃₋₆ monocyclic cycloalkyl;
or R₀₃, R₀₄ together with the carbon atom attached thereto form C₃₋₆ monocyclic cycloalkyl;
or R₀₅, R₀₆ together with the carbon atom attached thereto form C₃₋₆ monocyclic cycloalkyl;
L is a bond, (CR_{L1}R_{L2})ₙ, C(O), C(O)C(R_{L1}R_{L2}), or C(R_{L1}R_{L2})C(O); wherein R_{L1}, R_{L2} are each independently hydrogen, halo, or C₁₋₆ alkyl;
n is 1 or 2;
X₁ is NRₓ₁, O, or CRₓ₂Rₓ₃; wherein Rₓ₁ is hydrogen, or C₁₋₆ alkyl; Rₓ₂, Rₓ₃ are each independently hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{g}R^{h}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
X₂ is N or CRₓ₄; wherein Rₓ₄ is hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{g}R^{h}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
Rₐ is hydrogen, halo, cyano, C₁₋₆ alkyl, C₁₋₆ alkoxy, halo C₁₋₆ alkyl, halo C₁₋₆ alkoxy, C₃₋₆ monocyclic cycloalkyl, NR^{c}R^{d}, C₂₋₄ alkenyl, C₂₋₄ alkynyl, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, or -C₁₋₄ alkyl-halo C₁₋₆ alkoxy;
R_{b}' is C₆₋₁₀ aryl, C₅₋₁₀ heteroaryl, C₃₋₆ monocyclic heterocyclyl, pyrimidinonyl, or pyridonyl; the C₆₋₁₀ aryl, C₅₋₁₀ heteroaryl, C₃₋₆ monocyclic heterocyclyl, pyrimidinonyl, and pyridonyl are unsubstituted or substituted by 1, 2, 3, or 4 substituent(s) independently selected from the group S1, or substituted by 1, 2, 3, or 4 substituent(s) independently selected from deuterated C₁₋₆ alkyl and deuterated C₁₋₆ alkoxy; the substituents of the group S1 are halo, cyano, nitro, hydroxy, C₁₋₆ alkyl, C₁₋₆ alkoxy, halo C₁₋₆ alkyl, halo C₁₋₆ alkoxy, C₃₋₆ monocyclic cycloalkyl, NRⁱR^{j}, C(O)NR^{e}R^{f}, -SO₂C₁₋₃ alkyl, -SO₂halo C₁₋₃ alkyl, -SO₂NR^{e}R^{f}, -C₁₋₄ alkyl-hydroxy, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-NR^{e}R^{f}, -C₁₋₄ alkyl-C(O)NR^{e}R^{f}, -C₁₋₄ alkyl-SO₂C₁₋₃ alkyl, or C₂₋₄ alkynyl;
R_{c}' is C₁₋₆ alkyl, C₆₋₁₀ aryl, C₅₋₁₀ heteroaryl, C₃₋₆ monocyclic cycloalkyl, C₃₋₆ monocyclic heterocyclyl, 7- to 11-membered spirocycloalkyl, -C₁₋₄ alkyl-C₆₋₁₀ aryl, -C₁₋₄ alkyl-C₅₋₁₀ heteroaryl, -NR^{e}-C₆₋₁₀ aryl, -O-C₆₋₁₀ aryl, -C₁₋₄ alkyl-C₃₋₆ monocyclic heterocyclyl, -C₁₋₄ alkyl-C₃₋₆ monocyclic cycloalkyl, pyrimidinonyl, or pyridonyl;
wherein
the C₃₋₆ monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione;
the C₃₋₆ monocyclic heterocyclyl is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one;
the -C₁₋₄ alkyl- is unsubstituted or substituted by 1, 2, 3, or 4 substituent(s) independently selected from C₁₋₃ alkyl;
the C₁₋₆ alkyl, C₆₋₁₀ aryl, C₅₋₁₀ heteroaryl, 7- to 11-membered spirocycloalkyl, C₃₋₆ monocyclic cycloalkyl, C₃₋₆ monocyclic heterocyclyl, pyrimidinonyl, and pyridonyl are unsubstituted or substituted by 1, 2, 3, or 4 substituent(s) independently selected from the group S2, or substituted by 1, 2, 3, or 4 substituent(s) independently selected from deuterated C₁₋₆ alkyl and deuterated C₁₋₆ alkoxy; the substituents of the group S2 are halo, cyano, hydroxy, C₁₋₆ alkyl, C₁₋₆ alkoxy, halo C₁₋₆ alkyl, halo C₁₋₆ alkoxy, C₃₋₆ monocyclic cycloalkyl, C₃₋₆ monocyclic heterocyclyl, NRⁱR^{j}, C(O)NR^{e}R^{f}, -SO₂C₁₋₃ alkyl, -SO₂halo C₁₋₃ alkyl, -SO₂NR^{e}R^{f}, -C₁₋₄ alkyl-hydroxy, -C₁₋₄ alkyl-C₂₋₄ alkynyl, -C₁₋₄ alkyl-cyano, -C₁₋₄ alkyl-C₁₋₆ alkoxy, -C₁₋₄ alkyl-halo C₁₋₆ alkyl, -C₁₋₄ alkyl-halo C₁₋₆ alkoxy, -C₁₋₄ alkyl-C₃₋₆ monocyclic heterocyclyl, -C₁₋₄ alkyl-C₃₋₆ monocyclic cycloalkyl, -C₁₋₄ alkyl-NR^{e}R^{f}, -C₁₋₄ alkyl-C(O)NR^{e}R^{f}, -C₁₋₄ alkyl-SO₂C₁₋₃ alkyl, or C₂₋₄ alkynyl; wherein the C₃₋₆ monocyclic cycloalkyl in the substituents of the group S2 is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran; and the C₁₋₆ alkyl, C₁₋₆ alkoxy, -C₁₋₄ alkyl-, C₃₋₆ monocyclic cycloalkyl, C₃₋₆ monocyclic heterocyclyl in the substituents of the group S2 are optionally substituted by 1, 2, or 3 substituent(s) independently selected from the group consisting of halo, methyl, ethyl, propyl, isopropyl, trifluoromethyl, amino, N(CH₃)₂, hydroxy, carboxyl; wherein the C₃₋₆ monocyclic cycloalkyl is selected from the group consisting of: cyclopropyl, cyclobutyl, cyclopentyl, cyclohexyl; the C₃₋₆ monocyclic heterocyclyl is selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide, tetrahydropyran;
R^{a}, R^{b}, R^{c}, R^{d}, R^{e}, R^{f}, R^{g}, R^{h} are each independently hydrogen, or C₁₋₃ alkyl;
Rⁱ, R^{j} are each independently hydrogen, C₁₋₃ alkyl, -C(O)C₁₋₃ alkyl, -CO₂C₁₋₃ alkyl.

34. The compound or a pharmaceutically acceptable salt, stereoisomer, solvate or prodrug thereof according to claim 33, **characterized in that**, the compound of formula (II) has a structure as shown in formula (IIA) or formula (IIB) : 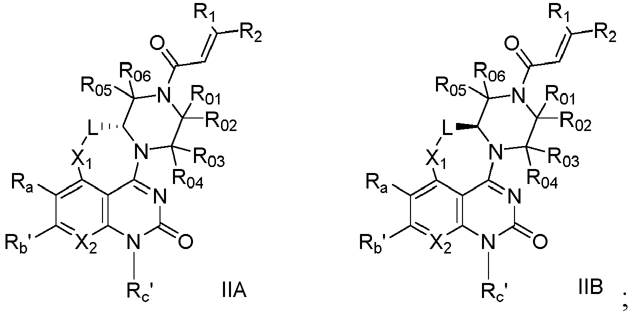 wherein each group is as defined in claim 33.

35. The compound or a pharmaceutically acceptable salt, stereoisomer, solvate or prodrug thereof according to claim 33, **characterized in that**, the compound of formula (II) is selected from the Table A-1.

36. The compound or a pharmaceutically acceptable salt, stereoisomer, solvate or prodrug thereof according to claim 33, **characterized in that**, the compound of formula (II) is selected from the Table A-2.

37. A pharmaceutical composition, comprising the compound or a pharmaceutically acceptable salt, stereoisomer, solvate or prodrug thereof according to any one of claims 33-36; and a pharmaceutically acceptable carrier.

38. Use of the compound or a pharmaceutically acceptable salt, stereoisomer, solvate or prodrug thereof according to any one of claims 33-36, or the pharmaceutical composition according to claim 37 in the preparation of a medicament for preventing and/or treating cancer.

39. Use of the compound or a pharmaceutically acceptable salt, stereoisomer, solvate or prodrug thereof according to any one of claims 33-36, or the pharmaceutical composition according to claim 37 in the preparation of an inhibitor of KRAS mutation (preferably, the KRAS mutation is KRAS G12C mutation).
